# EUROPEAN PATENT SPECIFICATION

(11) **EP 3 818 159 B1**
(45) Date of publication and mention of the grant of the patent: **09.12.2026**
(21) Application number: 19742115.9
(22) Date of filing: 03.07.2019
(51) Int. Cl.: C12N 15/113

(54) **OLIGONUCLEOTIDES FOR MODULATING TAU EXPRESSION**
OLIGONUKLEOTIDE ZUR MODULATION DER TAU-EXPRESSION
OLIGONUCLÉOTIDES POUR MODULER L'EXPRESSION DE TAU

(30) Priority: 03.07.2018 US 201862693851 P; 31.08.2018 US 201862726005 P
(43) Date of publication of application: 12.05.2021
(73) Proprietor: F. Hoffmann-La Roche AG, 4070 Basel (CH)
(72) Inventor: HAGEDORN, Peter, 2970 Hørsholm (DK); HØG, Anja Mølhart, 2970 Hørsholm (DK); OLSON, Richard E., Cambridge, Massachusetts 02139 (US); JENSEN, Marianne L., 2970 Hørsholm (DK)
(74) Representative: D Young & Co LLP
(86) International application number: PCT/EP2019/067799
(87) International publication number: WO 2020/007892

(56) References cited:
- WO-A1-2016/019063
- WO-A1-2016/126995
- WO-A1-2017/106370
- WO-A2-2015/002971
- SCHOCH KATHLEEN M ET AL: "Antisense Oligonucleotides: Translation from Mouse Models to Human Neurodegenerative Diseases", NEURON, vol. 94, no. 6, 21 June 2017 (2017-06-21), pages 1056 - 1070, XP085093439, ISSN: 0896-6273, DOI: 10.1016/J.NEURON.2017.04.010

## Description

### FIELD OF INVENTION

The present invention relates to oligonucleotides (oligomers) that are complementary to microtubule-associated protein Tau *(MAPT)* transcript, leading to reduction of the expression of Tau. Reduction of *MAPT* transcripts and/or Tau protein expression is beneficial for a range of medical disorders, such as such as Tauopathies, Alzheimzer's disease, fronto-temporal dementia (FTD), FTDP-17, progressive supranuclear palsy (PSP), chronic traumatic encephalopathy (CTE), corticobasal ganglionic degeneration (CBD), epilepsy, Dravet syndrome, depression, seizure disorders and movement disorders.

### BACKGROUND

Tau is a microtubule-associated protein (MAP) that interacts with tubulin and is involved in microtubule assembly and stabilization. Microtubules are critical structural components of the cellular cytoskeleton and are involved in various cellular processes, including mitosis, cytokinesis, and vesicular transport. Tau protein is present in multiple cell and tissue types, but is particularly abundant in neurons where it plays a critical role in regulating axonal transport and function.

Alterations in Tau expression levels and/or function contribute to the pathophysiology of various neurodegenerative disorders. For example, aggregates of misfolded and hyperphosphorylated Tau are found in the neurofibrillary inclusions associated with Alzheimer's disease (AD) and related Tauopathies such as progressive supranuclear palsy (PSP), corticobasal ganglionic degeneration (CBD), chronic traumatic encephalopathy (CTE), fronto- temporal dementia FTD) and FTD with parkinsonism linked to chromosome 17 (FTDP-17), Pick's disease (PiD), argyrophilic grain disease (AGD), tangle-predominant senile dementia (TPSD), primary age-related Tauopathy (PART), Down syndrome and lytico-bodig disease. Upregulation of pathological Tau is associated with infantile Tauopathies including hemimegalencephaly (HME), tuberous sclerosis complex; focal cortical dysplasia type 2b; and ganglioglioma. In addition, abnormal Tau expression and/or function may also be associated with other diseases such as Hallervorden-Spatz syndrome, also known as neurodegeneration with brain iron accumulation type 1 (NBIA1), gangliocytomas, and subacute sclerosing panencephalitis. Tau may also play a role in seizure disorders (e.g., epilepsy), network dysfunction (e.g., depression), and movement disorders (e.g., Parkinson's disease).

Antisense molecules as well as siRNA molecules have that can reduce Tau protein levels by targeting *MAPT* pre-mRNA or mRNA transcripts have been described, see for example De Vos et al 2013 Journal of Neuroscience Vol 33 pp 12887, WO2013/148260, WO2014/153236, WO2015/010135, WO2016/126995, WO2016/151523, WO2017/09679 and WO2018/064593.

Antisense oligonucleotides than can induce splice modulation of the *MAPT* transcript have also been described in Sud et al 2014 Mol Ther Nucl Acid 3 e180 and WO2016/019063.

Tau-associated disorders such as AD are the most common cause of dementia in the elderly, and robust and effective agents for the treatment of AD and related neurodegenerative diseases, including Tauopathies, seizure disorders, and movement disorders, are greatly needed.

### OBJECTIVE OF THE INVENTION

The present invention provides antisense oligonucleotides according to the claims which reduce Tau both *in vivo* and *in vitro.* Described herein are three specific target regions in the *MAPT* pre-mRNA located in intron 1 or 2 of the human *MAPT* pre-mRNA which may be targeted by antisense oligonucleotides to give effective Tau inhibition. In particular targeting position 12051 to 12111, 39562 to 39593 and or 72837 to 72940 of SEQ ID NO: 1 is advantageous in terms of reducing Tau. The invention also provides effective antisense oligonucleotide sequences and compounds according to the claims which are capable of reducing Tau, and their use in treatment of diseases or disorders such as neurodegenerative diseases including Tauopathies, Alzheimer's disease, FTDP-17, seizure disorders and movement disorders.

### SUMMARY OF INVENTION

Disclosed herein are oligonucleotides targeting a Tau encoding nucleic acid which is capable of modulating the expression of Tau and the use of the oligonucleotide to treat or prevent diseases related to the functioning of the Tau.

Accordingly, in a first aspect the invention provides oligonucleotides of 16 to 30 nucleotides in length which comprise a contiguous nucleotide sequence of at least 16 nucleotides in length with 100% complementarity to contiguous nucleotides within position 12060 to 12078 of SEQ ID NO: 1, wherein the antisense oligonucleotide is capable of reducing the expression of Tau.

The oligonucleotide can be an antisense oligonucleotide, preferably with a gapmer design. Preferably, the oligonucleotide is capable of inhibiting the expression of Tau by cleavage of a target nucleic acid. The cleavage is preferably achieved via nuclease recruitment.

In a further aspect, the invention provides a conjugate comprising the antisense oligonucleotide according to the invention, and at least one conjugate moiety covalently attached to said oligonucleotide.

In a further aspect, the invention provides a pharmaceutically acceptable salt of the antisense oligonucleotide according to the invention, or the conjugate according to the invention.

In a further aspect, the invention provides pharmaceutical compositions comprising the oligonucleotides of the invention and pharmaceutically acceptable diluents, carriers, salts and/or adjuvants.

In a further aspect, the invention provides an *in vitro* method for modulation of Tau expression in a target cell which is expressing Tau, by administering an oligonucleotide or composition of the invention in an effective amount to said cell.

In a further aspect, the invention provides the antisense oligonucleotide of the invention, the conjugate according to the invention, or the pharmaceutical composition of the invention, for use in medicine.

In a further aspect the oligonucleotide, conjugate or composition of the invention is used for the treatment or prevention of Alzheimer's disease (AD), progressive supranuclear palsy (PSP), fronto- temporal dementia (FTD) or FTDP-17.

### BRIEF DESCRIPTION OF FIGURES

**Figure 1****:** Screening result from oligonucleotide library (example 1) covering all intron regions on MAPT. Each dot represents an oligonucleotide compound, the x-axis illustrates its position on the MAPT transcript and the y- axis shows the amount of MAPT mRNA reminaing when compared to control (low number correspond to large reduction of MAPT). A, B and C indicate three regions on the MAPT transcript selected as target regions for further oligonucleotide compounds.
**Figure 2****:** Compound 9_103 (sequence of nucleobases is shown in SEQ ID NO 9)
**Figure 3****:** Compound 9_104 (sequence of nucleobases is shown in SEQ ID NO 9)
**Figure 4****:** Compound 11_1 (sequence of nucleobases is shown in SEQ ID NO 11)
**Figure 5****:** Compound 49_38 (sequence of nucleobases is shown in SEQ ID NO 49)
**Figure 6****:** Compound 49_189 (sequence of nucleobases is shown in SEQ ID NO 49)

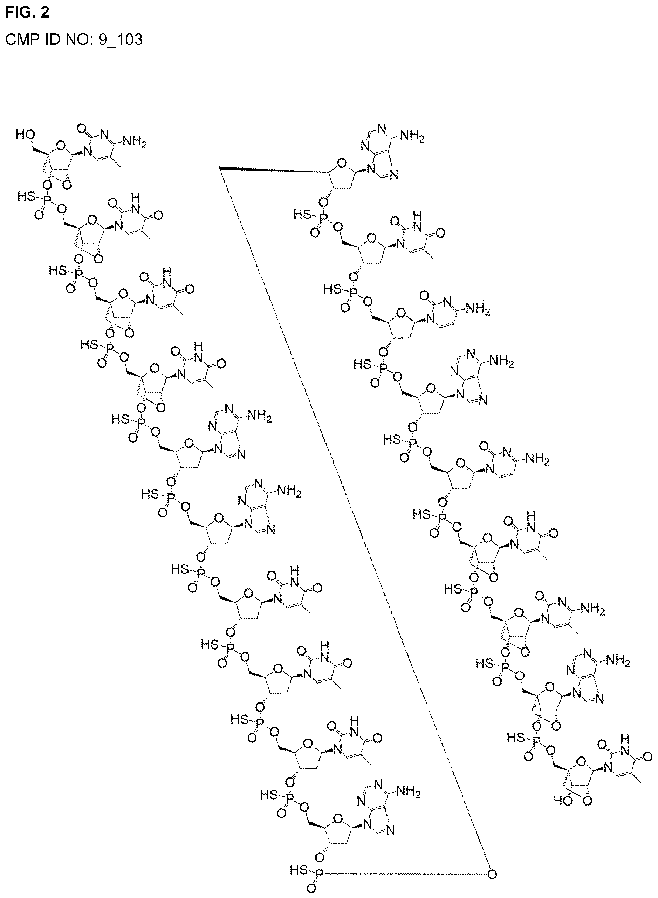

The compounds illustrated in figures 2, 3, 4, 5 and 6 are shown in the protonated form - the S atom on the phosphorothioate linkage is protonated - it will be understood that the presence of the proton will depend on the acidity of the environment of the molecule, and the presence of an alternative cation (e.g. when the oligonucleotide is in salt form). Protonated phosphorothioates exist in tautomeric forms.

### DEFINITIONS

### Oligonucleotide

The term "oligonucleotide" as used herein is defined as it is generally understood by the skilled person as a molecule comprising two or more covalently linked nucleosides. Such covalently bound nucleosides may also be referred to as nucleic acid molecules or oligomers. Oligonucleotides are commonly made in the laboratory by solid-phase chemical synthesis followed by purification and isolation. When referring to a sequence of the oligonucleotide, reference is made to the sequence or order of nucleobase moieties, or modifications thereof, of the covalently linked nucleotides or nucleosides. The oligonucleotide of the invention is man-made, and is chemically synthesized, and is typically purified or isolated. The oligonucleotide of the invention may comprise one or more modified nucleosides or nucleotides, such as 2' sugar modified nucleosides.

### Antisense oligonucleotides

The term "Antisense oligonucleotide" as used herein is defined as oligonucleotides capable of modulating expression of a target gene by hybridizing to a target nucleic acid, in particular to a contiguous sequence on a target nucleic acid. The antisense oligonucleotides are not essentially double stranded and are therefore not siRNAs or shRNAs. Preferably, the antisense oligonucleotides of the present invention are single stranded. It is understood that single stranded oligonucleotides of the present invention can form hairpins or intermolecular duplex structures (duplex between two molecules of the same oligonucleotide), as long as the degree of intra or inter self-complementarity is less than 50% across of the full length of the oligonucleotide.

Advantageously, the single stranded antisense oligonucleotide of the invention does not contain RNA nucleosides, since this will decrease nuclease resistance.

Advantageously, the antisense oligonucleotide of the invention comprises one or more modified nucleosides or nucleotides, such as 2' sugar modified nucleosides. Furthermore, it is advantageous that the nucleosides which are not modified are DNA nucleosides.

### Contiguous Nucleotide Sequence

The term "contiguous nucleotide sequence" refers to the region of the oligonucleotide which is complementary to the target nucleic acid or target sequence. The term is used interchangeably herein with the term "contiguous nucleobase sequence" and the term "oligonucleotide motif sequence". In some embodiments all the nucleotides of the oligonucleotide constitute the contiguous nucleotide sequence. In some embodiments the oligonucleotide comprises the contiguous nucleotide sequence, such as a F-G-F' gapmer region, and may optionally comprise further nucleotide(s), for example a nucleotide linker region which may be used to attach a functional group to the contiguous nucleotide sequence. The nucleotide linker region may or may not be complementary to the target nucleic acid. It is understood that the contiguous nucleotide sequence of the oligonucleotide cannot be longer than the oligonucleotide as such and that the oligonucleotide cannot be shorter than the contiguous nucleotide sequence.

### Nucleotides

Nucleotides are the building blocks of oligonucleotides and polynucleotides, and for the purposes of the present invention include both naturally occurring and non-naturally occurring nucleotides. In nature, nucleotides, such as DNA and RNA nucleotides comprise a ribose sugar moiety, a nucleobase moiety and one or more phosphate groups (which is absent in nucleosides). Nucleosides and nucleotides may also interchangeably be referred to as "units" or "monomers".

### Modified nucleoside

The term "modified nucleoside" or "nucleoside modification" as used herein refers to nucleosides modified as compared to the equivalent DNA or RNA nucleoside by the introduction of one or more modifications of the sugar moiety or the (nucleo)base moiety. In a preferred embodiment the modified nucleoside comprises a modified sugar moiety. The term modified nucleoside may also be used herein interchangeably with the term "nucleoside analogue" or modified "units" or modified "monomers". Nucleosides with an unmodified DNA or RNA sugar moiety are termed DNA or RNA nucleosides herein. Nucleosides with modifications in the base region of the DNA or RNA nucleoside are still generally termed DNA or RNA if they allow Watson Crick base pairing.

### Modified internucleoside linkage

The term "modified internucleoside linkage" is defined as generally understood by the skilled person as linkages other than phosphodiester (PO) linkages, that covalently couples two nucleosides together. The oligonucleotides of the invention may therefore comprise modified internucleoside linkages. In some embodiments, the modified internucleoside linkage increases the nuclease resistance of the oligonucleotide compared to a phosphodiester linkage. For naturally occurring oligonucleotides, the internucleoside linkage includes phosphate groups creating a phosphodiester bond between adjacent nucleosides. Modified internucleoside linkages are particularly useful in stabilizing oligonucleotides for in vivo use, and may serve to protect against nuclease cleavage at regions of DNA or RNA nucleosides in the oligonucleotide of the invention, for example within the gap region G of a gapmer oligonucleotide, as well as in regions of modified nucleosides, such as region F and **F'.**

In an embodiment, the oligonucleotide comprises one or more internucleoside linkages modified from the natural phosphodiester, such as one or more modified internucleoside linkages that is for example more resistant to nuclease attack. Nuclease resistance may be determined by incubating the oligonucleotide in blood serum or by using a nuclease resistance assay (e.g. snake venom phosphodiesterase (SVPD)), both are well known in the art. Internucleoside linkages which are capable of enhancing the nuclease resistance of an oligonucleotide are referred to as nuclease resistant internucleoside linkages. In some embodiments at least 50% of the internucleoside linkages in the oligonucleotide, or contiguous nucleotide sequence thereof, are modified, such as at least 60%, such as at least 70%, such as at least 75%, such as at least 80% or such as at least 90% of the internucleoside linkages in the oligonucleotide, or contiguous nucleotide sequence thereof, are modified. In some embodiments all of the internucleoside linkages of the oligonucleotide, or contiguous nucleotide sequence thereof, are modified. It will be recognized that, in some embodiments the nucleosides which link the oligonucleotide of the invention to a non-nucleotide functional group, such as a conjugate, may be phosphodiester. In some embodiments all of the internucleoside linkages of the oligonucleotide, or contiguous nucleotide sequence thereof, are nuclease resistant internucleoside linkages.

Modified internucleoside linkages may be selected from the group comprising phosphorothioate, diphosphorothioate and boranophosphate. In some embodiments, the modified internucleoside linkages are compatible with the RNaseH recruitment of the oligonucleotide of the invention, for example phosphorothioate, diphosphorothioate or boranophosphate.

In some embodiments the internucleoside linkage comprises sulphur (S), such as a phosphorothioate internucleoside linkage.

With the oligonucleotides of the invention it is advantageous to use phosphorothioate internucleoside linkages.

Phosphorothioate internucleoside linkages are particularly useful due to nuclease resistance, beneficial pharmacokinetics and ease of manufacture. In some embodiments at least 50% of the internucleoside linkages in the oligonucleotide, or contiguous nucleotide sequence thereof, are phosphorothioate, such as at least 60%, such as at least 70%, such as at least 75%, such as at least 80% or such as at least 90% of the internucleoside linkages in the oligonucleotide, or contiguous nucleotide sequence thereof, are phosphorothioate. In some embodiments all of the internucleoside linkages of the oligonucleotide, or contiguous nucleotide sequence thereof, are phosphorothioate.

In some embodiments, the oligonucleotide of the invention comprises both phosphorothioate internucleoside linkages and at least one phosphodiester linkage, such as 2, 3 or 4 phosphodiester linkages, in addition to the phosphorodithioate linkage(s). In a gapmer oligonucleotide, phosphodiester linkages, when present, are suitably not located between contiguous DNA nucleosides in the gap region G.

In some embodiments, the oligonucleotide comprises one or more neutral internucleoside linkage, particularly a internucleoside linkage selected from phosphotriester, methylphosphonate, MMI, amide-3, formacetal or thioformacetal.

Further internucleoside linkages are disclosed in WO2009/124238. In an embodiment the internucleoside linkage is selected from linkers disclosed in WO2007/031091. Particularly, the internucleoside linkage may be selected from -O-P(O)₂-O-, -O-P(O,S)-O-, -O-P(S)₂-O-, -S-P(O)₂-O-, -S-P(O,S)-O-, -S-P(S)₂-O-, -O-P(O)₂-S-, -O-P(O,S)-S-, -S-P(O)₂-S-, -O-PO(R^{H})-O-, 0-PO(OCH₃)-O-, -O-PO(NR^{H})-O-, -O-PO(OCH₂CH₂S-R)-O-, -O-PO(BH₃)-O-, -O-PO(NHR^{H})-O-, -OP(O)₂-NR^{H}-, -NR^{H}- P(O)₂-O-, -NR^{H}-CO-O-, -NR^{H}-CO-NR^{H}-, and/or the internucleoside linker may be selected form the group consisting of: -O-CO-O-, -O-CO-NR^{H}-, -NR^{H}-CO-CH₂-, -O-CH₂-CO-NR^{H}-, -O-CH₂-CH₂-NR^{H}-, - CO-NR^{H}-CH₂-, -CH₂-NR^{H}CO-, -O-CH₂-CH₂-S-, -S-CH₂-CH₂-O-, -S-CH₂-CH₂-S-, -CH₂-SO₂-CH₂-, -CH₂-CO-NR^{H}-, -O-CH₂-CH₂-NR^{H}-CO -, -CH₂-NCH₃-O-CH₂-, where R^{H} is selected from hydrogen and C1 -4-alkyl.

Nuclease resistant linkages, such as phosphorthioate linkages, are particularly useful in oligonucleotide regions capable of recruiting nuclease when forming a duplex with the target nucleic acid, such as region G for gapmers. Phosphorothioate linkages may, however, also be useful in non-nuclease recruiting regions and/or affinity enhancing regions such as regions F and F' for gapmers. Gapmer oligonucleotides may, in some embodiments comprise one or more phosphodiester linkages in region F or F', or both region F and F', where all the internucleoside linkages in region G may be phosphorothioate.

Advantageously, all the internucleoside linkages of the contiguous nucleotide sequence of the oligonucleotide are phosphorothioate, or all the internucleoside linkages of the oligonucleotide are phosphorothioate linkages.

### Nucleobase

The term nucleobase includes the purine (e.g. adenine and guanine) and pyrimidine (e.g. uracil, thymine and cytosine) moiety present in nucleosides and nucleotides which form hydrogen bonds in nucleic acid hybridization. In the context of the present invention the term nucleobase also encompasses modified nucleobases which may differ from naturally occurring nucleobases, but are functional during nucleic acid hybridization. In this context "nucleobase" refers to both naturally occurring nucleobases such as adenine, guanine, cytosine, thymidine, uracil, xanthine and hypoxanthine, as well as non-naturally occurring variants. Such variants are for example described in Hirao et al (2012) Accounts of Chemical Research vol 45 page 2055 and Bergstrom (2009) Current Protocols in Nucleic Acid Chemistry Suppl. 37 1.4.1.

In a some embodiments the nucleobase moiety is modified by changing the purine or pyrimidine into a modified purine or pyrimidine, such as substituted purine or substituted pyrimidine, such as a nucleobased selected from isocytosine, pseudoisocytosine, 5-methyl cytosine, 5-thiozolo-cytosine, 5-propynyl-cytosine, 5-propynyl-uracil, 5-bromouracil 5-thiazolo-uracil, 2-thio-uracil, 2'thio-thymine, inosine, diaminopurine, 6-aminopurine, 2-aminopurine, 2,6-diaminopurine and 2-chloro-6-aminopurine.

The nucleobase moieties may be indicated by the letter code for each corresponding nucleobase, e.g. A, T, G, C or U, wherein each letter may optionally include modified nucleobases of equivalent function. For example, in the exemplified oligonucleotides, the nucleobase moieties are selected from A, T, G, C, and 5-methyl cytosine. Optionally, for LNA gapmers, 5-methyl cytosine LNA nucleosides may be used.

### Modified oligonucleotide

The term modified oligonucleotide describes an oligonucleotide comprising one or more sugar-modified nucleosides and/or modified internucleoside linkages. The term chimeric" oligonucleotide is a term that has been used in the literature to describe oligonucleotides with modified nucleosides.

### Complementarity

The term "complementarity" describes the capacity for Watson-Crick base-pairing of nucleosides/nucleotides. Watson-Crick base pairs are guanine (G)-cytosine (C) and adenine (A) - thymine (T)/uracil (U). It will be understood that oligonucleotides may comprise nucleosides with modified nucleobases, for example 5-methyl cytosine is often used in place of cytosine, and as such the term complementarity encompasses Watson Crick base-paring between non-modified and modified nucleobases (see for example Hirao et al (2012) Accounts of Chemical Research vol 45 page 2055 and Bergstrom (2009) Current Protocols in Nucleic Acid Chemistry Suppl. 37 1.4.1).

The term "% complementary" as used herein, refers to the proportion of nucleotides (in percent) of a contiguous nucleotide sequence in a nucleic acid molecule (e.g. oligonucleotide) which across the contiguous nucleotide sequence, are complementary to a reference sequence (e.g. a target sequence or sequence motif). The percentage of complementarity is thus calculated by counting the number of aligned nucleobases that are complementary (from Watson Crick base pair) between the two sequences (when aligned with the target sequence 5'-3' and the oligonucleotide sequence from 3'-5'), dividing that number by the total number of nucleotides in the oligonucleotide and multiplying by 100. In such a comparison a nucleobase/nucleotide which does not align (form a base pair) is termed a mismatch. Insertions and deletions are not allowed in the calculation of % complementarity of a contiguous nucleotide sequence. It will be understood that in determining complementarity, chemical modifications of the nucleobases are disregarded as long as the functional capacity of the nucleobase to form Watson Crick base pairing is retained (e.g. 5'-methyl cytosine is considered identical to a cytosine for the purpose of calculating % identity).

The term "fully complementary", refers to 100% complementarity.

The following is an example of an oligonucleotide that is fully complementary to the target nucleic acid.

The following is an example of an oligonucleotide (SEQ ID NO: 49) that is fully complementary to the target nucleic acid (SEQ ID NO: 4).
5' gaaggttgaaatgagaattgatttgagttaaa 3¹ (SEQ ID NO: 4)
3' actcttaactaaactcaatt 5' (SEQ ID NO: 49)

### Identity

The term "Identity" as used herein, refers to the proportion of nucleotides (expressed in percent) of a contiguous nucleotide sequence in a nucleic acid molecule (e.g. oligonucleotide) which across the contiguous nucleotide sequence, are identical to a reference sequence (e.g. a sequence motif). The percentage of identity is thus calculated by counting the number of aligned nucleobases that are identical (a Match) between two sequences (in the contiguous nucleotide sequence of the compound of the invention and in the reference sequence), dividing that number by the total number of nucleotides in the oligonucleotide and multiplying by 100. Therefore, Percentage of Identity = (Matches x 100)/Length of aligned region (e.g. the contiguous nucleotide sequence). Insertions and deletions are not allowed in the calculation the percentage of identity of a contiguous nucleotide sequence. It will be understood that in determining identity, chemical modifications of the nucleobases are disregarded as long as the functional capacity of the nucleobase to form Watson Crick base pairing is retained (e.g. 5-methyl cytosine is considered identical to a cytosine for the purpose of calculating % identity).

### Hybridization

The term "hybridizing" or "hybridizes" as used herein is to be understood as two nucleic acid strands (e.g. an oligonucleotide and a target nucleic acid) forming hydrogen bonds between base pairs on opposite strands thereby forming a duplex. The affinity of the binding between two nucleic acid strands is the strength of the hybridization. It is often described in terms of the melting temperature (Tₘ) defined as the temperature at which half of the oligonucleotides are duplexed with the target nucleic acid. At physiological conditions Tₘ is not strictly proportional to the affinity (Mergny and Lacroix, 2003, Oligonucleotides 13:515-537). The standard state Gibbs free energy ΔG° is a more accurate representation of binding affinity and is related to the dissociation constant (K_{d}) of the reaction by ΔG°=-RTIn(K_{d}), where R is the gas constant and T is the absolute temperature. Therefore, a very low ΔG° of the reaction between an oligonucleotide and the target nucleic acid reflects a strong hybridization between the oligonucleotide and target nucleic acid. ΔG° is the energy associated with a reaction where aqueous concentrations are 1M, the pH is 7, and the temperature is 37°C. The hybridization of oligonucleotides to a target nucleic acid is a spontaneous reaction and for spontaneous reactions ΔG° is less than zero. ΔG° can be measured experimentally, for example, by use of the isothermal titration calorimetry (ITC) method as described in Hansen et al., 1965, Chem. Comm. 36-38 and Holdgate et al., 2005, Drug Discov Today*.* The skilled person will know that commercial equipment is available for ΔG° measurements. ΔG° can also be estimated numerically by using the nearest neighbor model as described by SantaLucia, 1998, Proc Natl Acad Sci USA. 95: 1460-1465 using appropriately derived thermodynamic parameters described by Sugimoto et al., 1995, Biochemistry 34:11211-11216 and McTigue et al., 2004, Biochemistry 43:5388-5405. In order to have the possibility of modulating its intended nucleic acid target by hybridization, oligonucleotides of the present invention hybridize to a target nucleic acid with estimated ΔG° values below -10 kcal for oligonucleotides that are 10-30 nucleotides in length. In some embodiments the degree or strength of hybridization is measured by the standard state Gibbs free energy ΔG°. The oligonucleotides may hybridize to a target nucleic acid with estimated ΔG° values below the range of -10 kcal, such as below -15 kcal, such as below -20 kcal and such as below -25 kcal for oligonucleotides that are 8-30 nucleotides in length. In some embodiments the oligonucleotides hybridize to a target nucleic acid with an estimated ΔG° value of -10 to -60 kcal, such as -12 to -40, such as from -15 to -30 kcal or-16 to -27 kcal such as -18 to -25 kcal.

### Target nucleic acid

According to the present invention, the target nucleic acid is a nucleic acid which encodes mammalian Tau and may for example be a gene, a RNA, a mRNA, and pre-mRNA, a mature mRNA or a cDNA sequence. The target may therefore be referred to as a Tau target nucleic acid or MAPT target nucleic acid, these terms can be used interchangeably. The oligonucleotide described herein may for example target exon regions of a mammalian MAPT, or may for example target intron region in the MAPT pre-mRNA (see Table 1).

**Table 1. human MAPT Exons and Introns**

| Exonic regions in the human Tau premRNA (SEQ ID NO 2) | | | Intronic regions in the human Tau premRNA (SEQ ID NO 2) | | |
|---|---|---|---|---|---|
| **ID** | **start** | **end** | **ID** | **start** | **end** |
| e1 | 1 | 303 | i1 | 304 | 67979 |
| e2 | 67980 | 68129 | i2 | 68130 | 77517 |
| e3 | 77518 | 77604 | i3 | 77605 | 80043 |
| e4 | 80044 | 80130 | i4 | 80131 | 84033 |
| e5 | 84034 | 84099 | i5 | 84100 | 88837 |
| e6 | 88838 | 89590 | i6 | 89591 | 92699 |
| e7 | 92700 | 92755 | i7 | 92756 | 95537 |
| e8 | 95538 | 95735 | i8 | 95736 | 97119 |
| e9 | 97120 | 97246 | i8 | 97247 | 102058 |
| e10 | 102059 | 102324 | i9 | 102325 | 115969 |
| e11 | 115970 | 116062 | i10 | 116063 | 119902 |
| e12 | 119903 | 119984 | i11 | 119985 | 124287 |
| e13 | 124288 | 124400 | i12 | 124401 | 129623 |
| e14 | 129624 | 134004 | | | |

Suitably, the target nucleic acid encodes a Tau protein, in particular mammalian Tau, such as human Tau (See for example tables 2 and 3) which provides pre-mRNA sequences for human, and monkey Tau).

In some examples, the target nucleic acid is selected from the group consisting of SEQ ID NO: 1 and 2 or naturally occurring variants thereof (e.g. sequences encoding a mammalian Tau protein. If employing the oligonucleotide of the invention in research or diagnostics the target nucleic acid may be a cDNA or a synthetic nucleic acid derived from DNA or RNA.

For *in vivo* or *in vitro* application, the oligonucleotide of the invention is typically capable of inhibiting the expression of the Tau protein in a cell which is expressing the MAPT target nucleic acid. The contiguous sequence of nucleobases of the oligonucleotide of the invention is typically complementary to the MAPT target nucleic acid, as measured across the length of the oligonucleotide, and optionally excluding nucleotide based linker regions which may link the oligonucleotide to an optional functional group such as a conjugate, or other non-complementary terminal nucleotides (e.g. region D' or D"). The target nucleic acid may, in some embodiments, be a RNA or DNA, such as a messenger RNA, such as a mature mRNA or a pre-mRNA.

In some examples the target nucleic acid is a RNA or DNA which encodes mammalian Tau protein, such as human Tau, e.g. the human MAPTpre- mRNA sequence, such as that disclosed as SEQ ID NO 1. Further information on exemplary target nucleic acids is provided in tables 2 and 3.

**Table 2. Genome and assembly information for Tau across species.**

| **Species** | **Chr.** | **Strand** | **Genomic coordinates** | | **Assembly** | **NCBI reference sequence* accession number for mRNA** |
|---|---|---|---|---|---|---|
| | | | **Start** | **End** | | |
| Human | 17 | fwd | 45894382 | 46028334 | GRCh38.p12 | NG_007398.1 |
| Cynomol gus monkey | 16 | fwd | 58257786 | 58390183 | Macaca_fascicularis _5.0 | From 58257786 to 58390183 in NC_022287.1 |

| | | | | | | |
|---|---|---|---|---|---|---|
| Fwd = forward strand. The genome coordinates provide the pre-mRNA sequence (genomic sequence). The NCBI reference provides the mRNA sequence (cDNA sequence). *The National Center for Biotechnology Information reference sequence database is a comprehensive, integrated, non-redundant, well-annotated set of reference sequences including genomic, transcript, and protein. It is hosted at www.ncbi.nlm.nih.gov/refseq. | | | | | | |

**Table 3. Sequence details for Tau/ MAPT across species.**

| **Species** | **RNA type** | **Length (nt)** | **SEQ ID NO** |
|---|---|---|---|
| Human | premRNA | 134004 | 1 |
| Monkey | premRNA | 132218 | 2 |

### Target Sequence

The term "target sequence" as used herein refers to a sequence of nucleotides present in the target nucleic acid which comprises the nucleobase sequence which is complementary to the oligonucleotide of the invention. In some embodiments, the target sequence consists of a region on the target nucleic acid with a nucleobase sequence that is complementary to the contiguous nucleotide sequence of the oligonucleotide of the invention. This region of the target nucleic acid may interchangeably be referred to as the target nucleotide sequence, target sequence or target region. In some embodiments the target sequence is longer than the complementary sequence of a single oligonucleotide, and may, for example represent a preferred region of the target nucleic acid which may be targeted by several oligonucleotides of the invention.

In some examples the target sequence is a sequence selected from any region in table 4 (R_1-R_2254). In particular, the target sequence may be selected from one of the region within the group of regions consisting of R_223, R_738 or R_1298.

**Table 4. Regions (reg.) on SEQ ID NO: 1 which may be targeted using an oligonucleotide described herein**

| **Reg.** | **length (nt)** | **Position on SEQ ID NO: 1** | | **Reg.** | **length (nt)** | **Position on SEQ ID NO: 1** | | **Reg.** | **length (nt)** | **Position on SEQ ID NO: 1** | |
|---|---|---|---|---|---|---|---|---|---|---|---|
| | | **start** | **end** | | | **start** | **end** | | | **start** | **end** |
| R_1 | 32 | 4 | 35 | R_752 | 23 | 40118 | 40140 | R_1503 | 88 | 85545 | 85632 |
| R_2 | 32 | 37 | 68 | R_753 | 40 | 40209 | 40248 | R_1504 | 20 | 85662 | 85681 |
| R_3 | 32 | 70 | 101 | R_754 | 24 | 40273 | 40296 | R_1505 | 75 | 85710 | 85784 |
| R_4 | 25 | 103 | 127 | R_755 | 63 | 40301 | 40363 | R_1506 | 35 | 85786 | 85820 |
| R_5 | 187 | 156 | 342 | R_756 | 35 | 40461 | 40495 | R_1507 | 24 | 85822 | 85845 |
| R_6 | 33 | 344 | 376 | R_757 | 27 | 40497 | 40523 | R_1508 | 24 | 85864 | 85887 |
| R_7 | 37 | 385 | 421 | R_758 | 33 | 40547 | 40579 | R_1509 | 20 | 85879 | 85898 |
| R_8 | 47 | 440 | 486 | R_759 | 42 | 40587 | 40628 | R_1510 | 41 | 85889 | 85929 |
| R_9 | 22 | 488 | 509 | R_760 | 41 | 40630 | 40670 | R_1511 | 25 | 85964 | 85988 |
| R_10 | 38 | 511 | 548 | R_761 | 34 | 40697 | 40730 | R_1512 | 23 | 85994 | 86016 |
| R_11 | 63 | 580 | 642 | R_762 | 57 | 40772 | 40828 | R_1513 | 56 | 86064 | 86119 |
| R_12 | 20 | 649 | 668 | R_763 | 36 | 40831 | 40866 | R_1514 | 71 | 86189 | 86259 |
| R_13 | 32 | 710 | 741 | R_764 | 60 | 40868 | 40927 | R_1515 | 32 | 86266 | 86297 |
| R_14 | 37 | 743 | 779 | R_765 | 28 | 40941 | 40968 | R_1516 | 54 | 86319 | 86372 |
| R_15 | 27 | 792 | 818 | R_766 | 29 | 40971 | 40999 | R_1517 | 38 | 86383 | 86420 |
| R_16 | 23 | 814 | 836 | R_767 | 96 | 41031 | 41126 | R_1518 | 31 | 86427 | 86457 |
| R_17 | 115 | 839 | 953 | R_768 | 43 | 41128 | 41170 | R_1519 | 33 | 86478 | 86510 |
| R_18 | 25 | 955 | 979 | R_769 | 22 | 41218 | 41239 | R_1520 | 36 | 86676 | 86711 |
| R_19 | 80 | 981 | 1060 | R_770 | 28 | 41266 | 41293 | R_1521 | 20 | 86715 | 86734 |
| R_20 | 23 | 1071 | 1093 | R_771 | 25 | 41311 | 41335 | R_1522 | 20 | 86742 | 86761 |
| R_21 | 26 | 1095 | 1120 | R_772 | 50 | 41356 | 41405 | R_1523 | 29 | 86809 | 86837 |
| R_22 | 32 | 1177 | 1208 | R_773 | 55 | 41425 | 41479 | R_1524 | 51 | 86873 | 86923 |
| R_23 | 78 | 1239 | 1316 | R_774 | 23 | 41483 | 41505 | R_1525 | 48 | 86939 | 86986 |
| R_24 | 34 | 1334 | 1367 | R_775 | 47 | 41518 | 41564 | R_1526 | 21 | 86989 | 87009 |
| R_25 | 68 | 1401 | 1468 | R_776 | 36 | 41586 | 41621 | R_1527 | 46 | 87080 | 87125 |
| R_26 | 82 | 1470 | 1551 | R_777 | 77 | 41641 | 41717 | R_1528 | 23 | 87140 | 87162 |
| R_27 | 95 | 1566 | 1660 | R_778 | 48 | 41762 | 41809 | R_1529 | 24 | 87164 | 87187 |
| R_28 | 43 | 1708 | 1750 | R_779 | 42 | 41830 | 41871 | R_1530 | 45 | 87209 | 87253 |
| R_29 | 71 | 1762 | 1832 | R_780 | 57 | 41888 | 41944 | R_1531 | 21 | 87261 | 87281 |
| R_30 | 37 | 1841 | 1877 | R_781 | 25 | 41964 | 41988 | R_1532 | 37 | 87297 | 87333 |
| R_31 | 26 | 1878 | 1903 | R_782 | 30 | 42005 | 42034 | R_1533 | 61 | 87367 | 87427 |
| R_32 | 21 | 1960 | 1980 | R_783 | 31 | 42096 | 42126 | R_1534 | 69 | 87595 | 87663 |
| R_33 | 20 | 1982 | 2001 | R_784 | 30 | 42141 | 42170 | R_1535 | 29 | 87665 | 87693 |
| R_34 | 27 | 2018 | 2044 | R_785 | 32 | 42172 | 42203 | R_1536 | 20 | 87679 | 87698 |
| R_35 | 22 | 2061 | 2082 | R_786 | 56 | 42279 | 42334 | R_1537 | 20 | 87760 | 87779 |
| R_36 | 24 | 2196 | 2219 | R_787 | 63 | 42336 | 42398 | R_1538 | 21 | 87915 | 87935 |
| R_37 | 30 | 2237 | 2266 | R_788 | 44 | 42439 | 42482 | R_1539 | 21 | 87952 | 87972 |
| R_38 | 27 | 2334 | 2360 | R_789 | 29 | 42486 | 42514 | R_1540 | 20 | 87962 | 87981 |
| R_39 | 22 | 2362 | 2383 | R_790 | 30 | 42518 | 42547 | R_1541 | 47 | 88017 | 88063 |
| R_40 | 22 | 2419 | 2440 | R_791 | 24 | 42581 | 42604 | R_1542 | 32 | 88099 | 88130 |
| R_41 | 31 | 2472 | 2502 | R_792 | 32 | 42631 | 42662 | R_1543 | 33 | 88133 | 88165 |
| R_42 | 21 | 2506 | 2526 | R_793 | 24 | 42681 | 42704 | R_1544 | 22 | 88176 | 88197 |
| R_43 | 21 | 2541 | 2561 | R_794 | 21 | 42712 | 42732 | R_1545 | 36 | 88216 | 88251 |
| R_44 | 31 | 2565 | 2595 | R_795 | 49 | 42745 | 42793 | R_1546 | 35 | 88279 | 88313 |
| R_45 | 21 | 2598 | 2618 | R_796 | 35 | 42841 | 42875 | R_1547 | 30 | 88353 | 88382 |
| R_46 | 28 | 2725 | 2752 | R_797 | 45 | 42877 | 42921 | R_1548 | 38 | 88384 | 88421 |
| R_47 | 38 | 2769 | 2806 | R_798 | 22 | 42937 | 42958 | R_1549 | 37 | 88439 | 88475 |
| R_48 | 59 | 2915 | 2973 | R_799 | 20 | 42969 | 42988 | R_1550 | 54 | 88493 | 88546 |
| R_49 | 50 | 2978 | 3027 | R_800 | 45 | 42976 | 43020 | R_1551 | 29 | 88561 | 88589 |
| R_50 | 21 | 3035 | 3055 | R_801 | 20 | 43035 | 43054 | R_1552 | 21 | 88594 | 88614 |
| R_51 | 24 | 3072 | 3095 | R_802 | 72 | 43047 | 43118 | R_1553 | 23 | 88617 | 88639 |
| R_52 | 22 | 3171 | 3192 | R_803 | 23 | 43136 | 43158 | R_1554 | 24 | 88648 | 88671 |
| R_53 | 28 | 3207 | 3234 | R_804 | 56 | 43188 | 43243 | R_1555 | 30 | 88678 | 88707 |
| R_54 | 25 | 3236 | 3260 | R_805 | 20 | 43239 | 43258 | R_1556 | 27 | 88715 | 88741 |
| R_55 | 33 | 3262 | 3294 | R_806 | 20 | 43279 | 43298 | R_1557 | 24 | 88774 | 88797 |
| R_56 | 58 | 3302 | 3359 | R_807 | 27 | 43304 | 43330 | R_1558 | 48 | 88820 | 88867 |
| R_57 | 21 | 3364 | 3384 | R_808 | 30 | 43346 | 43375 | R_1559 | 35 | 88877 | 88911 |
| R_58 | 36 | 3417 | 3452 | R_809 | 64 | 43408 | 43471 | R_1560 | 52 | 88919 | 88970 |
| R_59 | 56 | 3476 | 3531 | R_810 | 52 | 43481 | 43532 | R_1561 | 26 | 88978 | 89003 |
| R_60 | 20 | 3533 | 3552 | R_811 | 22 | 43538 | 43559 | R_1562 | 32 | 89011 | 89042 |
| R_61 | 20 | 3554 | 3573 | R_812 | 29 | 43561 | 43589 | R_1563 | 26 | 89044 | 89069 |
| R_62 | 22 | 3648 | 3669 | R_813 | 37 | 43593 | 43629 | R_1564 | 51 | 89100 | 89150 |
| R_63 | 21 | 3681 | 3701 | R_814 | 24 | 43637 | 43660 | R_1565 | 34 | 89196 | 89229 |
| R_64 | 20 | 3756 | 3775 | R_815 | 21 | 43697 | 43717 | R_1566 | 28 | 89231 | 89258 |
| R_65 | 24 | 3808 | 3831 | R_816 | 21 | 43719 | 43739 | R_1567 | 24 | 89261 | 89284 |
| R_66 | 35 | 3833 | 3867 | R_817 | 34 | 43772 | 43805 | R_1568 | 24 | 89286 | 89309 |
| R_67 | 46 | 3869 | 3914 | R_818 | 21 | 43818 | 43838 | R_1569 | 42 | 89374 | 89415 |
| R_68 | 27 | 3916 | 3942 | R_819 | 72 | 43916 | 43987 | R_1570 | 24 | 89430 | 89453 |
| R_69 | 21 | 3956 | 3976 | R_820 | 23 | 44002 | 44024 | R_1571 | 48 | 89466 | 89513 |
| R_70 | 41 | 4009 | 4049 | R_821 | 26 | 44041 | 44066 | R_1572 | 31 | 89528 | 89558 |
| R_71 | 29 | 4069 | 4097 | R_822 | 43 | 44103 | 44145 | R_1573 | 46 | 89563 | 89608 |
| R_72 | 37 | 4117 | 4153 | R_823 | 44 | 44167 | 44210 | R_1574 | 24 | 89610 | 89633 |
| R_73 | 23 | 4160 | 4182 | R_824 | 73 | 44216 | 44288 | R_1575 | 28 | 89725 | 89752 |
| R_74 | 38 | 4191 | 4228 | R_825 | 23 | 44284 | 44306 | R_1576 | 25 | 89754 | 89778 |
| R_75 | 24 | 4263 | 4286 | R_826 | 38 | 44298 | 44335 | R_1577 | 21 | 89780 | 89800 |
| R_76 | 75 | 4288 | 4362 | R_827 | 56 | 44380 | 44435 | R_1578 | 27 | 89802 | 89828 |
| R_77 | 40 | 4388 | 4427 | R_828 | 20 | 44449 | 44468 | R_1579 | 38 | 89833 | 89870 |
| R_78 | 46 | 4429 | 4474 | R_829 | 50 | 44463 | 44512 | R_1580 | 23 | 89882 | 89904 |
| R_79 | 44 | 4525 | 4568 | R_830 | 21 | 44530 | 44550 | R_1581 | 20 | 89961 | 89980 |
| R_80 | 28 | 4600 | 4627 | R_831 | 25 | 44543 | 44567 | R_1582 | 35 | 89982 | 90016 |
| R_81 | 38 | 4646 | 4683 | R_832 | 38 | 44552 | 44589 | R_1583 | 44 | 90049 | 90092 |
| R_82 | 26 | 4696 | 4721 | R_833 | 28 | 44610 | 44637 | R_1584 | 27 | 90129 | 90155 |
| R_83 | 32 | 4732 | 4763 | R_834 | 25 | 44629 | 44653 | R_1585 | 21 | 90264 | 90284 |
| R_84 | 35 | 4787 | 4821 | R_835 | 45 | 44651 | 44695 | R_1586 | 35 | 90287 | 90321 |
| R_85 | 20 | 4837 | 4856 | R_836 | 28 | 44763 | 44790 | R_1587 | 40 | 90444 | 90483 |
| R_86 | 36 | 4900 | 4935 | R_837 | 21 | 44820 | 44840 | R_1588 | 73 | 90558 | 90630 |
| R_87 | 27 | 5033 | 5059 | R_838 | 32 | 44857 | 44888 | R_1589 | 20 | 90632 | 90651 |
| R_88 | 28 | 5066 | 5093 | R_839 | 47 | 44888 | 44934 | R_1590 | 28 | 90702 | 90729 |
| R_89 | 46 | 5098 | 5143 | R_840 | 20 | 44994 | 45013 | R_1591 | 35 | 90771 | 90805 |
| R_90 | 24 | 5145 | 5168 | R_841 | 21 | 45032 | 45052 | R_1592 | 27 | 90794 | 90820 |
| R_91 | 20 | 5184 | 5203 | R_842 | 23 | 45054 | 45076 | R_1593 | 24 | 90814 | 90837 |
| R_92 | 40 | 5205 | 5244 | R_843 | 22 | 45078 | 45099 | R_1594 | 30 | 90827 | 90856 |
| R_93 | 28 | 5246 | 5273 | R_844 | 38 | 45129 | 45166 | R_1595 | 21 | 90839 | 90859 |
| R_94 | 20 | 5329 | 5348 | R_845 | 21 | 45203 | 45223 | R_1596 | 21 | 90876 | 90896 |
| R_95 | 58 | 5366 | 5423 | R_846 | 66 | 45238 | 45303 | R_1597 | 26 | 90901 | 90926 |
| R_96 | 41 | 5425 | 5465 | R_847 | 33 | 45304 | 45336 | R_1598 | 29 | 90972 | 91000 |
| R_97 | 58 | 5524 | 5581 | R_848 | 37 | 45338 | 45374 | R_1599 | 24 | 91032 | 91055 |
| R_98 | 20 | 5583 | 5602 | R_849 | 35 | 45391 | 45425 | R_1600 | 42 | 91057 | 91098 |
| R_99 | 30 | 5635 | 5664 | R_850 | 24 | 45526 | 45549 | R_1601 | 30 | 91135 | 91164 |
| R_100 | 51 | 5694 | 5744 | R_851 | 25 | 45551 | 45575 | R_1602 | 25 | 91189 | 91213 |
| R_101 | 42 | 5775 | 5816 | R_852 | 27 | 45673 | 45699 | R_1603 | 26 | 91247 | 91272 |
| R_102 | 53 | 5838 | 5890 | R_853 | 69 | 45708 | 45776 | R_1604 | 21 | 91274 | 91294 |
| R_103 | 32 | 5892 | 5923 | R_854 | 48 | 45821 | 45868 | R_1605 | 29 | 91296 | 91324 |
| R_104 | 53 | 5925 | 5977 | R_855 | 37 | 45907 | 45943 | R_1606 | 20 | 91396 | 91415 |
| R_105 | 28 | 6001 | 6028 | R_856 | 42 | 45987 | 46028 | R_1607 | 31 | 91471 | 91501 |
| R_106 | 21 | 6039 | 6059 | R_857 | 37 | 46043 | 46079 | R_1608 | 71 | 91521 | 91591 |
| R_107 | 64 | 6106 | 6169 | R_858 | 36 | 46104 | 46139 | R_1609 | 48 | 91667 | 91714 |
| R_108 | 65 | 6176 | 6240 | R_859 | 30 | 46146 | 46175 | R_1610 | 23 | 91755 | 91777 |
| R_109 | 35 | 6242 | 6276 | R_860 | 25 | 46178 | 46202 | R_1611 | 29 | 91788 | 91816 |
| R_110 | 29 | 6276 | 6304 | R_861 | 21 | 46261 | 46281 | R_1612 | 32 | 91858 | 91889 |
| R_111 | 38 | 6306 | 6343 | R_862 | 50 | 46304 | 46353 | R_1613 | 28 | 91915 | 91942 |
| R_112 | 22 | 6374 | 6395 | R_863 | 40 | 46373 | 46412 | R_1614 | 35 | 91965 | 91999 |
| R_113 | 22 | 6422 | 6443 | R_864 | 29 | 46435 | 46463 | R_1615 | 29 | 92052 | 92080 |
| R_114 | 28 | 6464 | 6491 | R_865 | 27 | 46465 | 46491 | R_1616 | 20 | 92131 | 92150 |
| R_115 | 23 | 6524 | 6546 | R_866 | 36 | 46522 | 46557 | R_1617 | 20 | 92152 | 92171 |
| R_116 | 23 | 6574 | 6596 | R_867 | 37 | 46590 | 46626 | R_1618 | 32 | 92181 | 92212 |
| R_117 | 54 | 6615 | 6668 | R_868 | 22 | 46663 | 46684 | R_1619 | 43 | 92227 | 92269 |
| R_118 | 28 | 6725 | 6752 | R_869 | 60 | 46686 | 46745 | R_1620 | 29 | 92271 | 92299 |
| R_119 | 49 | 6738 | 6786 | R_870 | 34 | 46811 | 46844 | R_1621 | 98 | 92306 | 92403 |
| R_120 | 25 | 6788 | 6812 | R_871 | 28 | 46845 | 46872 | R_1622 | 22 | 92420 | 92441 |
| R_121 | 59 | 6819 | 6877 | R_872 | 85 | 46896 | 46980 | R_1623 | 31 | 92463 | 92493 |
| R_122 | 22 | 6908 | 6929 | R_873 | 23 | 47027 | 47049 | R_1624 | 23 | 92495 | 92517 |
| R_123 | 26 | 6931 | 6956 | R_874 | 69 | 47051 | 47119 | R_1625 | 27 | 92574 | 92600 |
| R_124 | 24 | 6958 | 6981 | R_875 | 62 | 47178 | 47239 | R_1626 | 134 | 92643 | 92776 |
| R_125 | 35 | 6984 | 7018 | R_876 | 42 | 47430 | 47471 | R_1627 | 57 | 92793 | 92849 |
| R_126 | 32 | 7020 | 7051 | R_877 | 20 | 47473 | 47492 | R_1628 | 43 | 92866 | 92908 |
| R_127 | 23 | 7097 | 7119 | R_878 | 38 | 47519 | 47556 | R_1629 | 45 | 92910 | 92954 |
| R_128 | 83 | 7121 | 7203 | R_879 | 33 | 47605 | 47637 | R_1630 | 26 | 92956 | 92981 |
| R_129 | 21 | 7205 | 7225 | R_880 | 34 | 47652 | 47685 | R_1631 | 23 | 92983 | 93005 |
| R_130 | 32 | 7242 | 7273 | R_881 | 33 | 47699 | 47731 | R_1632 | 46 | 93007 | 93052 |
| R_131 | 20 | 7289 | 7308 | R_882 | 29 | 47733 | 47761 | R_1633 | 30 | 93022 | 93051 |
| R_132 | 21 | 7376 | 7396 | R_883 | 36 | 47769 | 47804 | R_1634 | 22 | 93094 | 93115 |
| R_133 | 20 | 7397 | 7416 | R_884 | 22 | 47806 | 47827 | R_1635 | 21 | 93117 | 93137 |
| R_134 | 23 | 7439 | 7461 | R_885 | 28 | 47848 | 47875 | R_1636 | 39 | 93139 | 93177 |
| R_135 | 23 | 7463 | 7485 | R_886 | 31 | 47999 | 48029 | R_1637 | 117 | 93214 | 93330 |
| R_136 | 28 | 7492 | 7519 | R_887 | 36 | 48043 | 48078 | R_1638 | 37 | 93359 | 93395 |
| R_137 | 26 | 7569 | 7594 | R_888 | 37 | 48080 | 48116 | R_1639 | 46 | 93409 | 93454 |
| R_138 | 38 | 7622 | 7659 | R_889 | 42 | 48118 | 48159 | R_1640 | 32 | 93508 | 93539 |
| R_139 | 25 | 7705 | 7729 | R_890 | 78 | 48195 | 48272 | R_1641 | 28 | 93541 | 93568 |
| R_140 | 20 | 7705 | 7724 | R_891 | 70 | 48294 | 48363 | R_1642 | 33 | 93570 | 93602 |
| R_141 | 28 | 7774 | 7801 | R_892 | 28 | 48377 | 48404 | R_1643 | 22 | 93647 | 93668 |
| R_142 | 20 | 7855 | 7874 | R_893 | 20 | 48406 | 48425 | R_1644 | 26 | 93674 | 93699 |
| R_143 | 23 | 7885 | 7907 | R_894 | 22 | 48438 | 48459 | R_1645 | 28 | 93716 | 93743 |
| R_144 | 35 | 7933 | 7967 | R_895 | 20 | 48485 | 48504 | R_1646 | 72 | 93770 | 93841 |
| R_145 | 21 | 7937 | 7957 | R_896 | 23 | 48532 | 48554 | R_1647 | 36 | 93897 | 93932 |
| R_146 | 20 | 7937 | 7956 | R_897 | 32 | 48564 | 48595 | R_1648 | 25 | 94007 | 94031 |
| R_147 | 23 | 7948 | 7970 | R_898 | 43 | 48627 | 48669 | R_1649 | 25 | 94121 | 94145 |
| R_148 | 26 | 7952 | 7977 | R_899 | 32 | 48671 | 48702 | R_1650 | 20 | 94227 | 94246 |
| R_149 | 25 | 7953 | 7977 | R_900 | 30 | 48744 | 48773 | R_1651 | 69 | 94295 | 94363 |
| R_150 | 30 | 8009 | 8038 | R_901 | 24 | 48782 | 48805 | R_1652 | 49 | 94371 | 94419 |
| R_151 | 31 | 8043 | 8073 | R_902 | 21 | 48797 | 48817 | R_1653 | 40 | 94426 | 94465 |
| R_152 | 20 | 8125 | 8144 | R_903 | 22 | 48802 | 48823 | R_1654 | 73 | 94478 | 94550 |
| R_153 | 21 | 8146 | 8166 | R_904 | 54 | 48808 | 48861 | R_1655 | 35 | 94571 | 94605 |
| R_154 | 36 | 8168 | 8203 | R_905 | 38 | 48924 | 48961 | R_1656 | 63 | 94607 | 94669 |
| R_155 | 44 | 8245 | 8288 | R_906 | 20 | 48966 | 48985 | R_1657 | 41 | 94788 | 94828 |
| R_156 | 29 | 8324 | 8352 | R_907 | 25 | 49010 | 49034 | R_1658 | 73 | 94844 | 94916 |
| R_157 | 43 | 8355 | 8397 | R_908 | 21 | 49067 | 49087 | R_1659 | 21 | 94929 | 94949 |
| R_158 | 23 | 8399 | 8421 | R_909 | 61 | 49145 | 49205 | R_1660 | 21 | 94979 | 94999 |
| R_159 | 26 | 8457 | 8482 | R_910 | 81 | 49207 | 49287 | R_1661 | 31 | 95087 | 95117 |
| R_160 | 54 | 8486 | 8539 | R_911 | 35 | 49289 | 49323 | R_1662 | 25 | 95173 | 95197 |
| R_161 | 43 | 8541 | 8583 | R_912 | 41 | 49325 | 49365 | R_1663 | 23 | 95244 | 95266 |
| R_162 | 26 | 8585 | 8610 | R_913 | 99 | 49400 | 49498 | R_1664 | 38 | 95278 | 95315 |
| R_163 | 26 | 8637 | 8662 | R_914 | 30 | 49507 | 49536 | R_1665 | 28 | 95355 | 95382 |
| R_164 | 37 | 8678 | 8714 | R_915 | 24 | 49538 | 49561 | R_1666 | 95 | 95390 | 95484 |
| R_165 | 24 | 8742 | 8765 | R_916 | 23 | 49563 | 49585 | R_1667 | 159 | 95486 | 95644 |
| R_166 | 37 | 8812 | 8848 | R_917 | 27 | 49612 | 49638 | R_1668 | 30 | 95646 | 95675 |
| R_167 | 37 | 8868 | 8904 | R_918 | 33 | 49654 | 49686 | R_1669 | 101 | 95695 | 95795 |
| R_168 | 21 | 9015 | 9035 | R_919 | 37 | 49697 | 49733 | R_1670 | 33 | 95807 | 95839 |
| R_169 | 28 | 9065 | 9092 | R_920 | 28 | 49751 | 49778 | R_1671 | 24 | 95863 | 95886 |
| R_170 | 20 | 9180 | 9199 | R_921 | 20 | 49870 | 49889 | R_1672 | 22 | 95888 | 95909 |
| R_171 | 23 | 9191 | 9213 | R_922 | 42 | 49890 | 49931 | R_1673 | 31 | 95915 | 95945 |
| R_172 | 24 | 9203 | 9226 | R_923 | 38 | 49964 | 50001 | R_1674 | 30 | 95951 | 95980 |
| R_173 | 28 | 9215 | 9242 | R_924 | 106 | 50003 | 50108 | R_1675 | 28 | 96033 | 96060 |
| R_174 | 21 | 9244 | 9264 | R_925 | 29 | 50110 | 50138 | R_1676 | 37 | 96057 | 96093 |
| R_175 | 23 | 9260 | 9282 | R_926 | 24 | 50394 | 50417 | R_1677 | 28 | 96159 | 96186 |
| R_176 | 25 | 9266 | 9290 | R_927 | 42 | 50473 | 50514 | R_1678 | 40 | 96287 | 96326 |
| R_177 | 23 | 9266 | 9288 | R_928 | 27 | 50578 | 50604 | R_1679 | 43 | 96331 | 96373 |
| R_178 | 24 | 9267 | 9290 | R_929 | 42 | 50606 | 50647 | R_1680 | 39 | 96450 | 96488 |
| R_179 | 21 | 9267 | 9287 | R_930 | 42 | 50692 | 50733 | R_1681 | 30 | 96492 | 96521 |
| R_180 | 22 | 9267 | 9288 | R_931 | 20 | 50763 | 50782 | R_1682 | 44 | 96523 | 96566 |
| R_181 | 23 | 9268 | 9290 | R_932 | 34 | 50808 | 50841 | R_1683 | 22 | 96589 | 96610 |
| R_182 | 21 | 9270 | 9290 | R_933 | 48 | 50847 | 50894 | R_1684 | 22 | 96655 | 96676 |
| R_183 | 23 | 9289 | 9311 | R_934 | 55 | 50955 | 51009 | R_1685 | 52 | 96714 | 96765 |
| R_184 | 20 | 9292 | 9311 | R_935 | 21 | 51011 | 51031 | R_1686 | 23 | 96776 | 96798 |
| R_185 | 22 | 9330 | 9351 | R_936 | 58 | 51071 | 51128 | R_1687 | 25 | 96798 | 96822 |
| R_186 | 20 | 9334 | 9353 | R_937 | 85 | 51138 | 51222 | R_1688 | 36 | 96838 | 96873 |
| R_187 | 22 | 10083 | 10104 | R_938 | 22 | 51273 | 51294 | R_1689 | 44 | 96895 | 96938 |
| R_188 | 23 | 10092 | 10114 | R_939 | 40 | 51330 | 51369 | R_1690 | 21 | 96940 | 96960 |
| R_189 | 38 | 10119 | 10156 | R_940 | 20 | 51343 | 51362 | R_1691 | 24 | 96993 | 97016 |
| R_190 | 20 | 10255 | 10274 | R_941 | 71 | 51498 | 51568 | R_1692 | 24 | 97038 | 97061 |
| R_191 | 21 | 10257 | 10277 | R_942 | 35 | 51570 | 51604 | R_1693 | 22 | 97073 | 97094 |
| R_192 | 28 | 10305 | 10332 | R_943 | 20 | 51639 | 51658 | R_1694 | 25 | 97106 | 97130 |
| R_193 | 63 | 10358 | 10420 | R_944 | 31 | 51680 | 51710 | R_1695 | 20 | 97132 | 97151 |
| R_194 | 28 | 10498 | 10525 | R_945 | 75 | 51712 | 51786 | R_1696 | 23 | 97162 | 97184 |
| R_195 | 27 | 10597 | 10623 | R_946 | 57 | 51788 | 51844 | R_1697 | 38 | 97186 | 97223 |
| R_196 | 24 | 10625 | 10648 | R_947 | 57 | 51846 | 51902 | R_1698 | 32 | 97225 | 97256 |
| R_197 | 56 | 10666 | 10721 | R_948 | 33 | 51928 | 51960 | R_1699 | 41 | 97258 | 97298 |
| R_198 | 27 | 10741 | 10767 | R_949 | 33 | 51962 | 51994 | R_1700 | 34 | 97300 | 97333 |
| R_199 | 21 | 10777 | 10797 | R_950 | 20 | 52012 | 52031 | R_1701 | 20 | 97342 | 97361 |
| R_200 | 38 | 10799 | 10836 | R_951 | 52 | 52024 | 52075 | R_1702 | 21 | 97486 | 97506 |
| R_201 | 30 | 10840 | 10869 | R_952 | 20 | 52183 | 52202 | R_1703 | 24 | 97532 | 97555 |
| R_202 | 24 | 10871 | 10894 | R_953 | 31 | 52316 | 52346 | R_1704 | 20 | 97592 | 97611 |
| R_203 | 30 | 10911 | 10940 | R_954 | 54 | 52348 | 52401 | R_1705 | 21 | 97606 | 97626 |
| R_204 | 49 | 10942 | 10990 | R_955 | 24 | 52408 | 52431 | R_1706 | 20 | 97690 | 97709 |
| R_205 | 21 | 10992 | 11012 | R_956 | 25 | 52433 | 52457 | R_1707 | 43 | 97694 | 97736 |
| R_206 | 69 | 11018 | 11086 | R_957 | 68 | 52452 | 52519 | R_1708 | 26 | 97740 | 97765 |
| R_207 | 30 | 11089 | 11118 | R_958 | 42 | 52521 | 52562 | R_1709 | 28 | 97767 | 97794 |
| R_208 | 42 | 11127 | 11168 | R_959 | 41 | 52569 | 52609 | R_1710 | 64 | 97820 | 97883 |
| R_209 | 25 | 11193 | 11217 | R_960 | 21 | 52626 | 52646 | R_1711 | 32 | 97928 | 97959 |
| R_210 | 68 | 11279 | 11346 | R_961 | 21 | 52676 | 52696 | R_1712 | 40 | 98008 | 98047 |
| R_211 | 42 | 11367 | 11408 | R_962 | 71 | 52704 | 52774 | R_1713 | 49 | 98103 | 98151 |
| R_212 | 43 | 11410 | 11452 | R_963 | 31 | 52784 | 52814 | R_1714 | 33 | 98166 | 98198 |
| R_213 | 54 | 11458 | 11511 | R_964 | 22 | 52826 | 52847 | R_1715 | 26 | 98200 | 98225 |
| R_214 | 79 | 11556 | 11634 | R_965 | 25 | 52874 | 52898 | R_1716 | 32 | 98324 | 98355 |
| R_215 | 37 | 11648 | 11684 | R_966 | 80 | 52915 | 52994 | R_1717 | 21 | 98333 | 98353 |
| R_216 | 31 | 11691 | 11721 | R_967 | 21 | 53027 | 53047 | R_1718 | 21 | 98467 | 98487 |
| R_217 | 28 | 11724 | 11751 | R_968 | 44 | 53130 | 53173 | R_1719 | 22 | 98506 | 98527 |
| R_218 | 81 | 11800 | 11880 | R_969 | 21 | 53175 | 53195 | R_1720 | 31 | 98577 | 98607 |
| R_219 | 20 | 11905 | 11924 | R_970 | 24 | 53181 | 53204 | R_1721 | 32 | 98681 | 98712 |
| R_220 | 21 | 11928 | 11948 | R_971 | 22 | 53233 | 53254 | R_1722 | 23 | 98751 | 98773 |
| R_221 | 50 | 11950 | 11999 | R_972 | 20 | 53262 | 53281 | R_1723 | 37 | 98789 | 98825 |
| R_222 | 20 | 12030 | 12049 | R_973 | 22 | 53315 | 53336 | R_1724 | 37 | 98930 | 98966 |
| R_223 | 61 | 12051 | 12111 | R_974 | 20 | 53352 | 53371 | R_1725 | 40 | 98969 | 99008 |
| R_224 | 23 | 12147 | 12169 | R_975 | 72 | 53390 | 53461 | R_1726 | 21 | 99015 | 99035 |
| R_225 | 25 | 12171 | 12195 | R_976 | 42 | 53473 | 53514 | R_1727 | 45 | 99231 | 99275 |
| R_226 | 23 | 12197 | 12219 | R_977 | 25 | 53534 | 53558 | R_1728 | 38 | 99345 | 99382 |
| R_227 | 45 | 12221 | 12265 | R_978 | 30 | 53560 | 53589 | R_1729 | 46 | 99387 | 99432 |
| R_228 | 43 | 12304 | 12346 | R_979 | 23 | 53600 | 53622 | R_1730 | 25 | 99434 | 99458 |
| R_229 | 51 | 12353 | 12403 | R_980 | 28 | 53637 | 53664 | R_1731 | 21 | 99515 | 99535 |
| R_230 | 23 | 12405 | 12427 | R_981 | 24 | 53696 | 53719 | R_1732 | 23 | 99565 | 99587 |
| R_231 | 62 | 12475 | 12536 | R_982 | 21 | 53738 | 53758 | R_1733 | 21 | 99658 | 99678 |
| R_232 | 28 | 12538 | 12565 | R_983 | 22 | 53753 | 53774 | R_1734 | 43 | 99718 | 99760 |
| R_233 | 28 | 12587 | 12614 | R_984 | 23 | 53759 | 53781 | R_1735 | 30 | 99762 | 99791 |
| R_234 | 21 | 12615 | 12635 | R_985 | 30 | 53793 | 53822 | R_1736 | 62 | 99820 | 99881 |
| R_235 | 29 | 12637 | 12665 | R_986 | 23 | 53895 | 53917 | R_1737 | 21 | 99933 | 99953 |
| R_236 | 38 | 12684 | 12721 | R_987 | 25 | 53910 | 53934 | R_1738 | 26 | 99986 | 100011 |
| R_237 | 34 | 12746 | 12779 | R_988 | 21 | 53979 | 53999 | R_1739 | 29 | 100013 | 100041 |
| R_238 | 20 | 12799 | 12818 | R_989 | 20 | 53996 | 54015 | R_1740 | 71 | 100063 | 100133 |
| R_239 | 33 | 12822 | 12854 | R_990 | 21 | 54027 | 54047 | R_1741 | 32 | 100169 | 100200 |
| R_240 | 37 | 12856 | 12892 | R_991 | 28 | 54049 | 54076 | R_1742 | 21 | 100248 | 100268 |
| R_241 | 20 | 12894 | 12913 | R_992 | 40 | 54162 | 54201 | R_1743 | 30 | 100263 | 100292 |
| R_242 | 23 | 12933 | 12955 | R_993 | 20 | 54218 | 54237 | R_1744 | 38 | 100296 | 100333 |
| R_243 | 50 | 13057 | 13106 | R_994 | 77 | 54239 | 54315 | R_1745 | 22 | 100359 | 100380 |
| R_244 | 37 | 13133 | 13169 | R_995 | 50 | 54317 | 54366 | R_1746 | 23 | 100375 | 100397 |
| R_245 | 51 | 13227 | 13277 | R_996 | 21 | 54368 | 54388 | R_1747 | 23 | 100384 | 100406 |
| R_246 | 22 | 13348 | 13369 | R_997 | 32 | 54406 | 54437 | R_1748 | 24 | 100639 | 100662 |
| R_247 | 29 | 13380 | 13408 | R_998 | 33 | 54439 | 54471 | R_1749 | 24 | 100645 | 100668 |
| R_248 | 41 | 13410 | 13450 | R_999 | 20 | 54507 | 54526 | R_1750 | 20 | 100666 | 100685 |
| R_249 | 32 | 13452 | 13483 | R_1000 | 55 | 54528 | 54582 | R_1751 | 23 | 100695 | 100717 |
| R_250 | 45 | 13483 | 13527 | R_1001 | 21 | 54584 | 54604 | R_1752 | 20 | 100746 | 100765 |
| R_251 | 32 | 13529 | 13560 | R_1002 | 42 | 54606 | 54647 | R_1753 | 34 | 100771 | 100804 |
| R_252 | 21 | 13569 | 13589 | R_1003 | 118 | 54651 | 54768 | R_1754 | 21 | 100801 | 100821 |
| R_253 | 50 | 13591 | 13640 | R_1004 | 23 | 54833 | 54855 | R_1755 | 26 | 100823 | 100848 |
| R_254 | 88 | 13770 | 13857 | R_1005 | 28 | 54857 | 54884 | R_1756 | 20 | 100857 | 100876 |
| R_255 | 20 | 13861 | 13880 | R_1006 | 57 | 54887 | 54943 | R_1757 | 34 | 100899 | 100932 |
| R_256 | 32 | 13882 | 13913 | R_1007 | 29 | 54973 | 55001 | R_1758 | 21 | 100965 | 100985 |
| R_257 | 55 | 13936 | 13990 | R_1008 | 21 | 55014 | 55034 | R_1759 | 32 | 101017 | 101048 |
| R_258 | 39 | 13992 | 14030 | R_1009 | 28 | 55074 | 55101 | R_1760 | 21 | 101085 | 101105 |
| R_259 | 34 | 14033 | 14066 | R_1010 | 21 | 55134 | 55154 | R_1761 | 26 | 101195 | 101220 |
| R_260 | 35 | 14068 | 14102 | R_1011 | 38 | 55171 | 55208 | R_1762 | 23 | 101227 | 101249 |
| R_261 | 27 | 14104 | 14130 | R_1012 | 31 | 55210 | 55240 | R_1763 | 30 | 101324 | 101353 |
| R_262 | 20 | 14140 | 14159 | R_1013 | 80 | 55248 | 55327 | R_1764 | 20 | 101357 | 101376 |
| R_263 | 51 | 14180 | 14230 | R_1014 | 25 | 55329 | 55353 | R_1765 | 21 | 101415 | 101435 |
| R_264 | 20 | 14232 | 14251 | R_1015 | 23 | 55365 | 55387 | R_1766 | 20 | 101444 | 101463 |
| R_265 | 107 | 14253 | 14359 | R_1016 | 43 | 55424 | 55466 | R_1767 | 37 | 101465 | 101501 |
| R_266 | 72 | 14367 | 14438 | R_1017 | 51 | 55539 | 55589 | R_1768 | 25 | 101497 | 101521 |
| R_267 | 69 | 14503 | 14571 | R_1018 | 27 | 55591 | 55617 | R_1769 | 42 | 101523 | 101564 |
| R_268 | 27 | 14595 | 14621 | R_1019 | 29 | 55619 | 55647 | R_1770 | 26 | 101576 | 101601 |
| R_269 | 35 | 14629 | 14663 | R_1020 | 30 | 55653 | 55682 | R_1771 | 34 | 101620 | 101653 |
| R_270 | 58 | 14732 | 14789 | R_1021 | 29 | 55724 | 55752 | R_1772 | 36 | 101679 | 101714 |
| R_271 | 25 | 14805 | 14829 | R_1022 | 33 | 55778 | 55810 | R_1773 | 39 | 101734 | 101772 |
| R_272 | 56 | 14851 | 14906 | R_1023 | 76 | 55848 | 55923 | R_1774 | 24 | 101779 | 101802 |
| R_273 | 53 | 14954 | 15006 | R_1024 | 33 | 55992 | 56024 | R_1775 | 71 | 101817 | 101887 |
| R_274 | 39 | 15026 | 15064 | R_1025 | 29 | 56026 | 56054 | R_1776 | 67 | 101913 | 101979 |
| R_275 | 21 | 15066 | 15086 | R_1026 | 59 | 56080 | 56138 | R_1777 | 28 | 101989 | 102016 |
| R_276 | 22 | 15138 | 15159 | R_1027 | 26 | 56155 | 56180 | R_1778 | 28 | 102025 | 102052 |
| R_277 | 107 | 15157 | 15263 | R_1028 | 22 | 56196 | 56217 | R_1779 | 33 | 102054 | 102086 |
| R_278 | 24 | 15249 | 15272 | R_1029 | 21 | 56225 | 56245 | R_1780 | 23 | 102088 | 102110 |
| R_279 | 22 | 15277 | 15298 | R_1030 | 31 | 56274 | 56304 | R_1781 | 44 | 102112 | 102155 |
| R_280 | 38 | 15300 | 15337 | R_1031 | 24 | 56338 | 56361 | R_1782 | 22 | 102161 | 102182 |
| R_281 | 24 | 15414 | 15437 | R_1032 | 22 | 56410 | 56431 | R_1783 | 65 | 102202 | 102266 |
| R_282 | 21 | 15476 | 15496 | R_1033 | 36 | 56433 | 56468 | R_1784 | 23 | 102268 | 102290 |
| R_283 | 23 | 15617 | 15639 | R_1034 | 22 | 56521 | 56542 | R_1785 | 35 | 102292 | 102326 |
| R_284 | 58 | 15671 | 15728 | R_1035 | 30 | 56567 | 56596 | R_1786 | 32 | 102352 | 102383 |
| R_285 | 36 | 15730 | 15765 | R_1036 | 55 | 56641 | 56695 | R_1787 | 29 | 102385 | 102413 |
| R_286 | 29 | 15840 | 15868 | R_1037 | 44 | 56697 | 56740 | R_1788 | 29 | 102526 | 102554 |
| R_287 | 27 | 15870 | 15896 | R_1038 | 43 | 56761 | 56803 | R_1789 | 77 | 102579 | 102655 |
| R_288 | 50 | 15926 | 15975 | R_1039 | 72 | 56805 | 56876 | R_1790 | 39 | 102744 | 102782 |
| R_289 | 27 | 16008 | 16034 | R_1040 | 30 | 56885 | 56914 | R_1791 | 32 | 102841 | 102872 |
| R_290 | 46 | 16109 | 16154 | R_1041 | 44 | 56916 | 56959 | R_1792 | 22 | 103017 | 103038 |
| R_291 | 27 | 16159 | 16185 | R_1042 | 67 | 56961 | 57027 | R_1793 | 20 | 103118 | 103137 |
| R_292 | 30 | 16245 | 16274 | R_1043 | 30 | 57033 | 57062 | R_1794 | 20 | 103196 | 103215 |
| R_293 | 44 | 16296 | 16339 | R_1044 | 20 | 57167 | 57186 | R_1795 | 23 | 103346 | 103368 |
| R_294 | 20 | 16316 | 16335 | R_1045 | 49 | 57211 | 57259 | R_1796 | 24 | 103400 | 103423 |
| R_295 | 48 | 16371 | 16418 | R_1046 | 24 | 57348 | 57371 | R_1797 | 27 | 103456 | 103482 |
| R_296 | 36 | 16447 | 16482 | R_1047 | 43 | 57434 | 57476 | R_1798 | 54 | 103494 | 103547 |
| R_297 | 36 | 16485 | 16520 | R_1048 | 73 | 57536 | 57608 | R_1799 | 21 | 103557 | 103577 |
| R_298 | 26 | 16532 | 16557 | R_1049 | 86 | 57641 | 57726 | R_1800 | 34 | 103637 | 103670 |
| R_299 | 21 | 16582 | 16602 | R_1050 | 27 | 57754 | 57780 | R_1801 | 58 | 103683 | 103740 |
| R_300 | 83 | 16604 | 16686 | R_1051 | 20 | 57786 | 57805 | R_1802 | 25 | 103782 | 103806 |
| R_301 | 63 | 16688 | 16750 | R_1052 | 21 | 57807 | 57827 | R_1803 | 20 | 103851 | 103870 |
| R_302 | 75 | 16766 | 16840 | R_1053 | 27 | 57829 | 57855 | R_1804 | 26 | 103876 | 103901 |
| R_303 | 24 | 16918 | 16941 | R_1054 | 41 | 57857 | 57897 | R_1805 | 21 | 103997 | 104017 |
| R_304 | 32 | 16947 | 16978 | R_1055 | 51 | 57899 | 57949 | R_1806 | 49 | 104093 | 104141 |
| R_305 | 31 | 17007 | 17037 | R_1056 | 26 | 57981 | 58006 | R_1807 | 61 | 104143 | 104203 |
| R_306 | 45 | 17039 | 17083 | R_1057 | 48 | 58008 | 58055 | R_1808 | 28 | 104263 | 104290 |
| R_307 | 25 | 17085 | 17109 | R_1058 | 26 | 58057 | 58082 | R_1809 | 22 | 104331 | 104352 |
| R_308 | 30 | 17111 | 17140 | R_1059 | 32 | 58097 | 58128 | R_1810 | 24 | 104354 | 104377 |
| R_309 | 29 | 17179 | 17207 | R_1060 | 40 | 58138 | 58177 | R_1811 | 36 | 104379 | 104414 |
| R_310 | 34 | 17292 | 17325 | R_1061 | 38 | 58192 | 58229 | R_1812 | 72 | 104416 | 104487 |
| R_311 | 28 | 17292 | 17319 | R_1062 | 26 | 58235 | 58260 | R_1813 | 23 | 104504 | 104526 |
| R_312 | 28 | 17309 | 17336 | R_1063 | 57 | 58375 | 58431 | R_1814 | 54 | 104544 | 104597 |
| R_313 | 21 | 17316 | 17336 | R_1064 | 25 | 58444 | 58468 | R_1815 | 20 | 104599 | 104618 |
| R_314 | 21 | 17319 | 17339 | R_1065 | 55 | 58484 | 58538 | R_1816 | 22 | 104632 | 104653 |
| R_315 | 22 | 17326 | 17347 | R_1066 | 26 | 58555 | 58580 | R_1817 | 25 | 104710 | 104734 |
| R_316 | 52 | 17349 | 17400 | R_1067 | 20 | 58582 | 58601 | R_1818 | 22 | 104738 | 104759 |
| R_317 | 20 | 17416 | 17435 | R_1068 | 23 | 58604 | 58626 | R_1819 | 40 | 104783 | 104822 |
| R_318 | 39 | 17445 | 17483 | R_1069 | 32 | 58650 | 58681 | R_1820 | 42 | 104824 | 104865 |
| R_319 | 43 | 17485 | 17527 | R_1070 | 70 | 58740 | 58809 | R_1821 | 21 | 104919 | 104939 |
| R_320 | 74 | 17587 | 17660 | R_1071 | 32 | 58889 | 58920 | R_1822 | 23 | 105014 | 105036 |
| R_321 | 38 | 17667 | 17704 | R_1072 | 25 | 58927 | 58951 | R_1823 | 58 | 105040 | 105097 |
| R_322 | 25 | 17706 | 17730 | R_1073 | 22 | 58953 | 58974 | R_1824 | 25 | 105111 | 105135 |
| R_323 | 45 | 17796 | 17840 | R_1074 | 35 | 58993 | 59027 | R_1825 | 50 | 105137 | 105186 |
| R_324 | 53 | 17855 | 17907 | R_1075 | 48 | 59029 | 59076 | R_1826 | 22 | 105188 | 105209 |
| R_325 | 44 | 17909 | 17952 | R_1076 | 45 | 59079 | 59123 | R_1827 | 40 | 105283 | 105322 |
| R_326 | 20 | 17954 | 17973 | R_1077 | 31 | 59125 | 59155 | R_1828 | 31 | 105393 | 105423 |
| R_327 | 34 | 17975 | 18008 | R_1078 | 31 | 59183 | 59213 | R_1829 | 29 | 105427 | 105455 |
| R_328 | 20 | 18010 | 18029 | R_1079 | 20 | 59243 | 59262 | R_1830 | 72 | 105457 | 105528 |
| R_329 | 46 | 18031 | 18076 | R_1080 | 35 | 59264 | 59298 | R_1831 | 30 | 105544 | 105573 |
| R_330 | 26 | 18078 | 18103 | R_1081 | 24 | 59303 | 59326 | R_1832 | 39 | 105683 | 105721 |
| R_331 | 29 | 18136 | 18164 | R_1082 | 39 | 59328 | 59366 | R_1833 | 36 | 105732 | 105767 |
| R_332 | 33 | 18208 | 18240 | R_1083 | 31 | 59380 | 59410 | R_1834 | 23 | 106011 | 106033 |
| R_333 | 54 | 18261 | 18314 | R_1084 | 20 | 59490 | 59509 | R_1835 | 45 | 106334 | 106378 |
| R_334 | 22 | 18333 | 18354 | R_1085 | 39 | 59551 | 59589 | R_1836 | 21 | 106380 | 106400 |
| R_335 | 34 | 18410 | 18443 | R_1086 | 76 | 59591 | 59666 | R_1837 | 23 | 106407 | 106429 |
| R_336 | 27 | 18446 | 18472 | R_1087 | 46 | 59713 | 59758 | R_1838 | 23 | 106475 | 106497 |
| R_337 | 86 | 18474 | 18559 | R_1088 | 26 | 59837 | 59862 | R_1839 | 47 | 106562 | 106608 |
| R_338 | 25 | 18590 | 18614 | R_1089 | 40 | 59878 | 59917 | R_1840 | 42 | 106645 | 106686 |
| R_339 | 21 | 18627 | 18647 | R_1090 | 23 | 59957 | 59979 | R_1841 | 44 | 106677 | 106720 |
| R_340 | 37 | 18650 | 18686 | R_1091 | 37 | 59998 | 60034 | R_1842 | 29 | 106677 | 106705 |
| R_341 | 33 | 18688 | 18720 | R_1092 | 63 | 60133 | 60195 | R_1843 | 22 | 106728 | 106749 |
| R_342 | 30 | 18742 | 18771 | R_1093 | 22 | 60201 | 60222 | R_1844 | 40 | 106783 | 106822 |
| R_343 | 20 | 18773 | 18792 | R_1094 | 23 | 60281 | 60303 | R_1845 | 22 | 106824 | 106845 |
| R_344 | 32 | 18782 | 18813 | R_1095 | 37 | 60291 | 60327 | R_1846 | 31 | 106847 | 106877 |
| R_345 | 20 | 18843 | 18862 | R_1096 | 27 | 60360 | 60386 | R_1847 | 31 | 106879 | 106909 |
| R_346 | 24 | 18864 | 18887 | R_1097 | 23 | 60429 | 60451 | R_1848 | 64 | 106923 | 106986 |
| R_347 | 24 | 18900 | 18923 | R_1098 | 52 | 60536 | 60587 | R_1849 | 35 | 106988 | 107022 |
| R_348 | 35 | 18935 | 18969 | R_1099 | 24 | 60605 | 60628 | R_1850 | 35 | 107046 | 107080 |
| R_349 | 38 | 18971 | 19008 | R_1100 | 28 | 60656 | 60683 | R_1851 | 26 | 107085 | 107110 |
| R_350 | 23 | 19080 | 19102 | R_1101 | 90 | 60703 | 60792 | R_1852 | 25 | 107122 | 107146 |
| R_351 | 51 | 19106 | 19156 | R_1102 | 48 | 60794 | 60841 | R_1853 | 40 | 107239 | 107278 |
| R_352 | 21 | 19158 | 19178 | R_1103 | 49 | 60841 | 60889 | R_1854 | 57 | 107338 | 107394 |
| R_353 | 25 | 19262 | 19286 | R_1104 | 31 | 60921 | 60951 | R_1855 | 36 | 107405 | 107440 |
| R_354 | 22 | 19310 | 19331 | R_1105 | 21 | 60953 | 60973 | R_1856 | 22 | 107442 | 107463 |
| R_355 | 28 | 19333 | 19360 | R_1106 | 30 | 60979 | 61008 | R_1857 | 22 | 107465 | 107486 |
| R_356 | 24 | 19362 | 19385 | R_1107 | 23 | 61040 | 61062 | R_1858 | 22 | 107506 | 107527 |
| R_357 | 44 | 19394 | 19437 | R_1108 | 20 | 61117 | 61136 | R_1859 | 28 | 107553 | 107580 |
| R_358 | 47 | 19493 | 19539 | R_1109 | 22 | 61148 | 61169 | R_1860 | 53 | 107582 | 107634 |
| R_359 | 26 | 19569 | 19594 | R_1110 | 106 | 61165 | 61270 | R_1861 | 37 | 107639 | 107675 |
| R_360 | 34 | 19624 | 19657 | R_1111 | 21 | 61274 | 61294 | R_1862 | 34 | 107679 | 107712 |
| R_361 | 38 | 19659 | 19696 | R_1112 | 25 | 61392 | 61416 | R_1863 | 36 | 107775 | 107810 |
| R_362 | 32 | 19713 | 19744 | R_1113 | 22 | 61447 | 61468 | R_1864 | 25 | 107868 | 107892 |
| R_363 | 56 | 19746 | 19801 | R_1114 | 25 | 61486 | 61510 | R_1865 | 24 | 107893 | 107916 |
| R_364 | 43 | 19839 | 19881 | R_1115 | 23 | 61495 | 61517 | R_1866 | 24 | 108016 | 108039 |
| R_365 | 24 | 19894 | 19917 | R_1116 | 27 | 61518 | 61544 | R_1867 | 42 | 108071 | 108112 |
| R_366 | 24 | 19960 | 19983 | R_1117 | 23 | 61586 | 61608 | R_1868 | 21 | 108176 | 108196 |
| R_367 | 21 | 19985 | 20005 | R_1118 | 32 | 61646 | 61677 | R_1869 | 30 | 108213 | 108242 |
| R_368 | 30 | 20006 | 20035 | R_1119 | 34 | 61784 | 61817 | R_1870 | 72 | 108263 | 108334 |
| R_369 | 21 | 20037 | 20057 | R_1120 | 23 | 61870 | 61892 | R_1871 | 32 | 108390 | 108421 |
| R_370 | 20 | 20069 | 20088 | R_1121 | 43 | 61904 | 61946 | R_1872 | 27 | 108441 | 108467 |
| R_371 | 20 | 20151 | 20170 | R_1122 | 22 | 61948 | 61969 | R_1873 | 31 | 108479 | 108509 |
| R_372 | 25 | 20182 | 20206 | R_1123 | 33 | 61997 | 62029 | R_1874 | 21 | 108524 | 108544 |
| R_373 | 22 | 20237 | 20258 | R_1124 | 21 | 62076 | 62096 | R_1875 | 58 | 108546 | 108603 |
| R_374 | 22 | 20267 | 20288 | R_1125 | 22 | 62103 | 62124 | R_1876 | 33 | 108669 | 108701 |
| R_375 | 27 | 20363 | 20389 | R_1126 | 20 | 62133 | 62152 | R_1877 | 26 | 108721 | 108746 |
| R_376 | 25 | 20375 | 20399 | R_1127 | 26 | 62162 | 62187 | R_1878 | 30 | 108822 | 108851 |
| R_377 | 21 | 20482 | 20502 | R_1128 | 20 | 62239 | 62258 | R_1879 | 32 | 108859 | 108890 |
| R_378 | 27 | 20485 | 20511 | R_1129 | 24 | 62243 | 62266 | R_1880 | 30 | 108909 | 108938 |
| R_379 | 22 | 20497 | 20518 | R_1130 | 20 | 62266 | 62285 | R_1881 | 41 | 108996 | 109036 |
| R_380 | 24 | 20566 | 20589 | R_1131 | 24 | 62307 | 62330 | R_1882 | 43 | 109038 | 109080 |
| R_381 | 22 | 20591 | 20612 | R_1132 | 27 | 62332 | 62358 | R_1883 | 22 | 109104 | 109125 |
| R_382 | 20 | 20610 | 20629 | R_1133 | 22 | 62433 | 62454 | R_1884 | 41 | 109145 | 109185 |
| R_383 | 22 | 20679 | 20700 | R_1134 | 22 | 62561 | 62582 | R_1885 | 25 | 109237 | 109261 |
| R_384 | 28 | 20702 | 20729 | R_1135 | 50 | 62600 | 62649 | R_1886 | 41 | 109263 | 109303 |
| R_385 | 35 | 20741 | 20775 | R_1136 | 29 | 62678 | 62706 | R_1887 | 34 | 109306 | 109339 |
| R_386 | 43 | 20790 | 20832 | R_1137 | 32 | 62708 | 62739 | R_1888 | 48 | 109355 | 109402 |
| R_387 | 35 | 20880 | 20914 | R_1138 | 20 | 62846 | 62865 | R_1889 | 20 | 109404 | 109423 |
| R_388 | 22 | 20892 | 20913 | R_1139 | 46 | 62871 | 62916 | R_1890 | 28 | 109425 | 109452 |
| R_389 | 21 | 21011 | 21031 | R_1140 | 23 | 62945 | 62967 | R_1891 | 31 | 109454 | 109484 |
| R_390 | 26 | 21138 | 21163 | R_1141 | 52 | 62978 | 63029 | R_1892 | 20 | 109494 | 109513 |
| R_391 | 20 | 21158 | 21177 | R_1142 | 43 | 63043 | 63085 | R_1893 | 25 | 109519 | 109543 |
| R_392 | 24 | 21248 | 21271 | R_1143 | 31 | 63087 | 63117 | R_1894 | 60 | 109554 | 109613 |
| R_393 | 26 | 21324 | 21349 | R_1144 | 35 | 63119 | 63153 | R_1895 | 34 | 109631 | 109664 |
| R_394 | 35 | 21351 | 21385 | R_1145 | 31 | 63155 | 63185 | R_1896 | 26 | 109666 | 109691 |
| R_395 | 29 | 21441 | 21469 | R_1146 | 54 | 63193 | 63246 | R_1897 | 22 | 109693 | 109714 |
| R_396 | 53 | 21557 | 21609 | R_1147 | 23 | 63249 | 63271 | R_1898 | 23 | 109757 | 109779 |
| R_397 | 31 | 21611 | 21641 | R_1148 | 29 | 63362 | 63390 | R_1899 | 34 | 109822 | 109855 |
| R_398 | 38 | 21645 | 21682 | R_1149 | 33 | 63404 | 63436 | R_1900 | 23 | 109866 | 109888 |
| R_399 | 40 | 21743 | 21782 | R_1150 | 33 | 63462 | 63494 | R_1901 | 140 | 109935 | 110074 |
| R_400 | 59 | 21819 | 21877 | R_1151 | 27 | 63501 | 63527 | R_1902 | 20 | 110077 | 110096 |
| R_401 | 20 | 21949 | 21968 | R_1152 | 29 | 63569 | 63597 | R_1903 | 29 | 110137 | 110165 |
| R_402 | 27 | 22001 | 22027 | R_1153 | 36 | 63599 | 63634 | R_1904 | 29 | 110216 | 110244 |
| R_403 | 63 | 22041 | 22103 | R_1154 | 20 | 63634 | 63653 | R_1905 | 32 | 110254 | 110285 |
| R_404 | 53 | 22125 | 22177 | R_1155 | 46 | 63769 | 63814 | R_1906 | 33 | 110294 | 110326 |
| R_405 | 48 | 22179 | 22226 | R_1156 | 20 | 63826 | 63845 | R_1907 | 31 | 110328 | 110358 |
| R_406 | 20 | 22247 | 22266 | R_1157 | 24 | 63848 | 63871 | R_1908 | 44 | 110383 | 110426 |
| R_407 | 48 | 22277 | 22324 | R_1158 | 54 | 63873 | 63926 | R_1909 | 24 | 110421 | 110444 |
| R_408 | 31 | 22334 | 22364 | R_1159 | 48 | 63941 | 63988 | R_1910 | 20 | 110563 | 110582 |
| R_409 | 105 | 22370 | 22474 | R_1160 | 45 | 63990 | 64034 | R_1911 | 32 | 110584 | 110615 |
| R_410 | 37 | 22475 | 22511 | R_1161 | 20 | 64059 | 64078 | R_1912 | 28 | 110598 | 110625 |
| R_411 | 32 | 22644 | 22675 | R_1162 | 20 | 64322 | 64341 | R_1913 | 54 | 110612 | 110665 |
| R_412 | 34 | 22686 | 22719 | R_1163 | 20 | 64382 | 64401 | R_1914 | 29 | 110781 | 110809 |
| R_413 | 28 | 22763 | 22790 | R_1164 | 24 | 64487 | 64510 | R_1915 | 51 | 110823 | 110873 |
| R_414 | 34 | 22792 | 22825 | R_1165 | 34 | 64532 | 64565 | R_1916 | 22 | 110875 | 110896 |
| R_415 | 22 | 22844 | 22865 | R_1166 | 27 | 64550 | 64576 | R_1917 | 27 | 110899 | 110925 |
| R_416 | 23 | 22875 | 22897 | R_1167 | 24 | 65195 | 65218 | R_1918 | 25 | 110992 | 111016 |
| R_417 | 27 | 22959 | 22985 | R_1168 | 20 | 65195 | 65214 | R_1919 | 38 | 111036 | 111073 |
| R_418 | 22 | 22990 | 23011 | R_1169 | 28 | 65736 | 65763 | R_1920 | 26 | 111108 | 111133 |
| R_419 | 23 | 23019 | 23041 | R_1170 | 30 | 65810 | 65839 | R_1921 | 20 | 111141 | 111160 |
| R_420 | 49 | 23066 | 23114 | R_1171 | 26 | 65850 | 65875 | R_1922 | 21 | 111162 | 111182 |
| R_421 | 35 | 23131 | 23165 | R_1172 | 32 | 65877 | 65908 | R_1923 | 35 | 111184 | 111218 |
| R_422 | 22 | 23168 | 23189 | R_1173 | 29 | 65917 | 65945 | R_1924 | 22 | 111234 | 111255 |
| R_423 | 46 | 23191 | 23236 | R_1174 | 55 | 66048 | 66102 | R_1925 | 20 | 111298 | 111317 |
| R_424 | 45 | 23238 | 23282 | R_1175 | 41 | 66123 | 66163 | R_1926 | 26 | 111319 | 111344 |
| R_425 | 23 | 23318 | 23340 | R_1176 | 37 | 66165 | 66201 | R_1927 | 61 | 111403 | 111463 |
| R_426 | 21 | 23497 | 23517 | R_1177 | 66 | 66203 | 66268 | R_1928 | 57 | 111467 | 111523 |
| R_427 | 24 | 23518 | 23541 | R_1178 | 49 | 66291 | 66339 | R_1929 | 23 | 111525 | 111547 |
| R_428 | 22 | 23562 | 23583 | R_1179 | 34 | 66392 | 66425 | R_1930 | 24 | 111567 | 111590 |
| R_429 | 26 | 23585 | 23610 | R_1180 | 45 | 66469 | 66513 | R_1931 | 26 | 111592 | 111617 |
| R_430 | 46 | 23626 | 23671 | R_1181 | 23 | 66545 | 66567 | R_1932 | 24 | 111631 | 111654 |
| R_431 | 34 | 23637 | 23670 | R_1182 | 27 | 66591 | 66617 | R_1933 | 22 | 111666 | 111687 |
| R_432 | 21 | 23650 | 23670 | R_1183 | 24 | 66635 | 66658 | R_1934 | 21 | 111692 | 111712 |
| R_433 | 28 | 23718 | 23745 | R_1184 | 22 | 66660 | 66681 | R_1935 | 49 | 111732 | 111780 |
| R_434 | 87 | 23748 | 23834 | R_1185 | 49 | 66690 | 66738 | R_1936 | 31 | 111815 | 111845 |
| R_435 | 41 | 23836 | 23876 | R_1186 | 29 | 66755 | 66783 | R_1937 | 21 | 111908 | 111928 |
| R_436 | 30 | 23889 | 23918 | R_1187 | 36 | 66789 | 66824 | R_1938 | 39 | 111934 | 111972 |
| R_437 | 83 | 23975 | 24057 | R_1188 | 23 | 66792 | 66814 | R_1939 | 26 | 111974 | 111999 |
| R_438 | 99 | 24059 | 24157 | R_1189 | 23 | 66865 | 66887 | R_1940 | 58 | 112001 | 112058 |
| R_439 | 37 | 24219 | 24255 | R_1190 | 27 | 66889 | 66915 | R_1941 | 28 | 112064 | 112091 |
| R_440 | 33 | 24319 | 24351 | R_1191 | 48 | 66991 | 67038 | R_1942 | 24 | 112066 | 112089 |
| R_441 | 20 | 24342 | 24361 | R_1192 | 24 | 67116 | 67139 | R_1943 | 21 | 112122 | 112142 |
| R_442 | 71 | 24354 | 24424 | R_1193 | 24 | 67155 | 67178 | R_1944 | 24 | 112157 | 112180 |
| R_443 | 28 | 24447 | 24474 | R_1194 | 27 | 67185 | 67211 | R_1945 | 21 | 112221 | 112241 |
| R_444 | 21 | 24515 | 24535 | R_1195 | 35 | 67231 | 67265 | R_1946 | 26 | 112253 | 112278 |
| R_445 | 31 | 24536 | 24566 | R_1196 | 20 | 67316 | 67335 | R_1947 | 23 | 112428 | 112450 |
| R_446 | 20 | 24552 | 24571 | R_1197 | 23 | 67337 | 67359 | R_1948 | 26 | 112444 | 112469 |
| R_447 | 26 | 24592 | 24617 | R_1198 | 31 | 67361 | 67391 | R_1949 | 30 | 112501 | 112530 |
| R_448 | 26 | 24656 | 24681 | R_1199 | 37 | 67467 | 67503 | R_1950 | 20 | 112511 | 112530 |
| R_449 | 25 | 24716 | 24740 | R_1200 | 27 | 67498 | 67524 | R_1951 | 69 | 112757 | 112825 |
| R_450 | 20 | 24721 | 24740 | R_1201 | 23 | 67499 | 67521 | R_1952 | 20 | 112884 | 112903 |
| R_451 | 57 | 24817 | 24873 | R_1202 | 37 | 67517 | 67553 | R_1953 | 44 | 112905 | 112948 |
| R_452 | 41 | 24903 | 24943 | R_1203 | 26 | 67604 | 67629 | R_1954 | 28 | 112979 | 113006 |
| R_453 | 26 | 24958 | 24983 | R_1204 | 25 | 67624 | 67648 | R_1955 | 62 | 113062 | 113123 |
| R_454 | 20 | 24985 | 25004 | R_1205 | 26 | 67708 | 67733 | R_1956 | 36 | 113141 | 113176 |
| R_455 | 48 | 25014 | 25061 | R_1206 | 21 | 67806 | 67826 | R_1957 | 23 | 113172 | 113194 |
| R_456 | 55 | 25122 | 25176 | R_1207 | 27 | 67877 | 67903 | R_1958 | 26 | 113203 | 113228 |
| R_457 | 29 | 25178 | 25206 | R_1208 | 43 | 67905 | 67947 | R_1959 | 37 | 113277 | 113313 |
| R_458 | 25 | 25249 | 25273 | R_1209 | 36 | 67987 | 68022 | R_1960 | 32 | 113364 | 113395 |
| R_459 | 30 | 25279 | 25308 | R_1210 | 50 | 68024 | 68073 | R_1961 | 43 | 113397 | 113439 |
| R_460 | 40 | 25310 | 25349 | R_1211 | 92 | 68092 | 68183 | R_1962 | 118 | 113452 | 113569 |
| R_461 | 53 | 25369 | 25421 | R_1212 | 24 | 68216 | 68239 | R_1963 | 46 | 113572 | 113617 |
| R_462 | 52 | 25427 | 25478 | R_1213 | 52 | 68257 | 68308 | R_1964 | 21 | 113628 | 113648 |
| R_463 | 66 | 25514 | 25579 | R_1214 | 32 | 68390 | 68421 | R_1965 | 21 | 113662 | 113682 |
| R_464 | 21 | 25618 | 25638 | R_1215 | 48 | 68442 | 68489 | R_1966 | 36 | 113690 | 113725 |
| R_465 | 51 | 25679 | 25729 | R_1216 | 20 | 68486 | 68505 | R_1967 | 32 | 113729 | 113760 |
| R_466 | 39 | 25731 | 25769 | R_1217 | 21 | 68546 | 68566 | R_1968 | 28 | 113782 | 113809 |
| R_467 | 28 | 25825 | 25852 | R_1218 | 25 | 68556 | 68580 | R_1969 | 21 | 113997 | 114017 |
| R_468 | 72 | 25881 | 25952 | R_1219 | 20 | 68561 | 68580 | R_1970 | 22 | 114007 | 114028 |
| R_469 | 23 | 25964 | 25986 | R_1220 | 23 | 68610 | 68632 | R_1971 | 57 | 114039 | 114095 |
| R_470 | 59 | 25988 | 26046 | R_1221 | 25 | 68679 | 68703 | R_1972 | 32 | 114174 | 114205 |
| R_471 | 25 | 26061 | 26085 | R_1222 | 35 | 68736 | 68770 | R_1973 | 28 | 114235 | 114262 |
| R_472 | 34 | 26088 | 26121 | R_1223 | 62 | 68806 | 68867 | R_1974 | 21 | 114349 | 114369 |
| R_473 | 24 | 26162 | 26185 | R_1224 | 22 | 68885 | 68906 | R_1975 | 38 | 114395 | 114432 |
| R_474 | 30 | 26194 | 26223 | R_1225 | 22 | 68908 | 68929 | R_1976 | 31 | 114434 | 114464 |
| R_475 | 28 | 26233 | 26260 | R_1226 | 20 | 68931 | 68950 | R_1977 | 20 | 114529 | 114548 |
| R_476 | 38 | 26335 | 26372 | R_1227 | 29 | 68950 | 68978 | R_1978 | 34 | 114624 | 114657 |
| R_477 | 24 | 26395 | 26418 | R_1228 | 34 | 69017 | 69050 | R_1979 | 65 | 114711 | 114775 |
| R_478 | 24 | 26455 | 26478 | R_1229 | 25 | 69053 | 69077 | R_1980 | 22 | 114904 | 114925 |
| R_479 | 27 | 26480 | 26506 | R_1230 | 20 | 69083 | 69102 | R_1981 | 42 | 114930 | 114971 |
| R_480 | 42 | 26521 | 26562 | R_1231 | 27 | 69123 | 69149 | R_1982 | 22 | 114982 | 115003 |
| R_481 | 67 | 26684 | 26750 | R_1232 | 30 | 69160 | 69189 | R_1983 | 20 | 115005 | 115024 |
| R_482 | 24 | 26752 | 26775 | R_1233 | 35 | 69210 | 69244 | R_1984 | 42 | 115026 | 115067 |
| R_483 | 35 | 26822 | 26856 | R_1234 | 53 | 69248 | 69300 | R_1985 | 28 | 115092 | 115119 |
| R_484 | 22 | 26937 | 26958 | R_1235 | 23 | 69304 | 69326 | R_1986 | 57 | 115121 | 115177 |
| R_485 | 38 | 26984 | 27021 | R_1236 | 34 | 69393 | 69426 | R_1987 | 28 | 115179 | 115206 |
| R_486 | 24 | 27022 | 27045 | R_1237 | 29 | 69428 | 69456 | R_1988 | 31 | 115228 | 115258 |
| R_487 | 54 | 27053 | 27106 | R_1238 | 45 | 69458 | 69502 | R_1989 | 24 | 115263 | 115286 |
| R_488 | 91 | 27154 | 27244 | R_1239 | 43 | 69547 | 69589 | R_1990 | 37 | 115306 | 115342 |
| R_489 | 35 | 27283 | 27317 | R_1240 | 20 | 69601 | 69620 | R_1991 | 44 | 115361 | 115404 |
| R_490 | 25 | 27339 | 27363 | R_1241 | 20 | 69633 | 69652 | R_1992 | 20 | 115467 | 115486 |
| R_491 | 75 | 27386 | 27460 | R_1242 | 29 | 69656 | 69684 | R_1993 | 30 | 115628 | 115657 |
| R_492 | 41 | 27493 | 27533 | R_1243 | 39 | 69705 | 69743 | R_1994 | 26 | 115665 | 115690 |
| R_493 | 22 | 27602 | 27623 | R_1244 | 42 | 69769 | 69810 | R_1995 | 34 | 115687 | 115720 |
| R_494 | 33 | 27631 | 27663 | R_1245 | 22 | 69829 | 69850 | R_1996 | 28 | 115804 | 115831 |
| R_495 | 23 | 27691 | 27713 | R_1246 | 28 | 69912 | 69939 | R_1997 | 26 | 115833 | 115858 |
| R_496 | 33 | 27736 | 27768 | R_1247 | 32 | 69941 | 69972 | R_1998 | 27 | 115937 | 115963 |
| R_497 | 24 | 27752 | 27775 | R_1248 | 31 | 70029 | 70059 | R_1999 | 119 | 115965 | 116083 |
| R_498 | 26 | 27777 | 27802 | R_1249 | 41 | 70065 | 70105 | R_2000 | 23 | 116085 | 116107 |
| R_499 | 20 | 27777 | 27796 | R_1250 | 27 | 70162 | 70188 | R_2001 | 42 | 116121 | 116162 |
| R_500 | 23 | 27778 | 27800 | R_1251 | 43 | 70200 | 70242 | R_2002 | 33 | 116193 | 116225 |
| R_501 | 30 | 27859 | 27888 | R_1252 | 20 | 70217 | 70236 | R_2003 | 24 | 116276 | 116299 |
| R_502 | 38 | 27909 | 27946 | R_1253 | 20 | 70345 | 70364 | R_2004 | 26 | 116356 | 116381 |
| R_503 | 49 | 27956 | 28004 | R_1254 | 35 | 70366 | 70400 | R_2005 | 29 | 116405 | 116433 |
| R_504 | 45 | 28071 | 28115 | R_1255 | 57 | 70433 | 70489 | R_2006 | 46 | 116441 | 116486 |
| R_505 | 33 | 28124 | 28156 | R_1256 | 21 | 70515 | 70535 | R_2007 | 29 | 116488 | 116516 |
| R_506 | 20 | 28152 | 28171 | R_1257 | 26 | 70537 | 70562 | R_2008 | 40 | 116518 | 116557 |
| R_507 | 24 | 28181 | 28204 | R_1258 | 40 | 70583 | 70622 | R_2009 | 46 | 116653 | 116698 |
| R_508 | 25 | 28251 | 28275 | R_1259 | 20 | 70657 | 70676 | R_2010 | 28 | 116700 | 116727 |
| R_509 | 33 | 28295 | 28327 | R_1260 | 22 | 70688 | 70709 | R_2011 | 46 | 116729 | 116774 |
| R_510 | 28 | 28345 | 28372 | R_1261 | 34 | 70723 | 70756 | R_2012 | 43 | 116927 | 116969 |
| R_511 | 51 | 28383 | 28433 | R_1262 | 23 | 70758 | 70780 | R_2013 | 32 | 116997 | 117028 |
| R_512 | 38 | 28441 | 28478 | R_1263 | 21 | 70782 | 70802 | R_2014 | 23 | 117043 | 117065 |
| R_513 | 24 | 28553 | 28576 | R_1264 | 21 | 70808 | 70828 | R_2015 | 35 | 117068 | 117102 |
| R_514 | 37 | 28598 | 28634 | R_1265 | 26 | 70818 | 70843 | R_2016 | 28 | 117148 | 117175 |
| R_515 | 35 | 28669 | 28703 | R_1266 | 31 | 70912 | 70942 | R_2017 | 36 | 117195 | 117230 |
| R_516 | 23 | 28733 | 28755 | R_1267 | 22 | 71039 | 71060 | R_2018 | 20 | 117243 | 117262 |
| R_517 | 31 | 28758 | 28788 | R_1268 | 25 | 71104 | 71128 | R_2019 | 37 | 117273 | 117309 |
| R_518 | 21 | 28857 | 28877 | R_1269 | 24 | 71195 | 71218 | R_2020 | 32 | 117329 | 117360 |
| R_519 | 38 | 28922 | 28959 | R_1270 | 43 | 71467 | 71509 | R_2021 | 59 | 117432 | 117490 |
| R_520 | 58 | 29019 | 29076 | R_1271 | 36 | 71519 | 71554 | R_2022 | 21 | 117509 | 117529 |
| R_521 | 22 | 29115 | 29136 | R_1272 | 24 | 71560 | 71583 | R_2023 | 23 | 117557 | 117579 |
| R_522 | 66 | 29198 | 29263 | R_1273 | 30 | 71606 | 71635 | R_2024 | 65 | 117580 | 117644 |
| R_523 | 24 | 29297 | 29320 | R_1274 | 21 | 71637 | 71657 | R_2025 | 27 | 117646 | 117672 |
| R_524 | 41 | 29335 | 29375 | R_1275 | 22 | 71672 | 71693 | R_2026 | 22 | 117708 | 117729 |
| R_525 | 21 | 29386 | 29406 | R_1276 | 56 | 71744 | 71799 | R_2027 | 47 | 117730 | 117776 |
| R_526 | 22 | 29433 | 29454 | R_1277 | 35 | 71827 | 71861 | R_2028 | 37 | 117778 | 117814 |
| R_527 | 40 | 29473 | 29512 | R_1278 | 21 | 71863 | 71883 | R_2029 | 24 | 117881 | 117904 |
| R_528 | 29 | 29531 | 29559 | R_1279 | 32 | 71913 | 71944 | R_2030 | 40 | 117904 | 117943 |
| R_529 | 41 | 29586 | 29626 | R_1280 | 25 | 71946 | 71970 | R_2031 | 30 | 117945 | 117974 |
| R_530 | 29 | 29635 | 29663 | R_1281 | 23 | 72022 | 72044 | R_2032 | 28 | 117993 | 118020 |
| R_531 | 36 | 29665 | 29700 | R_1282 | 28 | 72092 | 72119 | R_2033 | 48 | 118064 | 118111 |
| R_532 | 93 | 29750 | 29842 | R_1283 | 22 | 72095 | 72116 | R_2034 | 27 | 118113 | 118139 |
| R_533 | 35 | 29853 | 29887 | R_1284 | 21 | 72121 | 72141 | R_2035 | 27 | 118141 | 118167 |
| R_534 | 22 | 29907 | 29928 | R_1285 | 50 | 72147 | 72196 | R_2036 | 29 | 118169 | 118197 |
| R_535 | 77 | 29964 | 30040 | R_1286 | 31 | 72204 | 72234 | R_2037 | 33 | 118210 | 118242 |
| R_536 | 38 | 30093 | 30130 | R_1287 | 23 | 72230 | 72252 | R_2038 | 45 | 118386 | 118430 |
| R_537 | 30 | 30169 | 30198 | R_1288 | 36 | 72236 | 72271 | R_2039 | 48 | 118446 | 118493 |
| R_538 | 32 | 30210 | 30241 | R_1289 | 31 | 72285 | 72315 | R_2040 | 24 | 118532 | 118555 |
| R_539 | 20 | 30243 | 30262 | R_1290 | 85 | 72314 | 72398 | R_2041 | 46 | 118634 | 118679 |
| R_540 | 20 | 30303 | 30322 | R_1291 | 52 | 72400 | 72451 | R_2042 | 44 | 118774 | 118817 |
| R_541 | 23 | 30324 | 30346 | R_1292 | 37 | 72443 | 72479 | R_2043 | 54 | 118841 | 118894 |
| R_542 | 27 | 30362 | 30388 | R_1293 | 31 | 72482 | 72512 | R_2044 | 20 | 118912 | 118931 |
| R_543 | 30 | 30390 | 30419 | R_1294 | 40 | 72566 | 72605 | R_2045 | 21 | 118999 | 119019 |
| R_544 | 31 | 30462 | 30492 | R_1295 | 49 | 72607 | 72655 | R_2046 | 44 | 119283 | 119326 |
| R_545 | 22 | 30534 | 30555 | R_1296 | 86 | 72657 | 72742 | R_2047 | 33 | 119353 | 119385 |
| R_546 | 28 | 30557 | 30584 | R_1297 | 63 | 72752 | 72814 | R_2048 | 39 | 119392 | 119430 |
| R_547 | 24 | 30596 | 30619 | R_1298 | 125 | 72816 | 72940 | R_2049 | 65 | 119441 | 119505 |
| R_548 | 30 | 30626 | 30655 | R_1299 | 31 | 72955 | 72985 | R_2050 | 21 | 119566 | 119586 |
| R_549 | 41 | 30675 | 30715 | R_1300 | 20 | 72987 | 73006 | R_2051 | 55 | 119604 | 119658 |
| R_550 | 33 | 30726 | 30758 | R_1301 | 40 | 73008 | 73047 | R_2052 | 24 | 119660 | 119683 |
| R_551 | 29 | 30787 | 30815 | R_1302 | 24 | 73049 | 73072 | R_2053 | 42 | 119685 | 119726 |
| R_552 | 62 | 30819 | 30880 | R_1303 | 37 | 73118 | 73154 | R_2054 | 33 | 119736 | 119768 |
| R_553 | 79 | 30972 | 31050 | R_1304 | 26 | 73163 | 73188 | R_2055 | 32 | 119770 | 119801 |
| R_554 | 67 | 31053 | 31119 | R_1305 | 29 | 73212 | 73240 | R_2056 | 34 | 119804 | 119837 |
| R_555 | 56 | 31121 | 31176 | R_1306 | 22 | 73279 | 73300 | R_2057 | 116 | 119885 | 120000 |
| R_556 | 22 | 31178 | 31199 | R_1307 | 22 | 73315 | 73336 | R_2058 | 59 | 120128 | 120186 |
| R_557 | 22 | 31207 | 31228 | R_1308 | 30 | 73338 | 73367 | R_2059 | 34 | 120317 | 120350 |
| R_558 | 27 | 31227 | 31253 | R_1309 | 23 | 73387 | 73409 | R_2060 | 24 | 120530 | 120553 |
| R_559 | 27 | 31255 | 31281 | R_1310 | 52 | 73411 | 73462 | R_2061 | 22 | 120571 | 120592 |
| R_560 | 58 | 31310 | 31367 | R_1311 | 26 | 73498 | 73523 | R_2062 | 35 | 120611 | 120645 |
| R_561 | 26 | 31383 | 31408 | R_1312 | 24 | 73525 | 73548 | R_2063 | 98 | 120663 | 120760 |
| R_562 | 20 | 31419 | 31438 | R_1313 | 83 | 73562 | 73644 | R_2064 | 20 | 120924 | 120943 |
| R_563 | 36 | 31440 | 31475 | R_1314 | 36 | 73646 | 73681 | R_2065 | 22 | 121093 | 121114 |
| R_564 | 26 | 31503 | 31528 | R_1315 | 20 | 73703 | 73722 | R_2066 | 29 | 121117 | 121145 |
| R_565 | 34 | 31530 | 31563 | R_1316 | 27 | 73725 | 73751 | R_2067 | 39 | 121244 | 121282 |
| R_566 | 23 | 31585 | 31607 | R_1317 | 62 | 73776 | 73837 | R_2068 | 48 | 121365 | 121412 |
| R_567 | 21 | 31611 | 31631 | R_1318 | 20 | 73845 | 73864 | R_2069 | 37 | 121414 | 121450 |
| R_568 | 21 | 31614 | 31634 | R_1319 | 61 | 73894 | 73954 | R_2070 | 25 | 121649 | 121673 |
| R_569 | 32 | 31675 | 31706 | R_1320 | 91 | 73955 | 74045 | R_2071 | 40 | 121687 | 121726 |
| R_570 | 23 | 31708 | 31730 | R_1321 | 32 | 74079 | 74110 | R_2072 | 45 | 121728 | 121772 |
| R_571 | 39 | 31737 | 31775 | R_1322 | 28 | 74115 | 74142 | R_2073 | 22 | 121795 | 121816 |
| R_572 | 68 | 31763 | 31830 | R_1323 | 62 | 74144 | 74205 | R_2074 | 24 | 121939 | 121962 |
| R_573 | 27 | 31763 | 31789 | R_1324 | 27 | 74214 | 74240 | R_2075 | 28 | 122038 | 122065 |
| R_574 | 20 | 31803 | 31822 | R_1325 | 62 | 74244 | 74305 | R_2076 | 30 | 122218 | 122247 |
| R_575 | 23 | 31832 | 31854 | R_1326 | 28 | 74320 | 74347 | R_2077 | 27 | 122273 | 122299 |
| R_576 | 50 | 31952 | 32001 | R_1327 | 24 | 74350 | 74373 | R_2078 | 21 | 122301 | 122321 |
| R_577 | 22 | 32110 | 32131 | R_1328 | 46 | 74386 | 74431 | R_2079 | 30 | 122318 | 122347 |
| R_578 | 20 | 32114 | 32133 | R_1329 | 23 | 74433 | 74455 | R_2080 | 32 | 122356 | 122387 |
| R_579 | 35 | 32143 | 32177 | R_1330 | 31 | 74463 | 74493 | R_2081 | 21 | 122428 | 122448 |
| R_580 | 45 | 32179 | 32223 | R_1331 | 48 | 74497 | 74544 | R_2082 | 21 | 122432 | 122452 |
| R_581 | 26 | 32208 | 32233 | R_1332 | 40 | 74546 | 74585 | R_2083 | 24 | 123020 | 123043 |
| R_582 | 49 | 32225 | 32273 | R_1333 | 20 | 74604 | 74623 | R_2084 | 30 | 123038 | 123067 |
| R_583 | 27 | 32289 | 32315 | R_1334 | 65 | 74648 | 74712 | R_2085 | 26 | 123052 | 123077 |
| R_584 | 34 | 32317 | 32350 | R_1335 | 29 | 74725 | 74753 | R_2086 | 22 | 123258 | 123279 |
| R_585 | 32 | 32352 | 32383 | R_1336 | 35 | 74764 | 74798 | R_2087 | 28 | 123291 | 123318 |
| R_586 | 25 | 32390 | 32414 | R_1337 | 57 | 74805 | 74861 | R_2088 | 22 | 123402 | 123423 |
| R_587 | 46 | 32416 | 32461 | R_1338 | 56 | 74863 | 74918 | R_2089 | 27 | 123644 | 123670 |
| R_588 | 37 | 32497 | 32533 | R_1339 | 37 | 74936 | 74972 | R_2090 | 20 | 123819 | 123838 |
| R_589 | 37 | 32691 | 32727 | R_1340 | 28 | 74974 | 75001 | R_2091 | 26 | 123841 | 123866 |
| R_590 | 23 | 32753 | 32775 | R_1341 | 53 | 75003 | 75055 | R_2092 | 25 | 123965 | 123989 |
| R_591 | 38 | 32794 | 32831 | R_1342 | 22 | 75019 | 75040 | R_2093 | 24 | 123997 | 124020 |
| R_592 | 24 | 32835 | 32858 | R_1343 | 30 | 75097 | 75126 | R_2094 | 35 | 124034 | 124068 |
| R_593 | 55 | 32890 | 32944 | R_1344 | 51 | 75126 | 75176 | R_2095 | 44 | 124075 | 124118 |
| R_594 | 52 | 32959 | 33010 | R_1345 | 28 | 75362 | 75389 | R_2096 | 50 | 124156 | 124205 |
| R_595 | 37 | 33025 | 33061 | R_1346 | 29 | 75417 | 75445 | R_2097 | 75 | 124247 | 124321 |
| R_596 | 23 | 33063 | 33085 | R_1347 | 54 | 75482 | 75535 | R_2098 | 23 | 124353 | 124375 |
| R_597 | 62 | 33087 | 33148 | R_1348 | 27 | 75552 | 75578 | R_2099 | 34 | 124377 | 124410 |
| R_598 | 23 | 33160 | 33182 | R_1349 | 27 | 75580 | 75606 | R_2100 | 84 | 124472 | 124555 |
| R_599 | 21 | 33190 | 33210 | R_1350 | 26 | 75593 | 75618 | R_2101 | 20 | 124557 | 124576 |
| R_600 | 24 | 33222 | 33245 | R_1351 | 41 | 75815 | 75855 | R_2102 | 32 | 124648 | 124679 |
| R_601 | 56 | 33258 | 33313 | R_1352 | 30 | 75919 | 75948 | R_2103 | 22 | 124688 | 124709 |
| R_602 | 26 | 33317 | 33342 | R_1353 | 20 | 75944 | 75963 | R_2104 | 20 | 124700 | 124719 |
| R_603 | 25 | 33344 | 33368 | R_1354 | 37 | 75964 | 76000 | R_2105 | 35 | 124712 | 124746 |
| R_604 | 20 | 33379 | 33398 | R_1355 | 20 | 76123 | 76142 | R_2106 | 70 | 124748 | 124817 |
| R_605 | 22 | 33395 | 33416 | R_1356 | 30 | 76156 | 76185 | R_2107 | 21 | 124824 | 124844 |
| R_606 | 20 | 33395 | 33414 | R_1357 | 80 | 76199 | 76278 | R_2108 | 23 | 124859 | 124881 |
| R_607 | 22 | 33400 | 33421 | R_1358 | 23 | 76296 | 76318 | R_2109 | 35 | 124883 | 124917 |
| R_608 | 22 | 33457 | 33478 | R_1359 | 21 | 76327 | 76347 | R_2110 | 20 | 124919 | 124938 |
| R_609 | 22 | 33512 | 33533 | R_1360 | 24 | 76341 | 76364 | R_2111 | 57 | 124940 | 124996 |
| R_610 | 23 | 33532 | 33554 | R_1361 | 61 | 76366 | 76426 | R_2112 | 38 | 125015 | 125052 |
| R_611 | 24 | 33532 | 33555 | R_1362 | 26 | 76467 | 76492 | R_2113 | 21 | 125032 | 125052 |
| R_612 | 28 | 33535 | 33562 | R_1363 | 35 | 76520 | 76554 | R_2114 | 29 | 125064 | 125092 |
| R_613 | 21 | 33547 | 33567 | R_1364 | 58 | 76571 | 76628 | R_2115 | 37 | 125107 | 125143 |
| R_614 | 20 | 33548 | 33567 | R_1365 | 57 | 76697 | 76753 | R_2116 | 42 | 125198 | 125239 |
| R_615 | 23 | 33582 | 33604 | R_1366 | 22 | 76755 | 76776 | R_2117 | 50 | 125241 | 125290 |
| R_616 | 20 | 33588 | 33607 | R_1367 | 23 | 76822 | 76844 | R_2118 | 42 | 125292 | 125333 |
| R_617 | 24 | 33618 | 33641 | R_1368 | 42 | 76863 | 76904 | R_2119 | 31 | 125346 | 125376 |
| R_618 | 26 | 33675 | 33700 | R_1369 | 26 | 76906 | 76931 | R_2120 | 22 | 125378 | 125399 |
| R_619 | 29 | 33726 | 33754 | R_1370 | 51 | 76944 | 76994 | R_2121 | 46 | 125401 | 125446 |
| R_620 | 47 | 33775 | 33821 | R_1371 | 69 | 77037 | 77105 | R_2122 | 33 | 125700 | 125732 |
| R_621 | 20 | 33835 | 33854 | R_1372 | 26 | 77153 | 77178 | R_2123 | 32 | 125734 | 125765 |
| R_622 | 49 | 33856 | 33904 | R_1373 | 85 | 77180 | 77264 | R_2124 | 48 | 125803 | 125850 |
| R_623 | 64 | 33948 | 34011 | R_1374 | 35 | 77271 | 77305 | R_2125 | 35 | 125912 | 125946 |
| R_624 | 20 | 34025 | 34044 | R_1375 | 41 | 77307 | 77347 | R_2126 | 45 | 125948 | 125992 |
| R_625 | 20 | 34072 | 34091 | R_1376 | 27 | 77433 | 77459 | R_2127 | 73 | 126012 | 126084 |
| R_626 | 31 | 34139 | 34169 | R_1377 | 24 | 77462 | 77485 | R_2128 | 60 | 126087 | 126146 |
| R_627 | 78 | 34179 | 34256 | R_1378 | 30 | 77508 | 77537 | R_2129 | 32 | 126341 | 126372 |
| R_628 | 49 | 34258 | 34306 | R_1379 | 36 | 77561 | 77596 | R_2130 | 22 | 126374 | 126395 |
| R_629 | 29 | 34379 | 34407 | R_1380 | 39 | 77615 | 77653 | R_2131 | 25 | 126388 | 126412 |
| R_630 | 21 | 34417 | 34437 | R_1381 | 50 | 77655 | 77704 | R_2132 | 20 | 126473 | 126492 |
| R_631 | 27 | 34449 | 34475 | R_1382 | 20 | 77719 | 77738 | R_2133 | 22 | 126484 | 126505 |
| R_632 | 24 | 34495 | 34518 | R_1383 | 26 | 77762 | 77787 | R_2134 | 24 | 126660 | 126683 |
| R_633 | 21 | 34516 | 34536 | R_1384 | 29 | 77807 | 77835 | R_2135 | 23 | 126691 | 126713 |
| R_634 | 21 | 34562 | 34582 | R_1385 | 23 | 77837 | 77859 | R_2136 | 34 | 126715 | 126748 |
| R_635 | 21 | 34572 | 34592 | R_1386 | 26 | 77861 | 77886 | R_2137 | 22 | 126822 | 126843 |
| R_636 | 22 | 34576 | 34597 | R_1387 | 22 | 77910 | 77931 | R_2138 | 20 | 126885 | 126904 |
| R_637 | 32 | 34612 | 34643 | R_1388 | 45 | 77933 | 77977 | R_2139 | 38 | 127054 | 127091 |
| R_638 | 24 | 34646 | 34669 | R_1389 | 36 | 78017 | 78052 | R_2140 | 40 | 127111 | 127150 |
| R_639 | 65 | 34681 | 34745 | R_1390 | 24 | 78074 | 78097 | R_2141 | 30 | 127201 | 127230 |
| R_640 | 139 | 34765 | 34903 | R_1391 | 47 | 78136 | 78182 | R_2142 | 21 | 127232 | 127252 |
| R_641 | 60 | 34943 | 35002 | R_1392 | 93 | 78184 | 78276 | R_2143 | 76 | 127258 | 127333 |
| R_642 | 52 | 35012 | 35063 | R_1393 | 24 | 78282 | 78305 | R_2144 | 59 | 127359 | 127417 |
| R_643 | 83 | 35065 | 35147 | R_1394 | 99 | 78319 | 78417 | R_2145 | 33 | 127419 | 127451 |
| R_644 | 21 | 35160 | 35180 | R_1395 | 42 | 78420 | 78461 | R_2146 | 52 | 127567 | 127618 |
| R_645 | 29 | 35188 | 35216 | R_1396 | 23 | 78478 | 78500 | R_2147 | 38 | 127620 | 127657 |
| R_646 | 21 | 35218 | 35238 | R_1397 | 21 | 78647 | 78667 | R_2148 | 49 | 127656 | 127704 |
| R_647 | 59 | 35269 | 35327 | R_1398 | 34 | 78736 | 78769 | R_2149 | 37 | 127706 | 127742 |
| R_648 | 26 | 35330 | 35355 | R_1399 | 20 | 78891 | 78910 | R_2150 | 60 | 127761 | 127820 |
| R_649 | 44 | 35372 | 35415 | R_1400 | 26 | 78926 | 78951 | R_2151 | 25 | 127953 | 127977 |
| R_650 | 20 | 35417 | 35436 | R_1401 | 21 | 78953 | 78973 | R_2152 | 30 | 128097 | 128126 |
| R_651 | 43 | 35442 | 35484 | R_1402 | 69 | 78997 | 79065 | R_2153 | 40 | 128187 | 128226 |
| R_652 | 22 | 35482 | 35503 | R_1403 | 21 | 79067 | 79087 | R_2154 | 58 | 128237 | 128294 |
| R_653 | 74 | 35505 | 35578 | R_1404 | 25 | 79091 | 79115 | R_2155 | 20 | 128323 | 128342 |
| R_654 | 20 | 35599 | 35618 | R_1405 | 21 | 79122 | 79142 | R_2156 | 32 | 128408 | 128439 |
| R_655 | 25 | 35620 | 35644 | R_1406 | 24 | 79160 | 79183 | R_2157 | 37 | 128425 | 128461 |
| R_656 | 39 | 35654 | 35692 | R_1407 | 31 | 79187 | 79217 | R_2158 | 22 | 128463 | 128484 |
| R_657 | 26 | 35697 | 35722 | R_1408 | 75 | 79219 | 79293 | R_2159 | 56 | 128500 | 128555 |
| R_658 | 30 | 35724 | 35753 | R_1409 | 27 | 79308 | 79334 | R_2160 | 21 | 128565 | 128585 |
| R_659 | 23 | 35756 | 35778 | R_1410 | 71 | 79366 | 79436 | R_2161 | 29 | 128586 | 128614 |
| R_660 | 22 | 35777 | 35798 | R_1411 | 34 | 79469 | 79502 | R_2162 | 53 | 128631 | 128683 |
| R_661 | 40 | 35838 | 35877 | R_1412 | 41 | 79534 | 79574 | R_2163 | 59 | 128685 | 128743 |
| R_662 | 24 | 35879 | 35902 | R_1413 | 28 | 79576 | 79603 | R_2164 | 99 | 128738 | 128836 |
| R_663 | 20 | 35887 | 35906 | R_1414 | 23 | 79605 | 79627 | R_2165 | 23 | 128850 | 128872 |
| R_664 | 21 | 35894 | 35914 | R_1415 | 24 | 79712 | 79735 | R_2166 | 20 | 128896 | 128915 |
| R_665 | 62 | 35928 | 35989 | R_1416 | 35 | 79738 | 79772 | R_2167 | 63 | 128922 | 128984 |
| R_666 | 27 | 36002 | 36028 | R_1417 | 37 | 79793 | 79829 | R_2168 | 25 | 129031 | 129055 |
| R_667 | 20 | 36025 | 36044 | R_1418 | 38 | 79847 | 79884 | R_2169 | 28 | 129071 | 129098 |
| R_668 | 21 | 36030 | 36050 | R_1419 | 48 | 79924 | 79971 | R_2170 | 69 | 129104 | 129172 |
| R_669 | 64 | 36099 | 36162 | R_1420 | 31 | 80108 | 80138 | R_2171 | 27 | 129196 | 129222 |
| R_670 | 30 | 36171 | 36200 | R_1421 | 34 | 80140 | 80173 | R_2172 | 38 | 129235 | 129272 |
| R_671 | 39 | 36202 | 36240 | R_1422 | 77 | 80211 | 80287 | R_2173 | 30 | 129330 | 129359 |
| R_672 | 56 | 36242 | 36297 | R_1423 | 55 | 80307 | 80361 | R_2174 | 33 | 129345 | 129377 |
| R_673 | 47 | 36307 | 36353 | R_1424 | 26 | 80366 | 80391 | R_2175 | 40 | 129401 | 129440 |
| R_674 | 34 | 36404 | 36437 | R_1425 | 38 | 80419 | 80456 | R_2176 | 24 | 129427 | 129450 |
| R_675 | 22 | 36439 | 36460 | R_1426 | 20 | 80472 | 80491 | R_2177 | 22 | 129443 | 129464 |
| R_676 | 20 | 36493 | 36512 | R_1427 | 21 | 80505 | 80525 | R_2178 | 34 | 129488 | 129521 |
| R_677 | 24 | 36514 | 36537 | R_1428 | 40 | 80527 | 80566 | R_2179 | 79 | 129540 | 129618 |
| R_678 | 20 | 36568 | 36587 | R_1429 | 37 | 80571 | 80607 | R_2180 | 69 | 129617 | 129685 |
| R_679 | 32 | 36589 | 36620 | R_1430 | 40 | 80618 | 80657 | R_2181 | 29 | 129705 | 129733 |
| R_680 | 25 | 36622 | 36646 | R_1431 | 29 | 80671 | 80699 | R_2182 | 65 | 129735 | 129799 |
| R_681 | 22 | 36654 | 36675 | R_1432 | 36 | 80732 | 80767 | R_2183 | 48 | 129801 | 129848 |
| R_682 | 26 | 36678 | 36703 | R_1433 | 39 | 80791 | 80829 | R_2184 | 37 | 129884 | 129920 |
| R_683 | 28 | 36728 | 36755 | R_1434 | 37 | 80830 | 80866 | R_2185 | 42 | 129918 | 129959 |
| R_684 | 41 | 36790 | 36830 | R_1435 | 53 | 80868 | 80920 | R_2186 | 38 | 129988 | 130025 |
| R_685 | 60 | 36862 | 36921 | R_1436 | 30 | 80996 | 81025 | R_2187 | 26 | 130084 | 130109 |
| R_686 | 37 | 36940 | 36976 | R_1437 | 25 | 81027 | 81051 | R_2188 | 24 | 130125 | 130148 |
| R_687 | 55 | 37002 | 37056 | R_1438 | 55 | 81053 | 81107 | R_2189 | 36 | 130150 | 130185 |
| R_688 | 44 | 37124 | 37167 | R_1439 | 68 | 81109 | 81176 | R_2190 | 21 | 130247 | 130267 |
| R_689 | 29 | 37169 | 37197 | R_1440 | 24 | 81225 | 81248 | R_2191 | 80 | 130269 | 130348 |
| R_690 | 25 | 37232 | 37256 | R_1441 | 68 | 81264 | 81331 | R_2192 | 30 | 130384 | 130413 |
| R_691 | 21 | 37258 | 37278 | R_1442 | 23 | 81344 | 81366 | R_2193 | 21 | 130424 | 130444 |
| R_692 | 75 | 37280 | 37354 | R_1443 | 64 | 81377 | 81440 | R_2194 | 37 | 130564 | 130600 |
| R_693 | 93 | 37399 | 37491 | R_1444 | 26 | 81481 | 81506 | R_2195 | 21 | 130663 | 130683 |
| R_694 | 22 | 37465 | 37486 | R_1445 | 31 | 81571 | 81601 | R_2196 | 43 | 130690 | 130732 |
| R_695 | 21 | 37491 | 37511 | R_1446 | 44 | 81608 | 81651 | R_2197 | 61 | 130735 | 130795 |
| R_696 | 20 | 37543 | 37562 | R_1447 | 47 | 81694 | 81740 | R_2198 | 109 | 130797 | 130905 |
| R_697 | 23 | 37582 | 37604 | R_1448 | 27 | 81757 | 81783 | R_2199 | 51 | 130941 | 130991 |
| R_698 | 31 | 37608 | 37638 | R_1449 | 36 | 81780 | 81815 | R_2200 | 23 | 131025 | 131047 |
| R_699 | 21 | 37660 | 37680 | R_1450 | 25 | 81817 | 81841 | R_2201 | 21 | 131064 | 131084 |
| R_700 | 21 | 37720 | 37740 | R_1451 | 46 | 81866 | 81911 | R_2202 | 35 | 131119 | 131153 |
| R_701 | 35 | 37778 | 37812 | R_1452 | 23 | 81916 | 81938 | R_2203 | 62 | 131155 | 131216 |
| R_702 | 72 | 37825 | 37896 | R_1453 | 27 | 81946 | 81972 | R_2204 | 39 | 131269 | 131307 |
| R_703 | 35 | 37926 | 37960 | R_1454 | 20 | 82028 | 82047 | R_2205 | 22 | 131309 | 131330 |
| R_704 | 42 | 37962 | 38003 | R_1455 | 55 | 82049 | 82103 | R_2206 | 32 | 131350 | 131381 |
| R_705 | 20 | 38119 | 38138 | R_1456 | 71 | 82122 | 82192 | R_2207 | 52 | 131432 | 131483 |
| R_706 | 28 | 38162 | 38189 | R_1457 | 32 | 82216 | 82247 | R_2208 | 43 | 131501 | 131543 |
| R_707 | 23 | 38215 | 38237 | R_1458 | 47 | 82278 | 82324 | R_2209 | 20 | 131565 | 131584 |
| R_708 | 22 | 38249 | 38270 | R_1459 | 25 | 82498 | 82522 | R_2210 | 90 | 131606 | 131695 |
| R_709 | 79 | 38284 | 38362 | R_1460 | 27 | 82549 | 82575 | R_2211 | 79 | 131697 | 131775 |
| R_710 | 30 | 38419 | 38448 | R_1461 | 48 | 82606 | 82653 | R_2212 | 69 | 131758 | 131826 |
| R_711 | 25 | 38476 | 38500 | R_1462 | 26 | 82655 | 82680 | R_2213 | 20 | 131877 | 131896 |
| R_712 | 21 | 38486 | 38506 | R_1463 | 27 | 82699 | 82725 | R_2214 | 21 | 131898 | 131918 |
| R_713 | 22 | 38520 | 38541 | R_1464 | 67 | 82735 | 82801 | R_2215 | 23 | 131951 | 131973 |
| R_714 | 47 | 38548 | 38594 | R_1465 | 56 | 82833 | 82888 | R_2216 | 37 | 131975 | 132011 |
| R_715 | 22 | 38603 | 38624 | R_1466 | 29 | 82898 | 82926 | R_2217 | 25 | 132017 | 132041 |
| R_716 | 27 | 38623 | 38649 | R_1467 | 26 | 82928 | 82953 | R_2218 | 29 | 132061 | 132089 |
| R_717 | 22 | 38709 | 38730 | R_1468 | 45 | 82990 | 83034 | R_2219 | 22 | 132091 | 132112 |
| R_718 | 21 | 38734 | 38754 | R_1469 | 73 | 83083 | 83155 | R_2220 | 32 | 132138 | 132169 |
| R_719 | 46 | 38777 | 38822 | R_1470 | 39 | 83180 | 83218 | R_2221 | 36 | 132182 | 132217 |
| R_720 | 33 | 38853 | 38885 | R_1471 | 70 | 83255 | 83324 | R_2222 | 26 | 132253 | 132278 |
| R_721 | 27 | 38897 | 38923 | R_1472 | 35 | 83346 | 83380 | R_2223 | 48 | 132280 | 132327 |
| R_722 | 23 | 38982 | 39004 | R_1473 | 23 | 83409 | 83431 | R_2224 | 33 | 132403 | 132435 |
| R_723 | 26 | 39007 | 39032 | R_1474 | 111 | 83433 | 83543 | R_2225 | 58 | 132437 | 132494 |
| R_724 | 23 | 39007 | 39029 | R_1475 | 39 | 83553 | 83591 | R_2226 | 33 | 132496 | 132528 |
| R_725 | 20 | 39016 | 39035 | R_1476 | 54 | 83628 | 83681 | R_2227 | 60 | 132541 | 132600 |
| R_726 | 21 | 39026 | 39046 | R_1477 | 36 | 83710 | 83745 | R_2228 | 22 | 132619 | 132640 |
| R_727 | 30 | 39048 | 39077 | R_1478 | 32 | 83776 | 83807 | R_2229 | 23 | 132656 | 132678 |
| R_728 | 31 | 39140 | 39170 | R_1479 | 23 | 83809 | 83831 | R_2230 | 21 | 132758 | 132778 |
| R_729 | 24 | 39161 | 39184 | R_1480 | 53 | 83854 | 83906 | R_2231 | 39 | 132780 | 132818 |
| R_730 | 36 | 39188 | 39223 | R_1481 | 20 | 83960 | 83979 | R_2232 | 47 | 132827 | 132873 |
| R_731 | 28 | 39235 | 39262 | R_1482 | 43 | 83995 | 84037 | R_2233 | 27 | 132893 | 132919 |
| R_732 | 39 | 39264 | 39302 | R_1483 | 73 | 84051 | 84123 | R_2234 | 65 | 132917 | 132981 |
| R_733 | 52 | 39328 | 39379 | R_1484 | 40 | 84142 | 84181 | R_2235 | 20 | 132983 | 133002 |
| R_734 | 59 | 39391 | 39449 | R_1485 | 52 | 84217 | 84268 | R_2236 | 67 | 133014 | 133080 |
| R_735 | 30 | 39463 | 39492 | R_1486 | 28 | 84270 | 84297 | R_2237 | 46 | 133082 | 133127 |
| R_736 | 20 | 39492 | 39511 | R_1487 | 20 | 84354 | 84373 | R_2238 | 39 | 133129 | 133167 |
| R_737 | 20 | 39519 | 39538 | R_1488 | 21 | 84440 | 84460 | R_2239 | 31 | 133169 | 133199 |
| R_738 | 37 | 39557 | 39593 | R_1489 | 31 | 84488 | 84518 | R_2240 | 34 | 133201 | 133234 |
| R_739 | 34 | 39595 | 39628 | R_1490 | 22 | 84653 | 84674 | R_2241 | 27 | 133251 | 133277 |
| R_740 | 34 | 39639 | 39672 | R_1491 | 29 | 84727 | 84755 | R_2242 | 20 | 133282 | 133301 |
| R_741 | 26 | 39682 | 39707 | R_1492 | 38 | 84851 | 84888 | R_2243 | 37 | 133343 | 133379 |
| R_742 | 20 | 39709 | 39728 | R_1493 | 21 | 84887 | 84907 | R_2244 | 30 | 133404 | 133433 |
| R_743 | 23 | 39746 | 39768 | R_1494 | 58 | 84932 | 84989 | R_2245 | 77 | 133435 | 133511 |
| R_744 | 23 | 39753 | 39775 | R_1495 | 35 | 84991 | 85025 | R_2246 | 48 | 133528 | 133575 |
| R_745 | 20 | 39777 | 39796 | R_1496 | 24 | 85109 | 85132 | R_2247 | 22 | 133676 | 133697 |
| R_746 | 20 | 39798 | 39817 | R_1497 | 60 | 85135 | 85194 | R_2248 | 54 | 133710 | 133763 |
| R_747 | 41 | 39833 | 39873 | R_1498 | 27 | 85206 | 85232 | R_2249 | 20 | 133765 | 133784 |
| R_748 | 20 | 39876 | 39895 | R_1499 | 26 | 85239 | 85264 | R_2250 | 29 | 133786 | 133814 |
| R_749 | 36 | 39907 | 39942 | R_1500 | 32 | 85327 | 85358 | R_2251 | 40 | 133816 | 133855 |
| R_750 | 47 | 39990 | 40036 | R_1501 | 24 | 85390 | 85413 | R_2252 | 42 | 133857 | 133898 |
| R_751 | 36 | 40074 | 40109 | R_1502 | 24 | 85520 | 85543 | R_2253 | 63 | 133900 | 133962 |
| | | | | | | | | R_2254 | 40 | 133964 | 134003 |

In some examples the target sequence is a sequence selected from a human MAPT mRNA intron, such as a Tau human mRNA intron 1 or 2 (see table 1 above).

The oligonucleotide described herein comprises a contiguous nucleotide sequence which is complementary to or hybridizes to the target nucleic acid, such as a target sequence described herein.

The target sequence to which the oligonucleotide is complementary or hybridizes to generally comprises a contiguous nucleobases sequence of at least 10 nucleotides. The contiguous nucleotide sequence is between 10 to 100 nucleotides, such as 12 to 60, such as 13 to 50, such as 14 to 30, such as 15 to 25, such as 16 to 20 contiguous nucleotides.

In one example the target sequence is SEQ ID NO: 3, corresponding to region A. In certain embodiments the target sequence is position 12060 - 12078 of SEQ ID NO: 1.

In another example the target sequence is SEQ ID NO: 4, corresponding to region B. In certain examples the target sequence is selected from position 39562-39593 of SEQ ID NO: 1 such as positon 39573-39592 of SEQ ID NO: 1.

In another example the target sequence is SEQ ID NO: 5, corresponding to region C. In certain examples the target sequence is selected from position 72837-72940 of SEQ ID NO: 1 such as positon 72861-72891 or position 72862-72890 of SEQ ID NO: 1.

### Target Cell

The term a "target cell" as used herein refers to a cell which is expressing the target nucleic acid. In some embodiments the target cell may be *in vitro.* In some embodiments the target cell is a mammalian cell such as a rodent cell, such as a mouse cell or a rat cell, or a primate cell such as a monkey cell or a human cell.

In preferred embodiments the target cell expresses Tau mRNA, such as the Tau pre-mRNA or Tau mature mRNA. The poly A tail of Tau mRNA is typically disregarded for antisense oligonucleotide targeting.

### Naturally occurring variant

The term "naturally occurring variant" refers to variants of MAPT gene or transcripts which originate from the same genetic loci as the target nucleic acid, but may differ for example, by virtue of degeneracy of the genetic code causing a multiplicity of codons encoding the same amino acid, or due to alternative splicing of pre-mRNA, or the presence of polymorphisms, such as single nucleotide polymorphisms (SNPs), and allelic variants. Based on the presence of the sufficient complementary sequence to the oligonucleotide, the oligonucleotide of the invention may therefore target the target nucleic acid and naturally occurring variants thereof.

In some examples, the naturally occurring variants have at least 95% such as at least 98% or at least 99% homology to a mammalian MAPT target nucleic acid, such as a target nucleic acid selected form the group consisting of SEQ ID NO 1 and 2. In some examples the naturally occurring variants have at least 99% homology to the human MAPT target nucleic acid of SEQ ID NO: 1.

### Modulation of expression

The term "modulation of expression" as used herein is to be understood as an overall term for an oligonucleotide's ability to alter the amount of Tau when compared to the amount of Tau before administration of the oligonucleotide. Alternatively, modulation of expression may be determined by reference to a control experiment. It is generally understood that the control is an individual or target cell treated with a saline composition or an individual or target cell treated with a non-targeting oligonucleotide (mock).

One type of modulation is the ability of an oligonucleotide to inhibit, down-regulate, reduce, suppress, remove, stop, block, prevent, lessen, lower, avoid or terminate expression of Tau, e.g. by degradation of mRNA or blockage of transcription. Another type of modulation is an oligonucleotide's ability to restore, increase or enhance expression of Tau, e.g. by repair of splice sites or prevention of splicing or removal or blockage of inhibitory mechanisms such as microRNA repression.

### High affinity modified nucleosides

A high affinity modified nucleoside is a modified nucleotide which, when incorporated into the oligonucleotide enhances the affinity of the oligonucleotide for its complementary target, for example as measured by the melting temperature (T^{m}). A high affinity modified nucleoside of the present invention preferably result in an increase in melting temperature between +0.5 to +12°C, more preferably between +1.5 to +10°C and most preferably between+3 to +8°C per modified nucleoside. Numerous high affinity modified nucleosides are known in the art and include for example, many 2' substituted nucleosides as well as locked nucleic acids (LNA) (see e.g. Freier & Altmann; Nucl. Acid Res., 1997, 25, 4429-4443 and Uhlmann; Curr. Opinion in Drug Development, 2000, 3(2), 293-213).

### Sugar modifications

The oligomer of the invention may comprise one or more nucleosides which have a modified sugar moiety, *i.e.* a modification of the sugar moiety when compared to the ribose sugar moiety found in DNA and RNA.

Numerous nucleosides with modification of the ribose sugar moiety have been made, primarily with the aim of improving certain properties of oligonucleotides, such as affinity and/or nuclease resistance.

Such modifications include those where the ribose ring structure is modified, e.g. by replacement with a hexose ring (HNA), or a bicyclic ring, which typically have a biradicle bridge between the C2 and C4 carbons on the ribose ring (LNA), or an unlinked ribose ring which typically lacks a bond between the C2 and C3 carbons (e.g. UNA). Other sugar modified nucleosides include, for example, bicyclohexose nucleic acids (WO2011/017521) or tricyclic nucleic acids (WO2013/154798). Modified nucleosides also include nucleosides where the sugar moiety is replaced with a non-sugar moiety, for example in the case of peptide nucleic acids (PNA), or morpholino nucleic acids.

Sugar modifications also include modifications made via altering the substituent groups on the ribose ring to groups other than hydrogen, or the 2'-OH group naturally found in DNA and RNA nucleosides. Substituents may, for example be introduced at the 2', 3', 4' or 5' positions.

### 2' sugar modified nucleosides

A 2' sugar modified nucleoside is a nucleoside which has a substituent other than H or -OH at the 2' position (2' substituted nucleoside) or comprises a 2' linked biradicle capable of forming a bridge between the 2' carbon and a second carbon in the ribose ring, such as LNA (2' - 4' biradicle bridged) nucleosides.

Indeed, much focus has been spent on developing 2' sugar substituted nucleosides, and numerous 2' substituted nucleosides have been found to have beneficial properties when incorporated into oligonucleotides. For example, the 2' modified sugar may provide enhanced binding affinity and/or increased nuclease resistance to the oligonucleotide. Examples of 2' substituted modified nucleosides are 2'-O-alkyl-RNA, 2'-O-methyl-RNA, 2'-alkoxy-RNA, 2'-O-methoxyethyl-RNA (MOE), 2'-amino-DNA, 2'-Fluoro-RNA, and 2'-F-ANA nucleoside. For further examples, please see e.g. Freier & Altmann; Nucl. Acid Res., 1997, 25, 4429-4443 and Uhlmann; Curr. Opinion in Drug Development, 2000, 3(2), 293-213, and Deleavey and Damha, Chemistry and Biology 2012, 19, 937. Below are illustrations of some 2' substituted modified nucleosides.

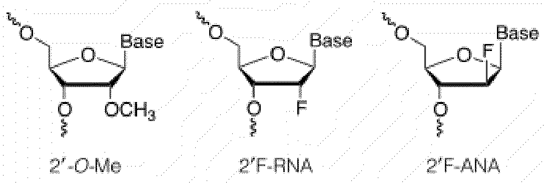

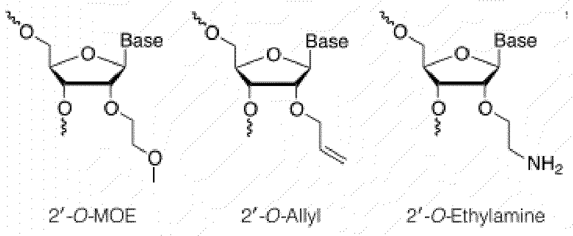

In relation to the present invention 2' substituted sugar modified nucleosides does not include 2' bridged nucleosides like LNA.

### Locked Nucleic Acid Nucleosides (LNA nucleoside)

A "LNA nucleoside" is a 2'-sugar modified nucleoside which comprises a biradical linking the C2' and C4' of the ribose sugar ring of said nucleoside (also referred to as a "2'- 4' bridge"), which restricts or locks the conformation of the ribose ring. These nucleosides are also termed bridged nucleic acid or bicyclic nucleic acid (BNA) in the literature. The locking of the conformation of the ribose is associated with an enhanced affinity of hybridization (duplex stabilization) when the LNA is incorporated into an oligonucleotide for a complementary RNA or DNA molecule. This can be routinely determined by measuring the melting temperature of the oligonucleotide/complement duplex.

Non limiting, exemplary LNA nucleosides are disclosed in WO 99/014226, WO 00/66604, WO 98/039352, WO 2004/046160, WO 00/047599, WO 2007/134181, WO 2010/077578, WO 2010/036698, WO 2007/090071, WO 2009/006478, WO 2011/156202, WO 2008/154401, WO 2009/067647, WO 2008/150729, Morita et al., Bioorganic & Med.Chem. Lett. 12, 73-76, Seth et al. J. Org. Chem. 2010, Vol 75(5) pp. 1569-81, Mitsuoka et al., Nucleic Acids Research 2009, 37(4), 1225-1238, and Wan and Seth, J. Medical Chemistry 2016, 59, 9645-9667.

The 2'-4' bridge comprises 2 to 4 bridging atoms and is in particular of formula -X-Y- wherein
X is oxygen, sulfur, -CR^{a}R^{b}-, -C(R^{a})=C(R^{b})-, -C(=CR^{a}R^{b})-, -C(R^{a})=N-, -Si(R^{a})₂-, -SO₂-, -NR^{a}-; -O-NR^{a}-, -NR^{a}-O-, -C(=J)-, Se, -O-NR^{a}-, -NR^{a}-CR^{a}R^{b}-, -N(R^{a})-O- or -O-CR^{a}R^{b}-; Y is oxygen, sulfur, -(CR^{a}R^{b})ₙ-, -CR^{a}R^{b}-O-CR^{a}R^{b}-, -C(R^{a})=C(R^{b})-, -C(R^{a})=N-, -Si(R^{a})₂-, -SO₂-, -NR^{a}-, -C(=J)-, Se, -O-NR^{a}-, -NR^{a}-CR^{a}R^{b}-, -N(R^{a})-O- or -O-CR^{a}R^{b}-;
with the proviso that -X-Y- is not -O-O-, Si(R^{a})₂-Si(R^{a})₂-, -SO₂-SO₂-, -C(R^{a})=C(R^{b})-C(R^{a})=C(R^{b}), -C(Ra)=N-C(Ra)=N-, -C(R^{a})=N-C(R^{a})=C(R^{b}) , -C(R^{a})=C(R^{b})-C(R^{a})=N- or -Se-Se-;
J is oxygen, sulfur, =CH₂ or =N(R^{a});
R^{a} and R^{b} are independently selected from hydrogen, halogen, hydroxyl, cyano, thiohydroxyl, alkyl, substituted alkyl, alkenyl, substituted alkenyl, alkynyl, substituted alkynyl, alkoxy, substituted alkoxy, alkoxyalkyl, alkenyloxy, carboxyl, alkoxycarbonyl, alkylcarbonyl, formyl, aryl, heterocyclyl, amino, alkylamino, carbamoyl, alkylaminocarbonyl, aminoalkylaminocarbonyl, alkylaminoalkylaminocarbonyl, alkylcarbonylamino, carbamido, alkanoyloxy, sulfonyl, alkylsulfonyloxy, nitro, azido, thiohydroxylsulfidealkylsulfanyl, aryloxycarbonyl, aryloxy, arylcarbonyl, heteroaryl, heteroaryloxycarbonyl, heteroaryloxy, heteroarylcarbonyl, -OC(=X^{a})R^{c}, -OC(=X^{a})NR^{c}R^{d} and -NR^{e}C(=X^{a})_{NR}c_{Rd};
or two geminal R^{a} and R^{b} together form optionally substituted methylene;
or two geminal R^{a} and R^{b}, together with the carbon atom to which they are attached, form cycloalkyl or halocycloalkyl, with only one carbon atom of -X-Y-;
wherein substituted alkyl, substituted alkenyl, substituted alkynyl, substituted alkoxy and substituted methylene are alkyl, alkenyl, alkynyl and methylene substituted with 1 to 3 substituents independently selected from halogen, hydroxyl, alkyl, alkenyl, alkynyl, alkoxy, alkoxyalkyl, alkenyloxy, carboxyl, alkoxycarbonyl, alkylcarbonyl, formyl, heterocylyl, aryl and heteroaryl;
X^{a} is oxygen, sulfur or -NR^{c};
R^{c}, R^{d} and R^{e} are independently selected from hydrogen and alkyl; and
n is 1, 2 or 3.

In a further particular embodiment of the invention, X is oxygen, sulfur, -NR^{a}-, -CR^{a}R^{b}- or - C(=CR^{a}R^{b})-, particularly oxygen, sulfur, -NH-, -CH₂- or -C(=CH₂)-, more particularly oxygen.

In another particular embodiment of the invention, Y is -CR^{a}R^{b}-, -CR^{a}R^{b}-CR^{a}R^{b}- or -CR^{a}R^{b}-CR^{a}R^{b}-CR^{a}R^{b}-, particularly -CH₂-CHCH₃-, -CHCH₃-CH₂-, -CH₂-CH₂- or -CH₂-CH₂-CH₂-.

In a particular embodiment of the invention, -X-Y- is -O-(CR^{a}R^{b})ₙ-, -S-CR^{a}R^{b}-, -N(R^{a})CR^{a}R^{b}-, - CR^{a}R^{b}-CR^{a}R^{b}-, -O-CR^{a}R^{b}-O-CR^{a}R^{b}-, -CR^{a}R^{b}-O-CR^{a}R^{b}-, -C(=CR^{a}R^{b})-CR^{a}R^{b}-, -N(R^{a})CR^{a}R^{b}-, -ON(R^{a})-CR^{a}R^{b}- or -N(R^{a})-O-CR^{a}R^{b}-.

In a particular embodiment of the invention, R^{a} and R^{b} are independently selected from the group consisting of hydrogen, halogen, hydroxyl, alkyl and alkoxyalkyl, in particular hydrogen, halogen, alkyl and alkoxyalkyl.

In another embodiment of the invention, R^{a} and R^{b} are independently selected from the group consisting of hydrogen, fluoro, hydroxyl, methyl and -CH₂-O-CH₃, in particular hydrogen, fluoro, methyl and -CH₂-O-CH₃.

Advantageously, one of R^{a} and R^{b} of -X-Y- is as defined above and the other ones are all hydrogen at the same time.

In a further particular embodiment of the invention, R^{a} is hydrogen or alkyl, in particular hydrogen or methyl.

In another particular embodiment of the invention, R^{b} is hydrogen or or alkyl, in particular hydrogen or methyl.

In a particular embodiment of the invention, one or both of R^{a} and R^{b} are hydrogen.

In a particular embodiment of the invention, only one of R^{a} and R^{b} is hydrogen.

In one particular embodiment of the invention, one of R^{a} and R^{b} is methyl and the other one is hydrogen.

In a particular embodiment of the invention, R^{a} and R^{b} are both methyl at the same time.

In a particular embodiment of the invention, -X-Y- is -O-CH₂-, -S-CH₂-, -S-CH(CH₃)-, -NH-CH₂-, - O-CH₂CH₂-, -O-CH(CH₂-O-CH₃)-, -O-CH(CH₂CH₃)-, -O-CH(CH₃)-, -O-CH₂₋O-CH₂-, -O-CH₂-O-CH₂-, -CH₂-O-CH₂-, -C(=CH₂)CH₂-, -C(=CH₂)CH(CH₃)-, -N(OCH₃)CH₂- or -N(CH₃)CH₂-;
In a particular embodiment of the invention, -X-Y- is -O-CR^{a}R^{b}-wherein R^{a} and R^{b} are independently selected from the group consisting of hydrogen, alkyl and alkoxyalkyl, in particular hydrogen, methyl and -CH₂-O-CH₃.

In a particular embodiment, -X-Y- is -O-CH₂- or -O-CH(CH₃)-, particularly -O-CH₂-.

The 2'- 4' bridge may be positioned either below the plane of the ribose ring (beta-D-configuration), or above the plane of the ring (alpha-L- configuration), as illustrated in formula (A) and formula (B) respectively.

The LNA nucleoside according to the invention is in particular of formula (A) or (B) 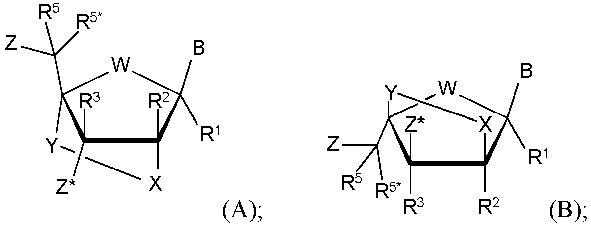 wherein
- W is: oxygen, sulfur, -N(R^{a})- or -CR^{a}R^{b}-, in particular oxygen;
- B is: a nucleobase or a modified nucleobase;
- Z is: an internucleoside linkage to an adjacent nucleoside or a 5'-terminal group;
- Z*: is an internucleoside linkage to an adjacent nucleoside or a 3'-terminal group;
- R¹, R², R³, R⁵ and R^{5*}: are independently selected from hydrogen, halogen, alkyl, haloalkyl, alkenyl, alkynyl, hydroxy, alkoxy, alkoxyalkyl, azido, alkenyloxy, carboxyl, alkoxycarbonyl, alkylcarbonyl, formyl and aryl; and
- X, Y, R^{a} and R^{b}: are as defined above.

In a particular embodiment, in the definition of -X-Y-, R^{a} is hydrogen or alkyl, in particular hydrogen or methyl. In another particular embodiment, in the definition of -X-Y-, R^{b} is hydrogen or alkyl, in particular hydrogen or methyl. In a further particular embodiment, in the definition of - X-Y-, one or both of R^{a} and R^{b} are hydrogen. In a particular embodiment, in the definition of -X-Y-, only one of R^{a} and R^{b} is hydrogen. In one particular embodiment, in the definition of -X-Y-, one of R^{a} and R^{b} is methyl and the other one is hydrogen. In a particular embodiment, in the definition of -X-Y-, R^{a} and R^{b} are both methyl at the same time.

In a further particular embodiment, in the definition of X, R^{a} is hydrogen or alkyl, in particular hydrogen or methyl. In another particular embodiment, in the definition of X, R^{b} is hydrogen or alkyl, in particular hydrogen or methyl. In a particular embodiment, in the definition of X, one or both of R^{a} and R^{b} are hydrogen. In a particular embodiment, in the definition of X, only one of R^{a} and R^{b} is hydrogen. In one particular embodiment, in the definition of X, one of R^{a} and R^{b} is methyl and the other one is hydrogen. In a particular embodiment, in the definition of X, R^{a} and R^{b} are both methyl at the same time.

In a further particular embodiment, in the definition of Y, R^{a} is hydrogen or alkyl, in particular hydrogen or methyl. In another particular embodiment, in the definition of Y, R^{b} is hydrogen or alkyl, in particular hydrogen or methyl. In a particular embodiment, in the definition of Y, one or both of R^{a} and R^{b} are hydrogen. In a particular embodiment, in the definition of Y, only one of R^{a} and R^{b} is hydrogen. In one particular embodiment, in the definition of Y, one of R^{a} and R^{b} is methyl and the other one is hydrogen. In a particular embodiment, in the definition of Y, R^{a} and R^{b} are both methyl at the same time.

In a particular embodiment of the invention R¹, R², R³, R⁵ and R^{5*} are independently selected from hydrogen and alkyl, in particular hydrogen and methyl.

In a further particular advantageous embodiment of the invention, R¹, R², R³, R⁵ and R^{5*} are all hydrogen at the same time.

In another particular embodiment of the invention, R¹, R², R³, are all hydrogen at the same time, one of R⁵ and R^{5*} is hydrogen and the other one is as defined above, in particular alkyl, more particularly methyl.

In a particular embodiment of the invention, R⁵ and R^{5*} are independently selected from hydrogen, halogen, alkyl, alkoxyalkyl and azido, in particular from hydrogen, fluoro, methyl, methoxyethyl and azido. In particular advantageous embodiments of the invention, one of R⁵ and R^{5*} is hydrogen and the other one is alkyl, in particular methyl, halogen, in particular fluoro, alkoxyalkyl, in particular methoxyethyl or azido; or R⁵ and R^{5*} are both hydrogen or halogen at the same time, in particular both hydrogen of fluoro at the same time. In such particular embodiments, W can advantageously be oxygen, and -X-Y- advantageously -O-CH₂-.

In a particular embodiment of the invention, -X-Y- is -O-CH₂-, W is oxygen and R¹, R², R³, R⁵ and R^{5*} are all hydrogen at the same time. Such LNA nucleosides are disclosed in WO 99/014226, WO 00/66604, WO 98/039352 and WO 2004/046160, and include what are commonly known in the art as beta-D-oxy LNA and alpha-L-oxy LNA nucleosides.

In another particular embodiment of the invention, -X-Y- is -S-CH₂-, W is oxygen and R¹, R², R³, R⁵ and R^{5*} are all hydrogen at the same time. Such thio LNA nucleosides are disclosed in WO 99/014226 and WO 2004/046160.

In another particular embodiment of the invention, -X-Y- is -NH-CH₂-, W is oxygen and R¹, R², R³, R⁵ and R^{5*} are all hydrogen at the same time. Such amino LNA nucleosides are disclosed in WO 99/014226 and WO 2004/046160.

In another particular embodiment of the invention, -X-Y- is -O-CH₂CH₂- or -OCH₂CH₂CH₂-, W is oxygen, and R¹, R², R³, R⁵ and R^{5*} are all hydrogen at the same time. Such LNA nucleosides are disclosed in WO 00/047599 and Morita et al., Bioorganic & Med.Chem. Lett. 12, 73-76, and include what are commonly known in the art as 2'-O-4'C-ethylene bridged nucleic acids (ENA).

In another particular embodiment of the invention, -X-Y- is -O-CH₂-, W is oxygen, R¹, R², R³ are all hydrogen at the same time, one of R⁵ and R^{5*} is hydrogen and the other one is not hydrogen, such as alkyl, for example methyl. Such 5' substituted LNA nucleosides are disclosed in WO 2007/134181.

In another particular embodiment of the invention, -X-Y- is -O-CR^{a}R^{b}-, wherein one or both of R^{a} and R^{b} are not hydrogen, in particular alkyl such as methyl, W is oxygen, R¹, R², R³ are all hydrogen at the same time, one of R⁵ and R^{5*} is hydrogen and the other one is not hydrogen, in particular alkyl, for example methyl. Such bis modified LNA nucleosides are disclosed in WO 2010/077578.

In another particular embodiment of the invention, -X-Y- is -O-CHR^{a}-, W is oxygen and R¹, R², R³, R⁵ and R^{5*} are all hydrogen at the same time. Such 6'-substituted LNA nucleosides are disclosed in WO 2010/036698 and WO 2007/090071. In such 6'-substituted LNA nucleosides, R^{a} is in particular C₁-C₆ alkyl, such as methyl.

In another particular embodiment of the invention, -X-Y- is -0-CH(CH₂-0-CH₃)- ("2' O-methoxyethyl bicyclic nucleic acid", Seth et al. J. Org. Chem. 2010, Vol 75(5) pp. 1569-81).

In another particular embodiment of the invention, -X-Y- is -O-CH(CH₂CH₃)- ("2'O-ethyl bicyclic nucleic acid", Seth at al., J. Org. Chem. 2010, Vol 75(5) pp. 1569-81).

In another particular embodiment of the invention, -X-Y- is -O-CH(CH₂-O-CH₃)-, W is oxygen and R¹, R², R³, R⁵ and R^{5*} are all hydrogen at the same time. Such LNA nucleosides are also known in the art as cyclic MOEs (cMOE) and are disclosed in WO 2007/090071.

In another particular embodiment of the invention, -X-Y- is -O-CH(CH₃)-.

In another particular embodiment of the invention, -X-Y- is -O-CH₂₋O-CH₂- (Seth et al., J. Org. Chem 2010 op. cit.)
In another particular embodiment of the invention, -X-Y- is -O-CH(CH₃)-, W is oxygen and R¹, R², R³, R⁵ and R^{5*} are all hydrogen at the same time. Such 6'-methyl LNA nucleosides are also known in the art as cET nucleosides, and may be either (S)-cET or (R)-cET diastereoisomers, as disclosed in WO 2007/090071 (beta-D) and WO 2010/036698 (alpha-L).

In another particular embodiment of the invention, -X-Y- is -O-CR^{a}R^{b}-, wherein neither R^{a} nor R^{b} is hydrogen, W is oxygen and R¹, R², R³, R⁵ and R^{5*} are all hydrogen at the same time. In a particular embodiment, R^{a} and R^{b} are both alkyl at the same time, in particular both methyl at the same time. Such 6'-di-substituted LNA nucleosides are disclosed in WO 2009/006478.

In another particular embodiment of the invention, -X-Y- is -S-CHR^{a}-, W is oxygen and R¹, R², R³, R⁵ and R^{5*} are all hydrogen at the same time. Such 6'-substituted thio LNA nucleosides are disclosed in WO 2011/156202. In a particular embodiment of such 6'-substituted thio LNA, R^{a} is alkyl, in particular methyl.

In a particular embodiment of the invention, -X-Y- is -C(=CH₂)C(R^{a}R^{b})-, -C(=CHF)C(R^{a}R^{b})- or - C(=CF₂)C(R^{a}R^{b})-, W is oxygen and R¹, R², R³, R⁵ and R^{5*} are all hydrogen at the same time. R^{a} and R^{b} are advantagesously independently selected from hydrogen, halogen, alkyl and alkoxyalkyl, in particular hydrogen, methyl, fluoro and methoxymethyl. R^{a} and R^{b} are in particular both hydrogen or methyl at the same time or one of R^{a} and R^{b} is hydrogen and the other one is methyl. Such vinyl carbo LNA nucleosides are disclosed in WO 2008/154401 and WO 2009/067647.

In a particular embodiment of the invention, -X-Y- is -N(OR^{a})-CH₂-, W is oxygen and R¹, R², R³, R⁵ and R^{5*} are all hydrogen at the same time. In a particular embodiment, R^{a} is alkyl such as methyl. Such LNA nucleosides are also known as N substituted LNAs and are disclosed in WO 2008/150729.

In a particular embodiment of the invention, -X-Y- is -O-N(R^{a})-, -N(R^{a})-O-, -NR^{a}-CR^{a}R^{b}-CR^{a}R^{b}- or -NR^{a}-CR^{a}R^{b}-, W is oxygen and R¹, R², R³, R⁵ and R^{5*} are all hydrogen at the same time. R^{a} and R^{b} are advantagesously independently selected from hydrogen, halogen, alkyl and alkoxyalkyl, in particular hydrogen, methyl, fluoro and methoxymethyl. In a particular embodiment, R^{a} is alkyl, such as methyl, R^{b} is hydrogen or methyl, in particular hydrogen. (Seth et al., J. Org. Chem 2010 op. cit.).

In a particular embodiment of the invention, -X-Y- is -O-N(CH₃)- (Seth et al., J. Org. Chem 2010 op. cit.).

In a particular embodiment of the invention, R⁵ and R^{5*} are both hydrogen at the same time. In another particular embodiment of the invention, one of R⁵ and R^{5*} is hydrogen and the other one is alkyl, such as methyl. In such embodiments, R¹, R² and R³ can be in particular hydrogen and -X-Y- can be in particular -O-CH₂- or -O-CHC(R^{a})₃-, such as -O-CH(CH₃)-.

In a particular embodiment of the invention, -X-Y- is -CR^{a}R^{b}-O-CR^{a}R^{b}-, such as -CH₂-O-CH₂-, W is oxygen and R¹, R², R³, R⁵ and R^{5*} are all hydrogen at the same time. In such particular embodiments, R^{a} can be in particular alkyl such as methyl, R^{b} hydrogen or methyl, in particular hydrogen. Such LNA nucleosides are also known as conformationally restricted nucleotides (CRNs) and are disclosed in WO 2013/036868.

In a particular embodiment of the invention, -X-Y- is -O-CR^{a}R^{b}-O-CR^{a}R^{b}-, such as -O-CH₂-O-CH₂-, W is oxygen and R¹, R², R³, R⁵ and R^{5*} are all hydrogen at the same time. R^{a} and R^{b} are advantageously independently selected from hydrogen, halogen, alkyl and alkoxyalkyl, in particular hydrogen, methyl, fluoro and methoxymethyl. In such a particular embodiment, R^{a} can be in particular alkyl such as methyl, R^{b} hydrogen or methyl, in particular hydrogen. Such LNA nucleosides are also known as COC nucleotides and are disclosed in Mitsuoka et al., Nucleic Acids Research 2009, 37(4), 1225-1238.

It will be recognized than, unless specified, the LNA nucleosides may be in the beta-D or alpha-L stereoisoform.

Particular examples of LNA nucleosides of the invention are presented in Scheme 1 (wherein B is as defined above).

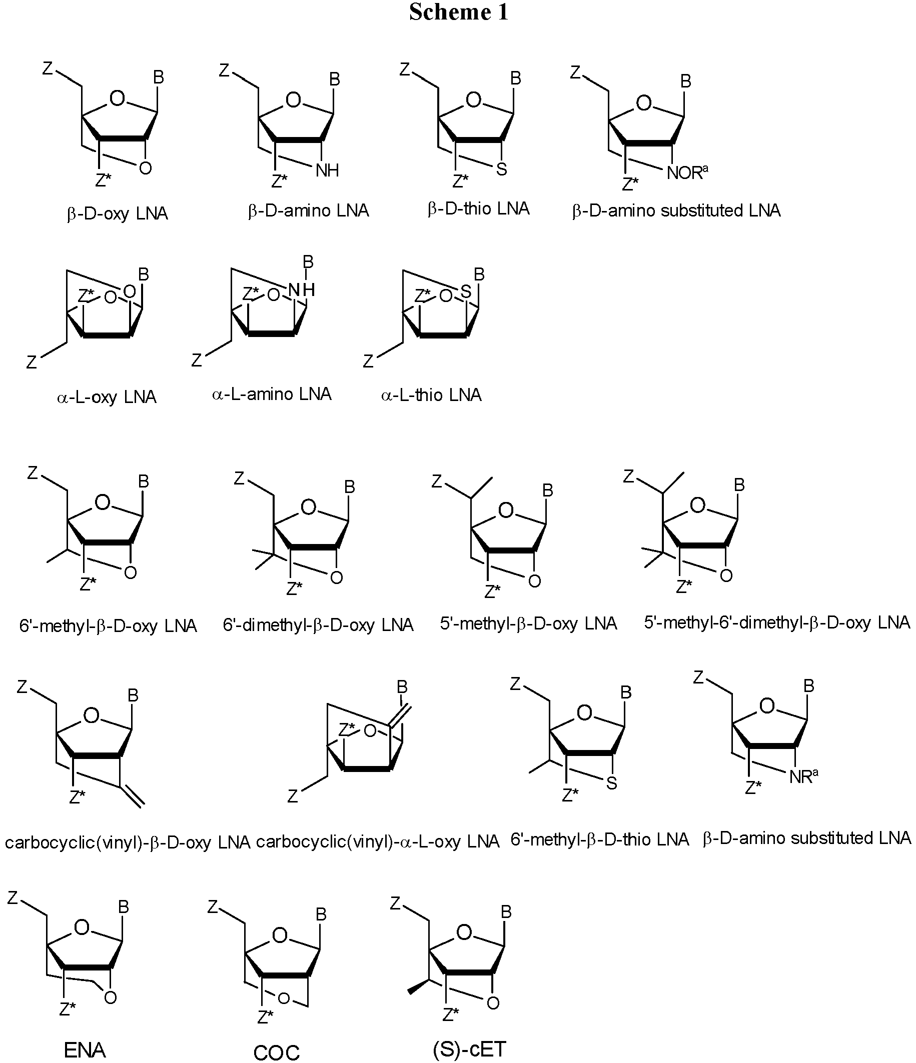

Particular LNA nucleosides are beta-D-oxy-LNA, 6'-methyl-beta-D-oxy LNA such as (S)-6'-methyl-beta-D-oxy-LNA (ScET) and ENA.

If one of the starting materials or compounds of the invention contain one or more functional groups which are not stable or are reactive under the reaction conditions of one or more reaction steps, appropriate protecting groups (as described e.g. in "Protective Groups in Organic Chemistry" by T. W. Greene and P. G. M. Wuts, 3rd Ed., 1999, Wiley, New York) can be introduced before the critical step applying methods well known in the art. Such protecting groups can be removed at a later stage of the synthesis using standard methods described in the literature. Examples of protecting groups are tert-butoxycarbonyl (Boc), 9-fluorenylmethyl carbamate (Fmoc), 2-trimethylsilylethyl carbamate (Teoc), carbobenzyloxy (Cbz) and p-methoxybenzyloxycarbonyl (Moz).

The compounds described herein can contain several asymmetric centers and can be present in the form of optically pure enantiomers, mixtures of enantiomers such as, for example, racemates, mixtures of diastereoisomers, diastereoisomeric racemates or mixtures of diastereoisomeric racemates.

The term "asymmetric carbon atom" means a carbon atom with four different substituents. According to the Cahn-Ingold-Prelog Convention an asymmetric carbon atom can be of the "R" or "S" configuration.

### Chemical group definitions

In the present description the term "alkyl", alone or in combination, signifies a straight-chain or branched-chain alkyl group with 1 to 8 carbon atoms, particularly a straight or branched-chain alkyl group with 1 to 6 carbon atoms and more particularly a straight or branched-chain alkyl group with 1 to 4 carbon atoms. Examples of straight-chain and branched-chain C₁-C₈ alkyl groups are methyl, ethyl, propyl, isopropyl, butyl, isobutyl, tert.-butyl, the isomeric pentyls, the isomeric hexyls, the isomeric heptyls and the isomeric octyls, particularly methyl, ethyl, propyl, butyl and pentyl. Particular examples of alkyl are methyl, ethyl and propyl.

The term "cycloalkyl", alone or in combination, signifies a cycloalkyl ring with 3 to 8 carbon atoms and particularly a cycloalkyl ring with 3 to 6 carbon atoms. Examples of cycloalkyl are cyclopropyl, cyclobutyl, cyclopentyl, cyclohexyl, cycloheptyl and cyclooctyl, more particularly cyclopropyl and cyclobutyl. A particular example of "cycloalkyl" is cyclopropyl.

The term "alkoxy", alone or in combination, signifies a group of the formula alkyl-O- in which the term "alkyl" has the previously given significance, such as methoxy, ethoxy, n-propoxy, isopropoxy, n-butoxy, isobutoxy, sec.butoxy and tert.butoxy. Particular "alkoxy" are methoxy and ethoxy. Methoxyethoxy is a particular example of "alkoxyalkoxy".

The term "oxy", alone or in combination, signifies the -O- group.

The term "alkenyl", alone or in combination, signifies a straight-chain or branched hydrocarbon residue comprising an olefinic bond and up to 8, preferably up to 6, particularly preferred up to 4 carbon atoms. Examples of alkenyl groups are ethenyl, 1-propenyl, 2-propenyl, isopropenyl, 1-butenyl, 2-butenyl, 3-butenyl and isobutenyl.

The term "alkynyl", alone or in combination, signifies a straight-chain or branched hydrocarbon residue comprising a triple bond and up to 8, preferably up to 6, particularly preferred up to 4 carbon atoms.

The terms "halogen" or "halo", alone or in combination, signifies fluorine, chlorine, bromine or iodine and particularly fluorine, chlorine or bromine, more particularly fluorine. The term "halo", in combination with another group, denotes the substitution of said group with at least one halogen, particularly substituted with one to five halogens, particularly one to four halogens, i.e. one, two, three or four halogens.

The term "haloalkyl", alone or in combination, denotes an alkyl group substituted with at least one halogen, particularly substituted with one to five halogens, particularly one to three halogens. Examples of haloalkyl include monofluoro-, difluoro- or trifluoro-methyl, -ethyl or - propyl, for example 3,3,3-trifluoropropyl, 2-fluoroethyl, 2,2,2-trifluoroethyl, fluoromethyl or trifluoromethyl. Fluoromethyl, difluoromethyl and trifluoromethyl are particular "haloalkyl".

The term "halocycloalkyl", alone or in combination, denotes a cycloalkyl group as defined above substituted with at least one halogen, particularly substituted with one to five halogens, particularly one to three halogens. Particular example of "halocycloalkyl" are halocyclopropyl, in particular fluorocyclopropyl, difluorocyclopropyl and trifluorocyclopropyl.

The terms "hydroxyl" and "hydroxy", alone or in combination, signify the -OH group.

The terms "thiohydroxyl" and "thiohydroxy", alone or in combination, signify the -SH group.

The term "carbonyl", alone or in combination, signifies the -C(O)- group.

The term "carboxy" or "carboxyl", alone or in combination, signifies the -COOH group.

The term "amino", alone or in combination, signifies the primary amino group (-NH₂), the secondary amino group (-NH-), or the tertiary amino group (-N-).

The term "alkylamino", alone or in combination, signifies an amino group as defined above substituted with one or two alkyl groups as defined above.

The term "sulfonyl", alone or in combination, means the -SO₂ group.

The term "sulfinyl", alone or in combination, signifies the -SO- group.

The term "sulfanyl", alone or in combination, signifies the -S- group.

The term "cyano", alone or in combination, signifies the -CN group.

The term "azido", alone or in combination, signifies the -N₃ group.

The term "nitro", alone or in combination, signifies the NO₂ group.

The term "formyl", alone or in combination, signifies the -C(O)H group.

The term "carbamoyl", alone or in combination, signifies the -C(O)NH₂ group.

The term "cabamido", alone or in combination, signifies the -NH-C(O)-NH₂ group.

The term "aryl", alone or in combination, denotes a monovalent aromatic carbocyclic mono- or bicyclic ring system comprising 6 to 10 carbon ring atoms, optionally substituted with 1 to 3 substituents independently selected from halogen, hydroxyl, alkyl, alkenyl, alkynyl, alkoxy, alkoxyalkyl, alkenyloxy, carboxyl, alkoxycarbonyl, alkylcarbonyl and formyl. Examples of aryl include phenyl and naphthyl, in particular phenyl.

The term "heteroaryl", alone or in combination, denotes a monovalent aromatic heterocyclic mono- or bicyclic ring system of 5 to 12 ring atoms, comprising 1, 2, 3 or 4 heteroatoms selected from N, O and S, the remaining ring atoms being carbon, optionally substituted with 1 to 3 substituents independently selected from halogen, hydroxyl, alkyl, alkenyl, alkynyl, alkoxy, alkoxyalkyl, alkenyloxy, carboxyl, alkoxycarbonyl, alkylcarbonyl and formyl. Examples of heteroaryl include pyrrolyl, furanyl, thienyl, imidazolyl, oxazolyl, thiazolyl, triazolyl, oxadiazolyl, thiadiazolyl, tetrazolyl, pyridinyl, pyrazinyl, pyrazolyl, pyridazinyl, pyrimidinyl, triazinyl, azepinyl, diazepinyl, isoxazolyl, benzofuranyl, isothiazolyl, benzothienyl, indolyl, isoindolyl, isobenzofuranyl, benzimidazolyl, benzoxazolyl, benzoisoxazolyl, benzothiazolyl, benzoisothiazolyl, benzooxadiazolyl, benzothiadiazolyl, benzotriazolyl, purinyl, quinolinyl, isoquinolinyl, quinazolinyl, quinoxalinyl, carbazolyl or acridinyl.

The term "heterocyclyl", alone or in combination, signifies a monovalent saturated or partly unsaturated mono- or bicyclic ring system of 4 to 12, in particular 4 to 9 ring atoms, comprising 1, 2,3 or 4 ring heteroatoms selected from N, O and S, the remaining ring atoms being carbon, optionally substituted with 1 to 3 substituents independently selected from halogen, hydroxyl, alkyl, alkenyl, alkynyl, alkoxy, alkoxyalkyl, alkenyloxy, carboxyl, alkoxycarbonyl, alkylcarbonyl and formyl. Examples for monocyclic saturated heterocyclyl are azetidinyl, pyrrolidinyl, tetrahydrofuranyl, tetrahydro-thienyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, or oxazepanyl. Examples for bicyclic saturated heterocycloalkyl are 8-aza-bicyclo[3.2.1]octyl, quinuclidinyl, 8-oxa-3-aza-bicyclo[3.2.1]octyl, 9-aza-bicyclo[3.3.1]nonyl, 3-oxa-9-aza-bicyclo[3.3.1]nonyl, or 3-thia-9-aza-bicyclo[3.3.1]nonyl. Examples for partly unsaturated heterocycloalkyl are dihydrofuryl, imidazolinyl, dihydro-oxazolyl, tetrahydro-pyridinyl or dihydropyranyl.

### Pharmaceutically acceptable salts

The term "pharmaceutically acceptable salts" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid, particularly hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein. In addition these salts may be prepared form addition of an inorganic base or an organic base to the free acid. Salts derived from an inorganic base include, but are not limited to, the sodium, potassium, lithium, ammonium, calcium, magnesium salts. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyamine resins. The compound of formula (I) can also be present in the form of zwitterions. Particularly preferred pharmaceutically acceptable salts of compounds of formula (I) are the salts of hydrochloric acid, hydrobromic acid, sulfuric acid, phosphoric acid and methanesulfonic acid.

### Protecting group

The term "protecting group", alone or in combination, signifies a group which selectively blocks a reactive site in a multifunctional compound such that a chemical reaction can be carried out selectively at another unprotected reactive site. Protecting groups can be removed. Exemplary protecting groups are amino-protecting groups, carboxy-protecting groups or hydroxy-protecting groups.

### Nuclease mediated degradation

Nuclease mediated degradation refers to an oligonucleotide capable of mediating degradation of a complementary nucleotide sequence when forming a duplex with such a sequence.

In some embodiments, the oligonucleotide may function via nuclease mediated degradation of the target nucleic acid, where the oligonucleotides of the invention are capable of recruiting a nuclease, particularly and endonuclease, preferably endoribonuclease (RNase), such as RNase H. Examples of oligonucleotide designs which operate via nuclease mediated mechanisms are oligonucleotides which typically comprise a region of at least 5 or 6 consecutive DNA nucleosides and are flanked on one side or both sides by affinity enhancing nucleosides, for example gapmers, headmers and tailmers.

### RNase H Activity and Recruitment

The RNase H activity of an antisense oligonucleotide refers to its ability to recruit RNase H when in a duplex with a complementary RNA molecule. WO01/23613 provides *in vitro* methods for determining RNaseH activity, which may be used to determine the ability to recruit RNaseH. Typically an oligonucleotide is deemed capable of recruiting RNase H if it, when provided with a complementary target nucleic acid sequence, has an initial rate, as measured in pmol/l/min, of at least 5%, such as at least 10% or more than 20% of the of the initial rate determined when using a oligonucleotide having the same base sequence as the modified oligonucleotide being tested, but containing only DNA monomers with phosphorothioate linkages between all monomers in the oligonucleotide, and using the methodology provided by Example 91 - 95 of WO01/23613. For use in determining RHase H activity, recombinant human RNase H1 is available from Lubio Science GmbH, Lucerne, Switzerland.

### Gapmer

The antisense oligonucleotide of the invention, or contiguous nucleotide sequence thereof, may be a gapmer, also termed gapmer oligonucleotide or gapmer designs. The antisense gapmers are commonly used to inhibit a target nucleic acid via RNase H mediated degradation. A gapmer oligonucleotide comprises at least three distinct structural regions a 5'-flank, a gap and a 3'-flank, F-G-F' in the '5 -> 3' orientation. The "gap" region (G) comprises a stretch of contiguous DNA nucleotides which enable the oligonucleotide to recruit RNase H. The gap region is flanked by a 5' flanking region (F) comprising one or more sugar modified nucleosides, advantageously high affinity sugar modified nucleosides, and by a 3' flanking region (F') comprising one or more sugar modified nucleosides, advantageously high affinity sugar modified nucleosides. The one or more sugar modified nucleosides in region F and F' enhance the affinity of the oligonucleotide for the target nucleic acid *(i.e.* are affinity enhancing sugar modified nucleosides). In some embodiments, the one or more sugar modified nucleosides in region F and F' are 2' sugar modified nucleosides, such as high affinity 2' sugar modifications, such as independently selected from LNA and 2'-MOE.

In a gapmer design, the 5' and 3' most nucleosides of the gap region are DNA nucleosides, and are positioned adjacent to a sugar modified nucleoside of the 5' (F) or 3' (F') region respectively. The flanks may further be defined by having at least one sugar modified nucleoside at the end most distant from the gap region, i.e. at the 5' end of the 5' flank and at the 3' end of the 3' flank.

Regions F-G-F' form a contiguous nucleotide sequence. Antisense oligonucleotides of the invention, or the contiguous nucleotide sequence thereof, may comprise a gapmer region of formula F-G-F'.

The overall length of the gapmer design F-G-F' may be, for example 12 to 32 nucleosides, such as 13 to 24, such as 14 to 22 nucleosides, Such as from 14 to17, such as 16 to18 nucleosides, such as 16 to 20 nuclotides.

By way of example, the gapmer oligonucleotide of the present invention can be represented by the following formulae:
F₁₋₈-G₆₋₁₆-F'₂₋₈, such as
F₂₋₈-G₆₋₁₄-F'₂₋₈, such as
F₃₋₈-G₆₋₁₄-F'₂₋₈
with the proviso that the overall length of the gapmer regions F-G-F' is at least 10, such as at least 12, such as at least 14 nucleotides in length.

In an aspect of the invention the antisense oligonucleotide or contiguous nucleotide sequence thereof consists of or comprises a gapmer of formula 5'-F-G-F'-3', where region F and F' independently comprise or consist of 1- 8 nucleosides, of which 2-4 are 2' sugar modified and defines the 5' and 3' end of the F and F' region, and G is a region between 6 and 16 nucleosides which are capable of recruiting RNaseH.

Regions F, G and F' are further defined below and can be incorporated into the F-G-F' formula.

### Gapmer - Region G

Region G (gap region) of the gapmer is a region of nucleosides which enables the oligonucleotide to recruit RNaseH, such as human RNase H1, typically DNA nucleosides. RNaseH is a cellular enzyme which recognizes the duplex between DNA and RNA, and enzymatically cleaves the RNA molecule. Suitably gapmers may have a gap region (G) of at least 5 or 6 contiguous DNA nucleosides, such as 5 - 16 contiguous DNA nucleosides, such as 6 - 15 contiguous DNA nucleosides, such as 7-14 contiguous DNA nucleosides, such as 8 - 12 contiguous DNA nucleotides, such as 8 - 12 contiguous DNA nucleotides in length. The gap region G may, in some embodiments consist of 6, 7, 8, 9, 10, 11, 12, 13, 14, 15 or 16 contiguous DNA nucleosides. Cytosine (C) DNA in the gap region may in some instances be methylated, such residues are either annotated as 5'-methyl-cytosine (^{me}C or with an e instead of a c). Methylation of cytosine DNA in the gap is advantageous if cg dinucleotides are present in the gap to reduce potential toxicity, the modification does not have significant impact on efficacy of the oligonucleotides. 5' substituted DNA nucleosides, such as 5' methyl DNA nucleoside have been reported for use in DNA gap regions (EP 2 742 136).

In some embodiments the gap region G may consist of 6, 7, 8, 9, 10, 11, 12, 13, 14, 15 or 16 contiguous phosphorothioate linked DNA nucleosides. In some embodiments, all internucleoside linkages in the gap are phosphorothioate linkages.

Whilst traditional gapmers have a DNA gap region, there are numerous examples of modified nucleosides which allow for RNaseH recruitment when they are used within the gap region. Modified nucleosides which have been reported as being capable of recruiting RNaseH when included within a gap region include, for example, alpha-L-LNA, C4' alkylated DNA (as described in WO 2009/090182 and Vester et al., Bioorg. Med. Chem. Lett. 18 (2008) 2296 - 2300), arabinose derived nucleosides like ANA and 2'F-ANA (Mangos et al. 2003 J. AM. CHEM. SOC. 125, 654-661), UNA (unlocked nucleic acid) (as described in Fluiter et al., Mol. Biosyst., 2009, 10, 1039). UNA is unlocked nucleic acid, typically where the bond between C2 and C3 of the ribose has been removed, forming an unlocked "sugar" residue. The modified nucleosides used in such gapmers may be nucleosides which adopt a 2' endo (DNA like) structure when introduced into the gap region, *i.e.* modifications which allow for RNaseH recruitment). In some embodiments the DNA Gap region (G) described herein may optionally contain 1 to 3 sugar modified nucleosides which adopt a 2' endo (DNA like) structure when introduced into the gap region.

### Region G - "Gap-breaker"

Alternatively, there are numerous reports of the insertion of a modified nucleoside which confers a 3' endo conformation into the gap region of gapmers, whilst retaining some RNaseH activity. Such gapmers with a gap region comprising one or more 3'endo modified nucleosides are referred to as "gap-breaker" or "gap-disrupted" gapmers, see for example WO2013/022984. Gap-breaker oligonucleotides retain sufficient region of DNA nucleosides within the gap region to allow for RNaseH recruitment. The ability of gapbreaker oligonucleotide design to recruit RNaseH is typically sequence or even compound specific - see Rukov et al. 2015 Nucl. Acids Res. Vol. 43 pp. 8476-8487, which discloses "gapbreaker" oligonucleotides which recruit RNaseH which in some instances provide a more specific cleavage of the target RNA. Modified nucleosides used within the gap region of gap-breaker oligonucleotides may for example be modified nucleosides which confer a 3'endo confirmation, such 2' -O-methyl (OMe) or 2'-O-MOE (MOE) nucleosides, or beta-D LNA nucleosides (the bridge between C2' and C4' of the ribose sugar ring of a nucleoside is in the beta conformation), such as beta-D-oxy LNA or ScET nucleosides.

As with gapmers containing region G described above, the gap region of gap-breaker or gap-disrupted gapmers, have a DNA nucleosides at the 5' end of the gap (adjacent to the 3' nucleoside of region F), and a DNA nucleoside at the 3' end of the gap (adjacent to the 5' nucleoside of region F'). Gapmers which comprise a disrupted gap typically retain a region of at least 3 or 4 contiguous DNA nucleosides at either the 5' end or 3' end of the gap region.

Exemplary designs for gap-breaker oligonucleotides include
F₁₋₈-[D₃₋₄-E₁- D ₃₋₄]₋F'₁₋₈
F₁₋₈- [D ₁₋₄-E₁- D ₃₋₄]-F'₁₋₈
F₁₋₈- [D ₃₋₄-E₁- D₁₋₄]-F'₁₋₈
wherein region G is within the brackets [Dₙ-Eᵣ- Dₘ], D is a contiguous sequence of DNA nucleosides, E is a modified nucleoside (the gap-breaker or gap-disrupting nucleoside), and F and F' are the flanking regions as defined herein, and with the proviso that the overall length of the gapmer regions F-G-F' is at least 12, such as at least 14 nucleotides in length.

In some embodiments, region G of a gap disrupted gapmer comprises at least 6 DNA nucleosides, such as 6, 7, 8, 9, 10, 11, 12, 13, 14, 15 or 16 DNA nucleosides. As described above, the DNA nucleosides may be contiguous or may optionally be interspersed with one or more modified nucleosides, with the proviso that the gap region G is capable of mediating RNaseH recruitment.

### Gapmer - flanking regions, F and F'

Region F is positioned immediately adjacent to the 5' DNA nucleoside of region G. The 3' most nucleoside of region F is a sugar modified nucleoside, such as a high affinity sugar modified nucleoside, for example a 2' substituted nucleoside, such as a MOE nucleoside, or an LNA nucleoside.

Region F' is positioned immediately adjacent to the 3' DNA nucleoside of region G. The 5' most nucleoside of region F' is a sugar modified nucleoside, such as a high affinity sugar modified nucleoside, for example a 2' substituted nucleoside, such as a MOE nucleoside, or an LNA nucleoside.

Region F is 1 - 8 contiguous nucleotides in length, such as 2-6, such as 3-4 contiguous nucleotides in length. Advantageously the 5' most nucleoside of region F is a sugar modified nucleoside. In some embodiments the two 5' most nucleoside of region F are sugar modified nucleoside. In some embodiments the 5' most nucleoside of region F is an LNA nucleoside. In some embodiments the two 5' most nucleoside of region F are LNA nucleosides. In some embodiments the two 5' most nucleoside of region F are 2' substituted nucleoside nucleosides, such as two 3' MOE nucleosides. In some embodiments the 5' most nucleoside of region F is a 2' substituted nucleoside, such as a MOE nucleoside.

Region F' is 2 - 8 contiguous nucleotides in length, such as 3-6, such as 4-5 contiguous nucleotides in length. Advantageously, embodiments the 3' most nucleoside of region F' is a sugar modified nucleoside. In some embodiments the two 3' most nucleoside of region F' are sugar modified nucleoside. In some embodiments the two 3' most nucleoside of region F' are LNA nucleosides. In some embodiments the 3' most nucleoside of region F' is an LNA nucleoside. In some embodiments the two 3' most nucleoside of region F' are 2' substituted nucleoside nucleosides, such as two 3' MOE nucleosides. In some embodiments the 3' most nucleoside of region F' is a 2' substituted nucleoside, such as a MOE nucleoside.

It should be noted that when the length of region F or F' is one, it is advantageously an LNA nucleoside.

In some embodiments, region F and F' independently consists of or comprises a contiguous sequence of sugar modified nucleosides. In some embodiments, the sugar modified nucleosides of region F may be independently selected from 2'-O-alkyl-RNA units, 2'-O-methyl-RNA, 2'-amino-DNA units, 2'-fluoro-DNA units, 2'-alkoxy-RNA, MOE units, LNA units, arabino nucleic acid (ANA) units and 2'-fluoro-ANA units.

In some embodiments, region F and F' independently comprises both LNA and a 2' substituted modified nucleosides (mixed wing design).

In some embodiments, region F and F' consists of only one type of sugar modified nucleosides, such as only MOE or only beta-D-oxy LNA or only ScET. Such designs are also termed uniform flanks or uniform gapmer design.

In some embodiments, all the nucleosides of region F or F', or F and F' are LNA nucleosides, such as independently selected from beta-D-oxy LNA, ENA or ScET nucleosides. In some embodiments region F consists of 1-5, such as 2-4, such as 3-4 such as 1, 2, 3, 4 or 5 contiguous LNA nucleosides. In some embodiments, all the nucleosides of region F and F' are beta-D-oxy LNA nucleosides.

In some embodiments, all the nucleosides of region F or F', or F and F' are 2' substituted nucleosides, such as OMe or MOE nucleosides. In some embodiments region F consists of 1, 2, 3, 4, 5, 6, 7, or 8 contiguous OMe or MOE nucleosides. In some embodiments only one of the flanking regions can consist of 2' substituted nucleosides, such as OMe or MOE nucleosides. In some embodiments it is the 5' (F) flanking region that consists 2' substituted nucleosides, such as OMe or MOE nucleosides whereas the 3' (F') flanking region comprises at least one LNA nucleoside, such as beta-D-oxy LNA nucleosides or cET nucleosides. In some embodiments it is the 3' (F') flanking region that consists 2' substituted nucleosides, such as OMe or MOE nucleosides whereas the 5' (F) flanking region comprises at least one LNA nucleoside, such as beta-D-oxy LNA nucleosides or cET nucleosides.

In some embodiments, all the modified nucleosides of region F and F' are LNA nucleosides, such as independently selected from beta-D-oxy LNA, ENA or ScET nucleosides, wherein region For F', or F and F' may optionally comprise DNA nucleosides (an alternating flank, see definition of these for more details). In some embodiments, all the modified nucleosides of region F and F' are beta-D-oxy LNA nucleosides, wherein region F or F', or F and F' may optionally comprise DNA nucleosides (an alternating flank, see definition of these for more details).

In some embodiments the 5' most and the 3' most nucleosides of region F and F' are LNA nucleosides, such as beta-D-oxy LNA nucleosides or ScET nucleosides.

In some embodiments, the internucleoside linkage between region F and region G is a phosphorothioate internucleoside linkage. In some embodiments, the internucleoside linkage between region F' and region G is a phosphorothioate internucleoside linkage. In some embodiments, the internucleoside linkages between the nucleosides of region F or F', F and F' are phosphorothioate internucleoside linkages.

### LNA Gapmer

An LNA gapmer is a gapmer wherein either one or both of region F and F' comprises or consists of LNA nucleosides. A beta-D-oxy gapmer is a gapmer wherein either one or both of region F and F' comprises or consists of beta-D-oxy LNA nucleosides.

In some embodiments the LNA gapmer is of formula: [LNA]₁₋₅-[region G] -[LNA]₁₋₅, wherein region G is as defined in the Gapmer region G definition.

In one embodiment the LNA gapmer is of the formula [LNA]₄-[region G]₁₀₋₁₂ -[LNA]₄

### MOE Gapmers

A MOE gapmers is a gapmer wherein regions F and F' consist of MOE nucleosides. In some embodiments the MOE gapmer is of design [MOE]₁₋₈-[Region G] ₅₋₁₆-[MOE] ₁₋₈, such as [MOE]₂₋₇-[Region G]₆₋₁₄-[MOE] ₂₋₇, such as [MOE]₃₋₆-[Region G]₈₋₁₂-[MOE] ₃₋₆, wherein region G is as defined in the Gapmer definition. MOE gapmers with a 5-10-5 design (MOE-DNA-MOE) have been widely used in the art.

### Mixed Wing Gapmer

A mixed wing gapmer is an LNA gapmer wherein one or both of region F and F' comprise a 2' substituted nucleoside, such as a 2' substituted nucleoside independently selected from the group consisting of 2'-O-alkyl-RNA units, 2'-O-methyl-RNA, 2'-amino-DNA units, 2'-fluoro-DNA units, 2'-alkoxy-RNA, MOE units, arabino nucleic acid (ANA) units and 2'-fluoro-ANA units, such as MOE nucleosides. In some embodiments wherein at least one of region F and F', or both region F and F' comprise at least one LNA nucleoside, the remaining nucleosides of region F and F' are independently selected from the group consisting of MOE and LNA. In some embodiments wherein at least one of region F and F', or both region F and F' comprise at least two LNA nucleosides, the remaining nucleosides of region F and F' are independently selected from the group consisting of MOE and LNA. In some mixed wing embodiments, one or both of region F and F' may further comprise one or more DNA nucleosides.

Mixed wing gapmer designs are disclosed in WO2008/049085 and WO2012/109395.

### Alternating Flank Gapmers

Flanking regions may comprise both LNA and DNA nucleoside and are referred to as "alternating flanks" as they comprise an alternating motif of LNA-DNA-LNA nucleosides. Gapmers comprising such alternating flanks are referred to as "alternating flank gapmers". "Alternative flank gapmers" are LNA gapmer oligonucleotides where at least one of the flanks (F or F') comprises DNA in addition to the LNA nucleoside(s). In some embodiments at least one of region F or F', or both region F and F', comprise both LNA nucleosides and DNA nucleosides. In such embodiments, the flanking region F or F', or both F and F' comprise at least three nucleosides, wherein the 5' and 3' most nucleosides of the F and/or F' region are LNA nucleosides.

Alternating flank LNA gapmers are disclosed in WO2016/127002.

An alternating flank region may comprise up to 3 contiguous DNA nucleosides, such as 1 to 2 or 1 or 2 or 3 contiguous DNA nucleosides.

The alternating flak can be annotated as a series of integers, representing a number of LNA nucleosides (L) followed by a number of DNA nucleosides (D), for example

[L]₁₋₃-[D]₁₋₄-[L]₁₋₃

[L]₁₋₂-[D]₁₋₂-[L]₁₋₂-[D]₁₋₂-[L]₁₋₂

In oligonucleotide designs these will often be represented as numbers such that 2-2-1 represents 5' [L]₂-[D]₂-[L] 3', and 1-1-1-1-1 represents 5' [L]-[D]-[L]-[D]-[L] 3'. The length of the flank (region F and F') in oligonucleotides with alternating flanks may independently be 3 to 10 nucleosides, such as 4 to 8, such as 5 to 6 nucleosides, such as 4, 5, 6 or 7 modified nucleosides. In some embodiments only one of the flanks in the gapmer oligonucleotide is alternating while the other is constituted of LNA nucleotides. It may be advantageous to have at least two LNA nucleosides at the 3' end of the 3' flank (F'), to confer additional exonuclease resistance. In one embodiment the flanks in the alternating flank gapmer have an overall length from 5-to 8 nucleosides of which 3 to 5 are LNA nucleosides. Some examples of oligonucleotides with alternating flanks are:

[L]₁₋₅-[D]₁₋₄-[L]₁₋₃-[G]₅₋₁₆-[L]₂₋₆

[L]₁₋₂-[D]₂₋₃-[L]₃₋₄--[G]₅₋₇-[L]₁₋₂-[D]₂₋₃-[L]₂₋₃

[L]₁₋₂-[D]₁₋₂-[L]₁₋₂-[D]₁₋₂-[L]₁₋₂-[G]₅₋₁₆-[L]₁₋₂-[D]₁₋₃-[L]₂₋₄

[L]₁₋₅-[G]₅₋₁₆-[L]-[D]-[L]-[D]-[L]₂

[L]₄-[G]₆₋₁₀-[L]-[D]₃-[L]₂

with the proviso that the overall length of the gapmer is at least 12, such as at least 14 nucleotides in length.

### Region D' or D" in an oligonucleotide

The oligonucleotide of the invention may in some embodiments comprise or consist of the contiguous nucleotide sequence of the oligonucleotide which is complementary to the target nucleic acid, such as the gapmer F-G-F', and further 5' and/or 3' nucleosides. The further 5' and/or 3' nucleosides may or may not be fully complementary to the target nucleic acid. Such further 5' and/or 3' nucleosides may be referred to as region D' and D" herein.

The addition of region D' or D" may be used for the purpose of joining the contiguous nucleotide sequence, such as the gapmer, to a conjugate moiety or another functional group. When used for joining the contiguous nucleotide sequence with a conjugate moiety is can serve as a biocleavable linker. Alternatively, it may be used to provide exonucleoase protection or for ease of synthesis or manufacture.

Region D' and D" can be attached to the 5' end of region F or the 3' end of region F', respectively to generate designs of the following formulas D'-F-G-F', F-G-F'-D" or D'-F-G-F'-D". In this instance the F-G-F' is the gapmer portion of the oligonucleotide and region D' or D" constitute a separate part of the oligonucleotide.

Region D' or D" may independently comprise or consist of 1, 2, 3, 4 or 5 additional nucleotides, which may be complementary or non-complementary to the target nucleic acid. The nucleotide adjacent to the F or F' region is not a sugar-modified nucleotide, such as a DNA or RNA or base modified versions of these. The D' or D' region may serve as a nuclease susceptible biocleavable linker (see definition of linkers). In some embodiments the additional 5' and/or 3' end nucleotides are linked with phosphodiester linkages, and are DNA or RNA. Nucleotide based biocleavable linkers suitable for use as region D' or D" are disclosed in WO2014/076195, which include by way of example a phosphodiester linked DNA dinucleotide. The use of biocleavable linkers in poly-oligonucleotide constructs is disclosed in WO2015/113922, where they are used to link multiple antisense constructs (e.g. gapmer regions) within a single oligonucleotide.

In one embodiment the oligonucleotide of the invention comprises a region D' and/or D" in addition to the contiguous nucleotide sequence which constitutes the gapmer.

In some embodiments, the oligonucleotide of the present invention can be represented by the following formulae:
F-G-F'; in particular F₂₋₈-G₆₋₁₆-F'₂₋₈
D'-F-G-F', in particular D'₂₋₃-F₁₋₈-G₆₋₁₆-F'₂₋₈
F-G-F'-D", in particular F₂₋₈-G₆₋₁₆-F'₂₋₈-D"₁₋₃
D'-F-G-F'-D", in particular D'₁₋₃- F₂₋₈-G₆₋₁₆-F'₂₋₈-D"₁₋₃

In some embodiments the internucleoside linkage positioned between region D' and region F is a phosphodiester linkage. In some embodiments the internucleoside linkage positioned between region F' and region D" is a phosphodiester linkage.

### Conjugate

The term conjugate as used herein refers to an oligonucleotide which is covalently linked to a non-nucleotide moiety (conjugate moiety or region C or third region).

Conjugation of the oligonucleotide of the invention to one or more non-nucleotide moieties may improve the pharmacology of the oligonucleotide, e.g. by affecting the activity, cellular distribution, cellular uptake or stability of the oligonucleotide. In some embodiments the conjugate moiety modify or enhance the pharmacokinetic properties of the oligonucleotide by improving cellular distribution, bioavailability, metabolism, excretion, permeability, and/or cellular uptake of the oligonucleotide. In particular, the conjugate may target the oligonucleotide to a specific organ, tissue or cell type and thereby enhance the effectiveness of the oligonucleotide in that organ, tissue or cell type. At the same time the conjugate may serve to reduce activity of the oligonucleotide in non-target cell types, tissues or organs, e.g. off target activity or activity in non-target cell types, tissues or organs.

Oligonucleotide conjugates and their synthesis has also been reported in comprehensive reviews by Manoharan in Antisense Drug Technology, Principles, Strategies, and Applications, S.T. Crooke, ed., Ch. 16, Marcel Dekker, Inc., 2001 and Manoharan, Antisense and Nucleic Acid Drug Development, 2002, 12, 103.

In an embodiment, the non-nucleotide moiety (conjugate moiety) is selected from the group consisting of carbohydrates (e.g. GalNAc), cell surface receptor ligands, drug substances, hormones, lipophilic substances, polymers, proteins, peptides, toxins (e.g. bacterial toxins), vitamins, viral proteins (e.g. capsids) or combinations thereof.

In some embodiments, the conjugate is an antibody or an antibody fragment which has a specific affinity for a transferrin receptor, for example as disclosed in WO 2012/143379. In some embodiments the non-nucleotide moiety is an antibody or antibody fragment, such as an antibody or antibody fragment that facilitates delivery across the blood-brain-barrier, in particular an antibody or antibody fragment targeting the transferrin receptor.

### Linkers

A linkage or linker is a connection between two atoms that links one chemical group or segment of interest to another chemical group or segment of interest via one or more covalent bonds. Conjugate moieties can be attached to the oligonucleotide directly or through a linking moiety (e.g. linker or tether). Linkers serve to covalently connect a third region, e.g. a conjugate moiety (Region C), to a first region, e.g. an oligonucleotide or contiguous nucleotide sequence complementary to the target nucleic acid (region A).

In some embodiments of the invention the conjugate or oligonucleotide conjugate of the invention may optionally, comprise a linker region (second region or region B and/or region Y) which is positioned between the oligonucleotide or contiguous nucleotide sequence complementary to the target nucleic acid (region A or first region) and the conjugate moiety (region C or third region).

Region B refers to biocleavable linkers comprising or consisting of a physiologically labile bond that is cleavable under conditions normally encountered or analogous to those encountered within a mammalian body. Conditions under which physiologically labile linkers undergo chemical transformation (e.g., cleavage) include chemical conditions such as pH, temperature, oxidative or reductive conditions or agents, and salt concentration found in or analogous to those encountered in mammalian cells. Mammalian intracellular conditions also include the presence of enzymatic activity normally present in a mammalian cell such as from proteolytic enzymes or hydrolytic enzymes or nucleases. In one embodiment the biocleavable linker is susceptible to S1 nuclease cleavage. In a preferred embodiment the nuclease susceptible linker comprises between 1 and 10 nucleosides, such as 1, 2, 3, 4, 5, 6, 7, 8, 9 or 10 nucleosides, more preferably between 2 and 6 nucleosides and most preferably between 2 and 4 linked nucleosides comprising at least two consecutive phosphodiester linkages, such as at least 3 or 4 or 5 consecutive phosphodiester linkages. Preferably the nucleosides are DNA or RNA. Phosphodiester containing biocleavable linkers are described in more detail in WO 2014/076195.

Region Y refers to linkers that are not necessarily biocleavable but primarily serve to covalently connect a conjugate moiety (region C or third region), to an oligonucleotide (region A or first region). The region Y linkers may comprise a chain structure or an oligomer of repeating units such as ethylene glycol, amino acid units or amino alkyl groups The oligonucleotide conjugates of the present invention can be constructed of the following regional elements A-C, A-B-C, A-BY-C, A-Y-B-C or A-Y-C. In some embodiments the linker (region Y) is an amino alkyl, such as a C2 - C36 amino alkyl group, including, for example C6 to C12 amino alkyl groups. In a preferred embodiment the linker (region Y) is a C6 amino alkyl group.

### Treatment

The term 'treatment' as used herein refers to both treatment of an existing disease (e.g. a disease or disorder as herein referred to), or prevention of a disease, *i.e.* prophylaxis. It will therefore be recognized that treatment as referred to herein may, in some embodiments, be prophylactic.

In some examples treatment is performed on a patient who has been diagnosed with a neurological disorder, such as a neurological disorder selected from the group consisting of neurodegenerative diseases including Tauopathies, Alzheimer's disease (AD), progressive supranuclear palsy (PSP), corticobasal ganglionic degeneration (CBD), chronic traumatic encephalopathy (CTE), fronto- temporal dementia FTD) and FTD with parkinsonism linked to chromosome 17 (FTDP-17), Pick's disease (PiD), argyrophilic grain disease (AGD), tangle-predominant senile dementia (TPSD), primary age-related Tauopathy (PART), Down syndrome and lytico-bodig disease. Upregulation of pathological Tau is associated with infantile Tauopathies including hemimegalencephaly (HME), tuberous sclerosis complex; focal cortical dysplasia type 2b; and ganglioglioma. In addition, abnormal Tau expression and/or function may also be associated with other diseases such as Hallervorden-Spatz syndrome, also known as neurodegeneration with brain iron accumulation type 1 (NBIA1), gangliocytomas, and subacute sclerosing panencephalitis. Tau may also play a role in seizure disorders (e.g., epilepsy), network dysfunction (e.g., depression), and movement disorders (e.g., Parkinson's disease).

### DETAILED DESCRIPTION OF THE INVENTION

### The Oligonucleotides of the Invention

The invention relates to oligonucleotides as defined by the claims, which are capable of reducing expression of Tau. The reduction is achieved by hybridizing to a target nucleic acid encoding Tau. The target nucleic acid may be a mammalian MAPT mRNA sequence, such as a sequence selected from the group consisting of SEQ ID NO: 1 and 2.

The oligonucleotide described herein is an antisense oligonucleotide, as defined by the claims, which targets MAPT resulting in reduced Tau expression.

In some embodiments the antisense oligonucleotide of the invention, as defined by the claims, is capable of modulating the expression of the target by inhibiting or down-regulating it. Preferably, such modulation produces an inhibition of expression of at least 20% compared to the normal expression level of the target, more preferably at least 30%, at least 40%, at least 50%, at least 60%, at least 70%, at least 80%, or at least 90% inhibition compared to the normal expression level of the target. In some embodiments oligonucleotides of the invention, as defined by the claims, may be capable of inhibiting expression levels of Tau mRNA by at least 60% or 70% *in vitro* following application of 5 µM oligonucleotide to primary neuronal cells. In some embodiments compounds of the invention, as defined by the claims, may be capable of inhibiting expression levels of Tau protein by at least 50% *in vitro* following application of 0.5 µM oligonucleotide to primary neuronal cells. Suitably, the examples provide assays which may be used to measure Tau RNA or protein inhibition (e.g. example 1 and 3). The target modulation is triggered by the hybridization between a contiguous nucleotide sequence of the oligonucleotide and the target nucleic acid. In some embodiments the oligonucleotide of the invention, as defined by the claims, comprises mismatches between the oligonucleotide and the target nucleic acid. Despite mismatches hybridization to the target nucleic acid may still be sufficient to show a desired modulation of Tau expression. Reduced binding affinity resulting from mismatches may advantageously be compensated by increased number of nucleotides in the oligonucleotide and/or an increased number of modified nucleosides capable of increasing the binding affinity to the target, such as 2' sugar modified nucleosides, including LNA, present within the oligonucleotide sequence.

An example of the present disclosure relates to an antisense oligonucleotide which comprises a contiguous nucleotide sequence of at least 10 nucleotides in length with at least 90% complementarity to SEQ ID NO: 3.

In some embodiments, the oligonucleotide comprises a contiguous sequence of 16 to 30 nucleotides in length, which is 100% complementary with a region of the target nucleic acid or a target sequence, as defined by the claims.

It is advantageous if the oligonucleotide of the invention, or contiguous nucleotide sequence thereof is fully complementary (100% complementary) to a region of the target nucleic acid, or in some embodiments may comprise one or two mismatches between the oligonucleotide and the target nucleic acid.

In some embodiments the oligonucleotide comprises a contiguous nucleotide sequence of 16 to 30 nucleotides in length with 100% complementary, to contiguous nucleotides within position 12060 to 12078 of SEQ ID NO: 1, wherein the antisense oligonucleotide is capable of reducing the expression of Tau.

In some embodiments the oligonucleotide sequence is 100% complementary to a corresponding target nucleic acid region present in SEQ ID NO: 1 and SEQ ID NO: 2.

It is advantageous if the antisense oligonucleotide is complementary to a target sequence selected from one of the regions listed in table 4. In some examples the contiguous nucleotide sequence of the antisense oligonucleotide is at least 90% complementary to, such as fully complementary to a target sequence selected R1-R2254 (table 4) In some embodiments the oligonucleotide sequence is 100% complementary to R_223 (see table 4).

In some embodiment the oligonucleotide or contiguous nucleotide sequence is fully complementary, to a region of the target nucleic acid, wherein the target nucleic acid region is position 12060 - 12078 of SEQ ID NO: 1, and wherein the antisense oligonucleotide is capable of reducing the expression of Tau.

In another example the oligonucleotide or contiguous nucleotide sequence is 90% complementary, such as fully complementary, to a region of the target nucleic acid, wherein the target nucleic acid region is selected from the group consisting of position 39562-39593 of SEQ ID NO: 1 such as positon 39573-39592 of SEQ ID NO: 1.

In another example of the oligonucleotide or contiguous nucleotide sequence is 90% complementary, such as fully complementary, to a region of the target nucleic acid, wherein the target nucleic acid region is selected from the group consisting of position 72837-72940 of SEQ ID NO: 1 such as positon 72861-72891 or position 72862-72890 of SEQ ID NO: 1.

In some embodiments the oligonucleotide comprises a contiguous nucleotide sequence of 16 to 22 nucleotides, such as 16 to 20 nucleotides, in length with 100% complementary, to contiguous nucleotides within position 12060 to 12078 of SEQ ID NO: 1.

In some examples, the oligonucleotide comprises or consists of 10 to 35 nucleotides in length, such as from 10 to 30, such as 10 to 25, such as from 10 to 22, such as from 10 to 20 or 10 to 18 contiguous nucleotides in length. In one embodiment, the oligonucleotide comprises or consists of 16 to 22 nucleotides in length. In a preferred embodiment, the oligonucleotide comprises or consists of 16 to 20 nucleotides in length.

In some embodiments, the oligonucleotide or contiguous nucleotide sequence thereof comprises or consists of 22 or less nucleotides, such as 20 or less nucleotides, such as 16, 17, 18, 19 or 20 nucleotides. It is to be understood that any range given herein includes the range endpoints. Accordingly, if an oligonucleotide is said to include from 10 to 30 nucleotides, both 10 and 30 nucleotides are included.

In some embodiments, the contiguous nucleotide sequence comprises or consists of 16, 17, 18, 19, 20, 21, 22, 23, 24, 25, 26, 27, 28, 29 or 30 contiguous nucleotides in length. In a preferred embodiment, the oligonucleotide comprises or consists of 16, 17, 18, 19 or 20 nucleotides in length.

In some examples, the oligonucleotide or contiguous nucleotide sequence comprises or consists of a sequence selected from the group consisting of sequences listed in table 5(Materials and Method section).

In some examples, the antisense oligonucleotide or contiguous nucleotide sequence comprises or consists of 10 to 30 nucleotides in length with at least 90% identity, preferably 100% identity, to a sequence selected from the group consisting of SEQ ID NO: 6 to 65 (see motif sequences listed in table 5).

In some examples, the antisense oligonucleotide or contiguous nucleotide sequence comprises or consists of 16 to 30 nucleotides in length with at least 90% identity, preferably 100% identity, to a sequence selected from the group consisting of SEQ ID NO: 9, 11, 49, 53, 56 and 62 (see motif sequences listed in table 5).

In some examples, the antisense oligonucleotide or contiguous nucleotide sequence comprises or consists of 10 to 30 nucleotides in length with at least 90% identity, preferably 100% identity, to a sequence selected from the group consisting of SEQ ID NO: 6 to 37 (see motif sequences listed in table 5).

In some embodiments, the antisense oligonucleotide or contiguous nucleotide sequence comprises or consists of 16 to 30 nucleotides in length with 100% identity to a sequence of SEQ ID NO: 9 or 11 (see motif sequences listed in table 5).

In some examples, the antisense oligonucleotide or contiguous nucleotide sequence comprises or consists of 10 to 30 nucleotides in length with at least 90% identity, preferably 100% identity, to a sequence selected from the group consisting of SEQ ID NO: 38 to 51 (see motif sequences listed in table 5).

In some examples, the antisense oligonucleotide or contiguous nucleotide sequence comprises or consists of 10 to 30 nucleotides in length with at least 90% identity, preferably 100% identity, to a sequence of SEQ ID NO: 49 or 51 (see motif sequences listed in table 5).

In some examples, the antisense oligonucleotide or contiguous nucleotide sequence comprises or consists of 10 to 30 nucleotides in length with at least 90% identity, preferably 100% identity, to a sequence selected from the group consisting of SEQ ID NO: 52 to 65 (see motif sequences listed in table 5).

In some examples, the antisense oligonucleotide or contiguous nucleotide sequence comprises or consists of 10 to 30 nucleotides in length with at least 90% identity, preferably 100% identity, to a sequence of SEQ ID NO: 56 or 62 (see motif sequences listed in table 5).

It is understood that the contiguous nucleobase sequences (motif sequence) can be modified to for example increase nuclease resistance and/or binding affinity to the target nucleic acid.

The pattern in which the modified nucleosides (such as high affinity modified nucleosides) are incorporated into the oligonucleotide sequence is generally termed oligonucleotide design.

The oligonucleotides of the invention are designed with modified nucleosides and DNA nucleosides. Advantageously, high affinity modified nucleosides are used.

In an embodiment, the oligonucleotide comprises at least 1 modified nucleoside, such as at least 2, at least 3, at least 4, at least 5, at least 6, at least 7, at least 8, at least 9, at least 10, at least 11, at least 12, at least 13, at least 14, at least 15 or at least 16 modified nucleosides. In an embodiment the oligonucleotide comprises from 1 to 10 modified nucleosides, such as from 2 to 9 modified nucleosides, such as from 3 to 8 modified nucleosides, such as from 4 to 7 modified nucleosides, such as 6 or 7 modified nucleosides. Suitable modifications are described in the "Definitions" section under "modified nucleoside", "high affinity modified nucleosides", "sugar modifications", "2' sugar modifications" and Locked nucleic acids (LNA)".

In an embodiment, the oligonucleotide comprises one or more sugar modified nucleosides, such as 2' sugar modified nucleosides. Preferably the oligonucleotide of the invention comprises one or more 2' sugar modified nucleoside independently selected from the group consisting of 2'-O-alkyl-RNA, 2'-O-methyl-RNA, 2'-alkoxy-RNA, 2'-O-methoxyethyl-RNA, 2'-amino-DNA, 2'-fluoro-DNA, arabino nucleic acid (ANA), 2'-fluoro-ANA and LNA nucleosides. It is advantageous if one or more of the modified nucleoside(s) is a locked nucleic acid (LNA).

In a further embodiment the oligonucleotide comprises at least one modified internucleoside linkage. Suitable internucleoside modifications are described in the "Definitions" section under "Modified internucleoside linkage". It is advantageous if at least 75%, such as 80%, such as all, the internucleoside linkages within the contiguous nucleotide sequence are phosphorothioate or boranophosphate internucleoside linkages. In some embodiments all the internucleotide linkages in the contiguous sequence of the oligonucleotide are phosphorothioate linkages.

In some embodiments, the oligonucleotide of the invention comprises at least one LNA nucleoside, such as 1, 2, 3, 4, 5, 6, 7, or 8 LNA nucleosides, such as from 2 to 6 LNA nucleosides, such as from 3 to 7 LNA nucleosides, 4 to 8 LNA nucleosides or 3, 4, 5, 6, 7 or 8 LNA nucleosides. In some embodiments, at least 75% of the modified nucleosides in the oligonucleotide are LNA nucleosides, such as 80%, such as 85%, such as 90% of the modified nucleosides are LNA nucleosides, in particular beta-D-oxy LNA or ScET. In a still further embodiment all the modified nucleosides in the oligonucleotide are LNA nucleosides. In a further embodiment, the oligonucleotide may comprise both beta-D-oxy-LNA, and one or more of the following LNA nucleosides: thio-LNA, amino-LNA, oxy-LNA, ScET and/or ENA in either the beta-D or alpha-L configurations or combinations thereof. In a further embodiment, all LNA cytosine units are 5-methyl-cytosine. It is advantageous for the nuclease stability of the oligonucleotide or contiguous nucleotide sequence to have at least 1 LNA nucleoside at the 5' end and at least 2 LNA nucleosides at the 3' end of the nucleotide sequence.

In an embodiment of the invention the oligonucleotide of the invention is capable of recruiting RNase H.

In the current invention an advantageous structural design is a gapmer design as described in the "Definitions" section under for example "Gapmer", "LNA Gapmer", "MOE gapmer" and "Mixed Wing Gapmer" "Alternating Flank Gapmer". The gapmer design includes gapmers with uniform flanks, mixed wing flanks, alternating flanks, and gapbreaker designs. In the present invention it is advantageous if the oligonucleotide of the invention is a gapmer with an F-G-F' design, particular gapmer of formula 5'-F-G-F'-3', where region F and F' independently comprise 1 - 8 nucleosides, of which 2 - 5 are 2' sugar modified and defines the 5' and 3' end of the F and F' region, and G is a region between 6 and 16 nucleosides which are capable of recruiting RNaseH, such as a region comprising 6 - 16 DNA nucleosides.

In some embodiments the gapmer is an LNA gapmer.

In some embodiments of the invention the LNA gapmer is selected from the following uniform flank designs 4-10-4, 3-11-4, 4-11-4, 4-12-4 or 4-14-2.

In some embodiments of the invention the LNA gapmer is selected from the following alternating flanks designs 3-1-3-10-2, 1-3-4-6-1-3-2, 1-2-1-2-2-8-4, or 3-3-1-8-2-1-2.

Table 5 (Materials and Method section) lists preferred designs of each motif sequence.

In all instances the F-G-F' design may further include region D' and/or D" as described in the "Definitions" section under "Region D' or D" in an oligonucleotide". In some embodiments the oligonucleotide of the invention has 1, 2 or 3 phosphodiester linked nucleoside units, such as DNA units, at the 5' or 3' end of the gapmer region.

Exemplary oligonucleotides may be selected from the group of oligonucleotide compounds with CMP-ID-NO: 6_1; 7_1; 8_1; 9_1; 9_2; 9_3; 9_4; 9_5; 9_6; 9_7; 9_8; 9_9; 9_10; 9_11; 9_12; 9_13; 9_14; 9_15; 9_16; 9_17; 9_18; 9_19; 9_20; 9_21; 9_22; 9_23; 9_24; 9_25; 9_26; 9_27; 9_28; 9_29; 9_30; 9_31; 9_32; 9_33; 9_34; 9_35; 9_36; 9_37; 9_38; 9_39; 9_40; 9_41; 9_42; 9_43; 9_44; 9_45; 9_46; 9_47; 9_48; 9_49; 9_50; 9_51; 9_52; 9_53; 9_54; 9_55; 9_56; 9_57; 9_58; 9_59; 9_60; 9_61; 9_62; 9_63; 9_64; 9_65; 9_66; 9_67; 9_68; 9_69; 9_70; 9_71; 9_72; 9_73; 9_74; 9_75; 9_76; 9_77; 9_78; 9_79; 9_80; 9_81; 9_82; 9_83; 9_84; 9_85; 9_86; 9_87; 9_88; 9_89; 9_90; 9_91; 9_92; 9_93; 9_94; 9_95; 9_96; 9_97; 9_98; 9_99; 9_100; 9_101; 9_102; 9_103; 9_104; 9_105; 9_106; 10_1; 10_2; 10_3; 10_4; 10_5; 10_6; 10_7; 10_8; 10_9; 10_10; 10_11; 10_12; 10_13; 10_14; 10_15; 10_16; 10_17; 10_18; 10_19; 10_20; 10_21; 10_22; 10_23; 10_24; 10_25; 10_26; 10_27; 10_28; 10_29; 10_30; 10_31; 10_32; 10_33; 10_34; 10_35; 10_36; 10_37; 10_38; 10_39; 10_40; 10_41; 10_42; 10_43; 10_44; 10_45; 10_46; 10_47; 10_48; 10_49; 10_50; 10_51; 10_52; 10_53; 10_54; 10_55; 10_56; 10_57; 10_58; 10_59; 10_60; 10_61; 10_62; 10_63; 10_64; 10_65; 10_66; 10_67; 10_68; 10_69; 10_70; 10_71; 10_72; 10_73; 10_74; 10_75; 10_76; 10_77; 10_78; 10_79; 10_80; 10_81; 10_82; 10_83; 10_84; 10_85; 10_86; 10_87; 10_88; 10_89; 11_1; 12_1; 13_1; 14_1; 15_1; 16_1; 17_1; 18_1; 19_1; 20_1; 21_1; 22_1; 23_1; 24_1; 24_2; 24_3; 24_4; 24_5; 24_6; 24_7; 24_8; 24_9; 24_10; 24_11; 24_12; 24_13; 24_14; 24_15; 24_16; 24_17; 24_18; 24_19; 24_20; 24_21; 24_22; 24_23; 24_24; 24_25; 24_26; 24_27; 24_28; 24_29; 24_30; 24_31; 24_32; 24_33; 24_34; 24_35; 24_36; 24_37; 24_38; 24_39; 24_40; 24_41; 24_42; 24_43; 24_44; 24_45; 24_46; 24_47; 24_48; 24_49; 24_50; 24_51; 24_52; 24_53; 24_54; 24_55; 24_56; 24_57; 24_58; 24_59; 24 _60; 24_61; 24_62; 25_1; 25_2; 25_3; 25_4; 25_5; 25_6; 25_7; 25_8; 25_9; 25_10; 25_11; 25_12; 25_13; 25_14; 25_15; 25_16; 25_17; 25_18; 25_19; 25_20; 25_21; 25_22; 25_23; 25_24; 25_25; 25_26; 25_27; 25_28; 25_29; 25_30; 25_31; 25_32; 25_33; 25_34; 25_35; 25_36; 25_37; 25_38; 25_39; 25_40; 25_41; 25_42; 25_43; 26_1; 26_2; 26_3; 26_4; 26_5; 26_6; 26_7; 26_8; 26_9; 26_10; 26_11; 26_12; 26_13; 26_14; 26_15; 26_16; 26_17; 26_18; 26_19; 26_20; 26_21; 26_22; 26_23; 26_24; 26_25; 26_26; 26_27; 26_28; 26_29; 26_30; 26_31; 27_1; 28_1; 28_2; 28_3; 28_4; 28_5; 28_6; 28_7; 28_8; 28_9; 28_10; 28_11; 28_12; 28_13; 28_14; 28_15; 28_16; 28_17; 28_18; 28_19; 28_20; 28_21; 28_22; 28_23; 28_24; 28_25; 28_26; 28_27; 28_28; 28_29; 28_30; 28_31; 28_32; 28_33; 29_1; 29_2; 29_3; 29_4; 29_5; 29_6; 29_7; 29_8; 29_9; 29_10; 29_11; 29_12; 29_13; 29_14; 30_1; 30_2; 30_3; 30_4; 30_5; 30_6; 30_7; 30_8; 30_9; 30_10; 30_11; 30_12; 30_13; 30_14; 30_15; 30_16; 30_17; 30_18; 30_19; 30_20; 30_21; 30_22; 30_23; 30_24; 30_25; 31_1; 31_2; 31_3; 32_1; 32_2; 32_3; 32_4; 32_5; 32_6; 32_7; 32_8; 32_9; 32_10; 32_11; 32_12; 32_13; 32_14; 32_15; 32_16; 32_17; 32_18; 32_19; 32_20; 32_21; 32_22; 32_23; 32_24; 32_25; 32_26; 32_27; 32_28; 32_29; 32_30; 32_31; 32_32; 32_33; 32_34; 32_35; 32_36; 32_37; 32_38; 32_39; 32_40; 32_41; 32_42; 32_43; 32_44; 32_45; 32_46; 32_47; 32_48; 32_49; 32_50; 32_51; 33_1; 33_2; 33_3; 33 _4; 33_5; 33_6; 33_7; 33_8; 33_9; 33_10; 33_11; 33_12; 33_13; 33_14; 33_15; 33_16; 33_17; 33_18; 33_19; 33_20; 33_21; 33_22; 33_23; 33_24; 33_25; 33_26; 33_27; 33_28; 33_29; 33_30; 33_31; 33_32; 33_33; 34_1; 35_1; 35_2; 35_3; 36_1; 37_1; 38_1; 39_1; 40_1; 41_1; 42_1; 43_1; 44_1; 45_1; 46_1; 47_1; 48_1; 49_1; 49_2; 49_3; 49_4; 49_5; 49_6; 49_7; 49_8; 49_9; 49_10; 49_11; 49_12; 49_13; 49_14; 49_15; 49_16; 49_17; 49_18; 49_19; 49_20; 49_21; 49_22; 49_23; 49_24; 49_25; 49_26; 49_27; 49_28; 49_29; 49_30; 49_31; 49_32; 49_33; 49_34; 49_35; 49_36; 49_37; 49_38; 49_39; 49_40; 49_41; 49_42; 49_43; 49_44; 49_45; 49_46; 49_47; 49_48; 49_49; 49_50; 49_51; 49_52; 49_53; 49_54; 49_55; 49_56; 49_57; 49_58; 49_59; 49_60; 49_61; 49_62; 49_63; 49_64; 49_65; 49_66; 49_67; 49_68; 49_69; 49_70; 49_71; 49_72; 49_73; 49_74; 49_75; 49_76; 49_77; 49_78; 49_79; 49_80; 49_81; 49_82; 49_83; 49_84; 49_85; 49_86; 49_87; 49_88; 49_89; 49_90; 49_91; 49_92; 49_93; 49_94; 49_95; 49_96; 49_97; 49_98; 49_99; 49_100; 49_101; 49_102; 49_103; 49_104; 49_105; 49_106; 49_107; 49_108; 49_109; 49_110; 49_111; 49_112; 49_113; 49_114; 49_115; 49_116; 49_117; 49_118; 49_119; 49_120; 49_121; 49_122; 49_123; 49_124; 49_125; 49_126; 49_127; 49_128; 49_129; 49_130; 49_131; 49_132; 49_133; 49_134; 49_135; 49_136; 49_137; 49_138; 49_139; 49_140; 49_141; 49_142; 49_143; 49_144; 49_145; 49_146; 49_147; 49_148; 49_149; 49_150; 49_151; 49_152; 49_153; 49_154; 49_155; 49_156; 49_157; 49_158; 49_159; 49_160; 49_161; 49_162; 49_163; 49_164; 49_165; 49_166; 49_167; 49_168; 49_169; 49_170; 49_171; 49_172; 49_173; 49_174; 49_175; 49_176; 49_177; 49_178; 49_179; 49_180; 49_181; 49_182; 49_183; 49_184; 49_185; 49_186; 49_187; 49_188; 49_189; 49_190; 49_191; 49_192; 50_1; 51_1; 52_1; 53_1; 54_1; 55_1; 56_1; 57_1; 58_1; 59_1; 60_1; 61_1; 62_1; 63_1; 64_1 and 65_1.

For example, the oligonucleotide is selected from the group of oligonucleotide compounds with CMP-ID-NO: 9_102; 9_103; 9_104; 11_1; 49_38; 49_51; 49_179; 49_189; 53_1; 56_1 and 62_1.

For certain embodiments of the invention, the oligonucleotide is selected from the group of oligonucleotide compounds with CMP-ID-NO: 9_102; 9_103; 9_104 and 11_1.

For example, the oligonucleotide is selected from the group of oligonucleotide compounds with CMP-ID-NO: 49_38; 49_51; 49_179 and 49_189.

For example, the oligonucleotide is selected from the group of oligonucleotide compounds with CMP-ID-NO: 53_1; 56_1 and 62_1.

A particular advantageous antisense oligonucleotide described herein is an oligonucleotide compound selected from the group consisting of

| | |
|---|---|
| CTTtAATttaatcactcAT | SEQ ID NO: 9; CMP ID NO: 9_102 |
| CTTTaatttaatcacTCAT | SEQ ID NO: 9; CMP ID NO: 9_103 |
| CTTTaatttaatcaCtCAT | SEQ ID NO: 9; CMP ID NO: 9_104 |
| CTTTaatttaatcaCTCA | SEQ ID NO: 11; CMP ID NO: 11_1 |
| TtaaCTCAaatcaaTtctCA | SEQ ID NO: 49; CMP ID NO: 49_38 |
| TtaActCAaatcaattCTCA | SEQ ID NO: 49; CMP ID NO: 49_51 |
| TTAactCaaatcaatTCtCA | SEQ ID NO: 49; CMP ID NO: 49_179 |
| TTAActcaaatcaattCTCA | SEQ ID NO: 49; CMP ID NO: 49_189 |
| CAACaccttttaattcATTA | SEQ ID NO: 53; CMP ID NO: 53_1 |
| CTCAtcaacaccttttaaTT | SEQ ID NO: 56; CMP ID NO: 56_1 |
| TTAactcatcaacaCCTT | SEQ ID NO: 62; CMP ID NO: 62_1 |

wherein capital letters are beta-D-oxy LNA nucleosides, lowercase letters are DNA nucleosides, all LNA C are 5-methyl cytosine, all internucleoside linkages are phosphorothioate internucleoside linkages.

In one embodiment the antisense oligonucleotide is CMP ID NO: 9_103 as shown in Fig. 2.

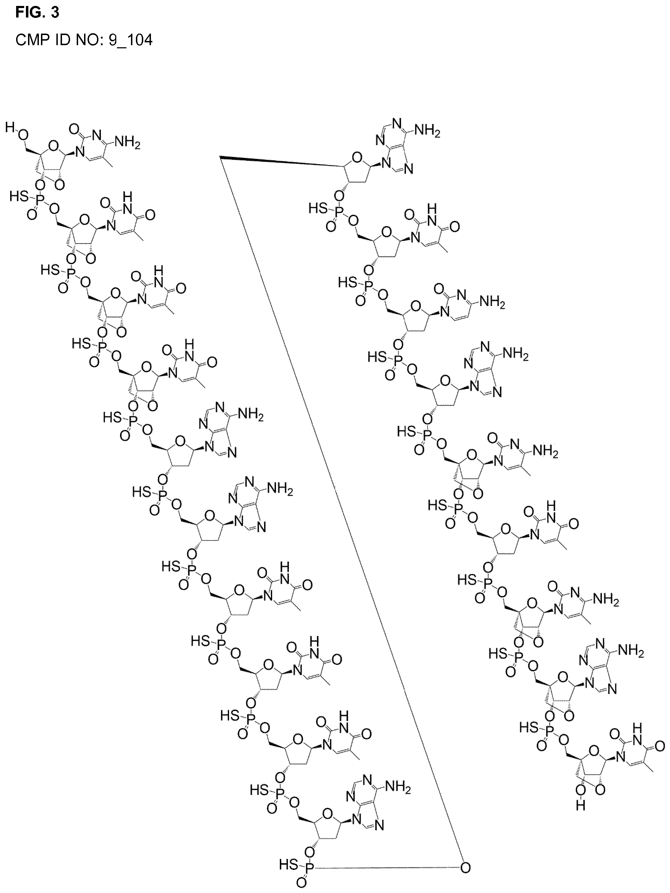

In one embodiment the antisense oligonucleotide is CMP ID NO: 9_104 as shown in Fig. 3.

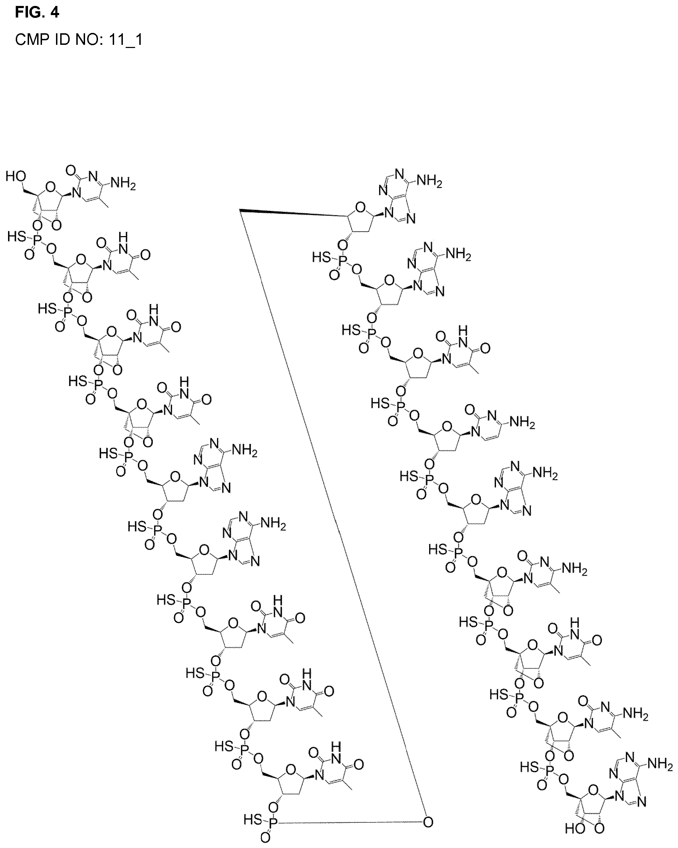

In one embodiment the antisense oligonucleotide is CMP ID NO: 11_1 as shown in Fig. 4.

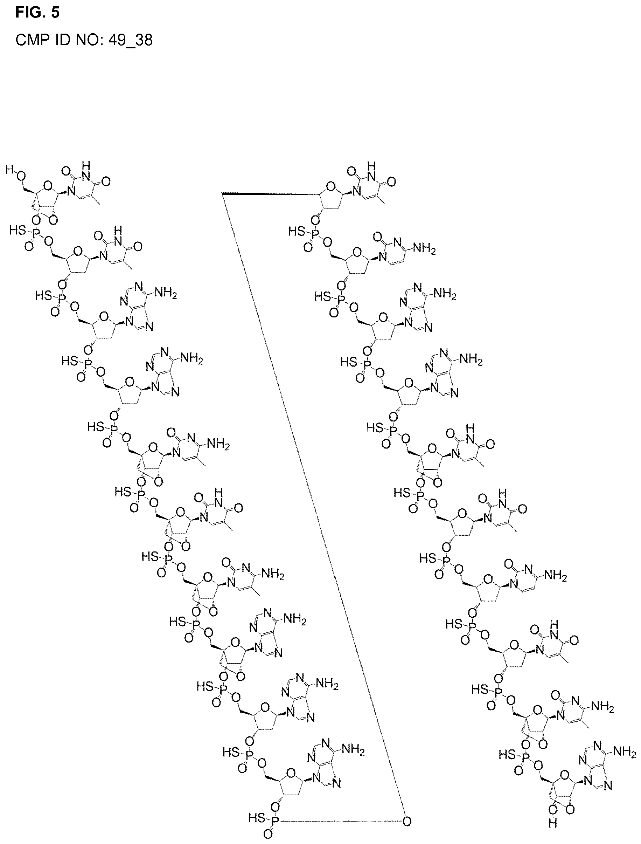

In one example the antisense oligonucleotide is CMP ID NO: 49_38 as shown in Fig. 5.

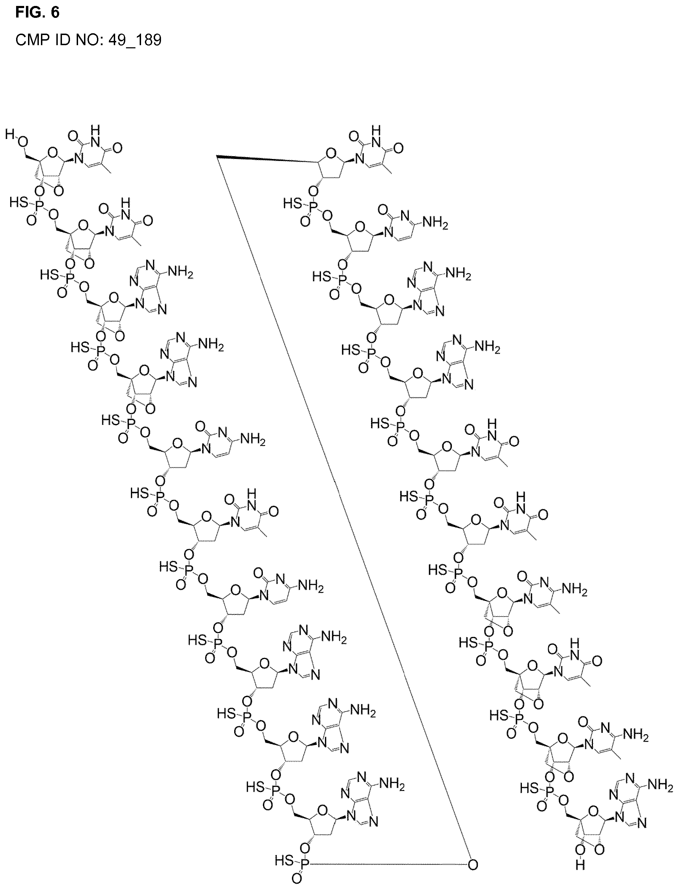

In one example the antisense oligonucleotide is CMP ID NO: 49_189 as shown in Fig. 6.

### Method of manufacture

Described herein are methods for manufacturing the oligonucleotides of the invention comprising reacting nucleotide units and thereby forming covalently linked contiguous nucleotide units comprised in the oligonucleotide. Preferably, the method uses phophoramidite chemistry (see for example Caruthers et al, 1987, Methods in Enzymology vol. 154, pages 287-313). In a further example the method further comprises reacting the contiguous nucleotide sequence with a conjugating moiety (ligand) to covalently attach the conjugate moiety to the oligonucleotide. In a further example a method is provided for manufacturing the composition of the invention, comprising mixing the oligonucleotide or conjugated oligonucleotide of the invention with a pharmaceutically acceptable diluent, solvent, carrier, salt and/or adjuvant.

### Pharmaceutical salt

The compounds according to the present invention may exist in the form of their pharmaceutically acceptable salts. The term "pharmaceutically acceptable salt" refers to conventional acid-addition salts or base-addition salts that retain the biological effectiveness and properties of the compounds of the present invention and are formed from suitable nontoxic organic or inorganic acids or organic or inorganic bases. Acid-addition salts include for example those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid, salicylic acid, methanesulfonic acid, oxalic acid, succinic acid, citric acid, malic acid, lactic acid, fumaric acid, and the like. Base-addition salts include those derived from ammonium, potassium, sodium and, quaternary ammonium hydroxides, such as for example, tetramethyl ammonium hydroxide. The chemical modification of a pharmaceutical compound into a salt is a technique well known to pharmaceutical chemists in order to obtain improved physical and chemical stability, hygroscopicity, flowability and solubility of compounds. It is for example described in Bastin, Organic Process Research & Development 2000, 4, 427-435 or in Ansel, In: Pharmaceutical Dosage Forms and Drug Delivery Systems, 6th ed. (1995), pp. 196 and 1456-1457. For example, the pharmaceutically acceptable salt of the compounds provided herein may be a sodium salt.

In a further aspect the invention provides a pharmaceutically acceptable salt of the antisense oligonucleotide or a conjugate thereof. In a preferred embodiment, the pharmaceutically acceptable salt is a sodium or a potassium salt.

### Pharmaceutical Composition

In a further aspect, the invention provides pharmaceutical compositions comprising any of the aforementioned oligonucleotides of the invention and/or oligonucleotide conjugates or salts thereof of the invention and a pharmaceutically acceptable diluent, carrier, salt and/or adjuvant. A pharmaceutically acceptable diluent includes phosphate-buffered saline (PBS) and pharmaceutically acceptable salts include, but are not limited to, sodium and potassium salts. In some embodiments the pharmaceutically acceptable diluent is sterile phosphate buffered saline. In some embodiments the oligonucleotide is used in the pharmaceutically acceptable diluent at a concentration of 50 - 300µM solution.

Suitable formulations for use in the present invention are found in Remington's Pharmaceutical Sciences, Mack Publishing Company, Philadelphia, Pa., 17th ed., 1985. For a brief review of methods for drug delivery, see, e.g., Langer (Science 249:1527-1533, 1990). WO 2007/031091 provides further suitable and preferred examples of pharmaceutically acceptable diluents, carriers and adjuvants. Suitable dosages, formulations, administration routes, compositions, dosage forms, combinations with other therapeutic agents, pro-drug formulations are also provided in WO2007/031091.

Oligonucleotides or oligonucleotide conjugates of the invention may be mixed with pharmaceutically acceptable active or inert substances for the preparation of pharmaceutical compositions or formulations. Compositions and methods for the formulation of pharmaceutical compositions are dependent upon a number of criteria, including, but not limited to, route of administration, extent of disease, or dose to be administered.

These compositions may be sterilized by conventional sterilization techniques, or may be sterile filtered. The resulting aqueous solutions may be packaged for use as is, or lyophilized, the lyophilized preparation being combined with a sterile aqueous carrier prior to administration. The pH of the preparations typically will be between 3 and 11, more preferably between 5 and 9 or between 6 and 8, and most preferably between 7 and 8, such as 7 to 7.5. The resulting compositions in solid form may be packaged in multiple single dose units, each containing a fixed amount of the above-mentioned agent or agents, such as in a sealed package of tablets or capsules. The composition in solid form can also be packaged in a container for a flexible quantity, such as in a squeezable tube designed for a topically applicable cream or ointment.

In some embodiments, the oligonucleotide or oligonucleotide conjugate of the invention is a prodrug. In particular, with respect to oligonucleotide conjugates the conjugate moiety is cleaved off the oligonucleotide once the prodrug is delivered to the site of action, e.g. the target cell.

### Applications

References to methods of treatment by therapy in this description are to be interpreted as references to compounds, pharmaceutical compositions, and medicaments of the present invention for use in those methods.

The oligonucleotides of the invention may be utilized as research reagents for, for example, diagnostics, therapeutics and prophylaxis.

In research, such oligonucleotides may be used to specifically modulate the synthesis of Tau protein in cells (e.g. *in vitro* cell cultures) and experimental animals thereby facilitating functional analysis of the target or an appraisal of its usefulness as a target for therapeutic intervention. Typically, the target modulation is achieved by degrading or inhibiting the mRNA producing the protein, thereby prevent protein formation or by degrading or inhibiting a modulator of the gene or mRNA producing the protein.

If employing the oligonucleotide of the invention in research or diagnostics the target nucleic acid may be a cDNA or a synthetic nucleic acid derived from DNA or RNA.

The present invention provides an *in vitro* method for modulating Tau expression in a target cell which is expressing Tau, said method comprising administering an oligonucleotide of the invention in an effective amount to said cell.

In some embodiments, the target cell, is a mammalian cell in particular a human cell. The target cell may be an *in vitro* cell culture. In preferred embodiments the target cell is present in the brain or central nervous system. In particular cells in the brain stem, cerebellum, cerebal cortex, frontal cortex, medulla/pons and midbrain and spinal cord are relevant target regions. For the treatment of progressive supranuclear palsy (PSP) target reduction in the brain regions medulla/pons and midbrain are advantageous. For the treatment of Alzheimer target reduction in the brain regions cerebal cortex, medulla/pons and midbrain are advantageous. In particular, in neurons, nerves cells, axons and basal ganglia are relevant cell types.

In diagnostics the oligonucleotides may be used to detect and quantitate MAPT expression in cell and tissues by northern blotting, *in-situ* hybridisation or similar techniques.

For therapeutics, the oligonucleotides may be administered to an animal or a human, suspected of having a disease or disorder, which can be treated by modulating the expression of Tau.

The invention provides methods for treating or preventing a disease, comprising administering a therapeutically or prophylactically effective amount of an oligonucleotide, an oligonucleotide conjugate or a pharmaceutical composition of the invention to a subject suffering from or susceptible to the disease.

The invention also relates to an oligonucleotide, a composition or a conjugate as defined herein for use as a medicament.

The oligonucleotide, oligonucleotide conjugate or a pharmaceutical composition according to the invention is typically administered in an effective amount.

Decribed herein is the use of the oligonucleotide or oligonucleotide conjugate of the invention as described for the manufacture of a medicament for the treatment of a disorder as referred to herein, or for a method of the treatment of as a disorder as referred to herein.

The disease or disorder, as referred to herein, is associated with expression of Tau. In some embodiments disease or disorder may be associated with a mutation in the Tau gene or a gene whose protein product is associated with or interacts with Tau. Therefore, in some embodiments, the target nucleic acid is a mutated form of the Tau sequence and in other examples, the target nucleic acid is a regulator of the Tau sequence.

The methods of the invention are preferably employed for treatment or prophylaxis against diseases caused by abnormal levels and/or activity of Tau.

Described herein is the use of an oligonucleotide, oligonucleotide conjugate or a pharmaceutical composition as defined herein for the manufacture of a medicament for the treatment of abnormal levels and/or activity of Tau.

In one embodiment, the invention relates to oligonucleotides, oligonucleotide conjugates or pharmaceutical compositions for use in the treatment of diseases or disorders selected from wherein the disease is selected from Tauopathies, Alzheimer's disease (AD), progressive supranuclear palsy (PSP), corticobasal ganglionic degeneration (CBD), chronic traumatic encephalopathy (CTE), fronto- temporal dementia (FTD), FTDP-17, Pick's disease (PiD), argyrophilic grain disease (AGD), tangle-predominant senile dementia (TPSD), primary age-related Tauopathy (PART), Down syndrome, lytico-bodig disease, infantile Tauopathies including hemimegalencephaly (HME), tuberous sclerosis complex, focal cortical dysplasia type 2b, ganglioglioma, Hallervorden-Spatz syndrome, neurodegeneration with brain iron accumulation type 1 (NBIA1), gangliocytomas, subacute sclerosing panencephalitis, seizure disorders (e.g., epilepsy), network dysfunction (e.g., depression) and movement disorders (e.g., Parkinson's disease).

In certain embodiments the disease is selected from Alzheimer's disease (AD), progressive supranuclear palsy (PSP), fronto- temporal dementia (FTD) or FTDP-17.

### Administration

The oligonucleotides or pharmaceutical compositions of the present invention may be administered via parenteral (such as, intravenous, subcutaneous, intra-muscular, intracerebral, intracerebroventricular intraocular, or intrathecal administration).

In some examples, the administration is via intrathecal administration.

Advantageously, e.g. for treatment of neurological disorders, the oligonucleotide or pharmaceutical compositions of the present invention are administered intrathecally or intracranially, e.g. via intracerebral or intraventricular administration.

Described herein is the use of the oligonucleotide or conjugate thereof, such as pharmaceutical salts or compositions of the invention, for the manufacture of a medicament wherein the medicament is in a dosage form for subcutaneous administration.

Described herein is the use of the oligonucleotide of the invention, or conjugate thereof, such as pharmaceutical salts or compositions of the invention, for the manufacture of a medicament wherein the medicament is in a dosage form for intrathecal administration.

Described herein is the use of the oligonucleotide or oligonucleotide conjugate of the invention as described for the manufacture of a medicament wherein the medicament is in a dosage form for intrathecal administration.

### Combination therapies

In some embodiments the oligonucleotide, oligonucleotide conjugate or pharmaceutical composition of the invention is for use in a combination treatment with another therapeutic agent. The therapeutic agent can for example be the standard of care for the diseases or disorders described above.

### EXAMPLES

### Materials and methods

### Oligonucleotide motif sequences and oligonucleotide compounds

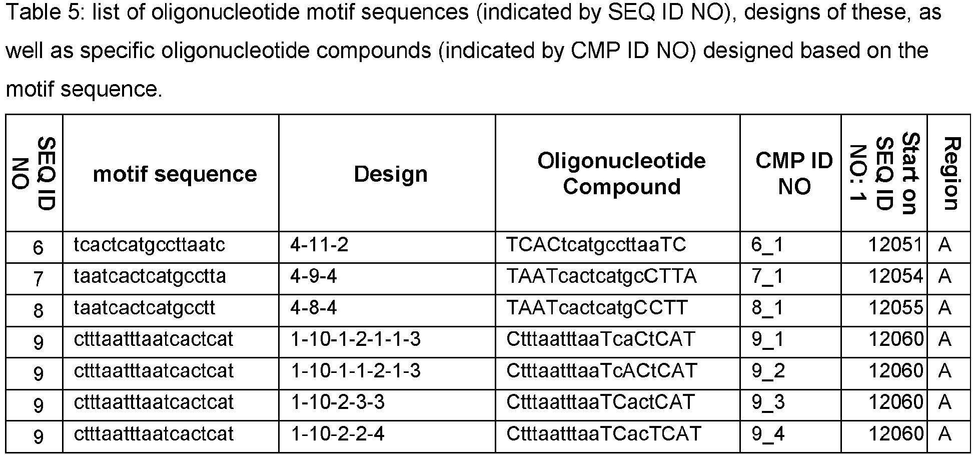

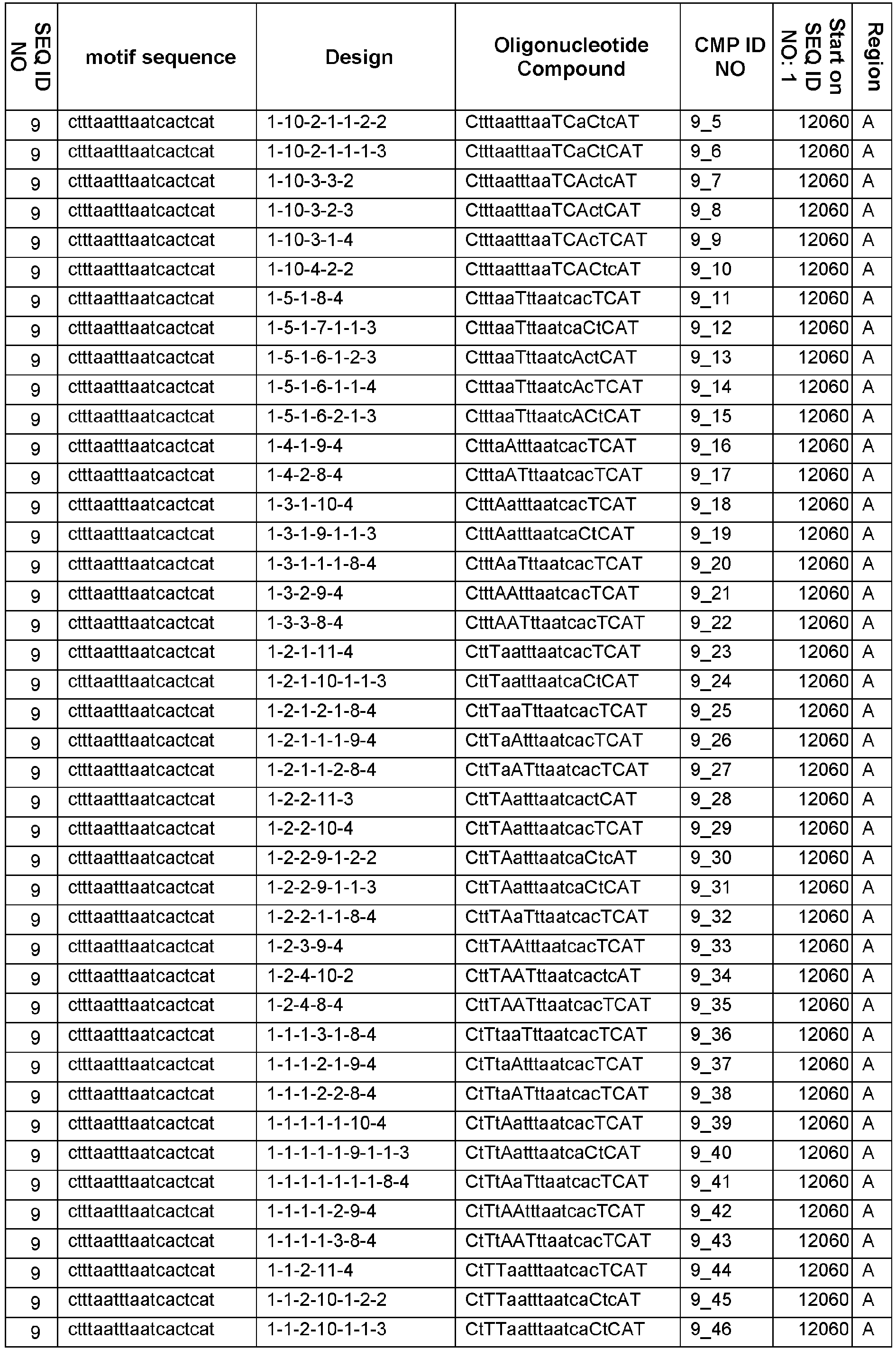

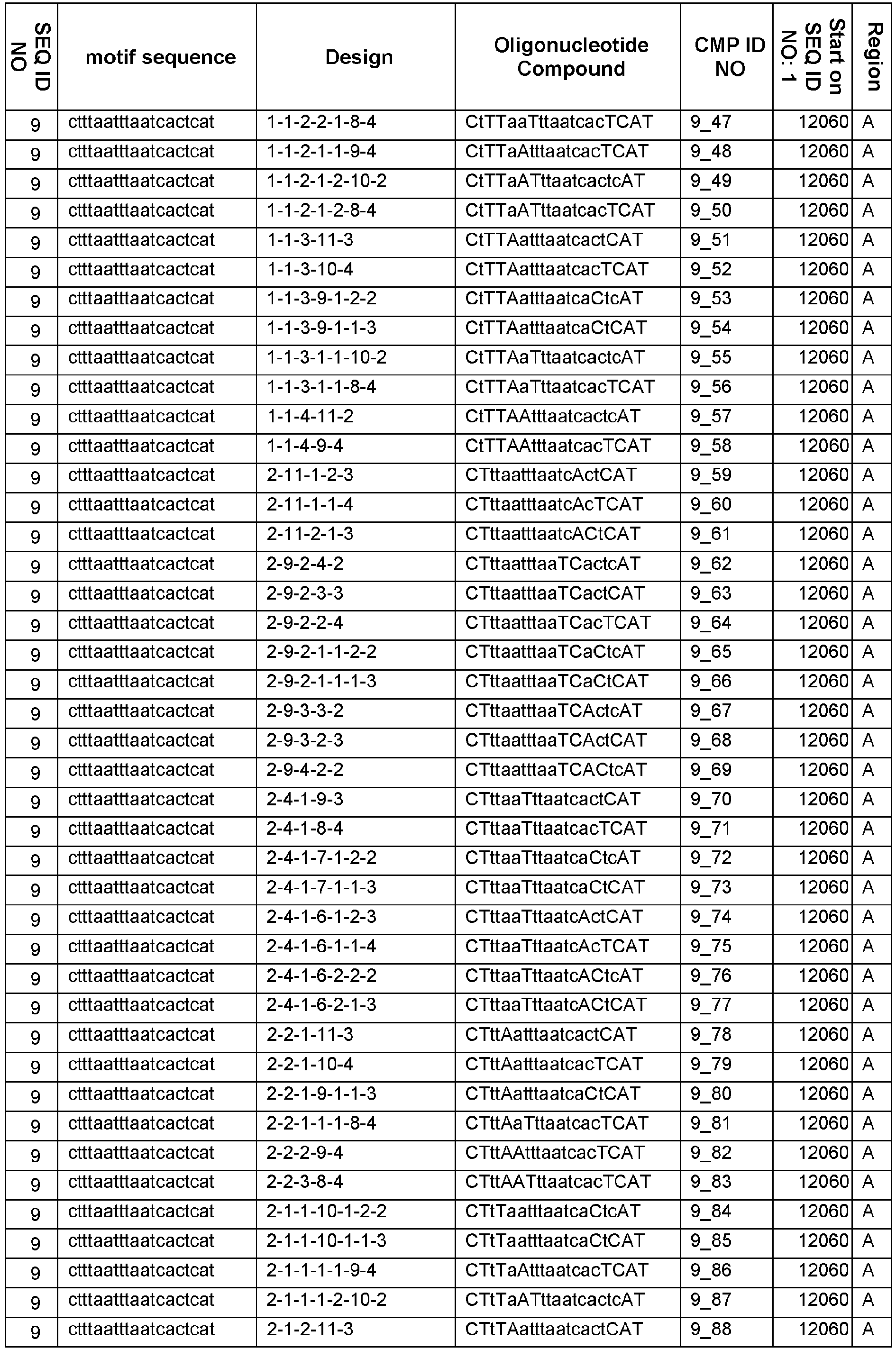

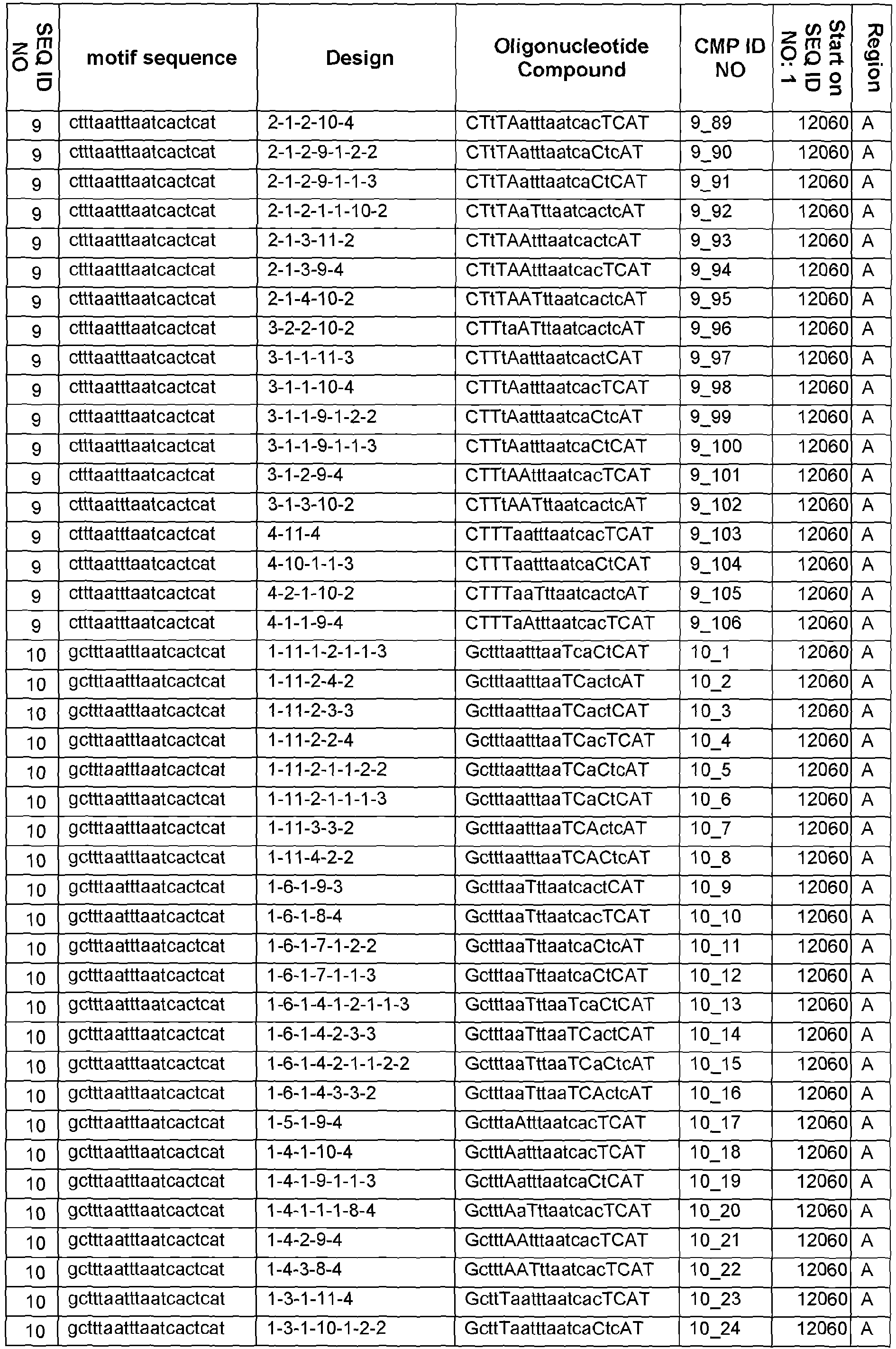

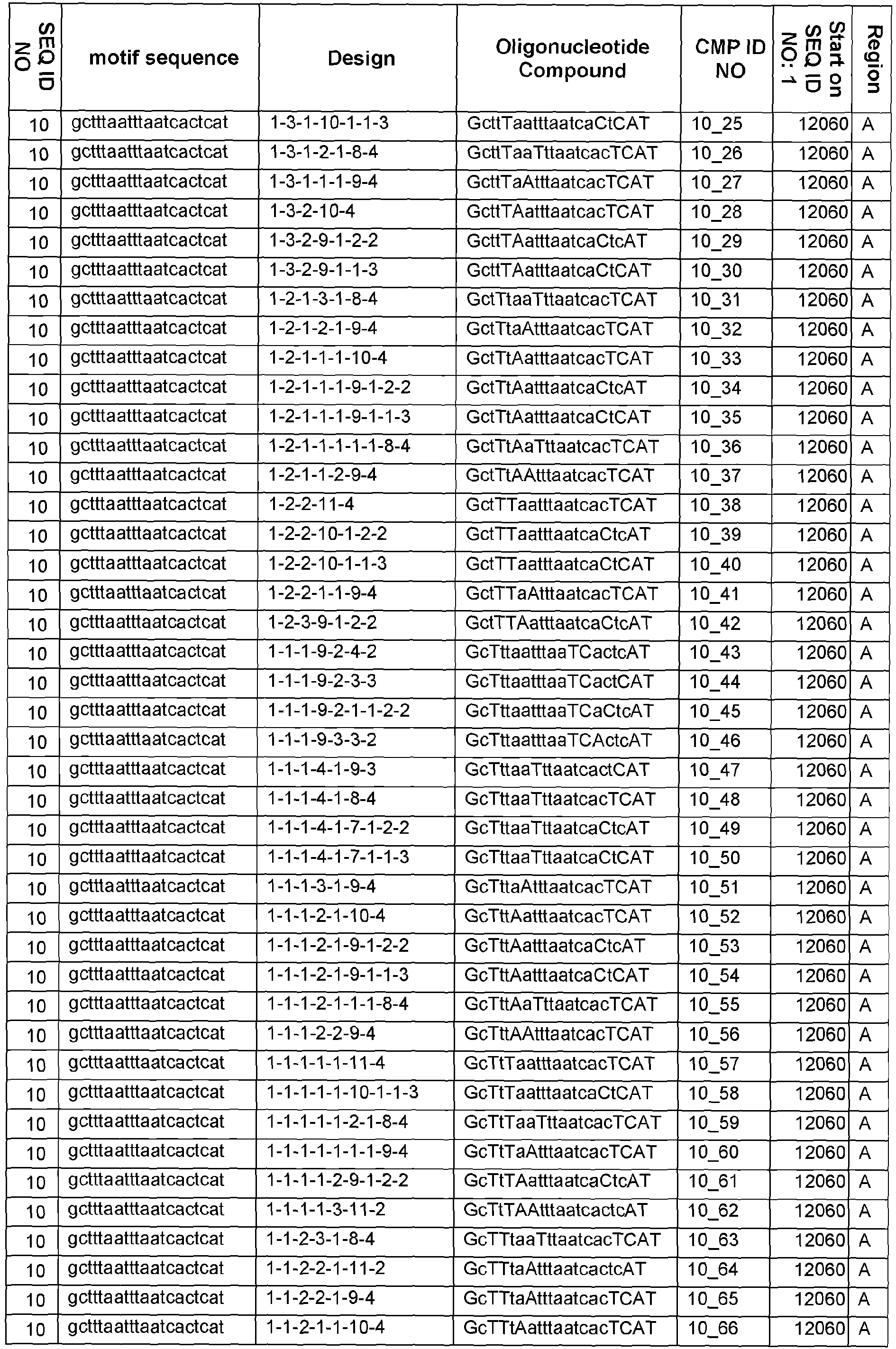

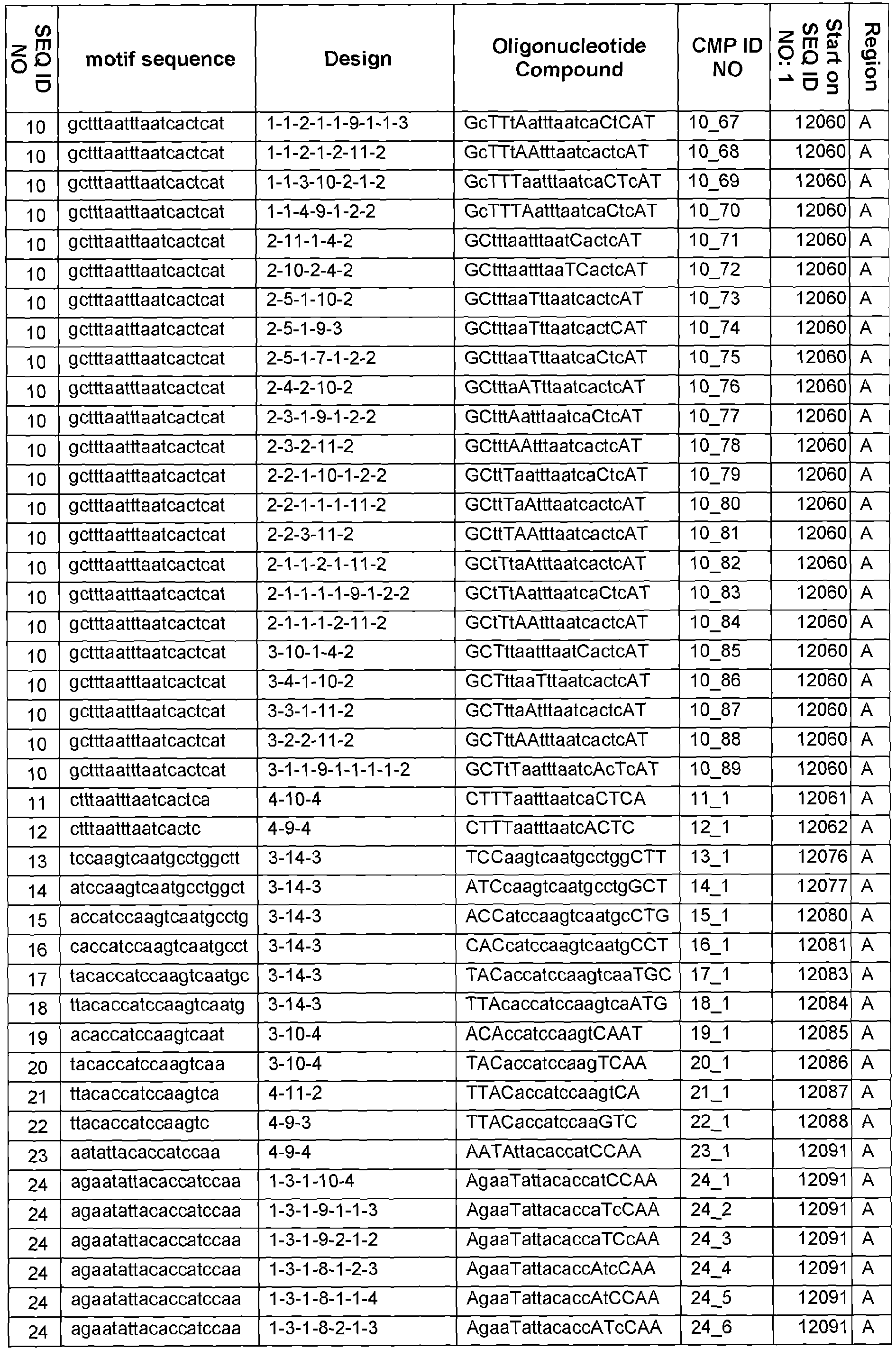

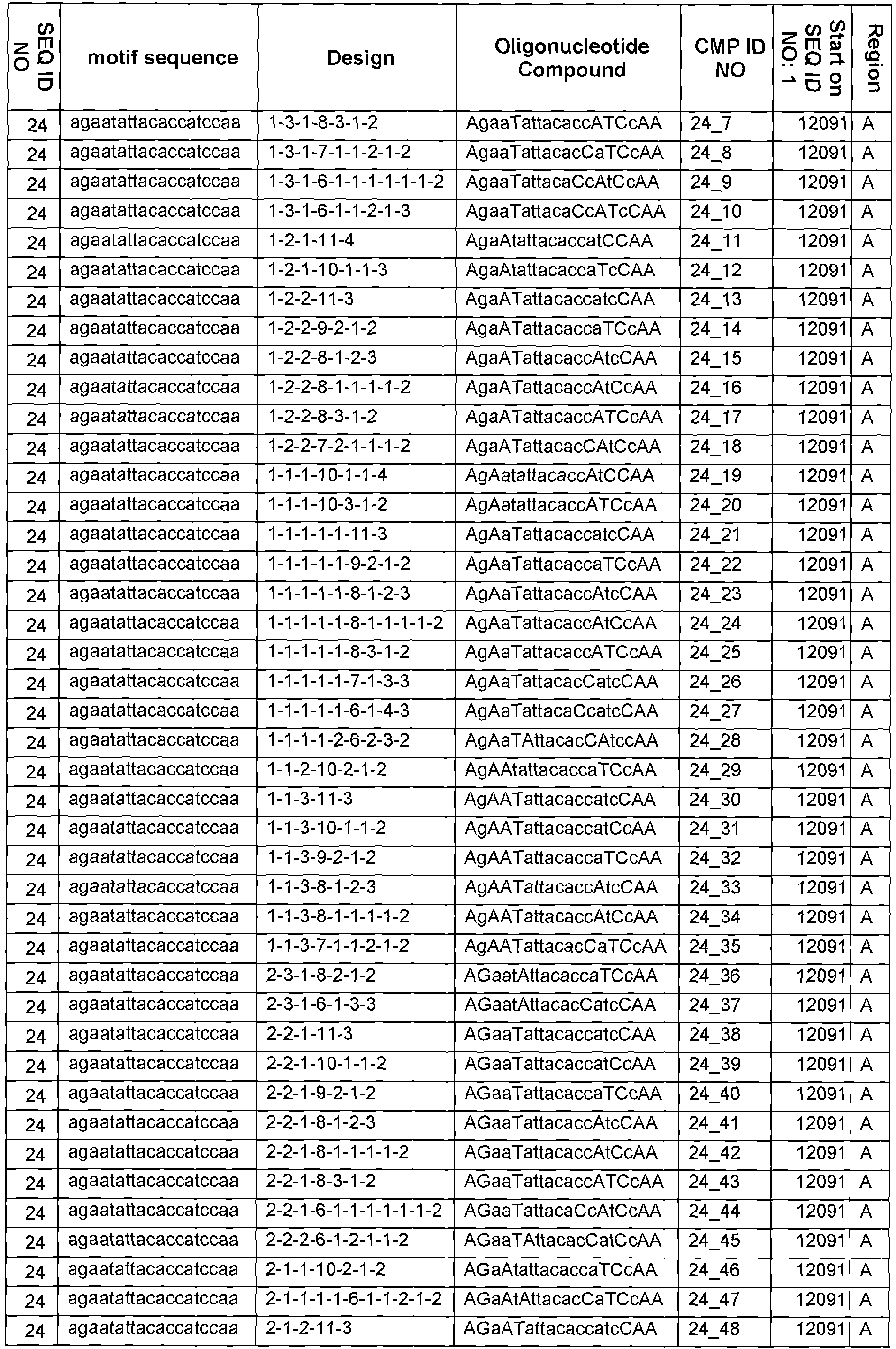

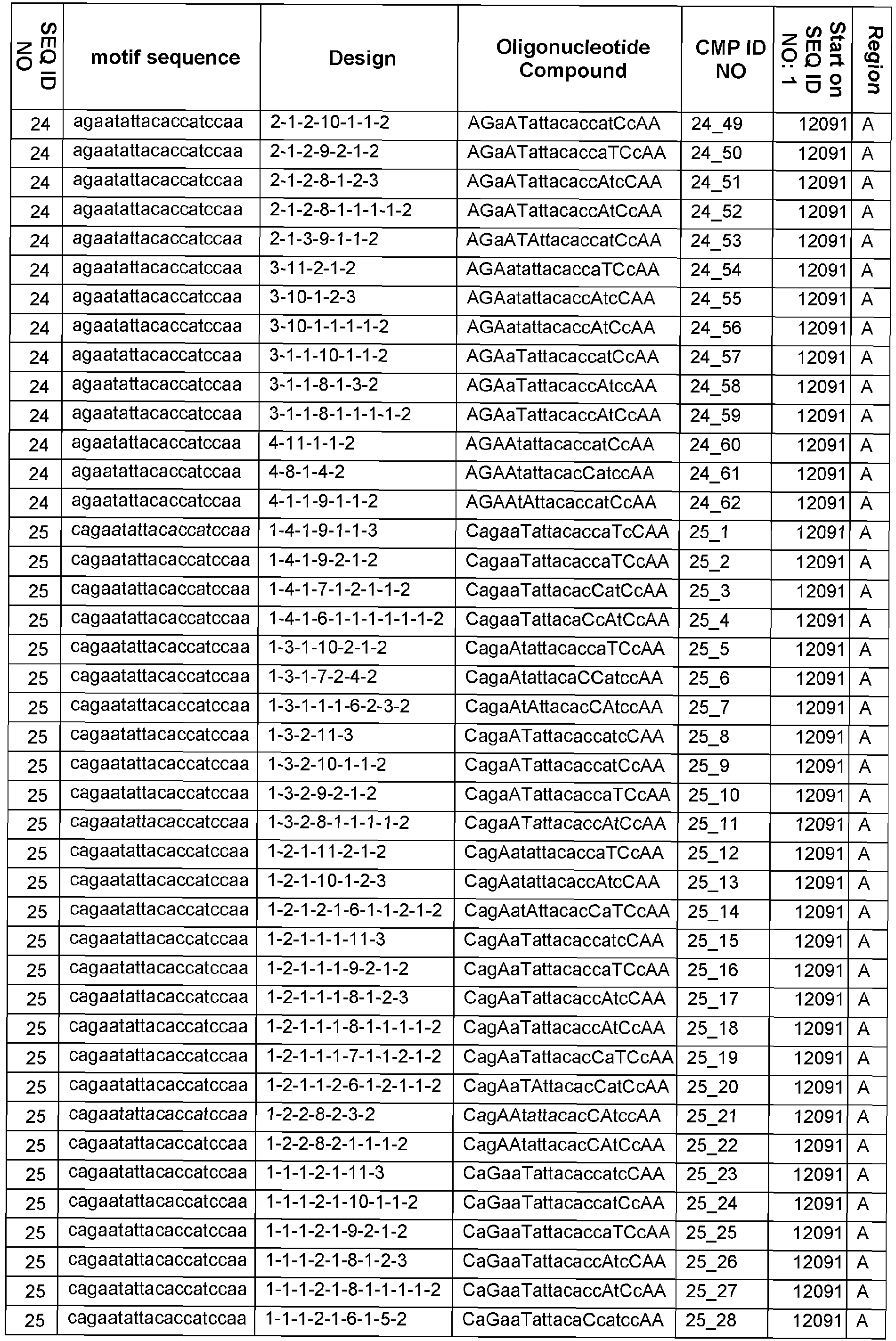

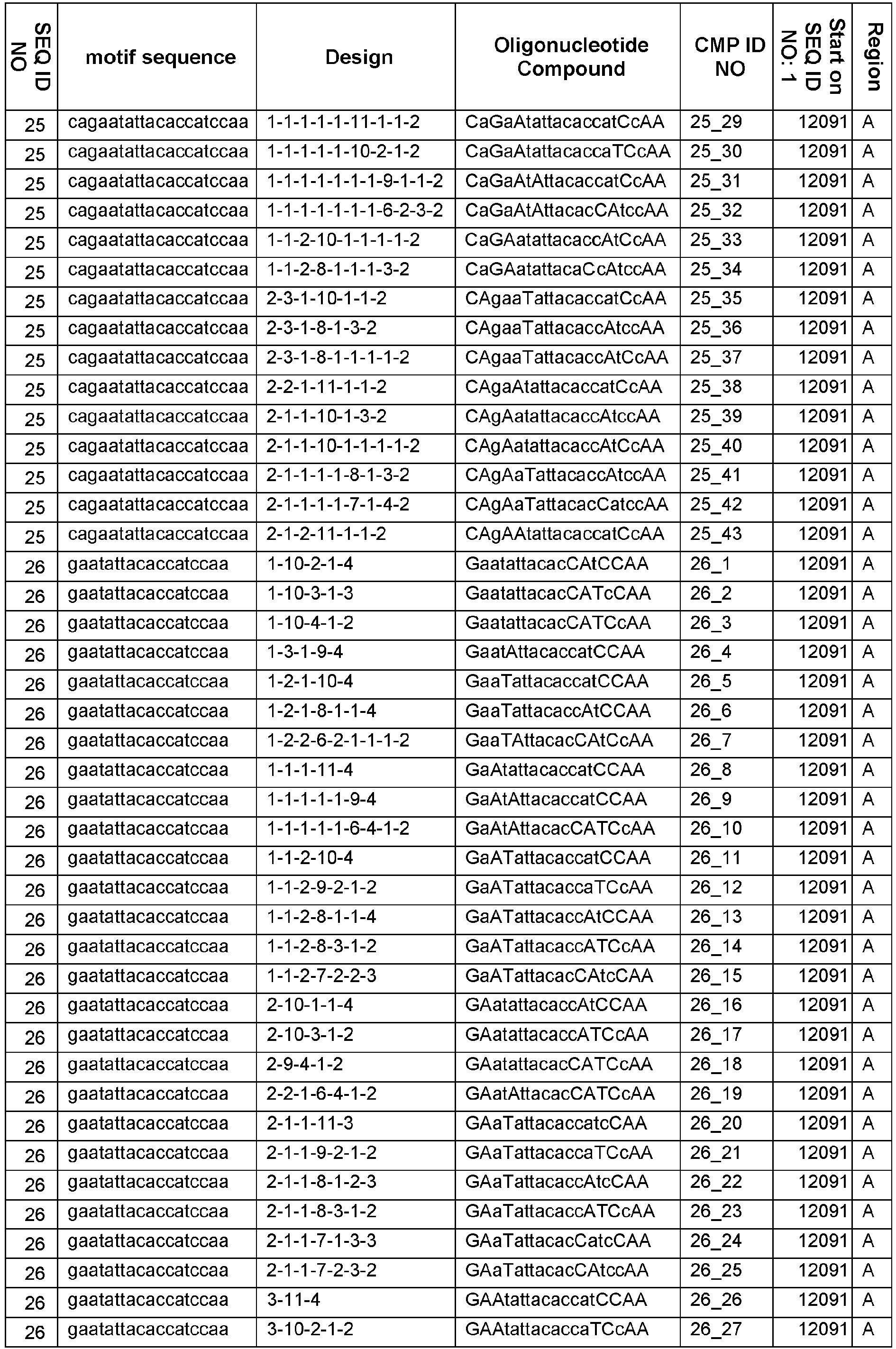

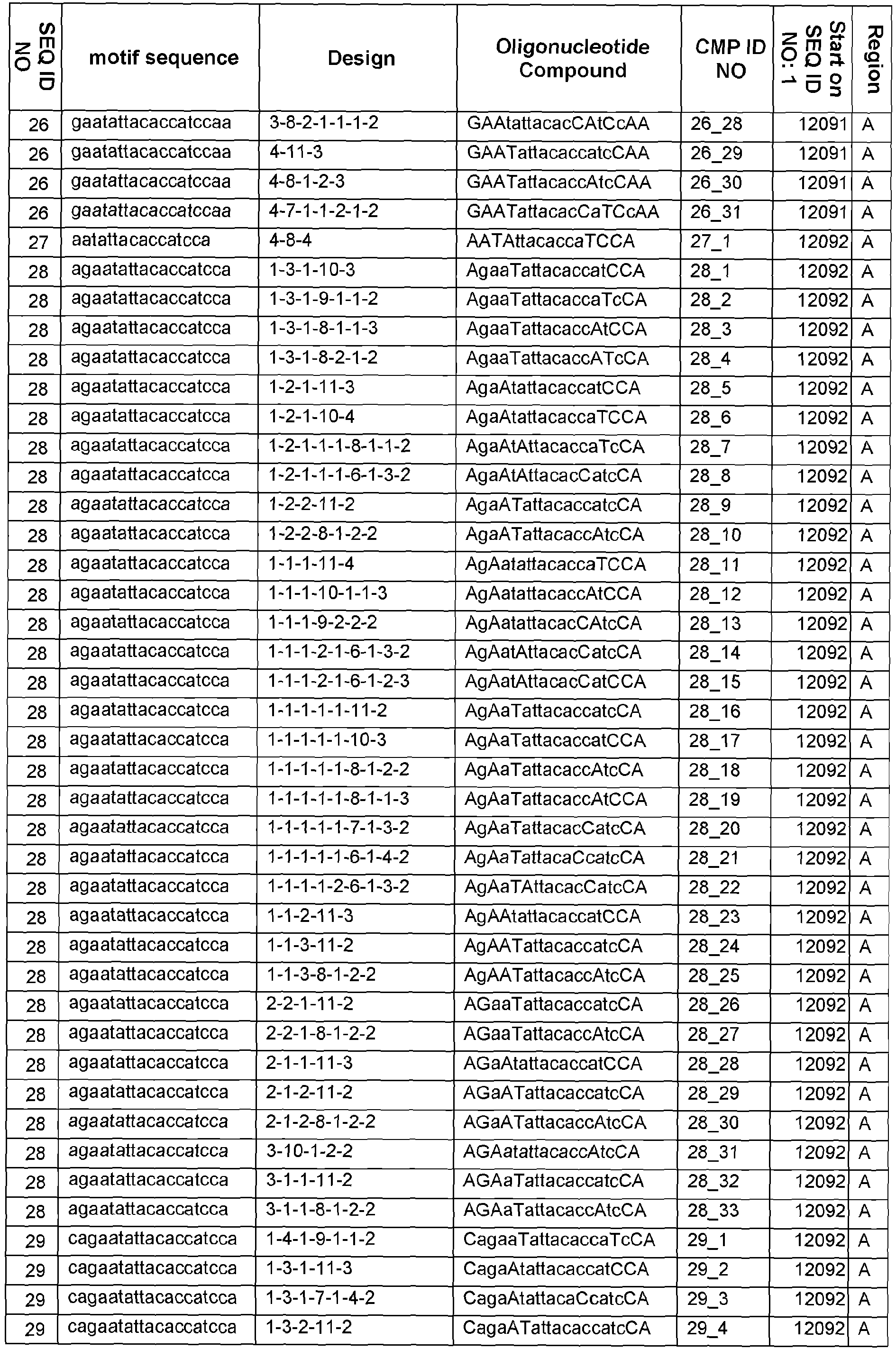

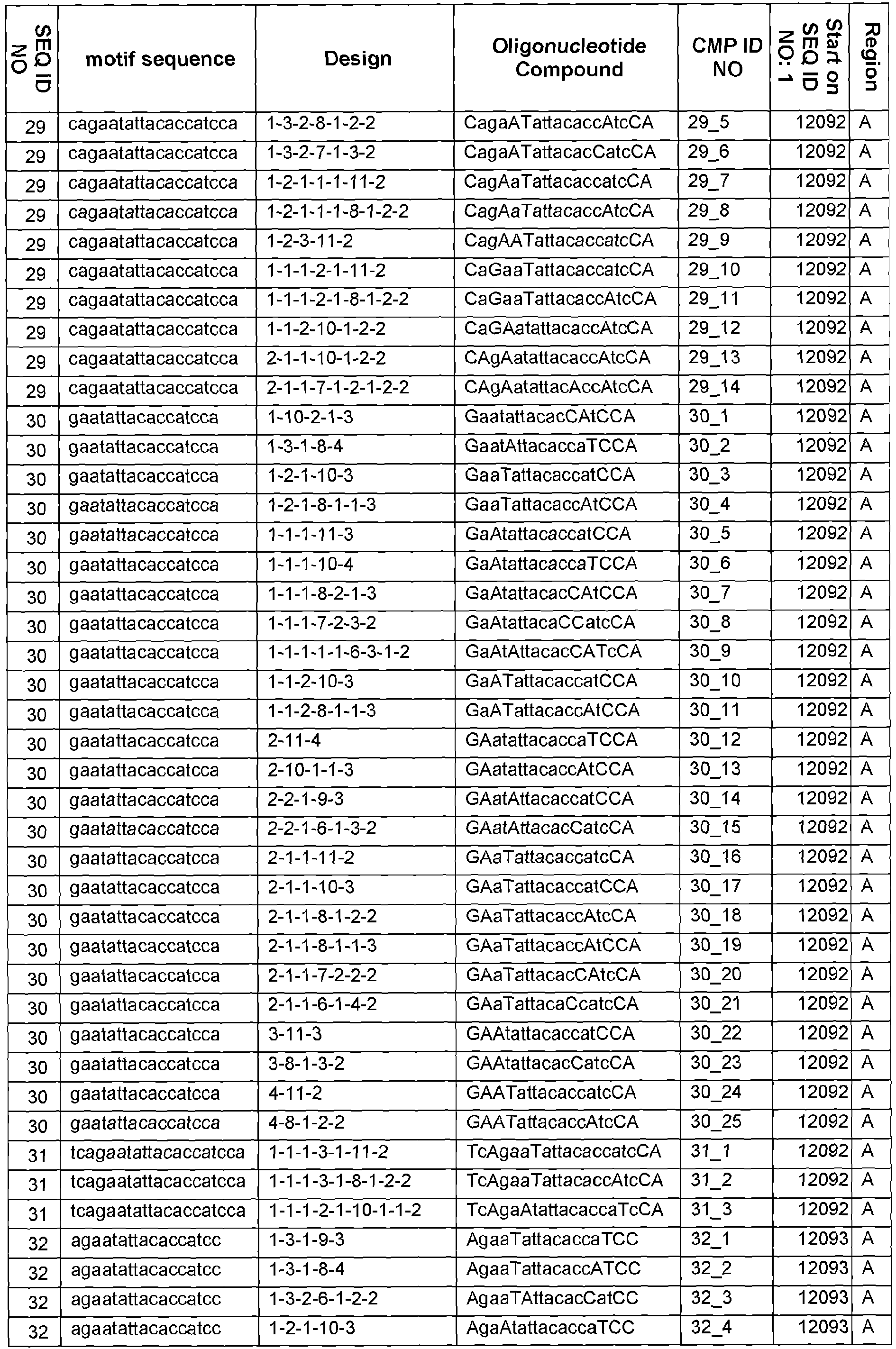

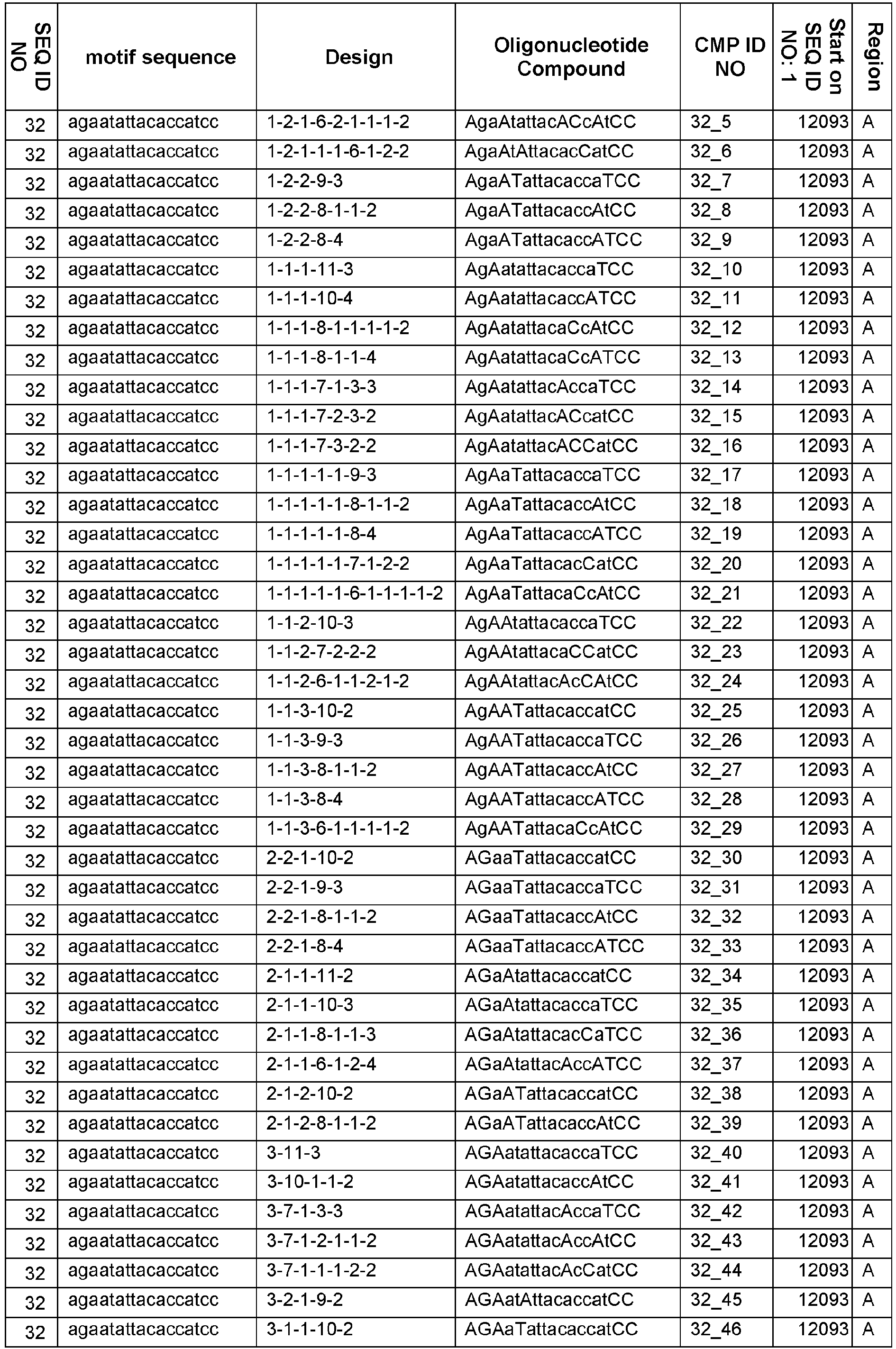

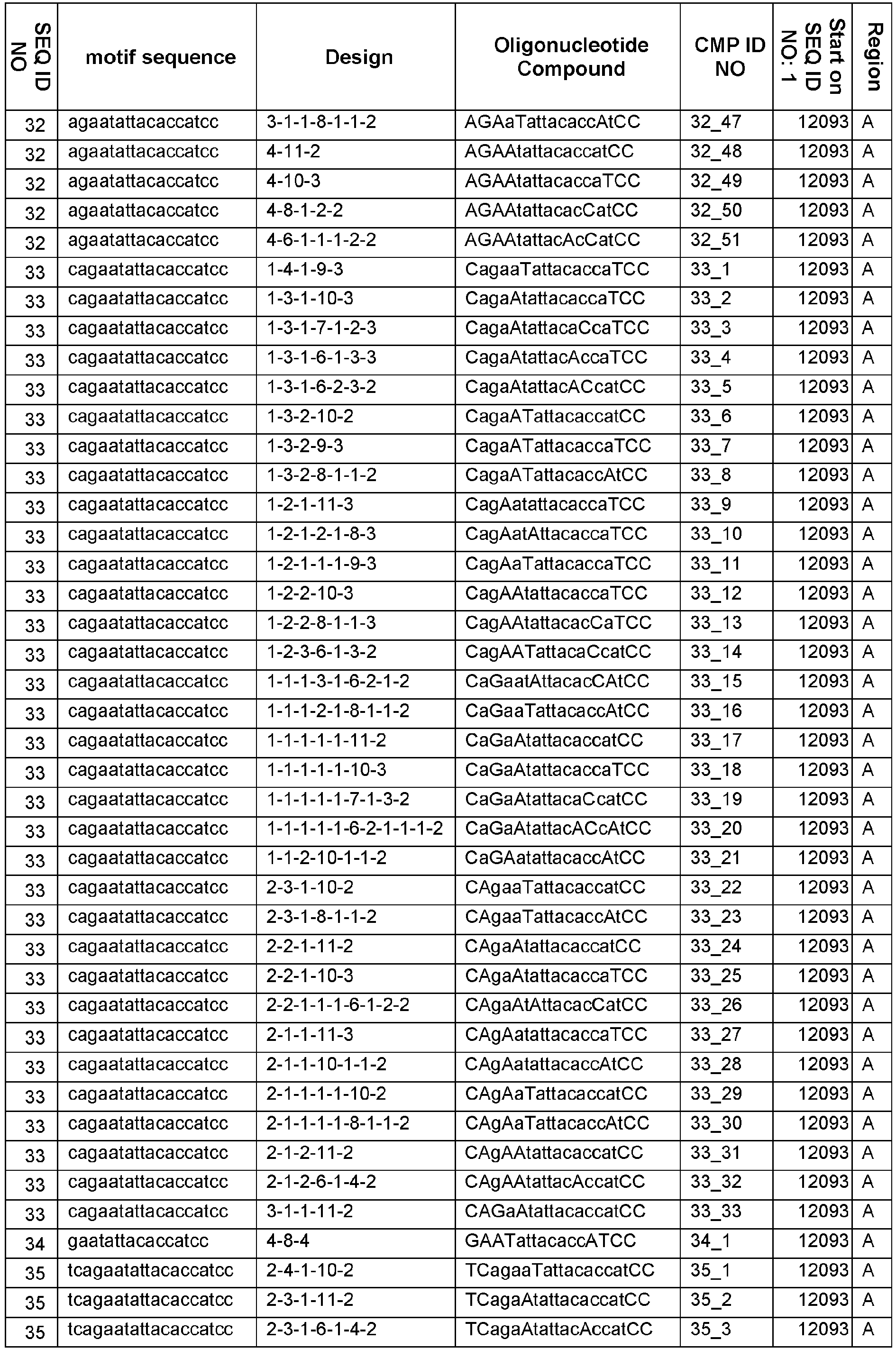

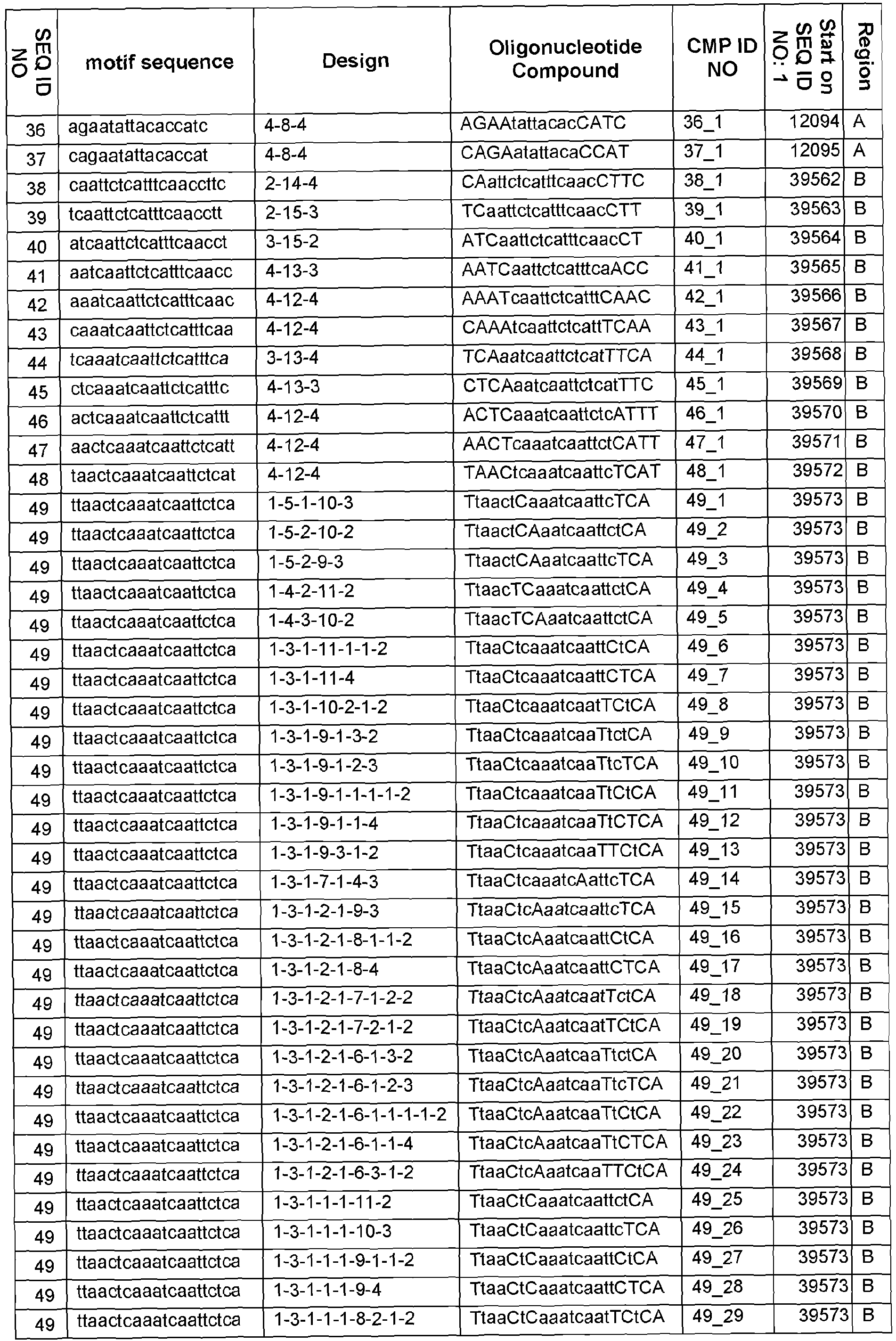

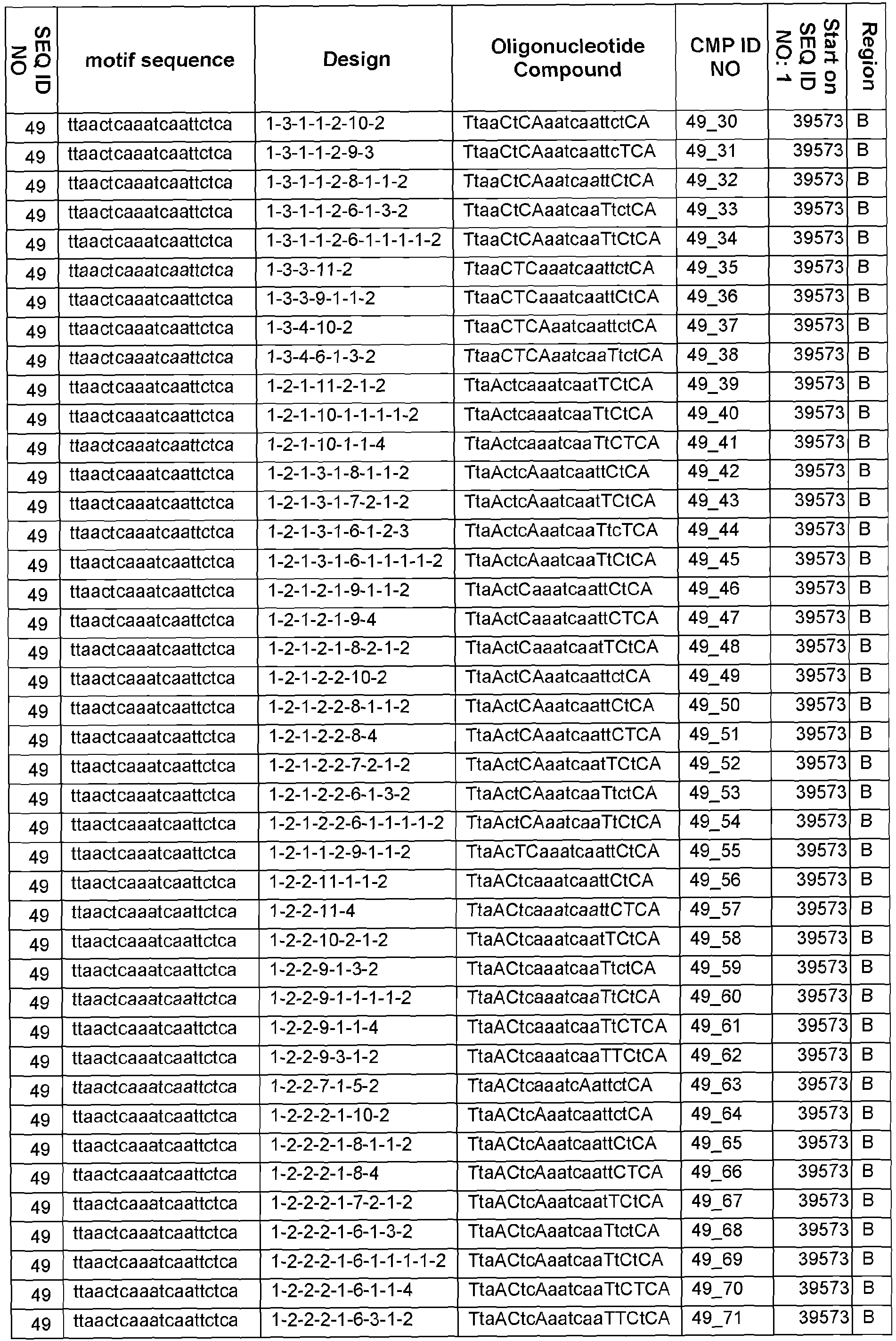

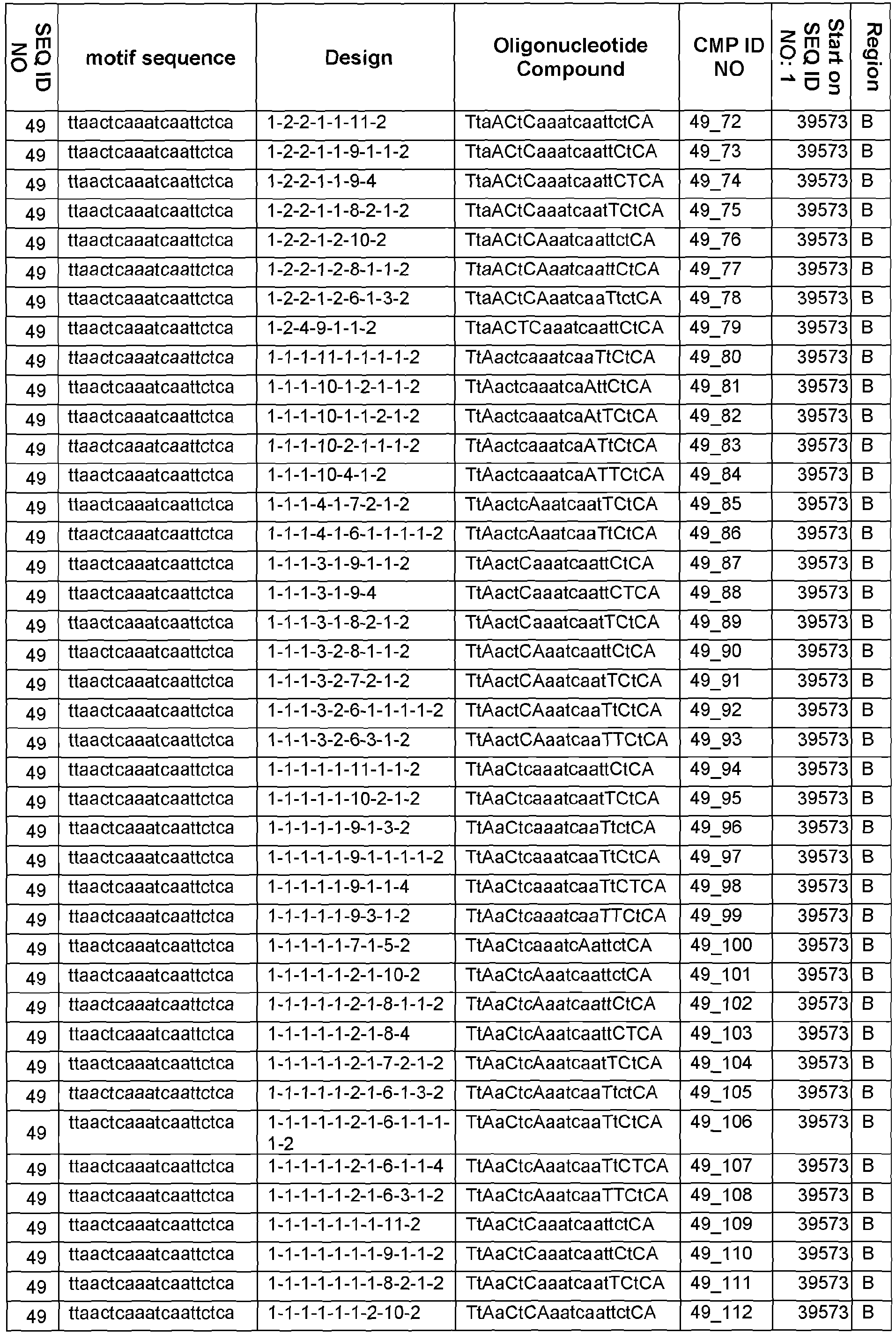

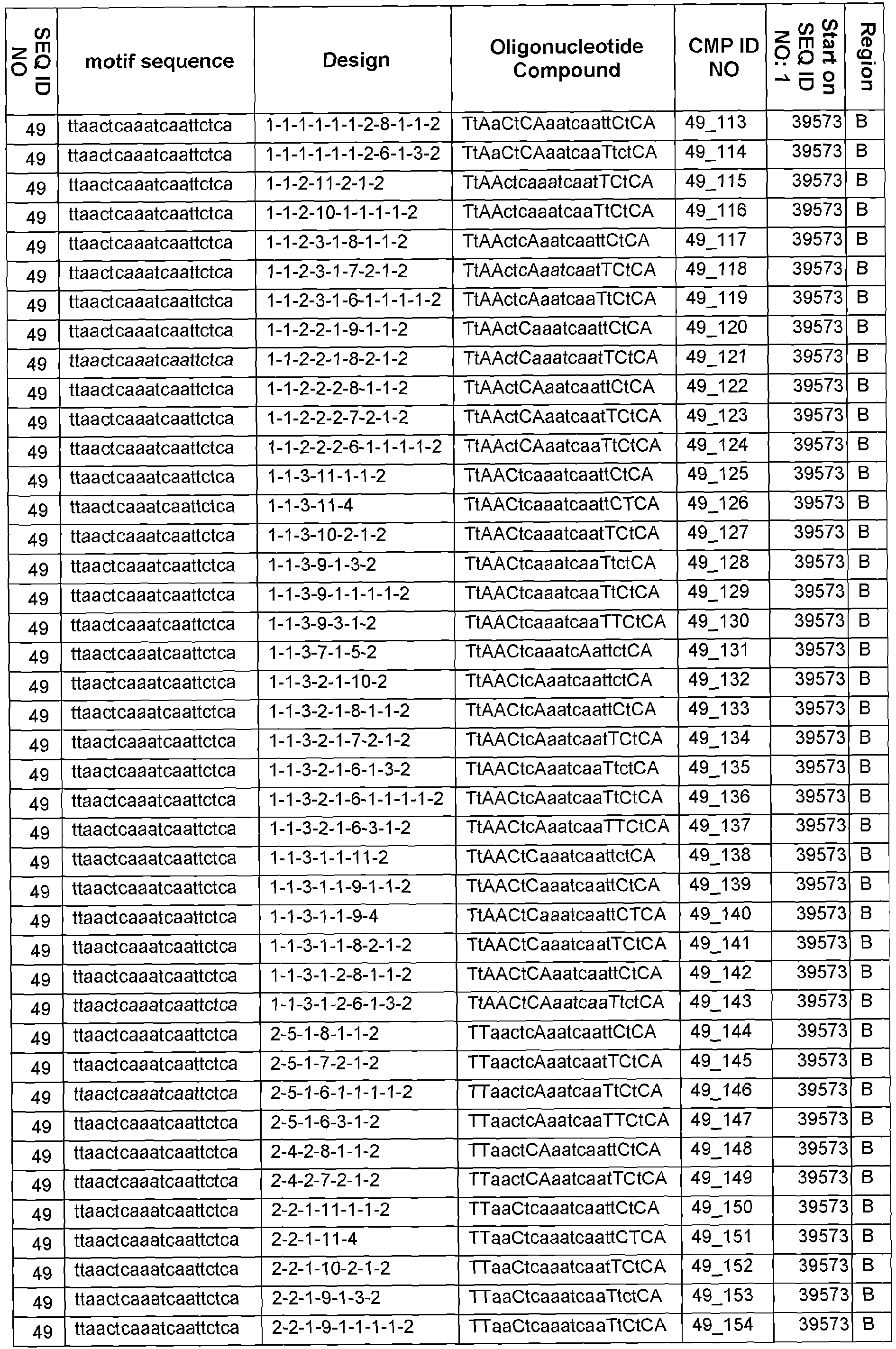

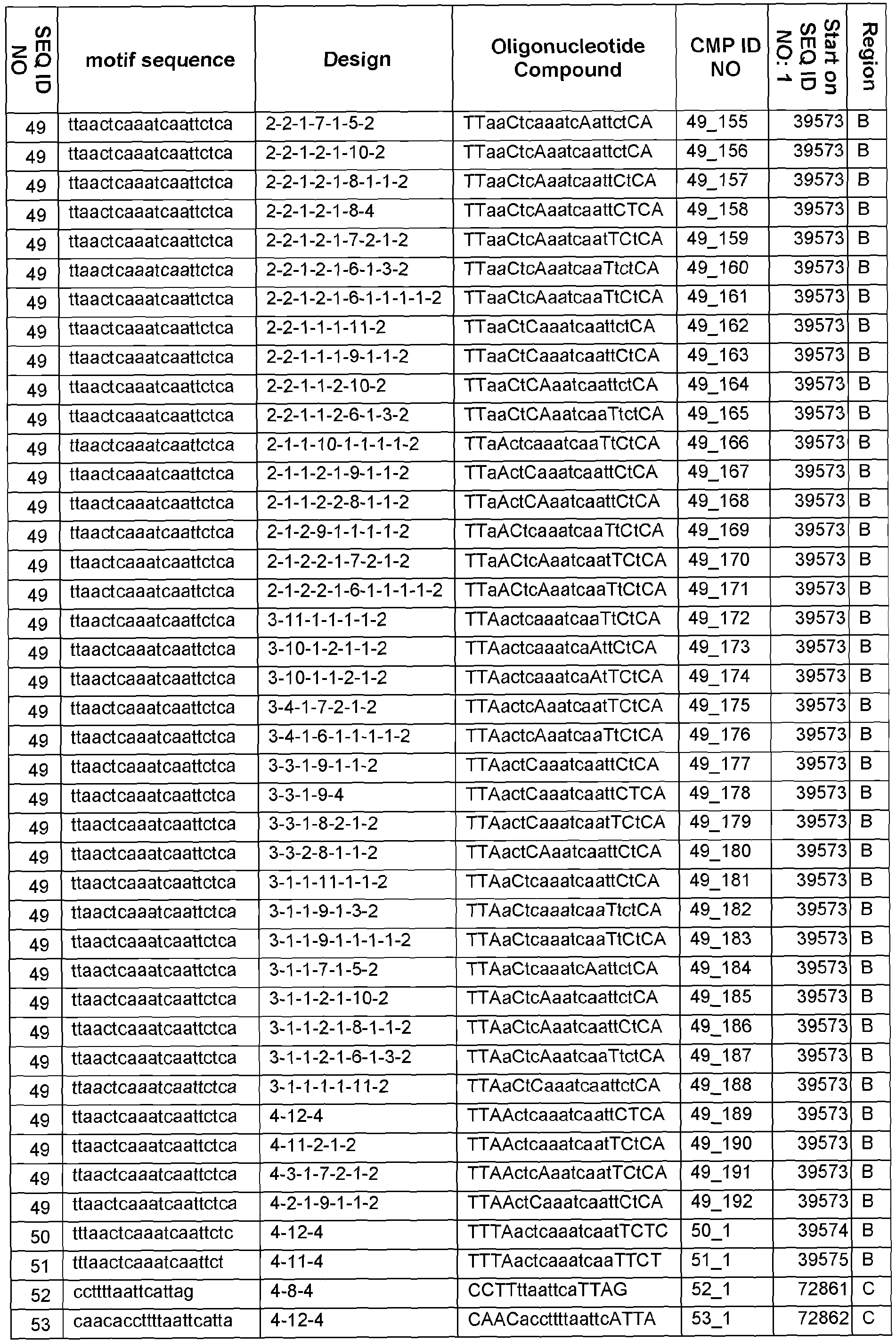

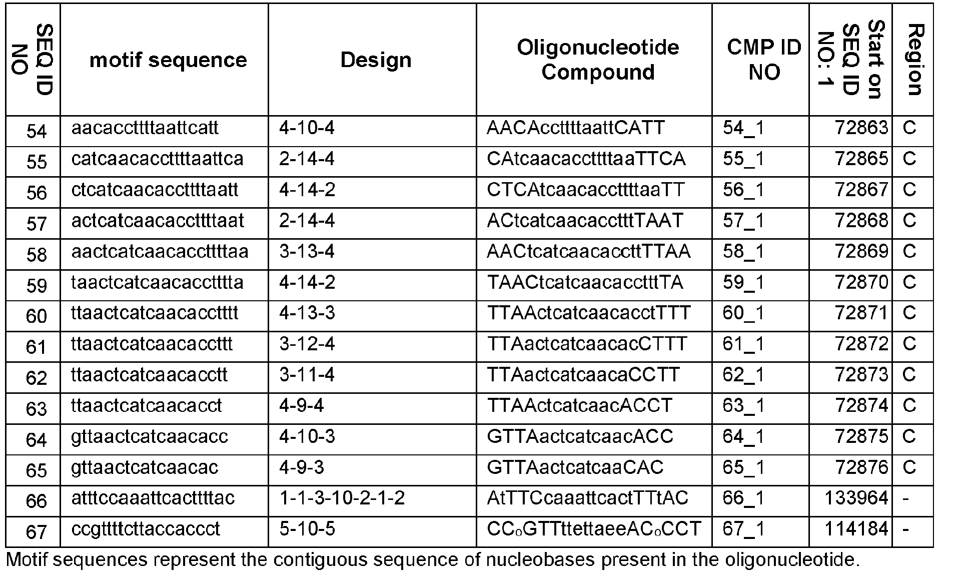

Designs refer to the gapmer design, F-G-F'. In classic gapmer design e.g. 3-10-3 all the nucleotides in the flanks (F and F') are constituted of the same 2'-sugar modified nucleoside, e.g. LNA, cET, or MOE, and a stretch of DNA in the middle forming the gap (G). In gapmers with alternating flank designs the flanks of oligonucleotide is annotated as a series of integers, representing a number of 2' sugar modified nucleosides (M) followed by a number of DNA nucleosides (D). For example a flank with a 2-2-1 motif represents 5' [M]₂-[D]₂-[M] 3' and a 1-1-1-1-1 motif represents 5' [M]-[D]-[M]-[D]-[M] 3'. Both flanks have a 2' sugar modified nucleoside at the 5' and 3' terminal. The gap region (G), which is constituted of a number of DNA nucleosides (typically between 6 and 16), is located between the flanks.

The heading "Oligonucleotide compound" in the table represents a specific design of the motif sequence. Capital letters represent beta-D-oxy LNA nucleosides, Underlined capital letter represent MOE nucleosides, lowercase letters represent DNA nucleosides, all LNA C are 5-methyl cytosine, e represents a 5-methyl cytosine DNA, all internucleoside linkages are phosphorothioate internucleoside linkages unless marked by a subscript letter between the nucleotides, subscript o represents a phosphodiester linkage.

### Oligonucleotide synthesis

Oligonucleotide synthesis is generally known in the art. Below is a protocol which may be applied. The oligonucleotides of the present invention may have been produced by slightly varying methods in terms of apparatus, support and concentrations used.

Oligonucleotides are synthesized on uridine universal supports using the phosphoramidite approach on an Oligomaker 48 at 1 µmol scale. At the end of the synthesis, the oligonucleotides are cleaved from the solid support using aqueous ammonia for 5-16hours at 60°C. The oligonucleotides are purified by reverse phase HPLC (RP-HPLC) or by solid phase extractions and characterized by UPLC, and the molecular mass is further confirmed by ESI-MS.

### Elongation of the oligonucleotide:

The coupling of β-cyanoethyl- phosphoramidites (DNA-A(Bz), DNA- G(ibu), DNA- C(Bz), DNA-T, LNA-5-methyl-C(Bz), LNA-A(Bz), LNA- G(dmf), or LNA-T) is performed by using a solution of 0.1 M of the 5'-O-DMT-protected amidite in acetonitrile and DCI (4,5-dicyanoimidazole) in acetonitrile (0.25 M) as activator. For the final cycle a phosphoramidite with desired modifications can be used, e.g. a C6 linker for attaching a conjugate group or a conjugate group as such. Thiolation for introduction of phosphorthioate linkages is carried out by using xanthane hydride (0.01 M in acetonitrile/pyridine 9:1). Phosphodiester linkages can be introduced using 0.02 M iodine in THF/Pyridine/water 7:2:1. The rest of the reagents are the ones typically used for oligonucleotide synthesis.

For post solid phase synthesis conjugation a commercially available C6 amino linker phorphoramidite can be used in the last cycle of the solid phase synthesis and after deprotection and cleavage from the solid support the aminolinked deprotected oligonucleotide is isolated. The conjugates are introduced via activation of the functional group using standard synthesis methods.

### Purification by RP-HPLC:

The crude compounds are purified by preparative RP-HPLC on a Phenomenex Jupiter C18 10µ 150x10 mm column. 0.1 M ammonium acetate pH 8 and acetonitrile is used as buffers at a flow rate of 5 mL/min. The collected fractions are lyophilized to give the purified compound typically as a white solid.

### Abbreviations:

- DCI:: 4,5-Dicyanoimidazole
- DCM:: Dichloromethane
- DMF:: Dimethylformamide
- DMT:: 4,4'-Dimethoxytrityl
- THF:: Tetrahydrofurane
- Bz:: Benzoyl
- Ibu:: Isobutyryl
- RP-HPLC:: Reverse phase high performance liquid chromatography

### Tₘ Assay:

Oligonucleotide and RNA target (phosphate linked, PO) duplexes are diluted to 3 mM in 500 ml RNase-free water and mixed with 500 ml 2x Tₘ-buffer (200mM NaCl, 0.2mM EDTA, 20mM Naphosphate, pH 7.0). The solution is heated to 95°C for 3 min and then allowed to anneal in room temperature for 30 min. The duplex melting temperatures (Tₘ) is measured on a Lambda 40 UV/VIS Spectrophotometer equipped with a Peltier temperature programmer PTP6 using PE Templab software (Perkin Elmer). The temperature is ramped up from 20°C to 95°C and then down to 25°C, recording absorption at 260 nm. First derivative and the local maximums of both the melting and annealing are used to assess the duplex Tₘ.

### Primary neuronal cell cultures

Primary neuronal cultures were established from the forebrain of E18 transgenic mice expressing the human tau transgene on a mouse tau knockout background. (Andorfer et al. J Neurochem 86:582-590 (2003)). Primary neurons were generated by papain digestion according to manufacturer's protocol (Worthington Biochemical Corporation, LK0031050). Briefly, forebrains were dissected from hTau mouse E18 BAC-Tg embryos expressing the entire human microtubule-associated protein Tau (MAPT) gene on a murine MAPT-null background and were incubated at 37 °C for 30-45 minutes in papain/DNase/Earle's balanced salt solution (EBSS) solution. After trituration and centrifugation of cell pellet, the reaction was stopped by incubation with EBSS containing protease inhibitors, bovine serum albumin (BSA) and DNase. The cells were triturated and washed with Neurobasal (NB, Invitrogen) supplemented with 2% B-27, 100 µg/ml penicillin, 85 µg/ml streptomycin, and 0.5mM glutamine.

### Transgenic Tau mouse (hTau mouse)

Male and female transgenic mice (30-40g) expressing a tau transgene derived from a human PAC, H1 haplotype driven by the tau promoter (Polydoro et. al., J. Neurosci. (2009) 29(34): 10741-9), and in which the native mouse Tau gene was deleted, were used to assess tolerability, pharmacodynamic endpoints and tissue drug concentrations.

Animals were held in colony rooms maintained at constant temperature (21 ± 2°C) and humidity (50 ± 10%) and illuminated for 12 hours per day (lights on at 0600 hours). All animals had ad *libitum* access to food and water throughout the studies. Behavioral studies were conducted between 0700 and 1500 hours.

Intracerebroventricular (ICV) injections were performed using a Hamilton micro syringe fitted with a 27 or 30-gauge needle, according to the method of Haley and McCormick. The needle was equipped with a polyethylene guard at 2.5 mm from the tip in order to limit its penetration into the brain. Mice were anesthetized using isoflurane anesthetic (1.5-4%). The mouse to be injected was held by the loose skin at the back of the neck with the thumb and first fingers of one hand. Applying gentle but firm pressure, the head of the animal was then immobilized by pressing against a firm flat level surface. The needle tip was then inserted through the scalp and the skull, about 1 mm lateral and 1 mm caudal to bregma. Once the needle was positioned, ASO was given in a volume of 5 microliters in saline vehicle and injected into the right (or left) lateral ventricle over 20-30 seconds. The needle was left in place for 10 seconds before removal. This procedure requires no surgery or incision. Animals were warmed on heating pads until they recovered from the procedure.

3 days and/or 4 weeks post administration mice were sacrificed with isoflurane overdose followed by rapid decapitation and brain tissue (right, frontal cortical region) was collected on dry ice for later Tau qPCR.

### Media used for cell culturing and differentiation of human stem cell derived neurons N2B27+ SFA Media=N2B27 + S,F,A cytokines

| **Cytokines used** | **Ref** | **Provider** | **Stock** | **Final use in N2B27(dilution)** |
|---|---|---|---|---|
| SHH (sonic hedgehog) | 100-45 | Peprotech | 100 ug/ml in PBS +0.1%BSA | 1:500 (200 ng/ml) |
| FGF8 | 100-25 | Peprotech | 100 ug/ml in | 1:1000 (100 ng/ml) |
| AA (Aa2-P) | A8960 | Sigma | PBS +0.1% BSA 100 mM in DMEM:F12 | 1:1000 |

### N2B27+ BGAA Media=N2B27 + B,G,Aa,cA cytokines +P/S+Laminin

| **Cytokines used** | **Ref** | **Provider** | **Stock** | **Final use in N2B27(dilution)** |
|---|---|---|---|---|
| BDNF | 450-02 | Peprotech | 20 ug/ml in PBS +0.1%BSA | 1:1000 |
| GDNF | 450-10 | Peprotech | 10 ug/ml in PBS +0.1%BSA | 1:1000 |
| AA (Aa2-P) | A8960 | Sigma | 100 mM in DMEM:F12 | 1:1000 |
| cAMP | D 009 | BIOLOG Life Science | 200 mM in water | 1:400 |
| PenStrep | 15140-122 | Gibco | | 1% |
| Laminin | 11243217001 | Roche | 1mg/ml | 1:500 |

### Example 1 in vitro screening of ASO's targeting MAPT introns

An antisense oligonucleotide (ASO) screening was performed in primary neuronal cells from humanized Tau mice with 807 ASO's targeting the MAPT introns.

The ability of ASOs to reduce MAPT mRNA *in vitro* was measured by QUANTIGENE^{®} analysis. Each tau mRNA reduction was standardized by subtracting an assay background signal and normalizing each well via the housekeeping gene tubulin mRNA signal.

Primary neuronal cell cultures were prepared as described in the "Materials and Method" section and plated on poly-D-lysine coated 384 well plates at 10,000 cells per well and maintained in Neurobasal media containing B27, glutamax and Penicillin-Streptomycin. ASO's were diluted in water and added to cells at DIV01 to a final concentration of 0.5 µM. Following ASO addition, neurons were incubated at 37°C and 5% CO₂ for 5 days to achieve steady state reduction of mRNA. Media was removed and cells were washed 1X in DPBS. Measurement of lysate messenger RNA was performed using the QUANTIGENE^{®} 2.0 Reagent System (AFFYMETRIX^{®}), which quantitates RNA using a branched DNA-signal amplification method reliant on the specifically designed RNA capture probe set. The cells were lysed using working cell lysis buffer solution made by adding 50µl proteinase K to 5ml of pre-warmed Lysis mix and diluted to 1:4 final dilution with dH₂0. The working lysis buffer was added to the plate (45 µl/ well), triturated to mix, sealed and incubated for 30 min at 55°C. Following lysis the wells were stored at -80°C or assayed immediately.

Lysates were diluted in lysis mix dependent on the specific capture probe used (tau or tubulin). 27 µl/well total was then added to the capture plate (384 well polystyrene plate coated with capture probes). Working probe sets reagents were generated by combining 2.2 ml of nuclease-free water, 1.2 ml of lysis mixture, 184 µl blocking reagent, and 66.8 µl of specific 2.0 probe set human MAPT catalogue #15486 and mouse beta 3 tubulin, catalogue #SB-17245, per manufacturer instructions (QUANTIGENE^{®} 2.0 AFFYMETRIX^{®}). Then 7 µl working probe set reagents were added to 27 µl lysate dilution (or 27 µl lysis mix for background samples) on the capture plate. Plates were centrifuged and then incubated for 16-20 hours at 55°C to hybridize (target RNA capture). Signal amplification and detection of target RNA began by washing plates with buffer 3 times to remove unbound material. 2.0 Pre-Amplifier hybridization reagent (30 µl/well) was added, incubated at 55°C for 1 hour then aspirated and wash buffer was added and aspirated 3 times. The 2.0 Amplifier hybridization reagent was then added as described (30 µl/well), incubated for 1 hour at 55°C and the wash was repeated as described previously. The 2.0 Label Probe hybridization reagent was added next (30 µl/well), incubated for 1 hour at 50°C and the wash was repeated as described previously. Lastly, the plates were centrifuged to remove any excess wash buffer and 2.0 Substrate was added (30 µl/well). Plates were incubated for 5 minutes at room temperature and plates were imaged on a PerkinElmer Envision multilabel reader in luminometer mode within 15 minutes.

For the gene of interest, the average assay background signal was subtracted from the average signal of each technical replicate. The background-subtracted, average signals for the gene of interest are divided by the background-subtracted average signal for the housekeeping tubulin RNA. The percent inhibition for the treated sample was calculated relative to untreated sample (i.e. the lower the value the larger the inhibition). Variability in background of untreated samples may result in percent inhibition of a treated sample that are equal to or higher than background, and in these cases, percent inhibition is expressed as 100% inhibition of control (i.e. no inhibition).

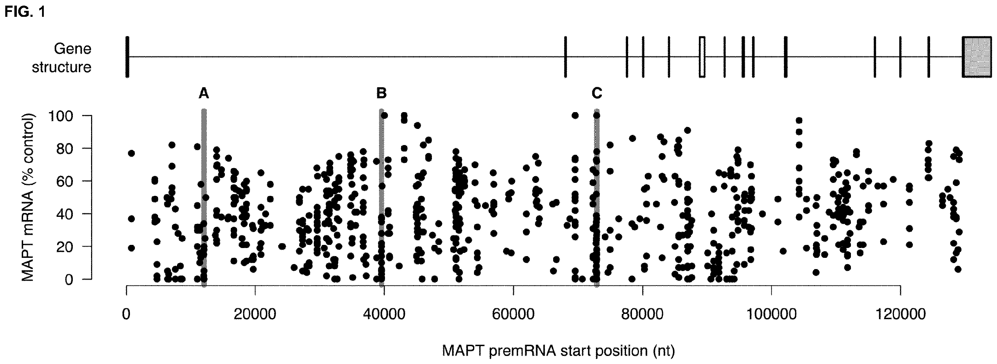

Figure 1 shows the MAPT mRNA reduction achieved by all 807 ASO's. In the figure three regions A, B and C on the MAPT target nucleic acid are indicated. These regions have a high prevalence of ASO's that reduce the target to 40% or less compared to control (100%).

### Example 2 in vitro screening of ASO's targeting selected regions on MAPT

Based on the screening in Example 1, a new library of ASO's were designed to target region A, B and C as illustrated in figure 1. The motif sequences and the oligonucleotide compounds are shown in table 5 above.

The screening was conducted as described in Example 1. The results are shown in table 6.

**Table 6 in vitro screening of anti-MAPT compounds**

| **CMP ID NO** | **Compound** | **% MAPT mRNA of control** |
|---|---|---|
| 6_1 | TCACtcatgccttaaTC | 2 |
| 7_1 | TAATcactcatgcCTTA | 15 |
| 8_1 | TAATcactcatgCCTT | 34 |
| 9_1 | CtttaatttaaTcaCtCAT | 41 |
| 9_2 | CtttaatttaaTcACtCAT | 36 |
| 9_3 | CtttaatttaaTCactCAT | 28 |
| 9_4 | CtttaatttaaTCacTCAT | 31 |
| 9_5 | CtttaatttaaTCaCtcAT | 28 |
| 9_6 | CtttaatttaaTCaCtCAT | 55 |
| 9_7 | CtttaatttaaTCActcAT | 30 |
| 9_8 | CtttaatttaaTCActCAT | 21 |
| 9_9 | CtttaatttaaTCAcTCAT | 61 |
| 9_10 | CtttaatttaaTCACtcAT | 24 |
| 9_11 | CtttaaTttaatcacTCAT | 14 |
| 9_12 | CtttaaTttaatcaCtCAT | 22 |
| 9_13 | CtttaaTttaatcActCAT | 33 |
| 9_14 | CtttaaTttaatcAcTCA T | 9 |
| 9_15 | CtttaaTttaatcACtCAT | 20 |
| 9_16 | CtttaAtttaatcacTCAT | 17 |
| 9_18 | CtttAatttaatcacTCAT | 10 |
| 9_19 | CtttAatttaatcaCtCAT | 17 |
| 9_20 | CtttAaTttaatcacTCAT | 0 |
| 9_21 | CtttAAtttaatcacTCAT | 3 |
| 9_22 | CtttAATttaatcacTCAT | 1 |
| 9_23 | CttTaatttaatcacTCAT | 13 |
| 9_24 | CttTaatttaatcaCtCAT | 13 |
| 9_25 | CttTaaTttaatcacTCAT | 4 |
| 9_26 | CttTaAtttaatcacTCAT | 4 |
| 9_27 | CttTaATttaatcacTCAT | 1 |
| 9_28 | CttTAatttaatcactCAT | 12 |
| 9_29 | CttTAatttaatcacTCAT | 1 |
| 9_30 | CttTAatttaatcaCtcAT | 15 |
| 9_31 | CttTAatttaatcaCtCAT | 4 |
| 9_32 | CttTAaTttaatcacTCAT | 1 |
| 9_33 | CttTAAtttaatcacTCAT | 1 |
| 9_34 | CttTAATttaatcactcAT | 4 |
| 9_35 | CttTAATttaatcacTCAT | 1 |
| 9_36 | CtTtaaTttaatcacTCAT | 5 |
| 9_37 | CtTtaAtttaatcacTCAT | 7 |
| 9_38 | CtTtaATttaatcacTCAT | 2 |
| 9_39 | CtTtAatttaatcacTCAT | 3 |
| 9_40 | CtTtAatttaatcaCtCAT | 9 |
| 9_41 | CtTtAaTttaatcacTCAT | 4 |
| 9_42 | CtTtAAtttaatcacTCAT | 1 |
| 9_43 | CtTtAATttaatcacTCAT | 1 |
| 9_44 | CtTTaatttaatcacTCAT | 2 |
| 9_45 | CtTTaatttaatcaCtcAT | 15 |
| 9_46 | CtTTaatttaatcaCtCAT | 3 |
| 9_47 | CtTTaaTttaatcacTCAT | 2 |
| 9_48 | CtTTaAtttaatcacTCAT | 1 |
| 9_49 | CtTTaATttaatcactcAT | 1 |
| 9_50 | CtTTaATttaatcacTCAT | 1 |
| 9_51 | CtTTAatttaatcactCAT | 1 |
| 9_52 | CtTTAatttaatcacTCAT | 1 |
| 9_53 | CtTTAatttaatcaCtcAT | 6 |
| 9_54 | CtTTAatttaatcaCtCAT | 2 |
| 9_56 | CtTTAaTttaatcacTCAT | 1 |
| 9_57 | CtTTAAtttaatcactcAT | 1 |
| 9_58 | CtTTAAtttaatcacTCAT | 1 |
| 9_59 | CTttaatttaatcActCAT | 39 |
| 9_60 | CTttaatttaatcAcTCAT | 10 |
| 9_61 | CTttaatttaatcACtCAT | 20 |
| 9_62 | CTttaatttaaTCactcAT | 26 |
| 9_63 | CTttaatttaaTCactCAT | 14 |
| 9_64 | CTttaatttaaTCacTCAT | 14 |
| 9_65 | CTttaatttaaTCaCtcAT | 15 |
| 9_66 | CTttaatttaaTCaCtCAT | 38 |
| 9_67 | CTttaatttaaTCActcAT | 9 |
| 9_68 | CTttaatttaaTCActCAT | 12 |
| 9_69 | CTttaatttaaTCACtcAT | 9 |
| 9_70 | CTttaaTttaatcactCAT | 42 |
| 9_71 | CTttaaTttaatcacTCAT | 6 |
| 9_72 | CTttaaTttaatcaCtcAT | 49 |
| 9_73 | CTttaaTttaatcaCtCAT | 15 |
| 9_74 | CTttaaTttaatcActCAT | 16 |
| 9_75 | CTttaaTttaatcAcTCAT | 12 |
| 9_76 | CTttaaTttaatcACtcAT | 32 |
| 9_77 | CTttaaTttaatcACtCAT | 15 |
| 9_78 | CTttAatttaatcactCAT | 21 |
| 9_79 | CTttAatttaatcacTCAT | 3 |
| 9_80 | CTttAatttaatcaCtCAT | 10 |
| 9_81 | CTttAaTttaatcacTCAT | 2 |
| 9_82 | CTttAAtttaatcacTCAT | 1 |
| 9_84 | CTtTaatttaatcaCtcAT | 22 |
| 9_85 | CTtTaatttaatcaCtCAT | 8 |
| 9_86 | CTtTaAtttaatcacTCAT | 2 |
| 9_89 | CTtTAatttaatcacTCAT | 1 |
| 9_90 | CTtTAatttaatcaCtcAT | 5 |
| 9_92 | CTtTAaTttaatcactcAT | 1 |
| 9_94 | CTtTAAtttaatcacTCAT | 1 |
| 9_97 | CTTtAatttaatcactCAT | 0 |
| 9_98 | CTTtAatttaatcacTCAT | 1 |
| 9_99 | CTTtAatttaatcaCtcAT | 7 |
| 9_100 | CTTtAatttaatcaCtCAT | 3 |
| 9_101 | CTTtAAtttaatcacTCAT | 1 |
| 9_103 | CTTTaatttaatcacTCAT | 0 |
| 9_105 | CTTTaaTttaatcactcAT | 0 |
| 9_106 | CTTTaAtttaatcacTCAT | 1 |
| 10_1 | GctttaatttaaTcaCtCAT | 35 |
| 10_2 | GctttaatttaaTCactcAT | 56 |
| 10_3 | GctttaatttaaTCactCAT | 18 |
| 10_4 | GctttaatttaaTCacTCAT | 21 |
| 10_5 | GctttaatttaaTCaCtcAT | 16 |
| 10_6 | GctttaatttaaTCaCtCAT | 35 |
| 10_7 | GctttaatttaaTCActcAT | 22 |
| 10_8 | GctttaatttaaTCACtcAT | 12 |
| 10_9 | GctttaaTttaatcactCAT | 61 |
| 10_10 | GctttaaTttaatcacTCAT | 19 |
| 10_11 | GctttaaTttaatcaCtcAT | 76 |
| 10_12 | GctttaaTttaatcaCtCAT | 12 |
| 10_13 | GctttaaTttaaTcaCtCAT | 15 |
| 10_14 | GctttaaTttaaTCactCAT | 7 |
| 10_15 | GctttaaTttaaTCaCtcAT | 14 |
| 10_16 | GctttaaTttaaTCActcAT | 10 |
| 10_17 | GetttaAtttaatcacTCAT | 28 |
| 10_18 | GetttAatttaatcacTCAT | 16 |
| 10_19 | GctttAatttaatcaCtCAT | 13 |
| 10_20 | GetttAaTttaatcacTCAT | 2 |
| 10_21 | GctttAAtttaatcacTCAT | 3 |
| 10_22 | GctttAATttaatcacTCAT | 1 |
| 10_23 | GcttTaatttaatcacTCAT | 18 |
| 10_24 | GcttTaatttaatcaCtcAT | 51 |
| 10_25 | GcttTaatttaatcaCtCAT | 8 |
| 10_26 | GcttTaaTttaatcacTCAT | 4 |
| 10_27 | GcttTaAtttaatcacTCAT | 3 |
| 10_28 | GcttTAatttaatcacTCAT | 2 |
| 10_29 | GcttTAatttaatcaCtcAT | 13 |
| 10_30 | GcttTAatttaatcaCtCAT | 3 |
| 10_31 | GctTtaaTttaatcacTCAT | 4 |
| 10_32 | GctTtaAtttaatcacTCAT | 6 |
| 10_33 | GctTtAatttaatcacTCAT | 3 |
| 10_34 | GctTtAatttaatcaCtcAT | 18 |
| 10_35 | GctTtAatttaatcaCtCAT | 6 |
| 10_36 | GctTtAaTttaatcacTCAT | 2 |
| 10_37 | GctTtAAtttaatcacTCAT | 1 |
| 10_38 | GctTTaatttaatcacTCAT | 1 |
| 10_39 | GctTTaatttaatcaCtcAT | 12 |
| 10_40 | GctTTaatttaatcaCtCAT | 3 |
| 10_41 | GctTTaAtttaatcacTCAT | 1 |
| 10_42 | GctTTAatttaatcaCtcAT | 5 |
| 10_43 | GcTttaatttaaTCactcAT | 15 |
| 10_44 | GcTttaatttaaTCactCAT | 11 |
| 10_45 | GcTttaatttaaTCaCtcAT | 15 |
| 10_46 | GcTttaatttaaTCActcAT | 7 |
| 10_47 | GcTttaaTttaatcactCAT | 23 |
| 10_48 | GcTttaaTttaatcacTCAT | 6 |
| 10_49 | GcTttaaTttaatcaCtcAT | 34 |
| 10_50 | GcTttaaTttaatcaCtCAT | 12 |
| 10_51 | GcTttaAtttaatcacTCAT | 10 |
| 10_52 | GcTttAatttaatcacTCAT | 5 |
| 10_53 | GcTttAatttaatcaCtcAT | 26 |
| 10_54 | GcTttAatttaatcaCtCAT | 10 |
| 10_55 | GcTttAaTttaatcacTCAT | 3 |
| 10_56 | GcTttAAtttaatcacTCAT | 2 |
| 10_57 | GcTtTaatttaatcacTCAT | 5 |
| 10_58 | GcTtTaatttaatcaCtCAT | 9 |
| 10_59 | GcTtTaaTttaatcacTCAT | 5 |
| 10_60 | GcTtTaAtttaatcacTCAT | 4 |
| 10_61 | GcTtTAatttaatcaCtcAT | 10 |
| 10_62 | GcTtTAAtttaatcactcAT | 4 |
| 10_63 | GcTTtaaTttaatcacTCAT | 2 |
| 10_64 | GcTTtaAtttaatcactcAT | 21 |
| 10_65 | GcTTtaAtttaatcacTCAT | 2 |
| 10_66 | GcTTtAatttaatcacTCAT | 2 |
| 10_67 | GcTTtAatttaatcaCtCAT | 1 |
| 10_68 | GcTTtAAtttaatcactcAT | 4 |
| 10_69 | GcTTTaatttaatcaCTcAT | 1 |
| 10_70 | GcTTTAatttaatcaCtcAT | 5 |
| 10_71 | GCtttaatttaatCactcAT | 71 |
| 10_72 | GCtttaatttaaTCactcAT | 22 |
| 10_73 | GCtttaaTttaatcactcAT | 76 |
| 10_74 | GCtttaaTttaatcactCAT | 25 |
| 10_75 | GCtttaaTttaatcaCtcAT | 43 |
| 10_76 | GCtttaATttaatcactcAT | 25 |
| 10_77 | GCtttAatttaatcaCtcAT | 13 |
| 10_78 | GCtttAAtttaatcactcAT | 22 |
| 10_79 | GCttTaatttaatcaCtcAT | 16 |
| 10_80 | GCttTaAtttaatcactcAT | 8 |
| 10_81 | GCttTAAtttaatcactcAT | 3 |
| 10_82 | GCtTtaAtttaatcactcAT | 21 |
| 10_83 | GCtTtAatttaatcaCtcAT | 7 |
| 10_84 | GCtTtAAtttaatcactcAT | 3 |
| 10_85 | GCTttaatttaatCactcAT | 29 |
| 10_86 | GCTttaaTttaatcactcAT | 32 |
| 10_87 | GCTttaAtttaatcactcAT | 38 |
| 10_88 | GCTttAAtttaatcactcAT | 6 |
| 10_89 | GCTtTaatttaatcAcTcAT | 9 |
| 11_1 | CTTTaatttaatcaCTCA | 0 |
| 12_1 | CTTTaatttaatcACTC | 0 |
| 19_1 | ACAccatccaagtCAAT | 20 |
| 20_1 | TACaccatccaagTCAA | 18 |
| 21_1 | TTACaccatccaagtCA | 0 |
| 22_1 | TTACaccatccaaGTC | 5 |
| 23_1 | AATAttacaccatCCAA | 0 |
| 24_1 | AgaaTattacaccatCCAA | 11 |
| 24_2 | AgaaTattacaccaTcCAA | 8 |
| 24_3 | AgaaTattacaccaTCcAA | 6 |
| 24_4 | AgaaTattacaccAtcCAA | 11 |
| 24_5 | AgaaTattacaccAtCCAA | 14 |
| 24_6 | AgaaTattacaccATcCAA | 6 |
| 24_7 | AgaaTattacaccATCcAA | 2 |
| 24_8 | AgaaTattacacCaTCcAA | 12 |
| 24_9 | AgaaTattacaCcAtCcAA | 11 |
| 24_10 | AqaaTattacaCcATcCAA | 18 |
| 24_11 | AgaAtattacaccatCCAA | 10 |
| 24_12 | AgaAtattacaccaTcCAA | 12 |
| 24_13 | AgaATattacaccatcCAA | 1 |
| 24_14 | AgaATattacaccaTCcAA | 1 |
| 24_15 | AgaATattacaccAtcCAA | 9 |
| 24_16 | AgaATattacaccAtCcAA | 0 |
| 24_17 | AgaATattacaccATCcAA | 10 |
| 24_18 | AgaATattacacCAtCcAA | 3 |
| 24_19 | AgAatattacaccAtCCAA | 10 |
| 24_20 | AgAatattacaccATCcAA | 13 |
| 24_21 | AgAaTattacaccatcCAA | 0 |
| 24_22 | AgAaTattacaccaTCcAA | 3 |
| 24_23 | AgAaTattacaccAtcCAA | 13 |
| 24_24 | AgAaTattacaccAtCcAA | 1 |
| 24_25 | AgAaTattacaccATCcAA | 8 |
| 24_26 | AgAaTattacacCatcCAA | 3 |
| 24_27 | AgAaTattacaCcatcCAA | 1 |
| 24_28 | AgAaTAttacacCAtccAA | 5 |
| 24_29 | AgAAtattacaccaTCcAA | 13 |
| 24_30 | AgAATattacaccatcCAA | 10 |
| 24_31 | AgAATattacaccatCcAA | 4 |
| 24_32 | AgAATattacaccaTCcAA | 12 |
| 24_33 | AgAATattacaccAtcCAA | 13 |
| 24_34 | AgAATattacaccAtCcAA | 5 |
| 24_35 | AgAATattacacCaTCcAA | 4 |
| 24_36 | AGaatAttacaccaTCcAA | 5 |
| 24_37 | AGaatAttacacCatcCAA | 2 |
| 24_38 | AGaaTattacaccatcCAA | 11 |
| 24_39 | AGaaTattacaccatCcAA | 3 |
| 24_40 | AGaaTattacaccaTCcAA | 17 |
| 24_41 | AGaaTattacaccAtcCAA | 9 |
| 24_42 | AGaaTattacaccAtCcAA | 2 |
| 24_43 | AGaaTattacaccATCcAA | 5 |
| 24_44 | AGaaTattacaCcAtCcAA | 9 |
| 24_45 | AGaaTAttacacCatCcAA | 3 |
| 24_46 | AGaAtattacaccaTCcAA | 9 |
| 24_47 | AGaAtAttacacCaTCcAA | 26 |
| 24_48 | AGaATattacaccatcCAA | 8 |
| 24_49 | AGaATattacaccatCcAA | 0 |
| 24_50 | AGaATattacaccaTCcAA | 2 |
| 24_51 | AGaATattacaccAtcCAA | 4 |
| 24_52 | AGaATattacaccAtCcAA | 0 |
| 24_53 | AGaATAttacaccatCcAA | 1 |
| 24_54 | AGAatattacaccaTCcAA | 5 |
| 24_55 | AGAatattacaccAtcCAA | 1 |
| 24_56 | AGAatattacaccAtCcAA | 0 |
| 24_57 | AGAaTattacaccatCcAA | 0 |
| 24_58 | AGAaTattacaccAtccAA | 13 |
| 24_59 | AGAaTattacaccAtCcAA | 11 |
| 24_60 | AGAAtattacaccatCcAA | 11 |
| 24_61 | AGAAtattacacCatccAA | 56 |
| 24_62 | AGAAtAttacaccatCcAA | 4 |
| 25_1 | CagaaTattacaccaTcCAA | 8 |
| 25_2 | CagaaTattacaccaTCcAA | 11 |
| 25_3 | CagaaTattacacCatCcAA | 9 |
| 25_4 | CaqaaTattacaCcAtCcAA | 12 |
| 25_5 | CaqaAtattacaccaTCcAA | 20 |
| 25_6 | CagaAtattacaCCatccAA | 10 |
| 25_7 | CagaAtAttacacCAtccAA | 6 |
| 25_8 | CagaATattacaccatcCAA | 5 |
| 25_9 | CaqaATattacaccatCcAA | 6 |
| 25_10 | CaqaATattacaccaTCcAA | 9 |
| 25_11 | CagaATattacaccAtCcAA | 12 |
| 25_12 | CagAatattacaccaTCcAA | 11 |
| 25_13 | CagAatattacaccAtcCAA | 2 |
| 25_14 | CaqAatAttacacCaTCcAA | 19 |
| 25_15 | CaqAaTattacaccatcCAA | 13 |
| 25_16 | CagAaTattacaccaTCcAA | 7 |
| 25_17 | CagAaTattacaccAtcCAA | 0 |
| 25_18 | CagAaTattacaccAtCcAA | 13 |
| 25_19 | CaqAaTattacacCaTCcAA | 6 |
| 25_20 | CagAaTAttacacCatCcAA | 12 |
| 25_21 | CagAAtattacacCAtccAA | 2 |
| 25_22 | CagAAtattacacCAtCcAA | 25 |
| 25_23 | CaGaaTattacaccatcCAA | 2 |
| 25_24 | CaGaaTattacaccatCcAA | 3 |
| 25_25 | CaGaaTattacaccaTCcAA | 5 |
| 25_26 | CaGaaTattacaccAtcCAA | 0 |
| 25_27 | CaGaaTattacaccAtCcAA | 10 |
| 25_28 | CaGaaTattacaCcatccAA | 4 |
| 25_29 | CaGaAtattacaccatCcAA | 6 |
| 25_30 | CaGaAtattacaccaTCcAA | 3 |
| 25_31 | CaGaAtAttacaccatCcAA | 6 |
| 25_32 | CaGaAtAttacacCAtccAA | 2 |
| 25_33 | CaGAatattacaccAtCcAA | 5 |
| 25_34 | CaGAatattacaCcAtccAA | 10 |
| 25_35 | CAgaaTattacaccatCcAA | 5 |
| 25_36 | CAgaaTattacaccAtccAA | 5 |
| 25_37 | CAgaaTattacaccAtCcAA | 3 |
| 25_38 | CAgaAtattacaccatCcAA | 26 |
| 25_39 | CAgAatattacaccAtccAA | 1 |
| 25_40 | CAgAatattacaccAtCcAA | 11 |
| 25_41 | CAgAaTattacaccAtccAA | 6 |
| 25_42 | CAgAaTattacacCatccAA | 73 |
| 25_43 | CAgAAtattacaccatCcAA | 1 |
| 26_1 | GaatattacacCAtCCAA | 11 |
| 26_2 | GaatattacacCA TcCAA | 13 |
| 26_3 | GaatattacacCA TCcAA | 10 |
| 26_4 | GaatAttacaccatCCAA | 0 |
| 26_5 | GaaTattacaccatCCAA | 2 |
| 26_6 | GaaTattacaccAtCCAA | 0 |
| 26_7 | GaaTAttacacCAtCcAA | 8 |
| 26_8 | GaAtattacaccatCCAA | 1 |
| 26_9 | GaAtAttacaccatCCAA | 1 |
| 26_10 | GaAtAttacacCATCcAA | 22 |
| 26_11 | GaATattacaccatCCAA | 1 |
| 26_12 | GaATattacaccaTCcAA | 2 |
| 26_13 | GaATattacaccAtCCAA | 3 |
| 26_14 | GaATattacaccATCcAA | 3 |
| 26_15 | GaATattacacCAtcCAA | 1 |
| 26_16 | GAatattacaccAtCCAA | 0 |
| 26_17 | GAatattacaccATCcAA | 1 |
| 26_18 | GAatattacacCATCcAA | 8 |
| 26_19 | GAatAttacacCATCcAA | 22 |
| 26_20 | GAaTattacaccatcCAA | 1 |
| 26_21 | GAaTattacaccaTCcAA | 1 |
| 26_22 | GAaTattacaccAtcCAA | 4 |
| 26_23 | GAaTattacaccATCcAA | 5 |
| 26_24 | GAaTattacacCatcCAA | 9 |
| 26_25 | GAaTattacacCAtccAA | 2 |
| 26_26 | GAAtattacaccatCCAA | 3 |
| 26_27 | GAAtattacaccaTCcAA | 3 |
| 26_28 | GAAtattacacCAtCcAA | 5 |
| 26_29 | GAATattacaccatcCAA | 0 |
| 26_30 | GAATattacaccAtcCAA | 0 |
| 26_31 | GAATattacacCaTCcAA | 24 |
| 27_1 | AATAttacaccaTCCA | 0 |
| 28_1 | AgaaTattacaccatCCA | 1 |
| 28_2 | AgaaTattacaccaTcCA | 6 |
| 28_3 | AgaaTattacaccAtCCA | 1 |
| 28_4 | AgaaTattacaccATcCA | 5 |
| 28_5 | AgaAtattacaccatCCA | 5 |
| 28_6 | AgaAtattacaccaTCCA | 6 |
| 28_7 | AgaAtAttacaccaTcCA | 3 |
| 28_8 | AgaAtAttacacCatcCA | 4 |
| 28_9 | AgaATattacaccatcCA | 2 |
| 28_10 | AgaATattacaccAtcCA | 0 |
| 28_11 | AgAatattacaccaTCCA | 8 |
| 28_12 | AgAatattacaccAtCCA | 1 |
| 28_13 | AgAatattacacCAtcCA | 1 |
| 28_14 | AgAatAttacacCatcCA | 3 |
| 28_15 | AgAatAttacacCatCCA | 6 |
| 28_16 | AgAaTattacaccatcCA | 3 |
| 28_17 | AgAaTattacaccatCCA | 1 |
| 28_18 | AgAaTattacaccAtcCA | 3 |
| 28_19 | AgAaTattacaccAtCCA | 0 |
| 28_20 | AgAaTattacacCatcCA | 6 |
| 28_21 | AgAaTattacaCcatcCA | 3 |
| 28_22 | AgAaTAttacacCatcCA | 5 |
| 28_23 | AgAAtattacaccatCCA | 0 |
| 28_24 | AgAATattacaccatcCA | 2 |
| 28_25 | AgAATattacaccAtcCA | 3 |
| 28_26 | AGaaTattacaccatcCA | 2 |
| 28_27 | AGaaTattacaccAtcCA | 1 |
| 28_28 | AGaAtattacaccatCCA | 1 |
| 28_29 | AGaATattacaccatcCA | 0 |
| 28_30 | AGaATattacaccAtcCA | 1 |
| 28_31 | AGAatattacaccAtcCA | 1 |
| 28_32 | AGAaTattacaccatcCA | 1 |
| 28_33 | AGAaTattacaccAtcCA | 5 |
| 29_1 | CagaaTattacaccaTcCA | 1 |
| 29_2 | CaqaAtattacaccatCCA | 4 |
| 29_3 | CaqaAtattacaCcatcCA | 15 |
| 29_4 | CagaATattacaccatcCA | 6 |
| 29_5 | CagaATattacaccAtcCA | 12 |
| 29_6 | CagaATattacacCatcCA | 3 |
| 29_7 | CaqAaTattacaccatcCA | 2 |
| 29_8 | CaqAaTattacaccAtcCA | 9 |
| 29_9 | CagAATattacaccatcCA | 0 |
| 29_10 | CaGaaTattacaccatcCA | 0 |
| 29_11 | CaGaaTattacaccAtcCA | 7 |
| 29_12 | CaGAatattacaccAtcCA | 4 |
| 29_13 | CAgAatattacaccAtcCA | 0 |
| 29_14 | CAgAatattacAccAtcCA | 2 |
| 30_1 | GaatattacacCAtCCA | 20 |
| 30_2 | GaatAttacaccaTCCA | 2 |
| 30_3 | GaaTattacaccatCCA | 1 |
| 30_4 | GaaTattacaccAtCCA | 1 |
| 30_5 | GaAtattacaccatCCA | 0 |
| 30_6 | GaAtattacaccaTCCA | 1 |
| 30_7 | GaAtattacacCAtCCA | 4 |
| 30_8 | GaAtattacaCCatcCA | 2 |
| 30_9 | GaAtAttacacCATcCA | 20 |
| 30_10 | GaATattacaccatCCA | 1 |
| 30_11 | GaATattacaccAtCCA | 4 |
| 30_12 | GAatattacaccaTCCA | 1 |
| 30_13 | GAatattacaccAtCCA | 1 |
| 30_14 | GAatAttacaccatCCA | 2 |
| 30_15 | GAatAttacacCatcCA | 3 |
| 30_16 | GAaTattacaccatcCA | 5 |
| 30_17 | GAaTattacaccatCCA | 0 |
| 30_18 | GAaTattacaccAtcCA | 5 |
| 30_19 | GAaTattacaccAtCCA | 3 |
| 30_20 | GAaTattacacCAtcCA | 2 |
| 30_21 | GAaTattacaCcatcCA | 2 |
| 30_22 | GAAtattacaccatCCA | 4 |
| 30_23 | GAAtattacacCatcCA | 2 |
| 30_24 | GAATattacaccatcCA | 1 |
| 30_25 | GAATattacaccAtcCA | 3 |
| 31_1 | TcAgaaTattacaccatcCA | 10 |
| 31_2 | TcAgaaTattacaccAtcCA | 3 |
| 31_3 | TcAgaAtattacaccaTcCA | 7 |
| 32_1 | AgaaTattacaccaTCC | 1 |
| 32_2 | AgaaTattacaccATCC | 1 |
| 32_3 | AgaaTAttacacCatCC | 0 |
| 32_4 | AgaAtattacaccaTCC | 5 |
| 32_5 | AgaAtattacACcAtCC | 39 |
| 32_6 | AgaAtAttacacCatCC | 7 |
| 32_7 | AgaATattacaccaTCC | 1 |
| 32_8 | AgaATattacaccAtCC | 0 |
| 32_9 | AgaATattacaccATCC | 1 |
| 32_10 | AqAatattacaccaTCC | 1 |
| 32_11 | AgAatattacaccATCC | 5 |
| 32_12 | AgAatattacaCcAtCC | 5 |
| 32_13 | AgAatattacaCcATCC | 15 |
| 32_14 | AgAatattacAccaTCC | 3 |
| 32_15 | AgAatattacACcatCC | 0 |
| 32_16 | AgAatattacACCatCC | 18 |
| 32_17 | AgAaTattacaccaTCC | 4 |
| 32_18 | AgAaTattacaccAtCC | 3 |
| 32_19 | AgAaTattacaccATCC | 3 |
| 32_20 | AgAaTattacacCatCC | 10 |
| 32_21 | AgAaTattacaCcAtCC | 18 |
| 32_22 | AgAAtattacaccaTCC | 5 |
| 32_23 | AgAAtattacaCCatCC | 6 |
| 32_24 | AgAAtattacAcCAtCC | 34 |
| 32_25 | AgAATattacaccatCC | 1 |
| 32_26 | AgAATattacaccaTCC | 1 |
| 32_27 | AgAATattacaccAtCC | 2 |
| 32_28 | AgAATattacaccATCC | 2 |
| 32_29 | AgAATattacaCcAtCC | 13 |
| 32_30 | AGaaTattacaccatCC | 5 |
| 32_31 | AGaaTattacaccaTCC | 0 |
| 32_32 | AGaaTattacaccAtCC | 4 |
| 32_33 | AGaaTattacaccATCC | 1 |
| 32_34 | AGaAtattacaccatCC | 2 |
| 32_35 | AGaAtattacaccaTCC | 1 |
| 32_36 | AGaAtattacacCaTCC | 4 |
| 32_37 | AGaAtattacAccATCC | 11 |
| 32_38 | AGaATattacaccatCC | 0 |
| 32_39 | AGaATattacaccAtCC | 0 |
| 32_40 | AGAatattacaccaTCC | 4 |
| 32_41 | AGAatattacaccAtCC | 0 |
| 32_42 | AGAatattacAccaTCC | 2 |
| 32_43 | AGAatattacAccAtCC | 10 |
| 32_44 | AGAatattacAcCatCC | 12 |
| 32_45 | AGAatAttacaccatCC | 3 |
| 32_46 | AGAaTattacaccatCC | 1 |
| 32_47 | AGAaTattacaccAtCC | 1 |
| 32_48 | AGAAtattacaccatCC | 0 |
| 32_49 | AGAAtattacaccaTCC | 0 |
| 32_50 | AGAAtattacacCatCC | 0 |
| 32_51 | AGAAtattacAcCatCC | 5 |
| 33_1 | CaqaaTattacaccaTCC | 7 |
| 33_2 | CagaAtattacaccaTCC | 55 |
| 33_3 | CagaAtattacaCcaTCC | 19 |
| 33_4 | CagaAtattacAccaTCC | 8 |
| 33_5 | CagaAtattacACcatCC | 20 |
| 33_6 | CaqaATattacaccatCC | 1 |
| 33_7 | CagaATattacaccaTCC | 2 |
| 33_8 | CagaATattacaccAtCC | 3 |
| 33_9 | CagAatattacaccaTCC | 1 |
| 33_10 | CagAatAttacaccaTCC | 10 |
| 33_11 | CagAaTattacaccaTCC | 0 |
| 33_12 | CagAAtattacaccaTCC | 11 |
| 33_13 | CagAAtattacacCaTCC | 4 |
| 33_14 | CagAATattacaCcatCC | 3 |
| 33_15 | CaGaatAttacacCAtCC | 5 |
| 33_16 | CaGaaTattacaccAtCC | 1 |
| 33_17 | CaGaAtattacaccatCC | 1 |
| 33_18 | CaGaAtattacaccaTCC | 14 |
| 33_19 | CaGaAtattacaCcatCC | 6 |
| 33_20 | CaGaAtattacACcAtCC | 53 |
| 33_21 | CaGAatattacaccAtCC | 0 |
| 33_22 | CAgaaTattacaccatCC | 0 |
| 33_23 | CAgaaTattacaccAtCC | 1 |
| 33_24 | CAgaAtattacaccatCC | 3 |
| 33_25 | CAgaAtattacaccaTCC | 61 |
| 33_26 | CAgaAtAttacacCatCC | 5 |
| 33_27 | CAgAatattacaccaTCC | 8 |
| 33_28 | CAgAatattacaccAtCC | 0 |
| 33_29 | CAgAaTattacaccatCC | 0 |
| 33_30 | CAgAaTattacaccAtCC | 1 |
| 33_31 | CAgAAtattacaccatCC | 13 |
| 33_32 | CAgAAtattacAccatCC | 1 |
| 33_33 | CAGaAtattacaccatCC | 10 |
| 34_1 | GAATattacaccATCC | 0 |
| 35_1 | TCagaaTattacaccatCC | 10 |
| 35_2 | TCagaAtattacaccatCC | 11 |
| 35_3 | TCagaAtattacAccatCC | 9 |
| 36_1 | AGAAtattacacCATC | 0 |
| 37_1 | CAGAatattacaCCAT | 0 |
| 38_1 | CAattctcatttcaacCTTC | 14 |
| 39_1 | TCaattctcatttcaacCTT | 35 |
| 40_1 | ATCaattctcatttcaacCT | 17 |
| 41_1 | AATCaattctcatttcaACC | 28 |
| 42_1 | AAATcaattctcatttCAAC | 38 |
| 43_1 | CAAAtcaattctcattTCAA | 22 |
| 44_1 | TCAaatcaattctcatTTCA | 0 |
| 45_1 | CTCAaatcaattctcatTTC | 6 |
| 46_1 | ACTCaaatcaattctcATTT | 5 |
| 47_1 | AACTcaaatcaattctCATT | 37 |
| 48_1 | TAACtcaaatcaattcTCAT | 20 |
| 49_1 | TtaactCaaatcaattcTCA | 46 |
| 49_2 | TtaactCAaatcaattctCA | 35 |
| 49_3 | TtaactCAaatcaattcTCA | 9 |
| 49_4 | TtaacTCaaatcaattctCA | 33 |
| 49_5 | TtaacTCAaatcaattctCA | 6 |
| 49_6 | TtaaCtcaaatcaattCtCA | 63 |
| 49_7 | TtaaCtcaaatcaattCTCA | 18 |
| 49_8 | TtaaCtcaaatcaatTCtCA | 19 |
| 49_9 | TtaaCtcaaatcaaTtctCA | 80 |
| 49_10 | TtaaCtcaaatcaaTtcTCA | 26 |
| 49_11 | TtaaCtcaaatcaaTtCtCA | 30 |
| 49_12 | TtaaCtcaaatcaaTtCTCA | 18 |
| 49_13 | TtaaCtcaaatcaaTTCtCA | 32 |
| 49_14 | TtaaCtcaaatcAattcTCA | 22 |
| 49_15 | TtaaCtcAaatcaattcTCA | 20 |
| 49_16 | TtaaCtcAaatcaattCtCA | 28 |
| 49_17 | TtaaCtcAaatcaattCTCA | 7 |
| 49_18 | TtaaCtcAaatcaatTctCA | 19 |
| 49_19 | TtaaCtcAaatcaatTCtCA | 9 |
| 49_20 | TtaaCtcAaatcaaTtctCA | 33 |
| 49_21 | TtaaCtcAaatcaaTtcTCA | 13 |
| 49_22 | TtaaCtcAaatcaaTtCtCA | 16 |
| 49_23 | TtaaCtcAaatcaaTtCTCA | 12 |
| 49_24 | TtaaCtcAaatcaaTTCtCA | 19 |
| 49_25 | TtaaCtCaaatcaattctCA | 33 |
| 49_26 | TtaaCtCaaatcaattcTCA | 14 |
| 49_27 | TtaaCtCaaatcaattCtCA | 17 |
| 49_28 | TtaaCtCaaatcaattCTCA | 7 |
| 49_29 | TtaaCtCaaatcaatTCtCA | 7 |
| 49_30 | TtaaCtCAaatcaattctCA | 7 |
| 49_32 | TtaaCtCAaatcaattCtCA | 10 |
| 49_33 | TtaaCtCAaatcaaTtctCA | 10 |
| 49_34 | TtaaCtCAaatcaaTtCtCA | 6 |
| 49_35 | TtaaCTCaaatcaattctCA | 10 |
| 49_36 | TtaaCTCaaatcaattCtCA | 7 |
| 49_37 | TtaaCTCAaatcaattctCA | 4 |
| 49_39 | TtaActcaaatcaatTCtCA | 24 |
| 49_40 | TtaActcaaatcaaTtCtCA | 26 |
| 49_41 | TtaActcaaatcaaTtCTCA | 17 |
| 49_42 | TtaActcAaatcaattCtCA | 33 |
| 49_43 | TtaActcAaatcaatTCtCA | 11 |
| 49_44 | TtaActcAaatcaaTtcTCA | 15 |
| 49_45 | TtaActcAaatcaaTtCtCA | 24 |
| 49_46 | TtaActCaaatcaattCtCA | 20 |
| 49_47 | TtaActCaaatcaattCTCA | 6 |
| 49_48 | TtaActCaaatcaatTCtCA | 6 |
| 49_49 | TtaActCAaatcaattctCA | 18 |
| 49_50 | TtaActCAaatcaattCtCA | 9 |
| 49_53 | TtaActCAaatcaaTtctCA | 12 |
| 49_54 | TtaActCAaatcaaTtCtCA | 6 |
| 49_55 | TtaAcTCaaatcaattCtCA | 7 |
| 49_56 | TtaACtcaaatcaattCtCA | 30 |
| 49_57 | TtaACtcaaatcaattCTCA | 7 |
| 49_58 | TtaACtcaaatcaatTCtCA | 11 |
| 49_59 | TtaACtcaaatcaaTtctCA | 47 |
| 49_60 | TtaACtcaaatcaaTtCtCA | 18 |
| 49_61 | TtaACtcaaatcaaTtCTCA | 9 |
| 49_62 | TtaACtcaaatcaaTTCtCA | 17 |
| 49_63 | TtaACtcaaatcAattctCA | 40 |
| 49_64 | TtaACtcAaatcaattctCA | 23 |
| 49_65 | TtaACtcAaatcaattCtCA | 13 |
| 49_67 | TtaACtcAaatcaatTCtCA | 4 |
| 49_68 | TtaACtcAaatcaaTtctCA | 19 |
| 49_69 | TtaACtcAaatcaaTtCtCA | 12 |
| 49_70 | TtaACtcAaatcaaTtCTCA | 9 |
| 49_71 | TtaACtcAaatcaaTTCtCA | 16 |
| 49_72 | TtaACtCaaatcaattctCA | 12 |
| 49_73 | TtaACtCaaatcaattCtCA | 9 |
| 49_74 | TtaACtCaaatcaattCTCA | 4 |
| 49_75 | TtaACtCaaatcaatTCtCA | 4 |
| 49_76 | TtaACtCAaatcaattctCA | 3 |
| 49_78 | TtaACtCAaatcaaTtctCA | 3 |
| 49_79 | TtaACTCaaatcaattCtCA | 6 |
| 49_80 | TtAactcaaatcaaTtCtCA | 11 |
| 49_81 | TtAactcaaatcaAttCtCA | 35 |
| 49_82 | TtAactcaaatcaAtTCtCA | 18 |
| 49_83 | TtAactcaaatcaATtCtCA | 21 |
| 49_84 | TtAactcaaatcaATTCtCA | 36 |
| 49_85 | TtAactcAaatcaatTCtCA | 7 |
| 49_86 | TtAactcAaatcaaTtCtCA | 6 |
| 49_87 | TtAactCaaatcaattCtCA | 19 |
| 49_88 | TtAactCaaatcaattCTCA | 7 |
| 49_89 | TtAactCaaatcaatTCtCA | 6 |
| 49_90 | TtAactCAaatcaattCtCA | 9 |
| 49_92 | TtAactCAaatcaaTtCtCA | 3 |
| 49_93 | TtAactCAaatcaaTTCtCA | 11 |
| 49_94 | TtAaCtcaaatcaattCtCA | 34 |
| 49_95 | TtAaCtcaaatcaatTCtCA | 11 |
| 49_96 | TtAaCtcaaatcaaTtctCA | 56 |
| 49_97 | TtAaCtcaaatcaaTtCtCA | 15 |
| 49_98 | TtAaCtcaaatcaaTtCTCA | 14 |
| 49_99 | TtAaCtcaaatcaaTTCtCA | 30 |
| 49_100 | TtAaCtcaaatcAattctCA | 46 |
| 49_101 | TtAaCtcAaatcaattctCA | 24 |
| 49_102 | TtAaCtcAaatcaattCtCA | 22 |
| 49_103 | TtAaCtcAaatcaattCTCA | 8 |
| 49_104 | TtAaCtcAaatcaatTCtCA | 6 |
| 49_105 | TtAaCtcAaatcaaTtctCA | 28 |
| 49_106 | TtAaCtcAaatcaaTtCtCA | 31 |
| 49_107 | TtAaCtcAaatcaaTtCTCA | 29 |
| 49_108 | TtAaCtcAaatcaaTTCtCA | 38 |
| 49_109 | TtAaCtCaaatcaattctCA | 21 |
| 49_110 | TtAaCtCaaatcaattCtCA | 19 |
| 49_111 | TtAaCtCaaatcaatTCtCA | 9 |
| 49_112 | TtAaCtCAaatcaattctCA | 10 |
| 49_113 | TtAaCtCAaatcaattCtCA | 10 |
| 49_114 | TtAaCtCAaatcaaTtctCA | 6 |
| 49_115 | TtAActcaaatcaatTCtCA | 6 |
| 49_116 | TtAActcaaatcaaTtCtCA | 9 |
| 49_117 | TtAActcAaatcaattCtCA | 11 |
| 49_118 | TtAActcAaatcaatTCtCA | 3 |
| 49_119 | TtAActcAaatcaaTtCtCA | 11 |
| 49_120 | TtAActCaaatcaattCtCA | 33 |
| 49_121 | TtAActCaaatcaatTCtCA | 2 |
| 49_123 | TtAActCAaatcaatTCtCA | 1 |
| 49_125 | TtAACtcaaatcaattCtCA | 6 |
| 49_126 | TtAACtcaaatcaattCTCA | 5 |
| 49_127 | TtAACtcaaatcaatTCtCA | 9 |
| 49_128 | TtAACtcaaatcaaTtctCA | 33 |
| 49_129 | TtAACtcaaatcaaTtCtCA | 12 |
| 49_130 | TtAACtcaaatcaaTTCtCA | 19 |
| 49_131 | TtAACtcaaatcAattctCA | 25 |
| 49_132 | TtAACtcAaatcaattctCA | 15 |
| 49_133 | TtAACtcAaatcaattCtCA | 6 |
| 49_134 | TtAACtcAaatcaatTCtCA | 10 |
| 49_135 | TtAACtcAaatcaaTtctCA | 15 |
| 49_136 | TtAACtcAaatcaaTtCtCA | 22 |
| 49_137 | TtAACtcAaatcaaTTCtCA | 33 |
| 49_138 | TtAACtCaaatcaattctCA | 8 |
| 49_139 | TtAACtCaaatcaattCtCA | 6 |
| 49_141 | TtAACtCaaatcaatTCtCA | 11 |
| 49_143 | TtAACtCAaatcaaTtctCA | 3 |
| 49_144 | TTaactcAaatcaattCtCA | 14 |
| 49_145 | TTaactcAaatcaatTCtCA | 6 |
| 49_146 | TTaactcAaatcaaTtCtCA | 6 |
| 49_147 | TTaactcAaatcaaTTCtCA | 9 |
| 49_148 | TTaactCAaatcaattCtCA | 6 |
| 49_149 | TTaactCAaatcaatTCtCA | 2 |
| 49_150 | TTaaCtcaaatcaattCtCA | 26 |
| 49_151 | TTaaCtcaaatcaattCTCA | 8 |
| 49_152 | TTaaCtcaaatcaatTCtCA | 11 |
| 49_153 | TTaaCtcaaatcaaTtctCA | 41 |
| 49_154 | TTaaCtcaaatcaaTtCtCA | 14 |
| 49_155 | TTaaCtcaaatcAattctCA | 38 |
| 49_156 | TTaaCtcAaatcaattctCA | 23 |
| 49_157 | TTaaCtcAaatcaattCtCA | 13 |
| 49_158 | TTaaCtcAaatcaattCTCA | 4 |
| 49_159 | TTaaCtcAaatcaatTCtCA | 6 |
| 49_160 | TTaaCtcAaatcaaTtctCA | 20 |
| 49_161 | TTaaCtcAaatcaaTtCtCA | 12 |
| 49_162 | TTaaCtCaaatcaattctCA | 18 |
| 49_163 | TTaaCtCaaatcaattCtCA | 10 |
| 49_164 | TTaaCtCAaatcaattctCA | 7 |
| 49_166 | TTaActcaaatcaaTtCtCA | 17 |
| 49_167 | TTaActCaaatcaattCtCA | 7 |
| 49_168 | TTaActCAaatcaattCtCA | 3 |
| 49_169 | TTaACtcaaatcaaTtCtCA | 12 |
| 49_170 | TTaACtcAaatcaatTCtCA | 9 |
| 49_171 | TTaACtcAaatcaaTtCtCA | 25 |
| 49_172 | TTAactcaaatcaaTtCtCA | 16 |
| 49_173 | TTAactcaaatcaAttCtCA | 27 |
| 49_174 | TTAactcaaatcaAtTCtCA | 14 |
| 49_175 | TTAactcAaatcaatTCtCA | 5 |
| 49_176 | TTAactcAaatcaaTtCtCA | 6 |
| 49_177 | TTAactCaaatcaattCtCA | 15 |
| 49_178 | TTAactCaaatcaattCTCA | 4 |
| 49_180 | TTAactCAaatcaattCtCA | 6 |
| 49_181 | TTAaCtcaaatcaattCtCA | 23 |
| 49_182 | TTAaCtcaaatcaaTtctCA | 38 |
| 49_183 | TTAaCtcaaatcaaTtCtCA | 17 |
| 49_184 | TT AaCtcaaatcAattctCA | 40 |
| 49_185 | TT AaCtcAaatcaattctCA | 19 |
| 49_186 | TTAaCtcAaatcaattCtCA | 13 |
| 49_187 | TTAaCtcAaatcaaTtctCA | 13 |
| 49_188 | TTAaCtCaaatcaattctCA | 18 |
| 49_189 | TTAActcaaatcaattCTCA | 3 |
| 49_190 | TTAActcaaatcaatTCtCA | 9 |
| 49_191 | TTAActcAaatcaatTCtCA | 3 |
| 49_192 | TTAActCaaatcaattCtCA | 6 |
| 50_1 | TTTAactcaaatcaatTCTC | 1 |
| 51_1 | TTTAactcaaatcaaTTCT | 10 |
| 52_1 | CCTTttaattcaTTAG | 72 |
| 53_1 | CAACaccttttaattcATTA | 0 |
| 54_1 | AACAccttttaattCATT | 27 |
| 55_1 | CAtcaacaccttttaaTTCA | 100 |
| 56_1 | CTCAtcaacaccttttaaTT | 15 |
| 57_1 | ACtcatcaacacctttTAAT | 37 |
| 58_1 | AACtcatcaacaccttTTAA | 16 |
| 59_1 | TAACtcatcaacacctttTA | 18 |
| 60_1 | TTAActcatcaacacctTTT | 12 |
| 61_1 | TTAactcatcaacacCTTT | 4 |
| 62_1 | TTAactcatcaacaCCTT | 3 |
| 63_1 | TTAActcatcaacACCT | 0 |
| 64_1 | GTTAactcatcaacACC | 29 |
| 65_1 | GTTAactcatcaaCAC | 78 |

### Example 3 IC50 values of selected oligonucleotides

The IC50 of some of the best performing oligonucleotides from Example 2 was determined *in vitro* in primary neuronal cells using a 96 well assay.

Primary neuronal cell cultures were prepared as described in the "Materials and Method" section and plated on poly-D-lysine coated 96 well plates at 50,000 cells per well and maintained in Neurobasal media containing B27, glutamax and Penicillin-Streptomycin. ASOs were diluted in water (for IC50 determinations) and added to cells at 1 day post plating (DIV01). For IC₅₀ determinations, neurons were treated with a top concentration of 0.5 to 5 µM and a concentration response dilution of about 1:4 was used to define the IC50. CMP ID NO: 66_1, corresponding to ASO-001933 in WO2016/126995, was included as a positive control. Following ASO treatment, neurons were incubated at 37°C for 5 days to achieve steady state reduction of mRNA. Media was removed and cells lysed as follows. Measurement of lysate messenger RNA was performed using the QUANTIGENE^{®} 2.0 Reagent System (AFFYMETRIX^{®}), which quantitated RNA using a branched DNA-signal amplification method reliant on the specifically designed RNA capture probe set. The working cell lysis buffer solution was made by adding 50 µl proteinase K to 5ml of pre-warmed Lysis mix and diluted to 1:4 final dilution with dH₂0. The working lysis buffer was added to the plate (150 µl/ well), triturated to mix, sealed and incubated for 30 min at 55°C. Following lysis the wells were stored at -80°C or assayed immediately.

Lysates were diluted in lysis mix dependent on the specific capture probe used (tau or tubulin). 80 µl/ well total were then added to the capture plate (96 well polystyrene plate coated with capture probes). Working probe sets reagents were generated by combining nuclease-free water 12.1 µl, lysis mixture 6.6 µl, blocking reagent 1 µl, specific 2.0 probe set 0.3 µl human MAPT catalogue #15486 and either mouse beta 3 tubulin, catalogue #SB-17245, per manufacturer instructions (QUANTIGENE^{®} 2.0 AFFYMETRIX^{®}). Then 20 µl working probe set reagents were added to 80 µl lysate dilution (or 80 µl lysis mix for background samples) on the capture plate. Plates were centrifuged and then incubated for 16-20 hours at 55°C to hybridize (target RNA capture). Signal amplification and detection of target RNA was begun by washing plates with buffer 3 times to remove unbound material. 2.0 Pre-Amplifier hybridization reagent (100 µl/well) was added, incubated at 55°C for 1 hour then aspirated and wash buffer was added and aspirated 3 times. The 2.0 Amplifier hybridization reagent was then added as described (100 µl/well), incubated for 1 hour at 55°C and the wash was repeated as described previously. The 2.0 Label Probe hybridization reagent was added next (100 µl/well), incubated for 1 hour at 50°C and the wash was repeated as described previously. Lastly, the plates were centrifuged to remove any excess wash buffer and 2.0 Substrate was added (100 µl/well).

Plates were incubated for 5 minutes at room temperature and plates were imaged on a PerkinElmer Envision multilabel reader in luminometer mode within 15 minutes.

Data determination: For the gene of interest, the average assay background signal was subtracted from the average signal of each technical replicate. The background-subtracted, average signals for the gene of interest are divided by the background-subtracted average signal for the housekeeping tubulin RNA. The percent inhibition for the treated sample was calculated relative to untreated sample (i.e. the lower the value the larger the inhibition). Variability in background of untreated samples may result in percent inhibition of a treated sample that are equal to or higher than background, and in these cases, percent inhibition is expressed as 100% inhibition of control (i.e. no inhibition). The results are shown in table 7.

**Table 7: IC50 of anti-MAPT compounds**

| **CMP ID NO** | **Compound** | **Region** | **IC50 (nM)** |
|---|---|---|---|
| 9_103 | CTTTaatttaatcacTCAT | A | 12.2 |
| 11_1 | CTTTaatttaatcaCTCA | A | 9.4 |
| 34_1 | GAATattacaccATCC | A | 32.0 |
| 37_1 | CAGAatattacaCCAT | A | 15.6 |
| 49_189 | TTAActcaaatcaattCTCA | B | 11.8 |
| 56_1 | CTCAtcaacaccttttaaTT | C | 44.0 |
| 62_1 | TTAactcatcaacaCCTT | C | 40.5 |
| 63_1 | TTAActcatcaacACCT | C | 37.1 |
| 66_1 | AtTTCcaaattcactTTtAC | - | 44.3 |

### Example 4 In vivo tolerability and in vivo Tau mRNA reduction

Some of the best performing oligonucleotides from Example 2 were tested *in vivo* in a humanized Tau mouse to assess acute tolerability in CNS as well as MAPT mRNA reduction 3 days or 28 days after a single injection.

Transgenic Tau mice were administered with 100 µg ASO by intracerebroventricular (ICV) injection (see Materials and Method section, Transgenic Tau mouse). CMP ID NO: 66_1, corresponding to ASO-001933 in WO2016/126995, was included as a positive control. Animals were observed for behavioral side effects for one hour following the single injection of ASO ICV. The acute tolerability for the severity of side effects was scored on a scale of zero (no side effects) to 20 (convulsions resulting in euthanasia). The tolerability scale was divided into 5 neurobehavioral categories: 1) hyperactivity 2) decreased activity and arousal 3) motor dysfunction /ataxia 4) abnormal posture and breathing and 5) tremor/convulsions. Each category was scored on a scale of 0-4, with the worst possible total score of 20. Animals were observed for changes in behavior in the home cage, and then they were removed from the home cage for more detailed observations which included measurement of grip strength and righting reflex. Data from acute tolerability of ASO of the invention are presented in table 8.

The MAPT mRNA reduction in right, frontal cortical region was analyzed by qPCR as follows. Collected mouse brain tissue (see Materials and Methods section, Transgenic Tau mouse) was homogenized in a 10x volume of a high salt /sucrose buffer (10 mM Tris-HCl, pH 7.4, 800 mM NaCl, 10% sucrose (w/v), 1 mM EGTA) supplemented with phosphatase inhibitor cocktail sets 2 and 3, 1 mM PMSF (Sigma, Saint Louis, MO), and complete protease inhibitor cocktail EDTA-free (Roche, Indianapolis, IN) using a Quiagen TissueLyzer II. The homogenate was centrifuged at 20,000 x g for 20 minutes at 4°C. The supernatant was centrifuged at 100,000 Xg for 1 hour at 4°C.

For cDNA synthesis and subsequent PCR, 300ng of RNA from brain tissue supernatants was added to 1 well of a 96 well plate (Axygen, PCR-96-C-S). To each well 7.5µl of master mix (5µL of 2.5mM NTP mix and 2.5µL random primers per reaction) was added and the plate was centrifuged at 1000 rpm and placed in thermocycler for 3 min at 70° C. Plates were immediately cooled on ice and 4µl of reaction master mix was added. Prior to PCR, plates were briefly centrifuged to collect sample in bottom of well. cDNA synthesis was carried out at 42° C for 60 min, 95° C for 10 min followed by a hold at 4° C. cDNA Samples were diluted 1:3 with molecular biology grade water and stored at -20° C until further use.

For PCR, each sample was run in triplicate with two probe sets (MAPT: Taqman Expression assays Hs00902193_m1; GAPDH Taqman Expression assays Hs01922876_u1). To each reaction 4 µl of previously diluted cDNA and 6 µL of master mix was added and plates were centrifuged. Samples were incubated at 95° C for 20 sec follow by 40 cycles at 95° C for 1 sec and 60° C for 20 sec.

Data were analyzed using the delta delta Ct method where each sample was first normalized to GAPDH and then expressed as percent of untreated control (percent inhibition). If the percent inhibition was equal to or higher than in control cells, percent inhibition was expressed as zero inhibition.

**Table 8 Acute tolerability in hTau mice and MAPT mRNA reduction 3 days and 4 weeks post treatment in vivo**

| **CMP ID NO** | **Compound** | **Region** | **Acute tolerability** | **% MAPT mRNA of saline** | |
|---|---|---|---|---|---|
| | | | | Day 3 | 4 weeks |
| 9_103 | CTTTaatttaatcacTCAT | A | 0.5 | 16 | 16 |
| 11_1 | CTTTaatttaatcaCTCA | A | 0.0 | 16 | 18 |
| 9_104 | CTTTaatttaatcaCtCAT | A | 0.25 | NA | 28 |
| 9_102 | CTTtAATttaatcactcAT | A | 1.75 | NA | 20 |
| 34_1 | GAATattacaccATCC | A | 0.0 | 36 | 20 |
| 9_91 | CTtTAatttaatcaCtCAT | A | 0.50 | NA | 84 |
| 9_83 | CTttAATttaatcacTCAT | A | 0.75 | NA | 31 |
| 9_017 | CtttaATttaatcacTCAT | A | 0.50 | NA | 65 |
| 9_88 | CTtTAatttaatcactCAT | A | 0.50 | NA | 43 |
| 9_96 | CTTtaATttaatcactcAT | A | 2.50 | NA | 54 |
| 9_95 | CTtTAATttaatcactcAT | A | 4.13 | NA | 34 |
| 9_93 | CTtTAAtttaatcactcAT | A | 1.88 | NA | 52 |
| 9_87 | CTtTaATttaatcactcAT | A | 1.63 | NA | 46 |
| 9_55 | CtTTAaTttaatcactcAT | A | 2.50 | NA | 54 |
| 37_1 | CAGAatattacaCCAT | A | 0.0 | 27 | NA |
| 49_189 | TTAActcaaatcaattCTCA | B | 0.0 | 29 | 29 |
| 49_38 | TtaaCTCAaatcaaTtctCA | B | 1.50 | NA | 18 |
| 49_179 | TTAactCaaatcaatTCtCA | B | 1.0 | NA | 32 |
| 49_51 | TtaActCAaatcaattCTCA | B | 1.25 | NA | 31 |
| 49_124 | TtAActCAaatcaaTtCtCA | B | 1.50 | NA | 48 |
| 49_165 | TTaaCtCAaatcaaTtctCA | B | 0.88 | NA | 44 |
| 49_91 | TtAactCAaatcaatTCtCA | B | 0.63 | NA | 60 |
| 49_52 | TtaActCAaatcaatTCtCA | B | 2.88 | NA | 56 |
| 49_140 | TtAACtCaaatcaattCTCA | B | 0.25 | NA | 43 |
| 49_66 | TtaACtcAaatcaattCTCA | B | 0.0 | NA | 36 |
| 49_142 | TtAACtCAaatcaattCtCA | B | 0.5 | NA | 36 |
| 49_122 | TtAActCAaatcaattCtCA | B | 0.75 | NA | 56 |
| 49_77 | TtaACtCAaatcaattCtCA | B | 1.13 | NA | 55 |
| 50_1 | TTTAactcaaatcaatTCTC | B | NA | 26 | NA |
| 53_1 | CAACaccttttaattcATTA | C | NA | 21 | NA |
| 56_1 | CTCAtcaacaccttttaaTT | C | 0.2 | 25 | NA |
| 62_1 | TTAactcatcaacaCCTT | C | 0.0 | 39 | 28 |
| 63_1 | TTAActcatcaacACCT | C | 0.5 | 13 | NA |
| 66_1 | AtTTCcaaattcactTTtAC | - | 0.83 | 37 | 44 |

| | | | | | |
|---|---|---|---|---|---|
| NA = not assessed | | | | | |

### Example 5: in vitro efficacy in human embryonic stem cell (hESC) derived neurons.

Selected ASO's from example 2 were tested at three different concentrations (200 nM, 8 nM and 0.32 nM) in an alternative *in vitro* assay using human embryonic stem cell (hESC) derived neurons. For comparative purposes two prior art oligonucleotides targeting MAPT were included, namely CMP ID NO: 66_1 corresponding to ASO-001933 in WO2016/126995 and CPM ID NO: 67:1 corresponding to compound No 814907 in WO2018/064593.

### Culturing and ASO treatment of human embryonic stem cells (ESCs):

Neural stem cells (NSCs) were derived from human ESCs according to published procedures (Chambers et al. 2009 Nat. Biotech. 7, 275-280). The neural stem cells (NSCs) were proliferated into ventralized progenitors during 1 week in SFA medium, and was then differentiated into neurons in BGAA medium during 6 weeks, for media content, please see the Materials and methods section.

Cells were seeded at a density of 10,000 cells/cm² in N2B27 + SFA medium in a flask coated with poly-ornithine and laminin. Media was changed at day 4. After 7 days in N2B27 + SFA medium cells were trypsinized, and seeded as ventralized progenitors in N2B27+BGAA media at a density of 50,000 cell/well in 96 well plates.

Media was changed twice a week and treatment with ASO was started at the first media change and continued for 6 weeks. Then cells were harvested as decribed below.

### qPCR analysis:

Treated neurons were harvested as follows: removal of media followed by addition of 125 µL PURELINK^{®}Pro 96 Lysis buffer and 125 µL 70% ethanol. RNA was purified according to the manufacture's instruction and eluted in a final volume of 50 µL water, resulting in an RNA concentration of 10-20 ng/µL. Next, RNA was diluted 10 fold in water prior to the one-step qPCR reaction.

For the one-step qPCR reaction, qPCR-mix (qScriptTMXLE 1-step RT-qPCR TOUGHMIX^{®}Low ROX from QauntaBio) was mixed with two Taqman probes at a ratio 10:1:1 (qPCR mix: probe1:probe2) to generate the mastermix. The qPCR was performed as technical replicates and Taqman probes were acquired from LifeTechnologies: MAPT_Hs00902193_m1; GAPDH 4325792 (house keeping gene used for normalization).

The mastermix (6 µL) and RNA (4µL, 1-2 ng/µL) were then mixed in a qPCR plate (MICROAMP^{®}optical 384 well, catalog no. 4309849). After sealing the plate, the plate was given a quick spin, 1000g for 1 minute at RT, and transferred to a ViiaTM 7 system (Applied Biosystems, Thermo). The following PCR conditions were used: 50°C for 15 minutes; 95°C for 3 minutes; 40 cycles of: 95°C for 5 sec, followed by a temperature decrease of 1.6 °C/sec, followed by 60 °C for 45 sec. The data was analyzed using the QuantStudioTM Real_time PCR Software. The percent inhibition for the ASO treated samples was calculated relative to the control treated samples (low values indicate high reduction of MAPT). The results are shown in table 9 as the average of the two technical repeats.

### Tau protein and pTau protein measurement in hESC neurons:

PBS-washed cells were extracted into a buffer containing Cytobuster protein extraction reagent (Merck-Millipore #71009), 1% Phosphatase Inhibitor Cocktail 3 (Sigma #P0044), 1% Proteases Inhibitor Set III (Calbiochem #539134), 1% DNAse-I (Roche # 4536282001) and 10 mM MgCl₂. The cell extract wss lysed by pipetting up and down and then stored at -20°C until use.

Total Tau levels in the cell extracts were measured by AlphaLISA using an in house assay format comprising the Tau-specific antibodies 5A6 (DSHB Antibody Registry ID: AB_528487) and Roche in house Tau monoclonal antibody Tau 4/2. The latter antibody was generated by immunizing mice with human full-length Tau i.e. longest human brain isoform, 441 amino acids. Tau 4/2 binds to an C-terminal epitope in Tau located between amino acids 369 and 441. Briefly, cell extracts were diluted into AlphaLISA Hi Block assay buffer (PerkinElmer AL004C) and mixed with biotinylated 5A6 and Tau 4/2-coated AlphaLlSa acceptor beads. After incubation for 1hr at room temperature, streptavidin-coated donor beads are added to the mixture. After incubation for 30 min, the samples were measured in an Envision plate reader (ex 680 nm, em 615 nm). A standard curve was constructed using recombinant human Tau (Merck-Millipore #AG960).

PhosphoTau (Tau-pS422) levels in the cell extracts were measured by AlphaLISA using the Roche in house assay format comprising Tau-specific antibody 5A6 (DSHB Antibody Registry ID: AB_528487) and Tau-pS422-specific antibody 5.6.11 (described in WO2010/142423 and Collin et al 2014 Brain vol 137 P 2834-2846). Cell extracts are diluted into assay buffer B before assay. Buffer B comprises 25 mM HEPES pH7.4, 0.5% Triton X-100, 0.1% Top Block (LuBio Science), 1 mg/ml Dextran500, 10 % ELISA Blocking Reagent (Roche). A standard curve was prepared using ERK-phosphorylated Tau prepared as follows: recombinant human Tau was produced as described in Grueninger et al (Neurobiology of Disease 37 [2010] pp294-306). Recombinant His-tagged ERK2 (produced in house) was activated by incubation with activated MEKK1 (produced in house). Activated ERK2 was then incubated with Tau at a molar ratio of 1:50 in buffer containing 2mM ATP. ERk2 was subsequently removed by passage over Ni-NTA agarose (Qiagen). The extent of phosphorylation at S422 was subsequently determined by mass spectroscopy.

The results are shown in table 9.

**Table 9 MAPT reduction and Tau protein reduction in hESC derived neurons following treatment at three different concentrations.**

| **CMP ID NO** | **MAPT as % of control** | | | **Total Tau protein % of control** | | | **PhosphoTau protein % of control** | | |
|---|---|---|---|---|---|---|---|---|---|
| ASO conc (nM) | 200 | 8 | 0.32 | 200 | 8 | 0.32 | 200 | 8 | 0.32 |
| 9_104 | 5.4 | 36.6 | 100.9 | 7.0 | 40.3 | 88.3 | 0.6 | 12.0 | 54.0 |
| 9_103 | 1.2 | 15.6 | 71.8 | 1.8 | 23.2 | 66.2 | 0.1 | 19.8 | 92.9 |
| 11_1 | 1.0 | 12.5 | 72.3 | 1.5 | 25.9 | 65.1 | 0.1 | 17.5 | 70.4 |
| 49_38 | 5.7 | 36.3 | 83.5 | 6.8 | 45.5 | 79.6 | 1.3 | 51.6 | 116.6 |
| 49_189 | 7.0 | 36.5 | 90.2 | 10.4 | 48.1 | 102.9 | 5.0 | 59.6 | 137.3 |
| 53_1 | 4.8 | 32.9 | 79.4 | 8.8 | 45.7 | 79.0 | 3.1 | 48.6 | 127.6 |
| 66_1 | 11.0 | 40.2 | 81.9 | 10.9 | 48.4 | 69.9 | 3.6 | 57.9 | 94.2 |
| 9_102 | 2.0 | 34.9 | 99.0 | 3.0 | 44.3 | 87.4 | 0.3 | 37.7 | 113.8 |
| 49_179 | 10.5 | 53.6 | 96.4 | 12.4 | 70.7 | 91.7 | 3.5 | 76.0 | 112.0 |
| 49_51 | 6.7 | 39.8 | 76.1 | 5.9 | 60.2 | 92.2 | 1.3 | 68.2 | 161.6 |
| 56_1 | 2.8 | 36.8 | 93.2 | 3.6 | 49.3 | 96.6 | 0.3 | 37.9 | 111.9 |
| 62_1 | 4.5 | 38.6 | 86.2 | 5.8 | 48.4 | 88.1 | 1.5 | 47.8 | 119.0 |
| 67_1 | 31.1 | 57.0 | 86.0 | 35.9 | 58.4 | 79.2 | 26.2 | 65.8 | 115.5 |

### Example 6: IC50 of selected compounds from Example 5

A selection of the efficacious ASO's from example 5 were tested in the same hESC derived neuron assay together with the two prior art controls (CMP ID 66_1 and CMP ID 67_1) to determine IC50 of the target mRNA reduction as well as the Tau protein reduction.

The experiment was conducted as described in example 5 using the following oligonucleotide concentrations: 1000, 200, 40, 8, 1.6, 0.32, 0.064, 0.0128, 0.00256 nM.

The IC50 values were fitted using the GraphPad PRISM software. The results are shown in table 10.

**Table 10 IC50 and max efficacy (as % of control) with respect to MAPT and TAU protein**

| **CMP ID NO** | **Compound** | **IC50 MAPT (nM)** | **Max efficacy MAPT** | **IC50 TAU (nM)** | **Max efficacy MAPT** |
|---|---|---|---|---|---|
| 9_103 | CTTTaatttaatcacTCAT | 2.0 | 0.6 | 1.4 | 1.1 |
| 49_38 | TtaaCTCAaatcaaTtctCA | 8.2 | 2.6 | 6.1 | 1.6 |
| 53_1 | CAACaccttttaattcATTA | 7.6 | 1.7 | 15.0 | 1.9 |
| 66_1 | AtTTCcaaattcactTTtAC | 9.7 | 8.1 | 11.8 | 4.9 |
| 67_1 | CCₒGTTttcettacceeACₒCCT | 17.7 | 22.6 | 43.3 | 23.4 |

From these data it can be seen that CMP ID NO 9_103 (of the invention) and 49_38 are more efficacious and have a better IC50 than the prior art compounds on all parameter, whereas CMP ID NO 53_1 seems to have a better maximal knockdown than the prior art compounds and a similar IC50 as CMP ID NO: 66_1.

### Example 7 in vivo activity in specific brain regions of hTau mouse

A selection of the ASO's from example 5 were tested for their ability to reduce the target *in vivo* in specific brain regions of a humanized Tau mouse (hTau mouse) four weeks after a single low dose ICV administration.

The humanized Tau mouse used in this example is an in house Roche hTau P301S transgenic mouse line which overexpresses human Tau (longest human brain isoform) with the point mutation P301S on a mouse Tau background.

Humanized Tau mice were administered with 25 µg ASO by intracerebroventricular (ICV) injection as described below. CMP ID NO: 66_1, corresponding to ASO-001933 in WO2016/126995, was included for comparative purposes.

### In vivo ICV mouse evaluation:

### Animal Care:

Animals of mixed sex with a weight of 16-23-grams were held in colony rooms maintained at constant temperature (22 ± 2°C) and humidity (55 ± 10%) and illuminated for 12 hours per day (lights on at 0600 hours). All animals had ad libitum access to food and water throughout the studies. All mouse protocols were approved by the Danish National Committee for Ethics in Animal Experiments.

### Intra-cerebroventricular injections:

The compounds were administered to mice by intracerebroventricular (ICV) injections. 6-8 mice of mixed sexes were included in each treatment group. Prior to the ICV dosing, the mice were weighed and anaesthetized with isofluran or Propofol (30mg/kg). Intracerebroventricular injections were performed using a Hamilton micro syringe with a FEP catheter fitted with a 23 gauge needle fixed in a stand adjusted to penetrate the correct distance (3.9mm) through the skin and skull and into the right lateral ventricle. The mouse to be injected was held at the scruff of the neck with the thumb and first fingers of one hand. Applying gentle but firm pressure, the head was pressed upwards so that the needle pierced the skull 1-2mm right of the midline (medio lateral) and 1-2mm behind the eye. The 5 µl bolus of test compound or vehicle was injected over 30 seconds with a previously determined infusion rate. To avoid reflux the mouse was held in this position for another 5 seconds before carefully being pulled downwards, away from the needle. This procedure required no surgery or incision. Animals were placed under a heating lamp until they recovered from the procedure.

At study termination (4 weeks), brain tissue (cortex, medulla/pons and midbrain) was collected on dry ice for analysis of tau mRNA and protein.

### Tissue homogenization:

Mouse brain tissue samples were homogenized in the MagNA Pure LC RNA Isolation Tissue Lysis Buffer (Roche, Indianapolis, IN) using a Qiagen TissueLyzer II. The homogenates were incubated for 30 minutes at room temperature for complete lysis. After lysis the homogenates were centrifuged for 3 minutes at 13000rpm and the supernatant used for analysis.

### RNA purification from tissue:

RNA was purified from 350µL of supernatant using the MagNA Pure 96 instrument using the kit Cellular RNA Large Volume Kit (Roche, Indianapolis, IN). RNA samples were normalized to 2ng/µL in RNase-Free water and stored at -20° C until further use. MAPT mRNA levels were quantified as described in example 5.

### Tau protein measurement from mouse brain tissue:

Pre-weighed frozen tissue was extracted with 10 volumes (wt/vol) of extraction buffer comprising 10 mM TrisCl pH 7.4, 800 mM NaCl, 1 mM EGTA, 10 % sucrose, 1% Phosphatase Inhibitor Cocktail 3 (Sigma #P0044), 1% Proteases Inhibitor Set III (Calbiochem #539134). A homogenate was prepared using the PreCellys tissue disruptor (20 sec, 6500 rpm). The homogenate was then centrifuged at 10'000xg for 20 min at 4°C and the supernatant retained for analysis.

Tau levels in the extracts were measured by AlphaLISA using the total Tau AlphaLISA kit supplied by Perkin Elmer (Cat. Nr. AL271C). The antibodies used in this assay were BT2 and Tau-12 provided with the kit, both of which bind to the central region of tau. Extracts were diluted into HiBlock assay buffer and 5 µl of each sample was then used in assay. The assay was otherwise performed as described by the supplier

Results from mRNA and protein quatification are shown in table 11

**Table 11: in vivo efficacy in selected brain regions 4 weeks after a single ICV dose of 25 µg ASO. MAPT mRNA as % control are shown for four brain regions and Tau protein as % of control is shown for one brain region**

| **CMP ID NO** | **mRNA %ctrl** | | | | | | | | **Protein %ctrl** | |
|---|---|---|---|---|---|---|---|---|---|---|
| | **Cortex A1** | | **Cortex A2** | | **Medulla-Pons** | | **Midbrain** | | **CortexB2** | |
| | Avg | Std | Avg | Std | Avg | Std | Avf | Std | Avg | Std |
| 9_104 | 74 | 12 | 77 | 14 | 68 | 19 | 65 | 18 | 73 | 18 |
| 9_103 | 80 | 13 | 80 | 10 | 66 | 17 | 64 | 12 | 69 | 16 |
| 11_1 | 58 | 12 | 62 | 15 | 54 | 16 | 48 | 19 | 63 | 11 |
| 49_38 | 63 | 12 | 67 | 9 | 55 | 18 | 49 | 16 | 76 | 15 |
| 49_189 | 75 | 5 | 70 | 10 | 54 | 4 | 55 | 6 | 84 | 13 |
| 53_1 | 80 | 10 | 93 | 7 | 81 | 12 | 81 | 16 | 101 | 11 |
| 66_1 | 94 | 20 | 98 | 6 | 101 | 4 | 99 | 11 | 112 | 8 |

From these data it can be observed that even at the fairly low concertation of 25 µg, reduction of more than 20 % is seen in most brain regions for the compounds of the invention, where as the control compound show vertually no reduction of the target at this concentration.

### Example 8 in vivo dose response and time course in the hTau mouse

The dose response of two ASO's (CMP ID NO: 9_103 and 49_189) was evaluated using three different doses (25, 50 and 100 µg) and target reduction was measure in specific brain regions 1 week and 4 weeks after administration. For comparative purposes two prior art compounds (CMP ID NO: 66_1_103 and 67_1) were included at some of the doses in the one-week study.

The experiment was essentially conducted as described in example 7. Tau protein was however not measured in the dose response study which was run for 1 week since the Tau protein has a half life beyond one week. The results are shown in Tables 12 and 13.

**Table 12 in vivo efficacy in selected brain regions 1 week after a single ICV dose at 25 µg, 50 µg or 100 µg ASO or 4weeks after a single ICV dose at 100 µg ASO. MAPT mRNA as % control are shown for four brain regions.**

| **Brain region** | **ASO conc µg** | **CMP ID NO** | **9_103** | **49_38** | **66_1** | **67_1** | **9_103** | **49_38** |
|---|---|---|---|---|---|---|---|---|
| | | **Time** | **1 week** | | | | **4 weeks** | |
| **Cortex A1** | 25 | Avg | 51 | 69 | NA | NA | NA | NA |
| | | Std | 13 | 8 | NA | NA | NA | NA |
| | 50 | Avg | 52 | 52 | 68 | NA | NA | NA |
| | | Std | 12 | 14 | 14 | NA | NA | NA |
| | 100 | Avg | 33 | 39 | 60 | 71 | 36 | 37 |
| | | Std | 10 | 24 | 12 | 25 | 17 | 26 |
| **Cortex A2** | 25 | Avg | 73 | 59 | NA | NA | NA | NA |
| | | Std | 12 | 12 | NA | NA | NA | NA |
| | 50 | Avg | 68 | 39 | 73 | NA | NA | NA |
| | | Std | 15 | 7 | 8 | NA | NA | NA |
| | 100 | Avg | 42 | 43 | 77 | 63 | 51 | 46 |
| | | Std | 21 | 30 | 12 | 20 | 13 | 30 |
| **Midbrain** | 25 | Avg | 79 | 43 | NA | NA | NA | NA |
| | | Std | 20 | 4 | NA | NA | NA | NA |
| | 50 | Avg | 50 | 26 | 68 | NA | NA | NA |
| | | Std | 14 | 6 | 11 | NA | NA | NA |
| | 100 | Avg | 51 | 38 | 78 | 76 | 60 | 38 |
| | | Std | 29 | 31 | 21 | 27 | 28 | 35 |
| **Medulla-Pons** | 25 | Avg | 81 | 41 | | NA | NA | NA |
| | | Std | 21 | 6 | | NA | NA | NA |
| | 50 | Avg | 57 | 26 | 70 | NA | NA | NA |
| | | Std | 18 | 5 | 10 | NA | NA | NA |
| | 100 | Avg | 58 | 37 | 80 | 82 | 61 | 40 |
| | | Std | 34 | 31 | 23 | 28 | 29 | 33 |

| | | | | | | | | |
|---|---|---|---|---|---|---|---|---|
| NA = not assessed | | | | | | | | |

**Table 13 in vivo reduction of Tau protein as % of control 4 weeks after a single ICV dose at 100 µg ASO.**

| **Brain region** | **Cortex B1** | |
|---|---|---|
| **CMP ID NO** | Avg | Std |
| **9_103** | 56 | 18 |
| **49_38** | 43 | 35 |

From the data in table 12 and 13 it can be seen that the compounds (9_103 is of the invention) perform significantly better than the prior art compounds, in particular when dosed at 100 µg. It can also be observed that the MAPT reduction is maintained over the 4 weeks. Furthermore, the compounds of the invention show a significant reduction of Tau protein after 4 weeks treatment with a single dose of 100 µg compound.

## Claims

1. An antisense oligonucleotide of 16 to 30 nucleotides in length, which comprises a contiguous nucleotide sequence of at least 16 nucleotides and 100% complementarity to contiguous nucleotides within position 12060 to 12078 of SEQ ID NO: 1, wherein the antisense oligonucleotide is capable of reducing the expression of Tau.

2. The antisense oligonucleotide of claim 1, wherein the oligonucleotide comprises a sequence selected from the group consisting of SEQ ID NO: 9 and SEQ ID NO: 11.

3. The antisense oligonucleotide of claim 1 or claim 2, wherein the contiguous nucleotide sequence comprises one or more 2' sugar modified nucleosides.

4. The antisense oligonucleotide of claim 3, wherein the one or more 2' sugar modified nucleoside is independently selected from the group consisting of 2'-O-alkyl-RNA, 2'-O-methyl-RNA, 2'-alkoxy-RNA, 2'-O-methoxyethyl-RNA, 2'-amino-DNA, 2'-fluoro-DNA, arabino nucleic acid (ANA), 2'-fluoro-ANA and LNA nucleosides.

5. The antisense oligonucleotide of claim 3 or claim 4, wherein the contiguous nucleotide sequence comprises 4 to 8 LNA nucleosides.

6. The antisense oligonucleotide of claim 5, wherein:
(a) the contiguous nucleotide sequence comprises 8 LNA nucleosides; and/or
(b) the LNA nucleoside is a beta-D-oxy-LNA nucleoside.

7. The antisense oligonucleotide of any one of claims 1 to 6, wherein the oligonucleotide comprises at least one modified internucleoside linkage, optionally wherein at least 80% of the internucleoside linkages within the contiguous nucleotide sequence are phosphorothioate internucleoside linkages.

8. The antisense oligonucleotide of any one of claims 1 to 7, wherein the oligonucleotide is capable of recruiting RNase H.

9. The antisense oligonucleotide of claim 8, wherein the antisense oligonucleotide, or contiguous nucleotide sequence thereof, consists of or comprises a gapmer of formula 5'-F-G-F'-3', where region F and F' independently comprise 1 to 8 nucleosides, of which 2 to 5 are 2' sugar modified and defines the 5' and 3' end of the F and F' region, and the G is a region between 6 and 16 nucleosides which are capable of recruiting RNaseH, such as a region comprising 6 to 16 DNA nucleosides.

10. The antisense oligonucleotide of claim 9, wherein:
(a) the 2' sugar modified nucleosides in region F and F' are identical LNA nucleosides; and/or
(b) all the nucleotides in the G region are DNA.

11. A conjugate comprising the antisense oligonucleotide according to any one of claims 1 to 10, and at least one conjugate moiety covalently attached to said oligonucleotide.

12. The conjugate of claim 11, wherein the conjugate moiety:
(a) is a non-nucleotide moiety;
(b) is an antibody or antibody fragment;
(c) is an antibody or antibody fragment that facilitates delivery across the blood-brain-barrier; and/or
(d) is an antibody or antibody fragment that targets or has a specific affinity for the transferrin receptor.

13. The conjugate of claim 11 or claim 12, wherein the conjugate comprises a linker which is positioned between the oligonucleotide and the conjugate moiety, optionally wherein wherein the linker is a physiologically labile or biocleavable linker.

14. A pharmaceutically acceptable salt of the antisense oligonucleotide according to any one of claims 1 to 10, or the conjugate according to any one of claims 11 to 13.

15. A pharmaceutical composition comprising the antisense oligonucleotide of any one of claims 1 to 10, or the conjugate of any one of claims 11 to 13, and a pharmaceutically acceptable diluent, solvent, carrier, salt and/or adjuvant.

16. An *in vitro* method for modulating Tau expression in a target cell which is expressing Tau, said method comprising administering an antisense oligonucleotide of any one of claims 1 to 10, the conjugate of any one of claims 11 to 13, or the pharmaceutical composition of claim 15 in an effective amount to said cell.

17. The antisense oligonucleotide of any one of claims 1 to 10, the conjugate according to any one of claims 11 to 13, or the pharmaceutical composition of claim 15, for use in medicine.

18. The antisense oligonucleotide of any one of claims 1 to 10, the conjugate according to any one of claims 11 to 13, or the pharmaceutical composition of claim 15, for use in the treatment or prevention of Alzheimer's disease (AD), progressive supranuclear palsy (PSP), fronto- temporal dementia (FTD) or FTDP-17.

19. The antisense oligonucleotide, conjugate, or pharmaceutical composition for use in the treatment of claim 18, wherein the disease is progressive supranuclear palsy (PSP).

20. The antisense oligonucleotide, conjugate, or pharmaceutical composition for use in the treatment of claim 18, wherein the disease is Alzheimer's disease (AD).

## Patentansprüche

1. Antisense-Oligonukleotid mit einer Länge von 16 bis 30 Nukleotiden, das eine zusammenhängende Nukleotidsequenz von mindestens 16 Nukleotiden und eine 100%-ige Komplementarität zu zusammenhängenden Nukleotiden innerhalb der Position 12060 bis 12078 von SEQ ID NO: 1 umfasst, wobei das Antisense-Oligonukleotid in der Lage ist, die Expression von Tau zu reduzieren.

2. Antisense-Oligonukleotid nach Anspruch 1, wobei das Oligonukleotid eine Sequenz umfasst, ausgewählt aus der Gruppe, bestehend aus SEQ ID NO: 9 und SEQ ID NO: 11.

3. Antisense-Oligonukleotid nach Anspruch 1 oder Anspruch 2, wobei die zusammenhängende Nukleotidsequenz ein oder mehrere 2'-zuckermodifizierte Nukleoside umfasst.

4. Antisense-Oligonukleotid nach Anspruch 3, wobei das eine oder die mehreren 2'-zuckermodifizierten Nukleoside unabhängig ausgewählt sind aus der Gruppe, bestehend aus 2'-O-Alkyl-RNA, 2'-O-Methyl-RNA, 2'-Alkoxy-RNA, 2'-O-Methoxyethyl-RNA, 2'-Amino-DNA, 2'-Fluor-DNA, Arabinonukleinsäure (ANA), 2'-Fluor-ANA und LNA-Nukleosiden.

5. Antisense-Oligonukleotid nach Anspruch 3 oder Anspruch 4, wobei die zusammenhängende Nukleotidsequenz 4 bis 8 LNA-Nukleoside umfasst.

6. Antisense-Oligonukleotid nach Anspruch 5, wobei:
(a) die zusammenhängende Nukleotidsequenz 8 LNA-Nukleoside umfasst; und/oder
(b) das LNA-Nukleosid ein beta-D-Oxy-LNA-Nukleosid ist.

7. Antisense-Oligonukleotid nach einem der Ansprüche 1 bis 6, wobei das Oligonukleotid mindestens eine modifizierte Internukleosidverknüpfung umfasst, wobei optional mindestens 80 % der Internukleosidverknüpfungen innerhalb der zusammenhängenden Nukleotidsequenz Phosphorothioat-Internukleosidverknüpfungen sind.

8. Antisense-Oligonukleotid nach einem der Ansprüche 1 bis 7, wobei das Oligonukleotid in der Lage ist, RNase H zu rekrutieren.

9. Antisense-Oligonukleotid nach Anspruch 8, wobei das Antisense-Oligonukleotid oder die zusammenhängende Nukleotidsequenz davon aus einem Gapmer der Formel 5'-F-G-F'-3' besteht oder dieses umfasst, wobei die Regionen F und F' unabhängig voneinander 1 bis 8 Nukleoside umfassen, von denen 2 bis 5 2'-zuckermodifiziert sind und das 5'- und 3'-Ende der F- und F'-Region definieren, und das G eine Region zwischen 6 und 16 Nukleosiden ist, die in der Lage sind, RNase H zu rekrutieren, wie etwa eine Region, die 6 bis 16 DNA-Nukleoside umfasst.

10. Antisense-Oligonukleotid nach Anspruch 9, wobei:
(a) die 2'-zuckermodifizierten Nukleoside in der Region F und F' identische LNA-Nukleoside sind; und/oder
(b) alle Nukleotide in der G-Region DNA sind.

11. Konjugat, umfassend das Antisense-Oligonukleotid nach einem der Ansprüche 1 bis 10 und mindestens eine Konjugateinheit, die kovalent an das Oligonukleotid gebunden ist.

12. Konjugat nach Anspruch 11, wobei die Konjugateinheit:
(a) eine Nicht-Nukleotideinheit ist;
(b) ein Antikörper oder Antikörperfragment ist;
(c) ein Antikörper oder Antikörperfragment ist, der/das die Abgabe durch die Blut-Hirn-Schranke erleichtert; und/oder
(d) ein Antikörper oder Antikörperfragment ist, der/das auf den Transferrinrezeptor abzielt oder eine spezifische Affinität für diesen aufweist.

13. Konjugat nach Anspruch 11 oder Anspruch 12, wobei das Konjugat einen Linker umfasst, der zwischen dem Oligonukleotid und der Konjugateinheit positioniert ist, wobei der Linker optional ein physiologisch labiler oder biologisch spaltbarer Linker ist.

14. Pharmazeutisch verträgliches Salz des Antisense-Oligonukleotids nach einem der Ansprüche 1 bis 10 oder des Konjugats nach einem der Ansprüche 11 bis 13.

15. Pharmazeutische Zusammensetzung, umfassend das Antisense-Oligonukleotid nach einem der Ansprüche 1 bis 10 oder das Konjugat nach einem der Ansprüche 11 bis 13 und ein pharmazeutisch verträgliches Verdünnungsmittel, Lösungsmittel, einen pharmazeutisch verträglichen Träger, ein pharmazeutisch verträgliches Salz und/oder Adjuvans.

16. In-vitro-Verfahren zum Modulieren der Tau-Expression in einer Zielzelle, die Tau exprimiert, wobei das Verfahren das Verabreichen eines Antisense-Oligonukleotids nach einem der Ansprüche 1 bis 10, des Konjugats nach einem der Ansprüche 11 bis 13 oder der pharmazeutischen Zusammensetzung nach Anspruch 15 in einer wirksamen Menge an die Zelle umfasst.

17. Antisense-Oligonukleotid nach einem der Ansprüche 1 bis 10, Konjugat nach einem der Ansprüche 11 bis 13 oder pharmazeutische Zusammensetzung nach Anspruch 15 zur Verwendung in der Medizin.

18. Antisense-Oligonukleotid nach einem der Ansprüche 1 bis 10, Konjugat nach einem der Ansprüche 11 bis 13 oder pharmazeutische Zusammensetzung nach Anspruch 15 zur Verwendung bei der Behandlung oder Prävention von Alzheimer-Krankheit (AD), progressiver supranukleärer Blickparese (PSP), frontotemporaler Demenz (FTD) oder FTDP-17.

19. Antisense-Oligonukleotid, Konjugat oder pharmazeutische Zusammensetzung zur Verwendung bei der Behandlung nach Anspruch 18, wobei die Krankheit progressive supranukleäre Blickparese (PSP) ist.

20. Antisense-Oligonukleotid, Konjugat oder pharmazeutische Zusammensetzung zur Verwendung bei der Behandlung nach Anspruch 18, wobei die Krankheit Alzheimer-Krankheit (AD) ist.

## Revendications

1. Oligonucléotide antisens de 16 à 30 nucléotides de longueur, qui comprend une séquence de nucléotides contigus d'au moins 16 nucléotides et de 100 % de complémentarité vis-à-vis de nucléotides contigus entre les positions 12060 et 12078 de la SEQ ID NO : 1, dans lequel l'oligonucléotide antisens est capable de réduire l'expression de Tau.

2. Oligonucléotide antisens selon la revendication 1, dans lequel l'oligonucléotide comprend une séquence choisie dans le groupe constitué par la SEQ ID NO : 9 et la SEQ ID NO : 11.

3. Oligonucléotide antisens selon la revendication 1 ou la revendication 2, dans lequel la séquence de nucléotides contigus comprend un ou plusieurs nucléosides modifiés au niveau du sucre en 2'.

4. Oligonucléotide antisens selon la revendication 3, dans lequel le ou les nucléosides modifiés au niveau du sucre en 2' sont indépendamment choisis dans le groupe constitué par les nucléosides 2'-O-alkyl-ARN, 2'-O-méthyl-ARN, 2'-alcoxy-ARN, 2'-O-méthoxyéthyl-ARN, 2'-amino-ADN, 2'-fluoro-ADN, acide arabinonucléique (ANA), 2'-fluoro-ANA et LNA.

5. Oligonucléotide antisens selon la revendication 3 ou la revendication 4, dans lequel la séquence de nucléotides contigus comprend 4 à 8 nucléosides LNA.

6. Oligonucléotide antisens selon la revendication 5, dans lequel :
(a) la séquence de nucléotides contigus comprend 8 nucléosides LNA ; et/ou
(b) le nucléoside LNA est un nucléoside bêta-D-oxy-LNA.

7. Oligonucléotide antisens selon l'une quelconque des revendications 1 à 6, dans lequel l'oligonucléotide comprend au moins une liaison internucléosidique modifiée, éventuellement dans lequel au moins 80 % des liaisons internucléosidiques au sein de la séquence de nucléotides contigus sont des liaisons internucléosidiques phosphorothioate.

8. Oligonucléotide antisens selon l'une quelconque des revendications 1 à 7, dans lequel l'oligonucléotide est capable de recruter la RNase H.

9. Oligonucléotide antisens selon la revendication 8, dans lequel l'oligonucléotide antisens, ou la séquence de nucléotides contigus de celui-ci, consiste en ou comprend un gapmère de formule 5'-F-G-F'-3', où les régions F et F' comprennent indépendamment 1 à 8 nucléosides, desquels 2 à 5 sont modifiés au niveau du sucre en 2' et définissent les extrémités 5' et 3' des régions F et F', et G représente une région entre 6 et 16 nucléosides qui sont capables de recruter la RNase H, telle qu'une région comprenant 6 à 16 nucléosides d'ADN.

10. Oligonucléotide antisens selon la revendication 9, dans lequel :
(a) les nucléosides modifiés au niveau du sucre en 2' dans les régions F et F' sont des nucléosides LNA identiques ; et/ou
(b) tous les nucléotides dans la région G sont de l'ADN.

11. Conjugué comprenant l'oligonucléotide antisens selon l'une quelconque des revendications 1 à 10, et au moins une fraction conjuguée liée de manière covalente audit oligonucléotide.

12. Conjugué selon la revendication 11, dans lequel la fraction conjuguée :
(a) est une fraction non nucléotidique ;
(b) est un anticorps ou un fragment d'anticorps ;
(c) est un anticorps ou un fragment d'anticorps qui facilite l'administration à travers la barrière hémato-encéphalique ; et/ou
(d) est un anticorps ou un fragment d'anticorps qui cible ou a une affinité spécifique pour le récepteur de la transferrine.

13. Conjugué selon la revendication 11 ou la revendication 12, dans lequel le conjugué comprend un lieur qui est positionné entre l'oligonucléotide et la fraction conjuguée, éventuellement dans lequel le lieur est un lieur physiologiquement labile ou bioclivable.

14. Sel pharmaceutiquement acceptable de l'oligonucléotide antisens selon l'une quelconque des revendications 1 à 10, ou du conjugué selon l'une quelconque des revendications 11 à 13.

15. Composition pharmaceutique comprenant l'oligonucléotide antisens selon l'une quelconque des revendications 1 à 10, ou le conjugué selon l'une quelconque des revendications 11 à 13, et un diluant, un solvant, un véhicule, un sel et/ou un adjuvant pharmaceutiquement acceptables.

16. Méthode *in vitro* pour moduler l'expression de Tau dans une cellule cible qui exprime Tau, ladite méthode comprenant l'administration à ladite cellule d'un oligonucléotide antisens selon l'une quelconque des revendications 1 à 10, du conjugué selon l'une quelconque des revendications 11 à 13 ou de la composition pharmaceutique selon la revendication 15 en une quantité efficace.

17. Oligonucléotide antisens selon l'une quelconque des revendications 1 à 10, conjugué selon l'une quelconque des revendications 11 à 13 ou composition pharmaceutique selon la revendication 15, pour une utilisation en médecine.

18. Oligonucléotide antisens selon l'une quelconque des revendications 1 à 10, conjugué selon l'une quelconque des revendications 11 à 13 ou composition pharmaceutique selon la revendication 15, pour une utilisation dans le traitement ou la prévention de la maladie d'Alzheimer (MA), de la paralysie supranucléaire progressive (PSP), de la démence fronto-temporale (FTD) ou de FTDP-17.

19. Oligonucléotide antisens, conjugué ou composition pharmaceutique pour une utilisation dans le traitement selon la revendication 18, dans lequel/laquelle la maladie est la paralysie supranucléaire progressive (PSP).

20. Oligonucléotide antisens, conjugué ou composition pharmaceutique pour une utilisation dans le traitement selon la revendication 18, dans lequel/laquelle la maladie est la maladie d'Alzheimer (MA).
